# EUROPEAN PATENT SPECIFICATION

(11) **EP 3 704 229 B1**
(45) Date of publication and mention of the grant of the patent: **20.12.2023**
(21) Application number: 18804489.5
(22) Date of filing: 01.11.2018
(51) Int. Cl.: C12N 5/0783, A61K 35/17, C07K 14/725

(54) **PROCESS FOR PRODUCING A T CELL COMPOSITION**
VERFAHREN ZUR HERSTELLUNG EINER T-ZELL-ZUSAMMENSETZUNG
PROCÉDÉ POUR LA PRODUCTION D'UNE COMPOSITION DE LYMPHOCYTES T

(30) Priority: 01.11.2017 US 201762580435 P; 10.11.2017 US 201762584687 P; 17.07.2018 US 201862699709 P; 28.07.2018 US 201862711494 P
(43) Date of publication of application: 09.09.2020
(73) Proprietor: Juno Therapeutics, Inc., Seattle, WA 98109 (US); Celgene Corporation, Summit, NJ 07901 (US)
(72) Inventor: BRAHMANDAM, Archana, Seattle Washington 98109 (US); THOMPSON, Lucas James, Seattle Washington 98109 (US); MORTENSEN, Deborah, San Diego California 92121 (US); FILVAROFF, Ellen, Summit New Jersey 07901 (US)
(74) Representative: Carpmaels & Ransford LLP
(86) International application number: PCT/US2018/058812
(87) International publication number: WO 2019/090004

(56) References cited:
- WO-A1-2015/188119
- US-A1- 2013 102 613
- Jillian Smith ET AL: "Abstract 3141: Restricting aerobic glycolysis provides functional improvement of chimeric antigen receptor (CAR)-modified T cells | Cancer Research", , 1 August 2015 (2015-08-01), XP055542922, Retrieved from the Internet: URL:http://cancerres.aacrjournals.org/cont ent/75/15_Supplement/3141 [retrieved on 2019-01-16]
- BRAHMANDAM A ET AL: "Enhanced functional profile of CAR-T cells generated in the presence of mTOR kinase inhibitor", JOURNAL FOR IMMUNOTHERAPY OF CANCER 20171101 BIOMED CENTRAL LTD. NLD, vol. 5, no. Supplement 2, 1 November 2017 (2017-11-01), XP055469477, ISSN: 2051-1426

## Description

### Field

The present disclosure describes methods for producing engineered T cells that express a recombinant receptor, such as for use in cell therapy. In some aspects, the methods include one or more steps for incubating the cells under stimulating conditions, introducing a recombinant polypeptide to the cells through transduction or transfection, and/or cultivating the cells under conditions that promote proliferation and/or expansion, in which one or more steps is carried out in the presence of an agent that inhibits mammalian target of rapamycin (mTOR) activity. In some aspects, cultivation is performed in the presence of an agent that inhibits mammalian target of rapamycin (mTOR) activity. In some aspects, the methods produce genetically engineered T cells with improved persistence and/or anti-tumor activity *in vivo.*

### Background

Various cell therapy methods are available for treating diseases and conditions. Among cell therapy methods are methods involving immune cells, such as T cells, genetically engineered with a recombinant receptor, such as a chimeric antigen receptor. Improved methods for manufacturing and/or engineering such cell therapies are needed, including to provide for a more efficient process and/or an improved cell composition product. Provided are methods for producing engineered cells, the engineered cells, compositions, kits and articles of manufacture and methods of treatment that meet such needs.

WO 2015/188119 A1 describes methods of manufacturing T cells for improved survival, expansion in vivo.

Smith et al., 2015 describes how modulating T cell metabolism by restricting aerobic glycolysis enhances anti-tumor activity of chimeric antigen receptor (CAR)-modified T cells.

US 2013/102613 A1 describes the treatment of non-Hodgkin lymphoma or multiple myeloma with TOR kinase inhibitors.

Brahmandam et al., 2017 describes the generation of enhanced CAR-T cells in the presence of mTOR kinase inhibitor.

### Summary

The present invention is set out in the appended set of claims.

In particular, the present invention provides a method for producing a composition of engineered cells, the method comprising:
(a) incubating, under stimulating conditions, an input composition comprising primary human T cells, said stimulating conditions comprising the presence of (i) a stimulatory reagent capable of activating one or more intracellular signaling domains of one or more components of a TCR complex and/or one or more intracellular signaling domains of one or more costimulatory molecules and (ii) an agent that inhibits mTOR activity, wherein the agent that inhibits mTOR activity is 2-(3-hydroxyphenyl)-9-(2-isopropylphenyl)-8-oxo-8,9-dihydro-7H-purine-6-carboxamide (Compound 63); and
(b) introducing a recombinant receptor into the stimulated composition, thereby generating an engineered composition comprising engineered T cells.

The present invention also provides a method for producing a composition of engineered cells, the method comprising cultivating, in the presence of an agent that inhibits mTOR activity, an engineered cell composition comprising enriched primary human T cells comprising T cells engineered with a recombinant receptor;
wherein the agent that inhibits mTOR activity is 2-(3-hydroxyphenyl)-9-(2-isopropylphenyl)-8-oxo-8,9-dihydro-7H-purine-6-carboxamide (Compound 63); and
wherein the method results in the proliferation or expansion of cells in the composition to produce an output composition comprising engineered T cells.

In certain embodiments, the method comprises cultivating, in the presence of an agent that inhibits mTOR activity, an engineered cell composition comprising enriched primary human T cells comprising cells engineered with a recombinant receptor, wherein cells in the composition have not been exposed to the agent prior to being cultivated; and wherein the method results in the proliferation or expansion of the cells in the composition to produce an output composition comprising engineered T cells. In particular embodiments of any of the provided methods, the primary T cells are CD4+ and/or CD8+ T cells. In some embodiments of any of the provided methods, the engineered T cell composition comprises enriched CD4+ T cells. In certain embodiments of any of the provided methods, the engineered T cell composition comprises enriched CD8+ T cells.

In certain embodiments, the method comprises cultivating, in the presence of an agent that inhibits mTOR activity, an engineered cell composition comprising enriched CD4+ and/or enriched CD8+ primary human T cells comprising T cells engineered with a recombinant receptor; wherein the method results in the proliferation or expansion of cells in the composition to produce an output composition comprising engineered enriched CD4+ and/or enriched CD8+ T cells. In some embodiments of any of the provided methods, the engineered T cell composition comprises greater than or greater than about 70%, greater than or greater than about 75%, greater than or greater than about 80%, greater than or greater than about 85%, greater than or greater than about 90%, greater than or greater than about 95% or greater than or greater than about 98% CD4+ primary human T cells; and/or the input composition consists essentially of CD4+ primary human T cells. In particular embodiments of any of the provided methods, the engineered T cell composition comprises greater than or greater than about 70%, greater than or greater than about 75%, greater than or greater than about 80%, greater than or greater than about 85%, greater than or greater than about 90%, greater than or greater than about 95% or greater than or greater than about 98% CD8+ primary human T cells; and/or the input composition consists essentially of CD8+ primary human T cells. In certain embodiments of any of the provided methods, the engineered T cell composition comprises greater than or greater than about 70%, greater than or greater than about 75%, greater than or greater than about 80%, greater than or greater than about 85%, greater than or greater than about 90%, greater than or greater than about 95% or greater than or greater than about 98% CD4+ and CD8+ primary human T cells; and/or the input composition consists essentially of CD4+ and CD8+ primary human T cells.

In certain embodiments of any of the provided methods, the cultivating is carried out in the presence of one or more cytokines. In some embodiments of any of the provided methods, the one or more cytokines comprise one or more of IL-2, IL-4, IL-7, IL-9, IL-12, IL-15, G-CSF, and GM-CSF. In particular embodiments of any of the provided methods, the one or more cytokines are recombinant cytokines.

In certain embodiments of any of the provided methods, prior to the cultivating, the method further comprises: (a) incubating, under stimulating conditions, an input composition comprising primary T cells, said stimulating conditions comprising the presence of a stimulatory reagent capable of activating one or more intracellular signaling domains of one or more components of a TCR complex and/or one or more intracellular signaling domains of one or more costimulatory molecules, thereby generating a stimulated composition; and (b) introducing a recombinant receptor into the stimulated composition, thereby generating an engineered composition comprising engineered T cells.

In some embodiments of any of the provided methods, the input composition, the stimulated composition, and/or the engineered composition comprises primary CD4+ and/or CD8+ T cells. In particular embodiments of any of the provided methods, the input composition, the stimulated composition, and/or the engineered composition comprises enriched CD4+ T cells. In certain embodiments of any of the provided methods, the input composition, the stimulated composition, and/or the engineered composition comprises enriched CD8+ T cells.

In certain embodiments of any of the provided methods for producing a composition of engineered cells, the method comprises: (a) incubating, under stimulating conditions, an input composition comprising primary human T cells enriched for CD4+ and/or CD8+ primary human T cells, said stimulating conditions comprising the presence of (i) a stimulatory reagent capable of activating one or more intracellular signaling domains of one or more components of a TCR complex and/or one or more intracellular signaling domains of one or more costimulatory molecules and (ii) an agent that inhibits mTOR activity; and (b) introducing a recombinant receptor into the stimulated composition, thereby generating an engineered composition comprising engineered T cells.

In some embodiments of any of the provided methods, the input composition, the stimulated composition, and/or the engineered composition comprises greater than or greater than about 70%, greater than or greater than about 75%, greater than or greater than about 80%, greater than or greater than about 85%, greater than or greater than about 90%, greater than or greater than about 95% or greater than or greater than about 98% CD4+ primary human T cells; and/or the input composition consists essentially of CD4+ primary human T cells. In particular embodiments of any of the provided methods, the input composition, the stimulated composition, and/or the engineered composition comprises greater than or greater than about 70%, greater than or greater than about 75%, greater than or greater than about 80%, greater than or greater than about 85%, greater than or greater than about 90%, greater than or greater than about 95% or greater than or greater than about 98% CD8+ primary human T cells; and/or the input composition consists essentially of CD8+ primary human T cells. In certain embodiments of any of the provided methods, the input composition, the stimulated composition, and/or the engineered composition comprises greater than or greater than about 70%, greater than or greater than about 75%, greater than or greater than about 80%, greater than or greater than about 85%, greater than or greater than about 90%, greater than or greater than about 95% or greater than or greater than about 98% CD4+ and CD8+ primary human T cells; and/or the input composition consists essentially of CD4+ and CD8+ primary human T cells.

In certain instances of any of the described methods, the agent that inhibits mTOR activity is a small molecule, a small organic molecule, a polynucleotide, an oligonucleotide, an siRNA, or a polypeptide. In some instances, the agent that inhibits mTOR activity is a small organic molecule. In some instances of any of the described methods, the agent that inhibits mTOR activity inhibits mTORC1 and/or mTORC2 kinase activity. In particular instances of any of the described methods, the agent that inhibits mTOR activity inhibits the activity of at least one additional kinase. In certain instances of any of the described methods, the at least one additional kinase is PI3K. In some instances of any of the described methods, the agent that inhibits mTOR activity is BEZ235, BGT226, GDC0980, NVP-BEZ235, PF-04691502, PI-103, SAR245409, SF1126, VS5584, or XL765.

The agent that inhibits mTOR activity: (i) does not inhibit PI3K activity; (ii) does not detectably inhibit PI3K activity at the IC₅₀ for mTOR activity; and/or (iii) does not detectably inhibit PI3K at all concentrations that detectably inhibit mTOR activity. The agent that inhibits mTOR activity inhibits mTORC1 and mTORC2 kinase activity. In some instances of any of the described methods, the agent that inhibits mTOR activity is a pyrazolopyrimidine, Torin l, Torkinib, PP30, Ku-0063794, WAY-600 (Wyeth), WAY-687 (Wyeth), WAY-354 (Wyeth), OSI-027, DS3078a, or AZD8055. In particular instances of any of the described methods, the agent that inhibits mTOR activity selectively inhibits mTORC1 activity.

In certain instances of any of the described methods, the agent that inhibits mTOR activity: (i) does not inhibit mTORC2 activity; (ii) does not detectably inhibit mTORC2 activity at the IC₅₀ for mTORC1 activity; and/or (iii) does not detectably inhibit mTORC2 at all concentrations that detectably inhibit mTORC1 activity. In some instances of any of the described methods, the agent that inhibits mTOR activity is rapamycin, temsirolimus, everolimus, deforolimus, or AZD8055.

In particular instances of any of the described methods, the agent comprises a formula set forth in Formula I, 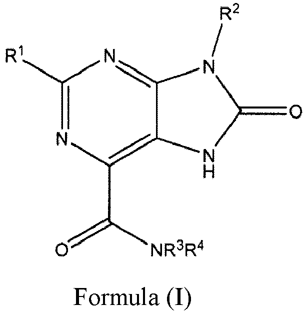 wherein R¹ is substituted or unsubstituted C₁₋₈alkyl, substituted or unsubstituted aryl, substituted or unsubstituted heteroaryl, substituted or unsubstituted cycloalkyl, or substituted or unsubstituted heterocycloalkyl, R² is substituted or unsubstituted C₁₋₈alkyl, substituted or unsubstituted aryl, substituted or unsubstituted heteroaryl, substituted or unsubstituted cycloalkyl, or substituted or unsubstituted heterocycloalkyl, and R³ and R⁴ are independently H or C₁₋₈ alkyl. In some embodiments, the agent that inhibits mTOR activity is or comprises a compound of Formula (I), or a pharmaceutically acceptable salt or solvate thereof. In some instances, the agent that inhibits mTOR activity is or comprises a compound of Formula (I), or a pharmaceutically acceptable salt thereof.

In certain instances of any of the described methods, R¹ is substituted aryl, substituted or unsubstituted heteroaryl, such as substituted phenyl. In some instances of any of the described methods, R² is substituted or unsubstituted aryl, and/or a substituted or unsubstituted phenyl. In some instances, R² is substituted or unsubstituted aryl, such as a substituted or unsubstituted phenyl. In particular instances of any of the described methods, groups that are substituted are substituted with one or more halogen; C₁₋₈ alkyl; C₂₋₈ alkenyl; C₂₋₈ alkynyl; hydroxyl; C₁₋₈ alkoxyl; amino; nitro; thiol; thioether; imine; cyano; amido; phosphonato; phosphine; carboxyl; thiocarbonyl; sulfonyl; sulfonamide; ketone; aldehyde; ester; carbonyl; haloalkyl; B(OH)₂; carbocyclic cycloalkyl, heterocycloalkyl, monocyclic or fused or non-fused polycyclic aryl or heteroaryl; amino; O-lower alkyl; O-aryl, aryl; aryl-lower alkyl; CO₂CH₃; CONH₂; OCH₂CONH₂; NH₂; SO₂NH₂; OCHF₂; CF₃; or OCF₃ groups. The agent that inhibits mTOR activity in any of the provided methods is Compound 63. The agent that inhibits mTOR activity in any of the provided methods is 2-(3-hydroxyphenyl)-9-(2-isopropylphenyl)-8-oxo-8,9-dihydro-7H-purine-6-carboxamide, or a pharmaceutically acceptable salt or solvate thereof. The agent that inhibits mTOR activity is 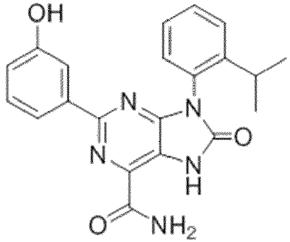 , or a pharmaceutically acceptable salt thereof.

Provided herein is a method for producing a composition of engineered cells, the method comprising cultivating, in the presence of an agent that inhibits mTOR activity, an engineered cell composition comprising enriched primary human T cells comprising T cells engineered with a recombinant receptor; wherein the agent that inhibits mTOR activity is Compound 63; and wherein the method results in the proliferation or expansion of cells in the composition to produce an output composition comprising engineered T cells.

In particular embodiments of any of the provided methods, the engineered cell composition is cultivated in the presence of between 500 nM and 2 µM, between 1 nM and 100 nM, between 50 nM and 250 nM, or between 100 nM and 500 nM of Compound 63.

In particular embodiments of any of the provided methods, prior to the cultivating, the method further comprises:(a) incubating, under stimulating conditions, an input composition comprising primary T cells in the presence of an agent that inhibits mTOR activity; wherein said stimulating conditions comprise the presence of a stimulatory reagent capable of activating one or more intracellular signaling domains of one or more components of a TCR complex and/or one or more intracellular signaling domains of one or more costimulatory molecules, thereby generating a stimulated composition; and wherein the agent that inhibits mTOR activity is Compound 63; and (b) introducing a recombinant receptor into the stimulated composition, thereby generating an engineered composition comprising engineered T cells.

Provided herein is a method for producing a composition of engineered cells, the method comprising: (a) incubating, under stimulating conditions, an input composition comprising primary human T cells, said stimulating conditions comprising the presence of (i) a stimulatory reagent capable of activating one or more intracellular signaling domains of one or more components of a TCR complex and/or one or more intracellular signaling domains of one or more costimulatory molecules and (ii) an agent that inhibits mTOR activity, wherein the agent that inhibits mTOR activity is Compound 63; and (b) introducing a recombinant receptor into the stimulated composition, thereby generating an engineered composition comprising engineered T cells. In some embodiments, the primary T cells are enriched in CD4+ and/or CD8+ T cells.

In certain embodiments of any of the provided methods, the stimulatory reagent comprises a primary agent that specifically binds to a member of a TCR complex, optionally that specifically binds to CD3. In some embodiments of any of the provided methods, the stimulatory reagent further comprises a secondary agent that specifically binds to a T cell costimulatory molecule, optionally wherein the costimulatory molecule is selected from CD28, CD137 (4-1-BB), OX40, or ICOS. In particular embodiments of any of the provided methods, the primary and/or secondary agents comprise an antibody, optionally wherein the stimulatory reagent comprises incubation with an anti-CD3 antibody and an anti-CD28 antibody, or an antigen-binding fragment thereof. In certain embodiments of any of the provided methods, the primary agent and/or secondary agent are present on the surface of a solid support. In some embodiments of any of the provided methods, the solid support is or comprises a bead. In particular embodiments of any of the provided methods, the bead comprises a diameter of greater than or greater than about 3.5 µm but no more than about 9 µm or no more than about 8 µm or no more than about 7 µm or no more than about 6 µm or no more than about 5 µm. In certain embodiments of any of the provided methods, the bead comprises a diameter of or about 4.5 µm. In some embodiments of any of the provided methods, the bead is inert. In particular embodiments of any of the provided methods, the bead is or comprises a polystyrene surface. In certain embodiments of any of the provided methods, the bead is magnetic or superparamagnetic. In some embodiments of any of the provided methods, the ratio of beads to cells is from or from about 4:1 to 0.25:1.

In particular embodiments of any of the provided methods, the introducing comprises transducing cells of the stimulated composition with a viral vector comprising a polynucleotide encoding the recombinant receptor. In certain embodiments of any of the provided methods, the viral vector is a retroviral vector. In some embodiments of any of the provided methods, the viral vector is a lentiviral vector or gammaretroviral vector. In particular embodiments of any of the provided methods, the introducing comprises transfecting the cells of the stimulated composition with a vector comprising a polynucleotide encoding the recombinant receptor. In certain embodiments of any of the provided methods, the vector is a transposon, optionally a Sleeping Beauty (SB) transposon or a Piggybac transposon.

In some embodiments of the provided methods, subsequent to the cultivating, the method further comprises collecting cells of the output composition. Particular embodiments of any of the provided methods further comprise formulating cells of the output composition for cryopreservation and/or administration to a subject, optionally in the presence of a pharmaceutically acceptable excipient. In certain embodiments of any of the provided methods, the cells of the output composition are formulated in the presence of a cryoprotectant. In some embodiments of any of the provided methods, the cryoprotectant comprises DMSO. In particular embodiments of any of the provided methods, the cells of the output composition are formulated in a container, optionally a vial or a bag.

Certain embodiments of the provided methods further comprise isolating the CD4+ and/or the CD8+ T cells from a biological sample prior to the incubating. In some embodiments of any of the provided methods, the isolating comprises, selecting cells based on surface expression of CD4 and/or CD8, optionally by positive or negative selection. In particular embodiments of any of the provided methods, the isolating comprises carrying out immunoaffinity-based selection. In certain embodiments of any of the provided methods, the biological sample comprises primary T cells obtained from a subject. In some embodiments of any of the provided methods, the biological sample is or comprises a whole blood sample, a buffy coat sample, a peripheral blood mononuclear cells (PBMC) sample, an unfractionated T cell sample, a lymphocyte sample, a white blood cell sample, an apheresis product, or a leukapheresis product.

In particular embodiments of any of the provided methods, the recombinant receptor is capable of binding to a target antigen that is associated with, specific to, and/or expressed on a cell or tissue of a disease, disorder or condition. In certain embodiments of any of the provided methods, the disease, disorder or condition is an infectious disease or disorder, an autoimmune disease, an inflammatory disease, or a tumor or a cancer. In some embodiments of any of the provided methods, the target antigen is a tumor antigen.

In particular embodiments of any of the provided methods, the target antigen is selected from among 5T4, 8H9, avb6 integrin, B7-H6, B cell maturation antigen (BCMA), CA9, a cancer-testes antigen, carbonic anhydrase 9 (CAIX), CCL-1, CD19, CD20, CD22, CEA, hepatitis B surface antigen, CD23, CD24, CD30, CD33, CD38, CD44, CD44v6, CD44v7/8, CD123, CD138, CD171, carcinoembryonic antigen (CEA), CE7, a cyclin, cyclin A2, c-Met, dual antigen, EGFR, epithelial glycoprotein 2 (EPG-2), epithelial glycoprotein 40 (EPG-40), EPHa2, ephrinB2, erb-B2, erb-B3, erb-B4, erbB dimers, EGFR vIII, estrogen receptor, Fetal AchR, folate receptor alpha, folate binding protein (FBP), FCRL5, FCRH5, fetal acetylcholine receptor, G250/CAIX, GD2, GD3, G Protein Coupled Receptor 5D (GPRC5D), gp100, Her2/neu (receptor tyrosine kinase erbB2), HMW-MAA, IL-22R-alpha, IL-13 receptor alpha 2 (IL-13Ra2), kinase insert domain receptor (kdr), kappa light chain, Lewis Y, L1-cell adhesion molecule (L1-CAM), Melanoma-associated antigen (MAGE)-A1, MAGE-A3, MAGE-A6, MART-1, mesothelin, murine CMV, mucin 1 (MUC1), MUC16, NCAM, NKG2D, NKG2D ligands, NY-ESO-1, O-acetylated GD2 (OGD2), oncofetal antigen, Preferentially expressed antigen of melanoma (PRAME), PSCA, progesterone receptor, survivin, ROR1, TAG72, tEGFR, VEGF receptors, VEGF-R2, Wilms Tumor 1 (WT-1), a pathogen-specific antigen and an antigen associated with a universal tag.

In certain embodiments of any of the provided methods, the recombinant receptor is or comprises a functional non-TCR antigen receptor or a TCR or antigen-binding fragment thereof. In some embodiments of any of the provided methods, the recombinant receptor is a chimeric antigen receptor (CAR). In particular embodiments of any of the provided methods, the recombinant receptor is an anti-CD19 CAR. In certain embodiments of any of the provided methods, the chimeric antigen receptor comprises an extracellular domain comprising an antigen-binding domain.

In some embodiments of any of the provided methods, the antigen-binding domain is or comprises an antibody or an antibody fragment thereof, which optionally is a single chain fragment. In particular embodiments of any of the provided methods, the fragment comprises antibody variable regions joined by a flexible linker. In certain embodiments of any of the provided methods, the fragment comprises an scFv. In some embodiments of any of the provided methods, the chimeric antigen receptor further comprises a spacer and/or a hinge region. In particular embodiments of any of the provided methods, the chimeric antigen receptor comprises an intracellular signaling region. In certain embodiments of any of the provided methods, the intracellular signaling region comprises an intracellular signaling domain. In some embodiments of any of the provided methods, the intracellular signaling domain is or comprises a primary signaling domain, a signaling domain that is capable of inducing a primary activation signal in a T cell, a signaling domain of a T cell receptor (TCR) component, and/or a signaling domain comprising an immunoreceptor tyrosine-based activation motif (ITAM). In particular embodiments of any of the provided methods, the intracellular signaling domain is or comprises an intracellular signaling domain of a CD3 chain, optionally a CD3-zeta (CD3ζ) chain, or a signaling portion thereof.

In certain embodiments of any of the provided methods, wherein the chimeric antigen receptor further comprises a transmembrane domain disposed between the extracellular domain and the intracellular signaling region. In some embodiments of any of the provided methods, the intracellular signaling region further comprises a costimulatory signaling region. In certain embodiments of any of the provided methods, the costimulatory signaling region comprises an intracellular signaling domain of a T cell costimulatory molecule or a signaling portion thereof. In particular embodiments of any of the provided methods, the costimulatory signaling region comprises an intracellular signaling domain of a CD28, a 4-1BB or an ICOS or a signaling portion thereof. In some embodiments of any of the provided methods, the costimulatory signaling region is between the transmembrane domain and the intracellular signaling region.

In certain embodiments of any of the provided methods, the primary T cells comprise separate compositions of enriched CD4+ T cells and enriched CD8+ T cells, and wherein the compositions of enriched CD4+ T cells and enriched CD8+ T cells are cultivated separately. In particular embodiments of any of the provided methods, the primary T cells comprise separate compositions of enriched CD4+ T cells and enriched CD8+ T cells, and wherein the compositions are mixed so as to cultivate the enriched CD4+ T cells and enriched CD8+ T cells together.

Described herein as part of the disclosure is a composition comprising engineered cells produced by any method provided herein. In particular instances, the composition further comprised a pharmaceutically acceptable carrier. In some instances, the composition comprises a cryoprotectant, optionally DMSO.

Described herein as part of the disclosure is an article of manufacture, comprising any composition described herein and instructions for administering the output composition to a subject. In certain instances, the subject has a disease or condition, optionally wherein the recombinant receptor specifically recognizes or specifically bind to an antigen associated with, or expressed or present on cells of, the disease or condition. In particular instances, the output composition is a composition of engineered CD4+ T cells. In some instances, the output composition is an engineered composition of CD8+ T cells. In certain instances, the output composition is an engineered composition of CD4+ and CD8+ T cells.

Described herein as part of the disclosure is an article of manufacture comprising a composition of engineered CD4+ T cells produced by a method provided herein, a composition of engineered CD8+ T cells produced by a method provided herein, and instructions for administering the engineered CD4+ T cells and the engineered CD8+ T cells to a subject. In certain instances, the instructions specify separately administering the CD4+ T cells and CD8+ T cells to the subject. In certain instances, the instructions specify administering the CD4+ T cells and the CD8+ T cells to the subject at a desired ratio. Also described herein as part of the disclosure is an article of manufacture comprising a composition of engineered CD4+ and CD8+ T cells produced by a method provided herein, and instructions for administering the engineered CD4+ and CD8+ T cells to a subject.

Described herein as part of the disclosure is a long-term stimulation method for assessing a cell composition including incubating, for a period of time of at least 10 days, an input composition under conditions to stimulate a CAR-dependent activity in cells in the input composition, said input composition containing T cells expressing a chimeric antigen receptor (CAR) containing an extracellular antigen-binding domain that specifically binds or recognizes an antigen, thereby producing an output composition; and assessing one or more phenotype or activity of one or more cells of the output composition.

In some instances, the conditions to stimulate a CAR-dependent activity includes the presence of a binding molecule that specifically binds to the antigen-binding domain of the CAR. In some instances, the binding molecule is attached to a support. In some instances, the support is a solid support. In some instances, the solid support is the surface of a well of a microplate or a bead. In some instances, the solid support is a microplate having the binding molecule attached to the microplate, and the incubation is carried out in the microplate. In some instances, the solid support is a bead having attached the binding molecule, and the incubation is carried out in the presence of a plurality of the beads.

In some instances , the binding molecule is or comprises a recombinant antigen or a portion thereof recognized by the antigen-binding domain. In some instances, the recombinant antigen or portion thereof is BCMA, or is a portion thereof recognized by the antigen-binding domain. In some instances, the binding molecule is or includes an anti-iditopytic antibody or antigen-binding fragment thereof that specifically binds to the antigen-binding domain. In some instances, the antigen-binding domain of the antigen receptor is or comprises antibody SJ25C1 or an antigen-binding fragment thereof. In some instances, the antigen-binding domain of the antigen receptor is or includes antibody FMC63 or an antigen-binding fragment thereof.

In some instances, the method is carried out in vitro or ex vivo.

In some instances, the input composition is incubated in the presence of a media that does not comprise recombinant cytokines. In some instances, the incubation is carried out continuously or is not interrupted for the period of time. In some instances, during the incubation, cells are not replated, media is not changed and binding molecule is not added.

In some instances, the method includes assessing one or more phenotypes of activation, exhaustion or differentiation state of the one or more cells of the output composition. In some instances, the phenotype is exhaustion and the assessing includes measuring the expression, optionally surface expression, of one or more markers selected from CTLA-4, FOXP3, PD-1, TIGIT, LAB-3, 2B4, BTLA, TIM3, VISTA, or CD96. In some instances, the phenotype is activation and the assessing includes measuring the expression, optionally surface expression, of one or more markers selected from CD25, CD26, CD27, CD28, CD30, CD71, CD154, CD40L, CD127, LAG3, or Ki67. In some instances, the phenotype is differentiation state and the assessing includes measuring one or more markers selected from (i) one or more of CD25, CD45RO, CD56, KLRG1, CD95 and/or (ii) one or of CD45RA, CD27, CD28, CD62L, and CCR7, optionally wherein the one or more markers are markers are positively or inversely associated with naive-like T cells.

In some instances , the method includes assessing one or more activities of the one or more cells of the output composition. In some instances, the one or more activities comprises a CAR-dependent activity, optionally an antigen-stimulated activity. In some instances, the one or more activities comprises cytolytic activity or cytokine production.

In some instances , the period of time is at least or at least about 11 days, 12 days, 13 days, 14 days, or 15 days. In some embodiments, the period of time is or is about 11 days, 12 days, 13 days, 14 days or 15 days.

In some instances , the input composition contains cells that have been exposed or contacted with a test agent or compound prior to the incubation, optionally wherein the exposing or contacting is carried out during one or more steps of a process for producing the input composition comprising the T cells expressing the CAR. In some instances, the method is carried out on a plurality of input compositions, each of said input compositions of the plurality being produced by a different process.

In some instances , the described method further includes comparing the phenotype or activity of the output composition to the phenotype or activity of a control composition, optionally wherein the control composition is a composition of T cells that have been incubated for the at least 10 days under the same conditions to stimulate the CAR-dependent activity, said composition of T cells having not been produced in the presence of the test agent or compound or having been produced by an alternative process compared to the input composition. In some instances, the method further includes identifying an output composition that exhibits reduced exhaustion, reduced activation or decreased differentiation, such as compared to the control compositions. In some instances, the decreased differentiation comprises increased expression of one more naive-like T cell markers.

### Brief Description of the Drawings

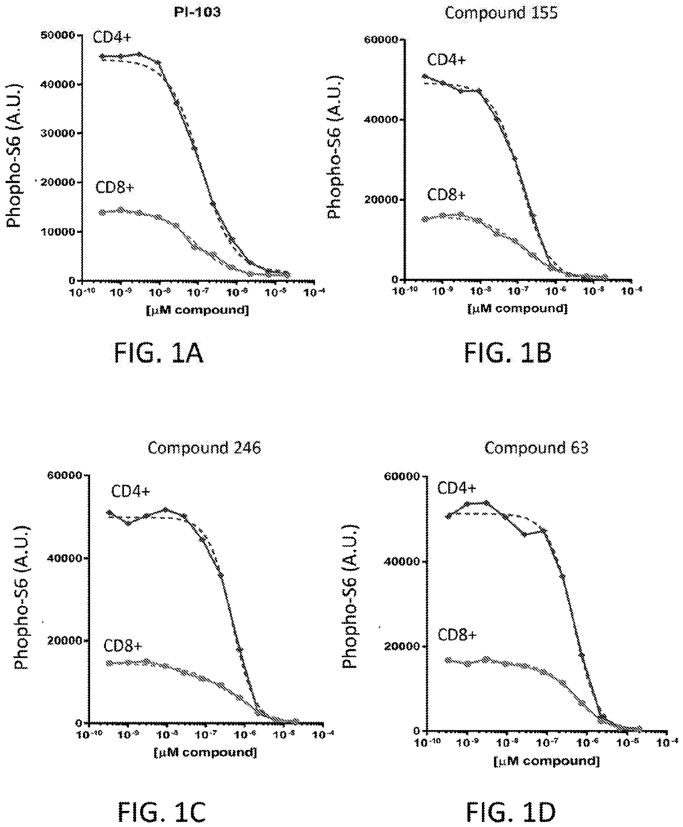

FIGS. 1A-1D shows graphs displaying the levels phosphorylated S6 detected in CD4+ and CD8+ T cells incubated with anti-CD3 and anti-CD28 antibody conjugated magnetic beads in the presence of varying concentrations of PI-103 (FIG. 1A), Compound 155 (FIG. 1B), Compound 246 (FIG. 1C), or Compound 63 (FIG. 1D).

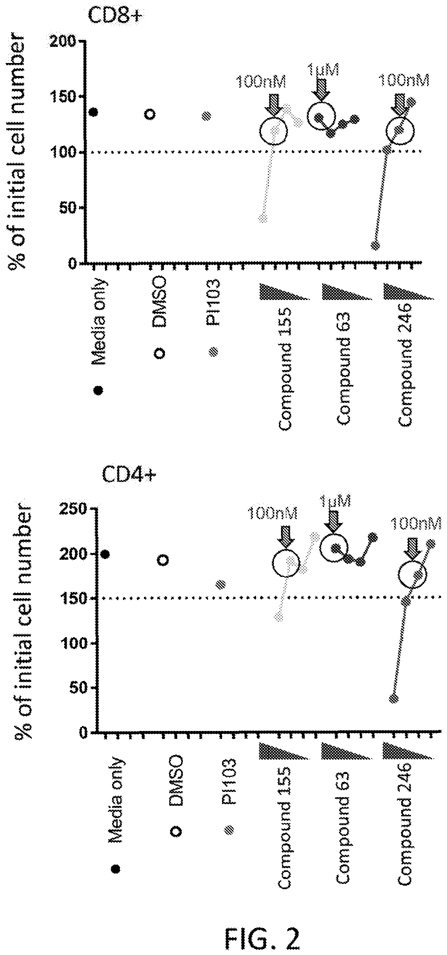

FIG. 2 shows graphs displaying the percent of initial cell number for engineered CD8+ (top panel) and CD4+ (bottom panel) T cells that are present following an incubation in a media only control, or in the presence of DMSO, PI-103, or varying concentrations of Compound 155, Compound 63, or Compound 246. Arrows indicate the highest tolerated dose of Compound 155, Compound 63, and Compound 246 that resulted in similar levels of CD8 and CD4 T cell expansion. Dotted horizontal lines indicate 70% of the mean values of the media and DMSO controls.

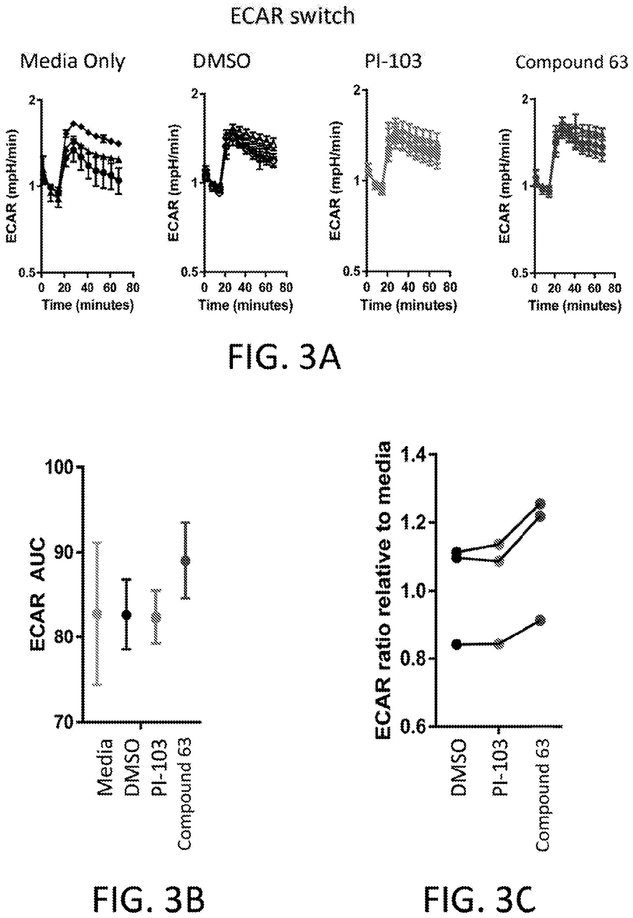

FIGS. 3A-3C show graphs of the cellular glycolytic metabolism in CD8+ T cells among the generated anti-CD19 CAR-T cell composition. FIG. 3A provides graphs displaying the extracellular acidification rate (ECAR) in real time of CD8+ CAR-T cells among anti-CD19 CAR-T cells generated by expansion in the presence of media only, DMSO, PI-103, or Compound 63. FIG. 3B displays the area under the curve (AUC) calculated for the ECAR rates (mpH/min) relative to media only controls. FIG. 3C shows maximal ECAR glycolytic burst ratio relative to media only controls.

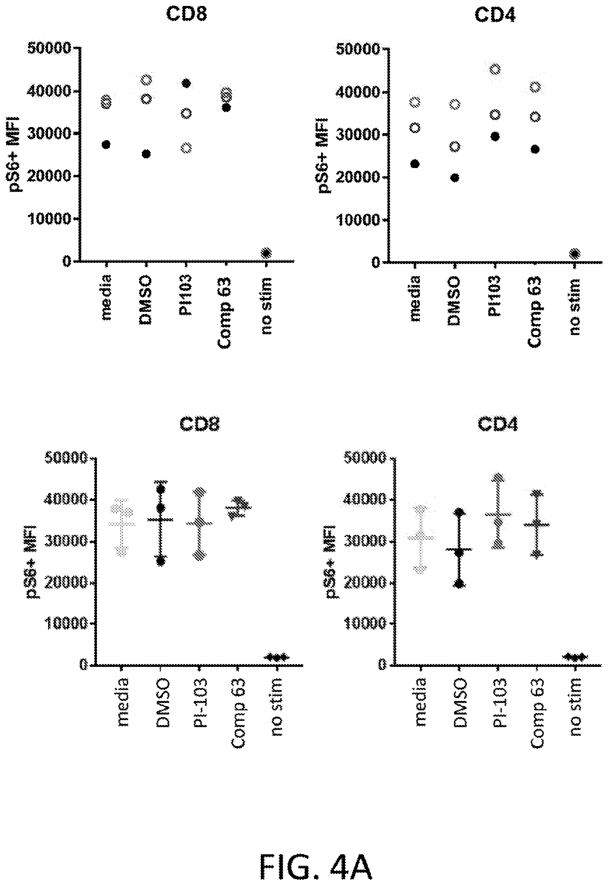

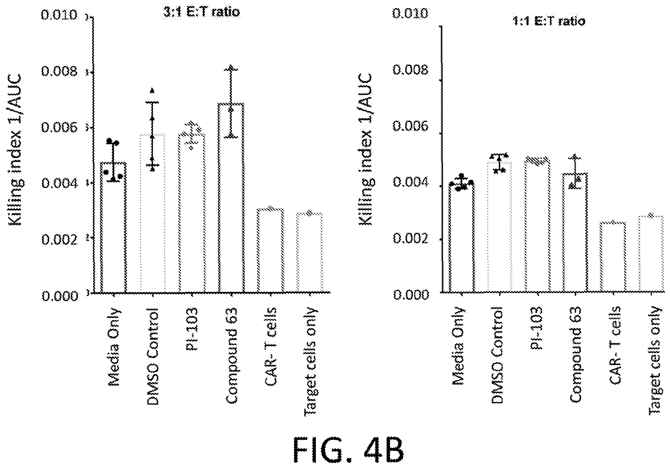

FIGS. 4A-4B provide graphs displaying the results of assays performed on anti-CD19 CAR-T cell compositions that were generated by expansion in the presence of media only, DMSO, PI-103, or Compound 63. FIG. 4A shows the mean fluorescent intensity of phospho-S6 staining in CD8+ and CD4+ T cells of the generated anti-CD19 CAR-T cell compositions following co-culture with irradiated K-562 cells transduced to express CD19 (irradiated K562-CD19 target cells) and cells that were not exposed to an antigen (no stim). Top panels display individual data points measured from separate cell compositions. Bottom panels display mean values +/- standard deviation. FIG. 4B provides graphs displaying the cytolytic activity of the generated CAR-T cell compositions co-cultured with K562-CD19 target cells at ratio of 3:1 or 1:1 effector cells to target cells. Measurements from generated CAR-T cell compositions and CD19 expressing K562 cells are provided as controls.

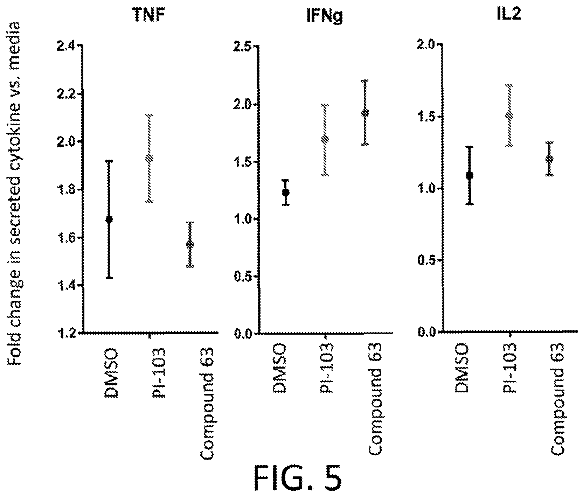

FIG. 5 provides graphs displaying secretion of TNF-alpha (left panel), IFN-gamma (middle panel), and IL-2 (right panel), of anti-CD 19 CAR-T cell compositions that were co-cultured with irradiated K562-CD19 target cells. The fold-change of cytokine production observed in co-culture supernatants from generated anti-CD19 CAR-T cell compositions expanded in the presence of PI-103, Compound 63 or DMSO vehicle compared to cells expanded in media only is displayed.

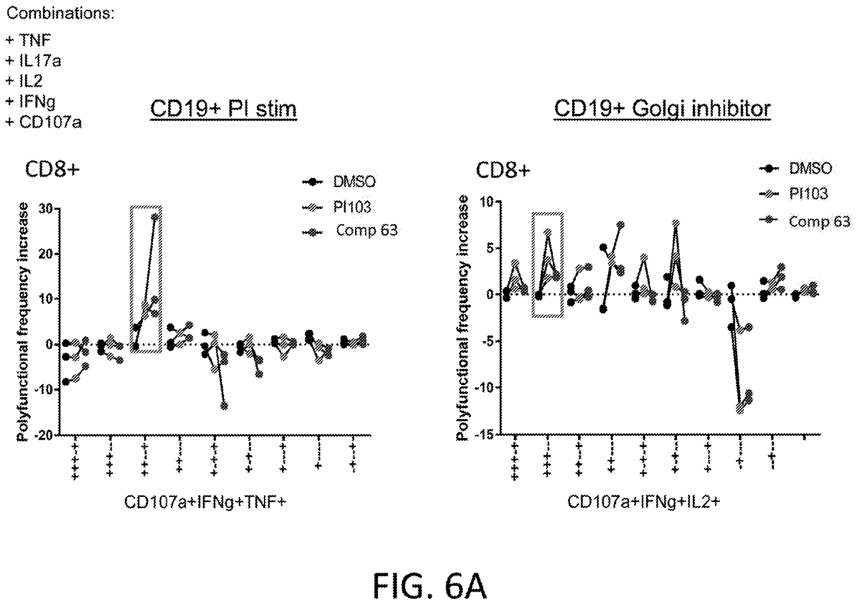

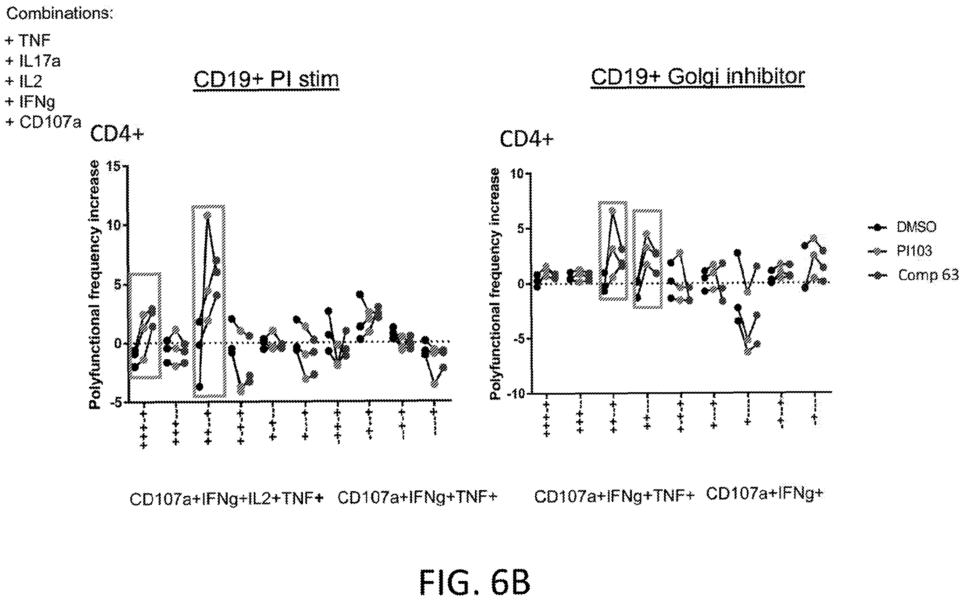

FIGS. 6A and 6B show graphs displaying the polyfunctional cytokine profiles of CD8+ (FIG. 6A) and CD4+ (FIG. 6B) T cells from generated anti-CD 19 CAR-T cell compositions that were co-cultured with irradiated K562-CD19 target cells and then further incubated PMA/Ionomycin (left panels) or Golgi inhibitor (right panels). The increased frequency of cells positively staining for different combinations of CD107a, IFN-gamma (IFNg), IL-2, IL-17a, and TNF-alpha (TNFa) in generated anti-CD19 CAR-T cell compositions expanded in the presence of PI-103, Compound 63 or DMSO vehicle as compared to cells expanded in media only is displayed.

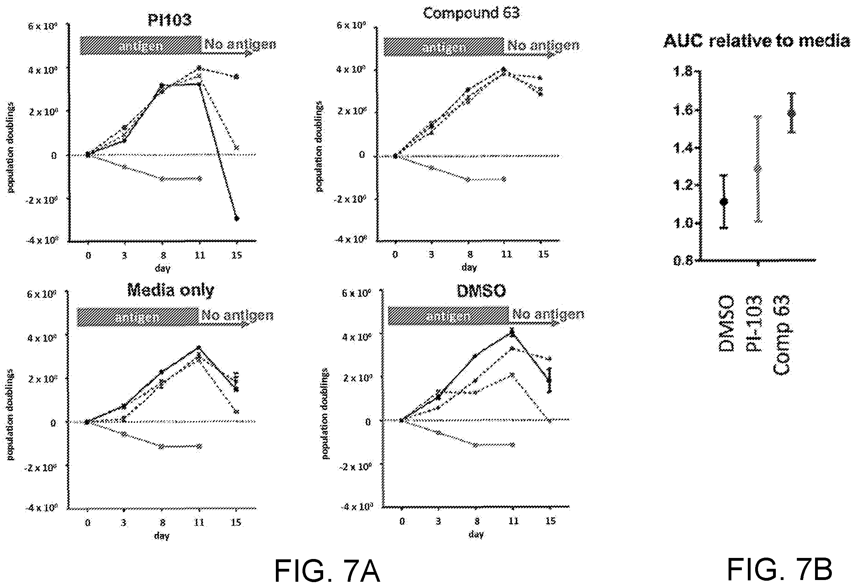

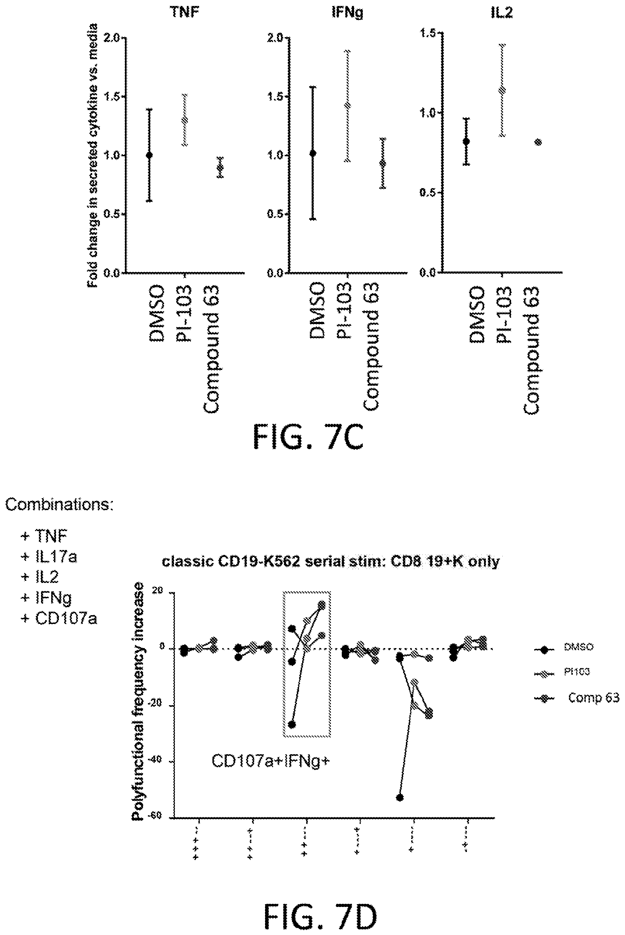

FIGS. 7A-7D show graphs displaying activity of T cells from generated anti-CD19 CAR-T cell compositions that were expanded in the presence of media only, DMSO, PI-103, or Compound 63 following repeated stimulations. FIG. 7A shows population doubling of the T cells from generated anti-CD19 CAR-T cell compositions co-cultured with irradiated K562-CD19 target cells. After 4 rounds of restimulation, T cells were re-cultured without targets cells at day 11. FIG. 7B displays the area under the curve (AUC) calculated for population doublings relative to media only controls. FIG. 7C shows a graph displaying the production of TNF-alpha (TNF), IFN-gamma (IFNg), and IL-2 by T cells from generated anti-CD19 CAR-T cell compositions following stimulation with a 16 hour co-culture with irradiated K562-CD19 target cells following 4 rounds of repeated stimulations with irradiated K562-CD19 target cells. The fold change of extracellular TNF-alpha (TNF), IFN-gamma (IFNg), and IL-2 as compared to the media only condition is shown. FIG. 7D shows graphs depicting the polyfunctional cytokine profiles of CD8+ T cells from generated anti-CD19 CAR-T cell compositions that followed 4 rounds of re-stimulation with irradiated K562-CD19 target cells.

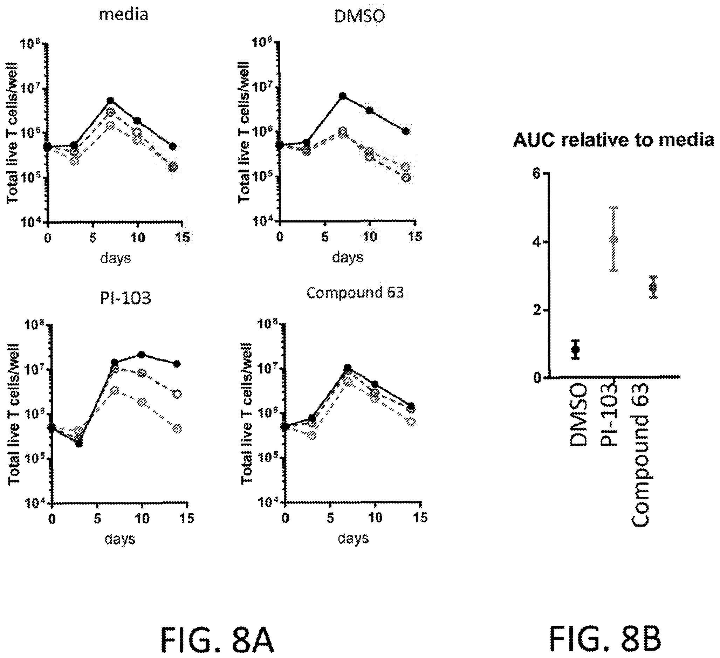

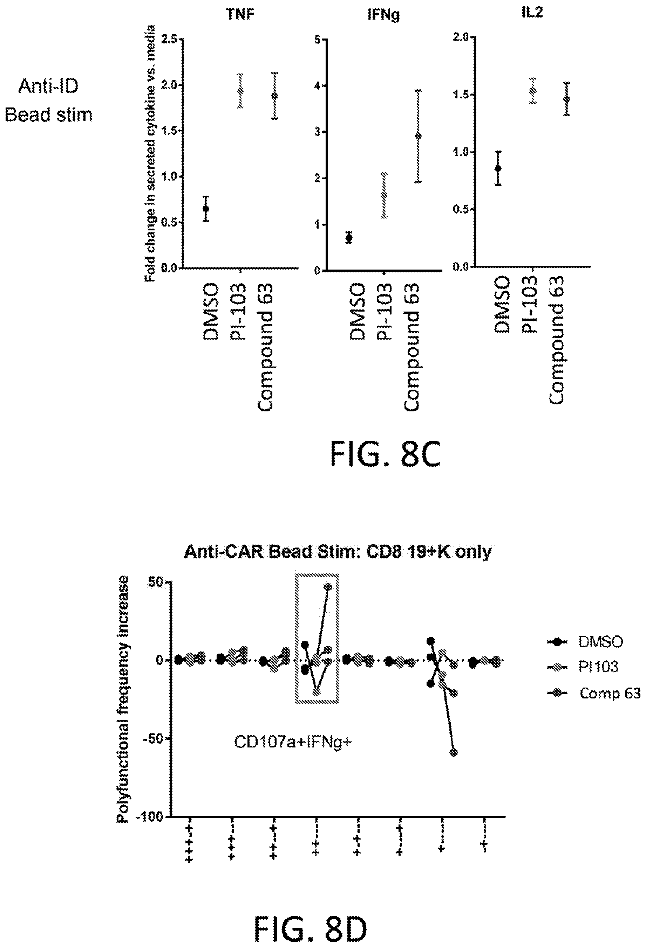

FIGS. 8A-8D show graphs displaying activity of T cells from generated anti-CD19 CAR-T cell compositions that were expanded in the presence of media only, DMSO, PI-103, or Compound 63 following stimulation with beads surface conjugated with anti-idiotype antibody specific to the anti-CD19 CAR. FIG. 8A shows the total live T cell counts per well of T cells from generated anti-CD 19 CAR-T cell compositions co-cultured with beads surface conjugated with the anti-idiotype antibody. FIG. 8B displays the area under the curve (AUC) calculated for the live T cell counts relative to media only controls. FIG. 8C shows a graph displaying the production of TNF-alpha (TNF), IFN-gamma (IFNg), and IL-2 by T cells from generated anti-CD19 CAR-T cell compositions following stimulation with a 16 hour co-culture with irradiated K562-CD19 target cells that followed a 15 day incubation with beads surface conjugated to the anti-idiotype antibody. The fold change of extracellular TNF-alpha (TNF), IFN-gamma (IFNg), and IL-2 as compared to the media only condition is shown. FIG. 8D shows graphs depicting the polyfunctional cytokine profiles of CD8+ T cells from generated anti-CD 19 CAR-T cell compositions that followed a 15 day incubation with beads conjugated with the anti-idiotype antibody.

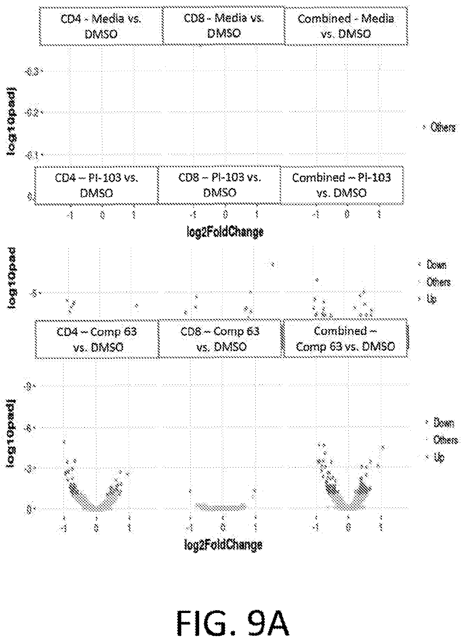

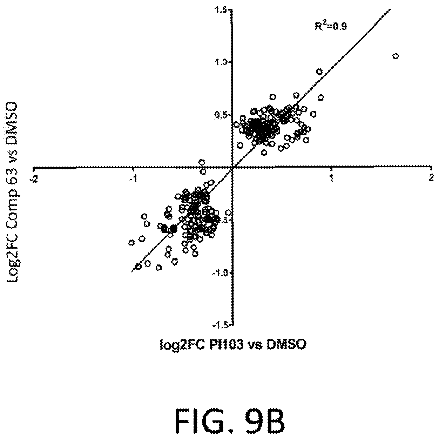

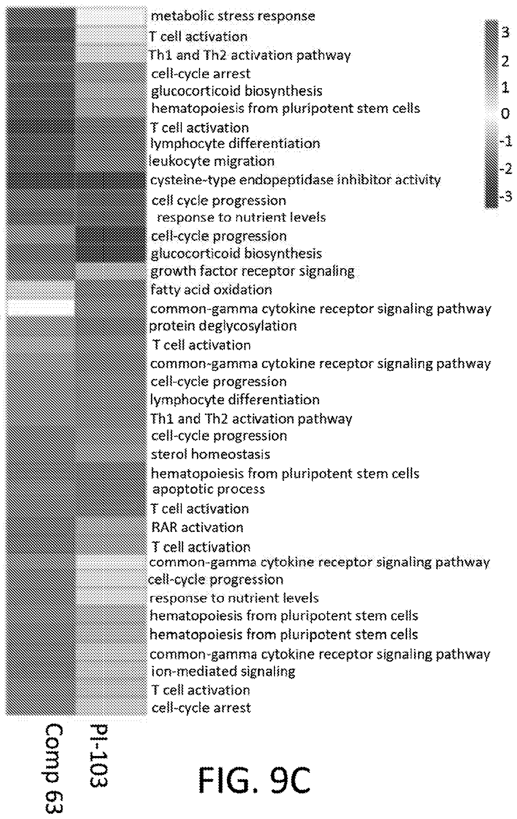

FIGS 9A-9C show graphs displaying results of an RNA-Seq analysis of CD4+ and CD8+ T cells from generated anti-CD19 CAR-T cell compositions that were expanded in the presence of media only, DMSO, PI-103, or Compound 63. FIG. 9A shows volcano plots depicting the differentially expressed gene expression by CD4+ (left panels), CD8+ (middle panels), and combined CD4+ and CD8+ (right panels) T cells from generated anti-CD 19 CAR-T cell compositions. Differential gene expression is shown for T cells expanded in media only (top row), PI-103 (middle row), and Compound 63 (bottom row) as compared to T cells expanded with DMSO. FIG. 9B shows a graph depicting log2 fold change (Log2FC) of differentially expressed genes measured in T cells from generated anti-CD 19 CAR-T cell compositions that were expanded in the presence of PI-103 (X-axis) and Compound 63 (Y-axis) relative to the expression in T cells from generated anti-CD 19 CAR-T cell compositions that were expanded in the presence of DMSO. FIG. 9C shows a table and graph depicting exemplary identified gene ontology (GO) categories and their corresponding Z-scores.

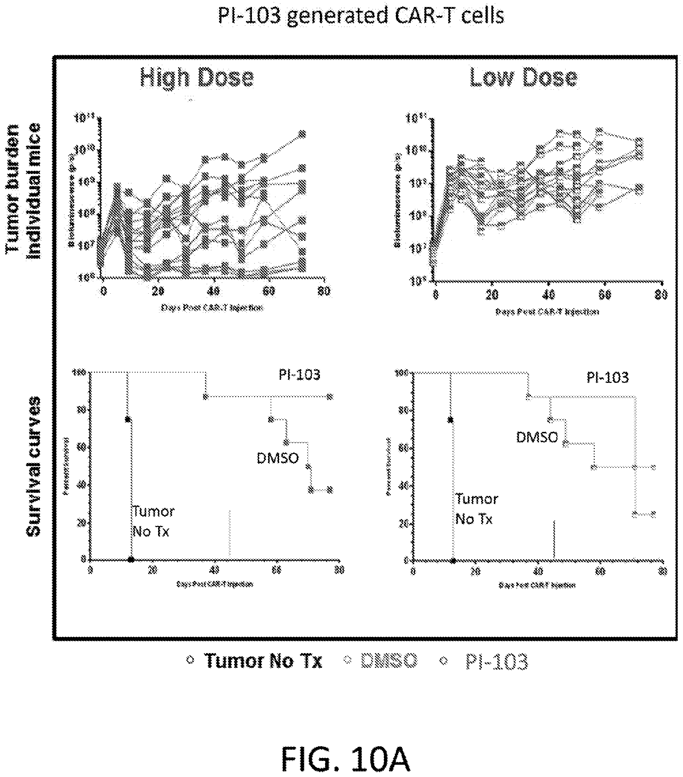

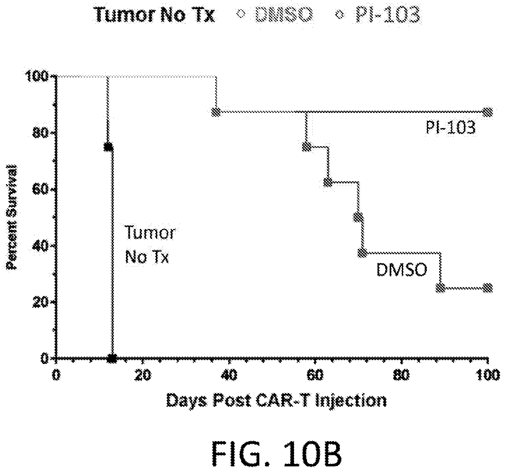

FIGS. 10A and 10B show graphs displaying the tumor burden and survival in tumor bearing mice following treatment with anti-CD19 CAR-T cells. FIG. 10A show graphs displaying the tumor burden and survival to day 80 in tumor bearing mice following treatment with anti-CD 19 CAR-T cells. FIG. 10B shows a graph displaying survival to day 100 in tumor bearing mice following treatment with anti-CD19 CAR-T cells. Nod scid gamma (NSG) immunodeficient mice were implanted with Raji cells that expressed firefly luciferase and received either no treatment, or treatment with a high (left panels) or low (right panels) dose of anti-CD19 CAR-T cell that were expanded in the presence of DMSO or PI-103. Tumor burden of individual mice as measured by bioluminescence (top panels) and survival curves of the treatment groups (bottom panels) are shown at the indicated times as shown in the FIG. 10A and 10B.

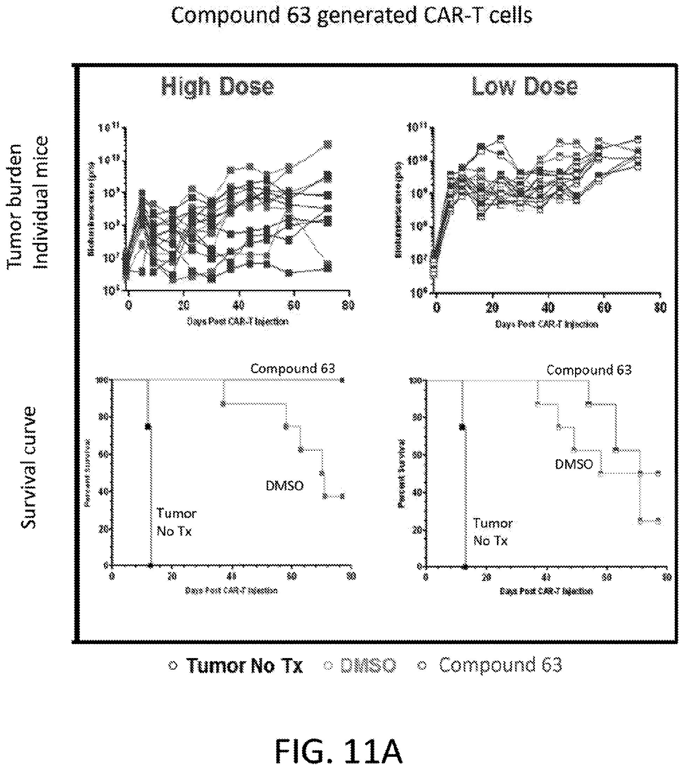

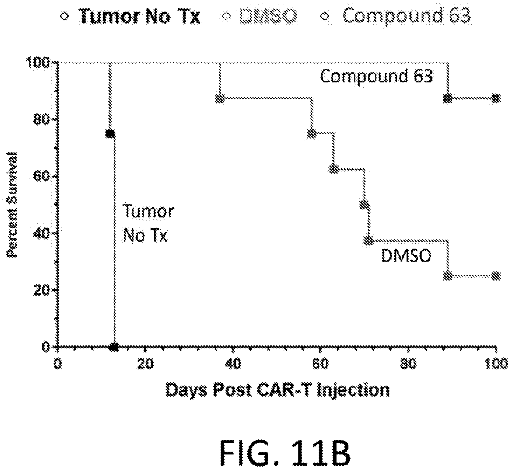

FIGS. 11A and 11B show graphs displaying the tumor burden and survival in tumor bearing mice following treatment with anti-CD19 CAR-T cells. FIG. 11A shows graphs displaying the tumor burden and survival to day 80 in tumor bearing mice following treatment with anti-CD19 CAR-T cells. FIG. 10B shows a graph displaying survival to day 100 in tumor bearing mice following treatment with anti-CD19 CAR-T cells. NSG immunodeficient mice were implanted with Raji cells that expressed firefly luciferase and received either no treatment, or treatment with a high (left panels) or low (right panels) dose of anti-CD19 CAR-T cell that were expanded in the presence of DMSO or Compound 63. Tumor burden of individual mice as measured by bioluminescence (top panels) and survival curves of the treatment groups (bottom panels) are shown at the indicated times as shown in the FIG. 11A and 11B.

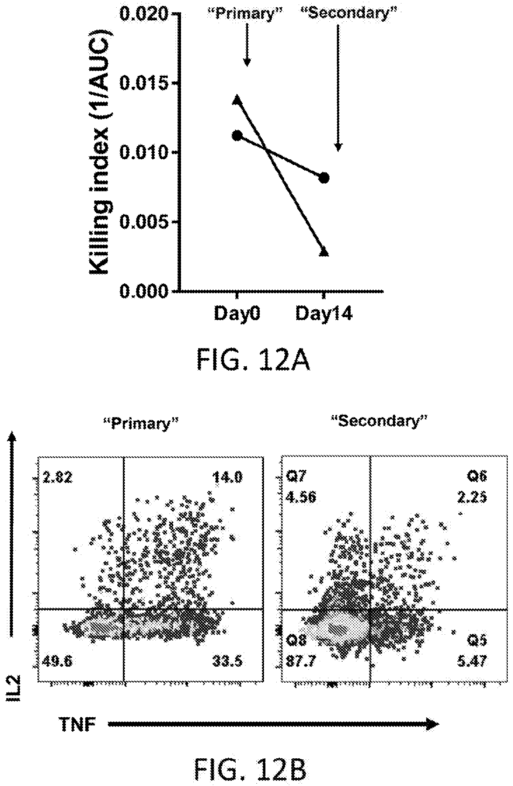

FIGS. 12A and 12B present graphs showing activity of T cell compositions containing anti-CD19 CAR+ T cells. Cells were either incubated with anti-CD19 antibody anti-ID conjugated beads for 14 days (Day 14; secondary) or were not incubated prior to assessing activity. Results from a cytotoxicity assay (FIG. 12A) and an internal cytokine staining (ICS) assay following exposure to CD19 expressing cells (FIG. 12B) are shown.

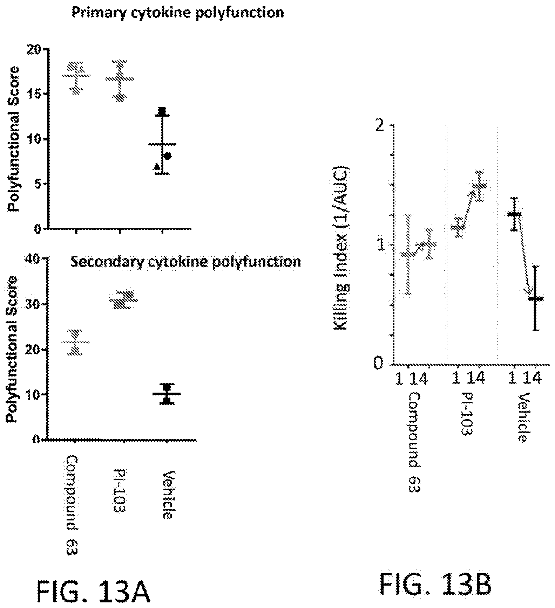

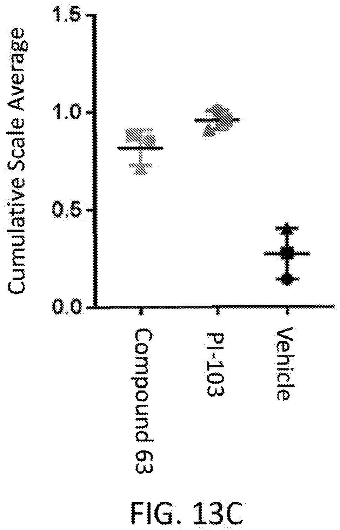

FIGS. 13A-13C present graphs showing characteristics of T cell compositions containing anti-CD19 CAR+ T cells during or following incubation with anti-CD19 antibody anti-ID conjugated beads for 14 days. Results from T cell compositions that were generated in the presence of PI-103, Compound 63, or a vehicle are shown. FIGS. 13A and 13B show activity in response to exposure to CD19 cells of T cell compositions that were not incubated (primary) or incubated for 14 days (secondary). Results of polyfunctional staining by ICS (FIG. 13A) and a cytolytic activity (FIG. 13B) following exposure to CD19 expressing cells are shown. FIG. 13C depicts levels of secreted cytokine from supernatant of cell compositions containing anti-CD19 CAR expressing cells that were incubated at a ratio of 1:1 with CD19 expressing cells for 20 hours. Amounts of IL2, TNF, and IFN-gamma were measured and the average of the scaled scores for all three cytokines is shown.

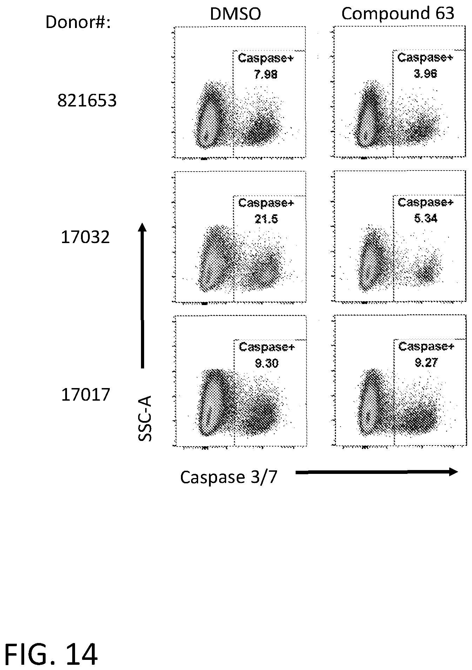

FIG. 14 depicts expression of the pro-apoptotic intracellular caspase 3 on thawed CAR-T cells prepared in the presence of DMSO or Compound 63. FACS plots show viable CD3+CAR+ T cells, prepared from three different donors.

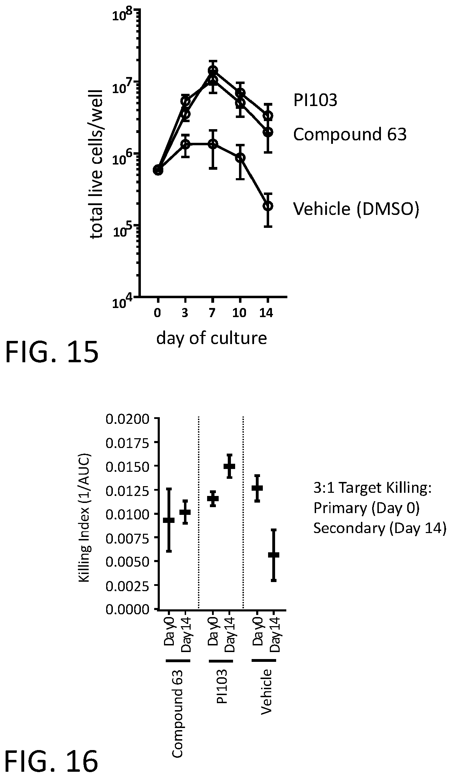

FIG. 15 presents a graph showing viable cell numbers over time of CAR-T cells prepared in the presence of DMSO, PI103, or Compound 63. Symbols represent mean and SEM of culture wells from three individual donors.
FIG. 16 presents a graph showing target cell killing by thawed CAR-T cells prepared in the presence of DMSO, PI103,or Compound 63. Killing assays were set up directly post-thaw (day 0) or at the end of the CAR stimulation culture (day 14) described in FIG. 15. Symbols represent mean and SEM of culture wells from three individual donors.

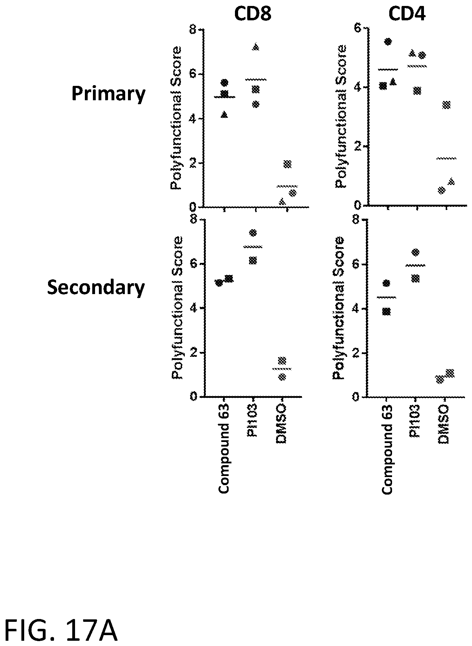

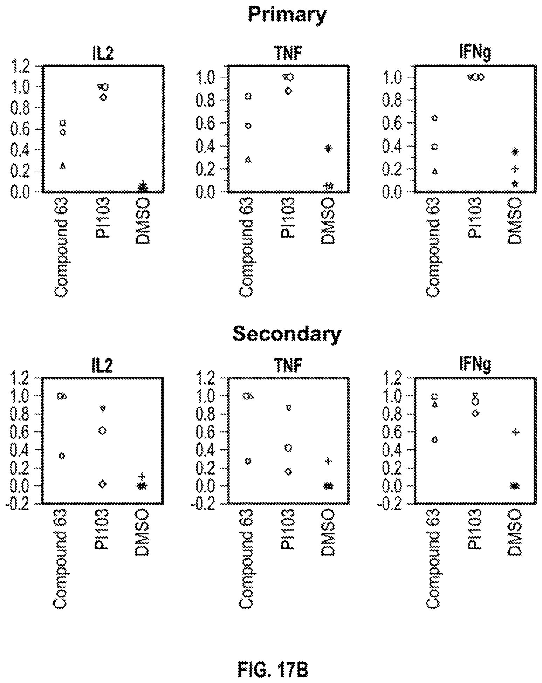

FIGS. 17A-17B present graphs showing that CAR-T cells prepared in the presence of PI103 or Compound 63 have improved and sustained effector cytokine profiles. CAR-T cells were mixed 1: 1 (CAR-T:target) with antigen-bearing target cells directly post thaw ("Primary", top panels) or at the end of the CAR stimulation culture as described in FIG. 15 ("Secondary", bottom panels). T cell polyfunctionality was assessed by intracellular expression of cytokines IL2, TNF and IFNg by FACS from CAR-T cells incubated with targets in the presence of a golgi inhibitor for 5 hours (FIG. 17A). Cytokine secretion into culture supernatants from CAR-T cells incubated with targets for 20 hours was also measured (FIG. 17B). Assay values were normalized and rank-scored by feature scaling within each donor cohort.

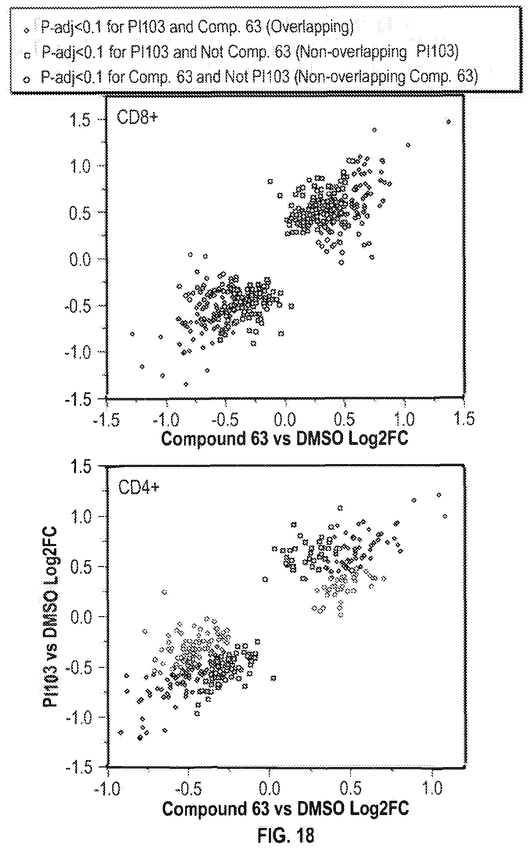

FIG. 18 presents results of a differential Expression (DESeq2) analysis of RNAseq performed on enriched CD8+ and CD4+ CAR-T cells generated in the presence of PI103 or Compound 63 as described in FIG. 15. Differential expression of genes with adjusted p-value (p-adj)<0.1 for PI103 vs DMSO or Compound 63 vs DMSO were selected and log2 fold-change gene expression values are shown. Genes significantly differentially expressed in PI103-treated cells only are shown with squares. Genes significantly differentially expressed in Compound 63-treated cells only are shownwith circles. Genes expressed in both T cell compositions are shown with diamonds. The differential expression values are the mean of three individual donors per group.

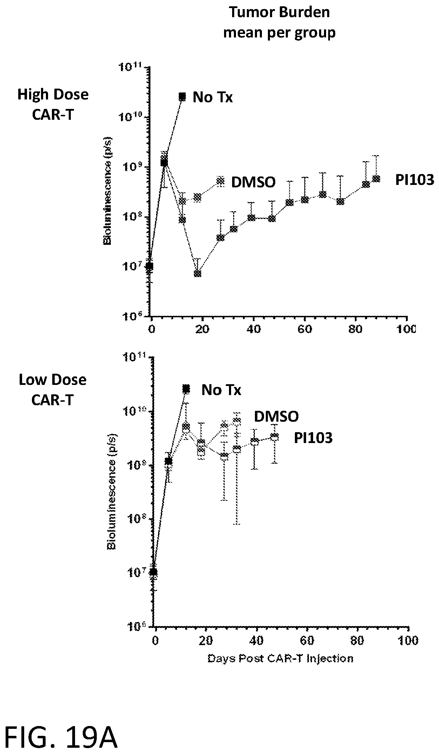

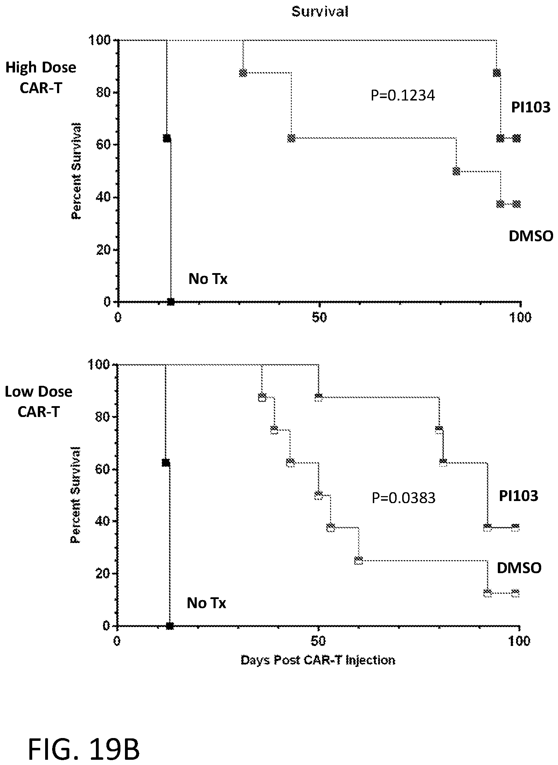

FIGS. 19A-19B show graphs displaying the tumor burden and survival in tumor bearing mice following treatment with anti-CD19 CAR-T cells. FIG. 19A show graphs displaying the tumor burden in mice following treatment with either a high dose (1 × 10⁶ CAR-T cells/mouse) or a low dose (2.5 × 10⁵ CAR-T cells/mouse) of anti-CD19 CAR-T cells. FIG. 19B show graphs displaying survival of tumor bearing mice following treatment with either a high dose or a low dose of anti-CD19 CAR-T cells.

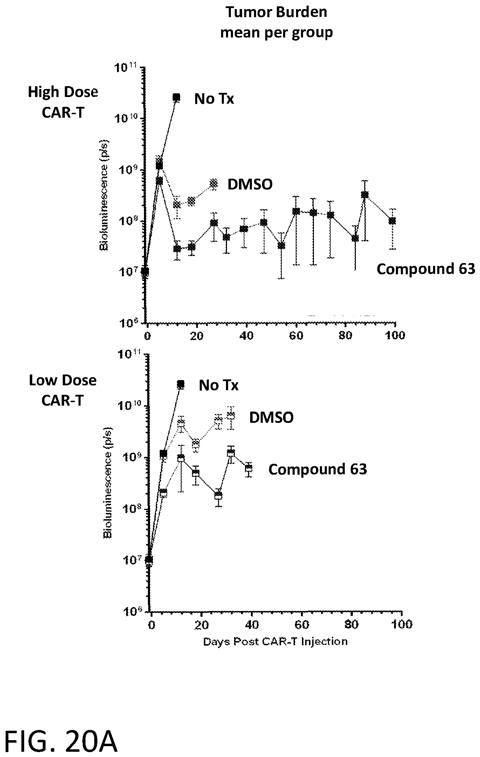

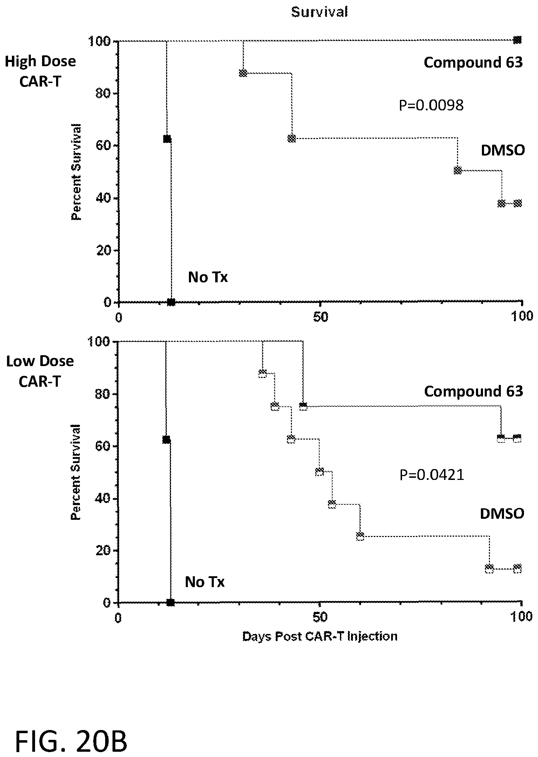

FIGS. 20A-20B show graphs displaying the tumor burden and survival in tumor bearing mice following treatment with anti-CD19 CAR-T cells. FIG. 20A show graphs displaying the tumor burden in mice following treatment with either a high dose (1 × 10⁶ CAR-T cells/mouse) or a low dose (2.5 × 10⁵ CAR-T cells/mouse) of anti-CD19 CAR-T cells. FIG. 20B show graphs displaying survival of tumor bearing mice following treatment with either a high dose or a low dose of anti-CD19 CAR-T cells.

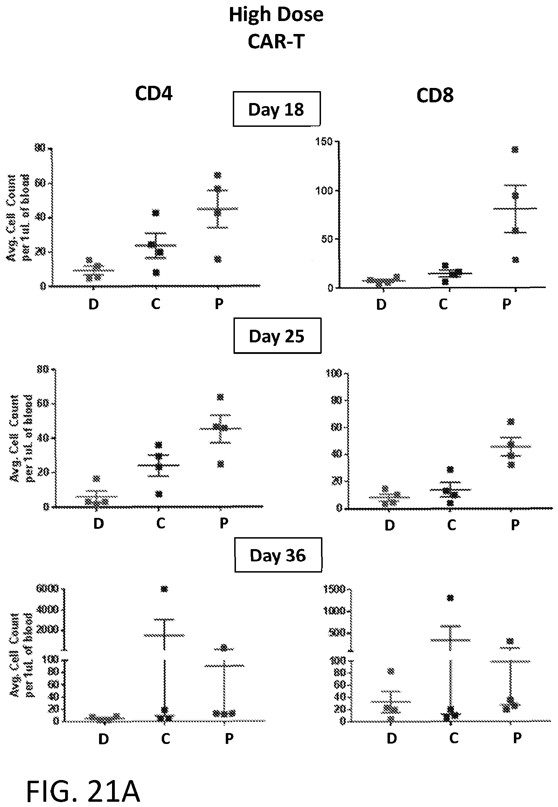

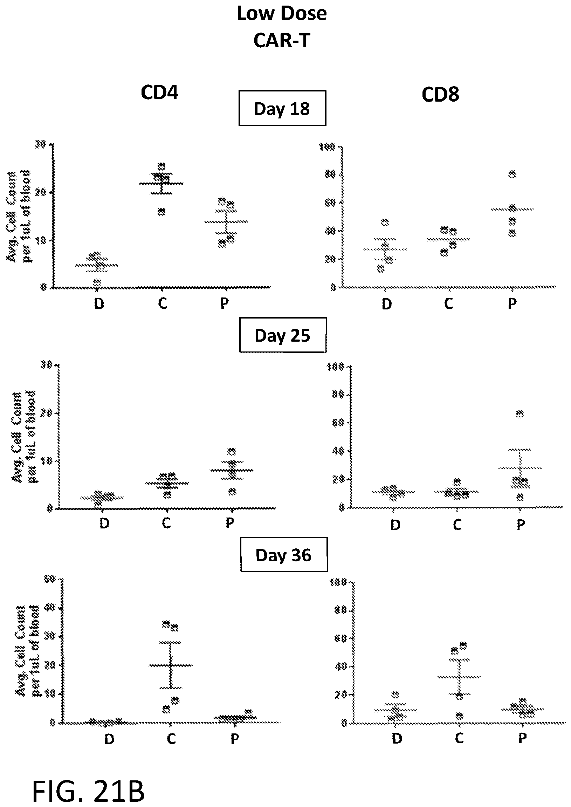

FIGS. 21A-21B show graphs illustrating the persistence of anti-CD19 CAR-T cells over time in blood of recipient mice. FIG. 21A shows CD4+ and CD8+ T cell counts at day 18, day 25 and day 36 post-injection in mice that received a high dose (1 × 10⁶ CAR-T cells/mouse) of CAR-T cells prepared in the presence of DMSO (D), Compound 63 (C), or PI103 (P). FIG. 21B shows CD4+ and CD8+ T cell counts at day 18, day 25 and day 36 post-injection in mice that received a low dose (2.5 × 10⁵ CAR-T cells/mouse) of CAR-T cells prepared in the presence of DMSO (D), Compound 63 (C), or PI103 (P).

### Detailed Description

### Embodiments of the invention are described in some of the instances and aspects below.

Disclosed herein are methods of producing a composition of engineered cells, such as engineered CD4+ and/or CD8+ cells, expressing a recombinant receptor in which one or more steps of the process, such as incubating, stimulating and/or cultivating the cells, is carried out in the presence of an agent that inhibits mTOR activity. In certain instances, the engineered cell composition is a cell composition of enriched CD4+ or enriched CD8+ primary T cells that include T cells engineered with a recombinant receptor. In certain instances, the T cells are human T cells. In some aspects, cultivating the cells, such as for expansion and/or proliferation of the cells, is carried out in the presence of an agent that inhibits mTOR activity. In some instances, the cells in the composition have not been contacted with, incubated with, and/or exposed to the agent prior to being cultivated. In certain instances, the cultivation results in the proliferation or expansion of the cells in the composition to produce an output composition comprising engineered CD4+ and/or CD8+ T cells.

Manufacture of genetically engineered T cells, such as CAR-T cells, for use in cell therapy involves isolation of cells, activation of cells, transduction or engineering of cells with a recombinant receptor and expansion of T cells for clinical dosing. This process may result in a portion of the engineered T cell drug product that, in some cases, may include cells that have been driven to a state of terminal exhaustion and/or in which the cells lack persistence and/or do not exhibit optimal efficacy. In some cases, multipotency and T cell replicative potential are diminished. In some aspects, an engineered T cell drug product containing exhausted cells may, in some cases, limit the potential efficacy of the T cell drug product. Alternative methods for manufacture of engineered cell therapies are needed that minimize or reduce the percentage or number of cells that are or are likely to become exhausted, lack persistence and/or have decreased efficacy when administered to a subject.

The methods disclosed herein are based on observations that the persistence, lack of exhaustion and/or efficacy of engineered cells, e.g. CAR-T cells, is improved by manufacturing or producing the cells in the presence of an agent that inhibits mTOR activity. In some aspects, the agent is an inhibitor of mammalian target of rapamycin (mTOR), such as mammalian, e.g. human, mTOR. In some instances, the agent is specific to mTOR and does not inhibit or have target activity against other related kinases, such as a kinase of the phosphoinositide 3-kinase (PI3-kinase) family. mTOR is an evolutionarily conserved kinase with substantial sequence homology with the phosphoinositide 3-kinase (PI3-kinase) family. Unlike PI3K, mTOR is not a lipid kinase but rather a serine-threonine protein kinase. In mammalian cells, mTOR is encoded as a single gene whose protein product signals via two distinct complexes (mTORC1 and mTORC2). In general, mTOR is thought to regulate various cellular processes including cell growth and proliferation. In T cells, mTOR may be activated by various stimuli that include activation of cytokine receptors and T cell receptors.

In some aspects, the disclosed methods are used in connection with a process whereby engineered T cells are incubated, cultivated, and/or cultured in the presence of an agent that inhibits mTOR activity, which may, in some aspects, improve or enhance expansion, persistence, and/or efficacy, e.g. anti-tumor activity, of the cells. In some aspects, an agent that inhibits mTORC1 and mTORC2 kinase activity is used. In certain instances, the agent is a selective mTOR inhibitor, e.g., it does not inhibit an additional kinase, e.g., PI3K. In some aspects, the agent is Compound 63, Compound 155, or Compound 246.

In some aspects, the disclosed methods produce compositions of cells that include primary T cells engineered to express a recombinant receptor ("engineered cells"), such as for use in cell therapy, that contain fewer exhausted cells and/or fewer cells that display markers or phenotypes associated with exhaustion as compared to compositions of engineered cells that are produced by alternative methods, such as alternative methods that are not carried out in the presence of an agent that inhibits mTOR activity. In particular instances, the engineered cells produced by the disclosed methods contain an increased percentage of memory-like T cells, such as long-lived memory T cells, compared to cells from compositions of engineered cells produced by alternative processes, such as methods that are not carried out in the presence of an agent that inhibits mTOR activity, e.g., kinase activity such as mTORC1 or mTORC2 kinase activity. In certain aspects, the engineered cells produced by the disclosed methods are less differentiated than engineered cells produced by alternative methods. In some aspects, the disclosed methods produce engineered cells with improved or enhanced expansion, persistence, and/or anti-tumor activity as compared to engineered cells that are generated by alternative methods, such as alternative methods that are not carried out in the presence of an agent that inhibits mTOR activity. Thus, in some aspects, the disclosed methods may generate compositions of engineered cells with improved therapeutic efficacy as compared to engineered cell compositions produced by alternative methods, such as alternative methods that are not carried out in the presence of an agent that inhibits mTOR activity. In certain instances, the disclosed methods may generate compositions of engineered cells with an improved clinical durability of response as compared to engineered cell compositions produced by alternative methods. In some of any such instances, the comparison to an alternative process is to the same process that differs only in that the alternative process is not carried out in the presence of an agent that inhibits mTOR activity.

If a definition set forth herein is contrary to or otherwise inconsistent with a definition set forth in the patents, applications, published applications and other publications referred to herein , the definition set forth herein prevails.

The section headings used herein are for organizational purposes only and are not to be construed as limiting the subject matter described.

### I. PROCESS FOR ISOLATING, CULTURING, AND ENGINEERING CELLS FOR ADOPTIVE CELL THERAPY

Disclosed herein are methods for generating an output composition of engineered cells, such as engineered primary CD4+ T cells and/or engineered CD8+ T cells, that express a recombinant protein, e.g., a recombinant receptor such as a T cell receptor (TCR) or a chimeric antigen receptor (CAR). In some instances, the methods disclosed herein are used in connection with a process that includes incubating, cultivating, and/or culturing cells in the presence of an agent that inhibits mTOR activity, such as any described herein, e.g. Compound 63, to generate the output composition of engineered cells. In some instances, the methods are used in connection with a process that includes cultivating a composition of engineered cells, such as under conditions that promote expansion and/or proliferation, in the presence of an agent that inhibits mTOR activity, such as any described herein, e.g. Compound 63, to generate the output composition of engineered cells. In some instances, the agent that inhibits mTOR activity inhibits activity of one or more additional kinases. In some instances, the agent selectively and/or specifically inhibits mTOR activity. In certain instances, the agent that inhibits mTOR activity is an agent as described herein, such as in Section II. In some instances, the agent inhibits mTOR kinase activity. In certain instances, the agent that inhibits mTORC1 and/or mTORC2. In particular instances, the agent is Compound 63, Compound 155, or Compound 246. In certain instances, the agent is Compound 63.

In some instances, the methods of generating or producing engineered cells, e.g., engineered CD4+ T cells and/or engineered CD8+ T cells, include one or more of isolating cells from a subject, preparing, processing, incubating under stimulating conditions, and/or engineering (e.g. transducing) the cells. In some instances, the method includes processing steps carried out in an order in which: input cells, e.g. primary cells, are first isolated, such as selected or separated, from a biological sample; input cells are incubated under stimulating conditions, engineered with vector particles, e.g., viral vector particles, to introduce a recombinant polynucleotide into the cells, e.g., by transduction or transfection; cultivating the engineered cells, e.g., transduced cells, such as to expand the cells; and collecting, harvesting, and/or filling a container with all or a portion of the cells for formulating the cells in an output composition. In some instances, the cells of the generated output composition are re-introduced into the same subject, before or after cryopreservation. In some instances, the output compositions of engineered cells are suitable for use in a therapy, e.g., an autologous cell therapy.

In some instances, one or more steps or stages are performed in the presence of an agent that inhibits mTOR activity, such as any described herein, e.g. Compound 63. In some instances, one or more steps of isolating, enriching, incubating, engineering, transducing, transfecting, cultivating, culturing, collecting, formulating, storing, and/or cryofreezing cells of the cells compositions are performed in the presence of an agent that inhibits mTOR activity. In certain instances, the cells are cultivated in the presence of an agent that inhibits mTOR activity.

In some instances, engineered cells are cultivated in the presence of an agent that inhibits mTOR activity, such as any described herein, e.g. Compound 63, and, in certain instances, the cells have not been contacted with, incubated with, and/or exposed to an agent that inhibits mTOR activity prior to the cultivation. In some instances, the input cells, the stimulated cells, and/or the engineered cells have not, prior to cultivating the cells to induce or stimulate their expansion or proliferation, been contacted, exposed, and/or incubated with an agent that inhibits mTOR activity. In certain instances, the steps of isolating, enriching, incubating under one or more activating conditions, engineering, transducing, transfecting, formulating, storing, and/or cryofreezing are not performed in the presence of an mTOR inhibitor.

In particular instances, the disclosed methods are used in connection with generating output compositions of cells expressing a recombinant receptor from an initial and/or input composition of cells. In some instances, the composition of cells is a composition of enriched T cells, enriched CD4+ T cells, and/or enriched CD8+ T cells (herein after also referred to as compositions of enriched T cells, compositions of enriched CD4+ T cells, and compositions of enriched CD8+ T cells, respectively). In certain instances, the composition of enriched T cells, enriched CD4+ T cells, or enriched CD8+ T cells is cultivated in the presence of an agent that inhibits mTOR activity. In some instances, a composition of enriched CD4+ T cells is cultivated in the presence of an agent that inhibits mTOR activity, such as any described herein, e.g. Compound 63. In certain instances, a composition of enriched CD8+ T cells is cultivated in the presence of an agent that inhibits mTOR activity.

In some instances, the disclosed methods are used in connection with incubating an input composition that includes primary T cells under stimulatory conditions. In some instances, the stimulatory conditions are or include incubating the input composition in the present of a stimulatory reagent. In certain instances, the stimulatory reagent is or includes beads, e.g., paramagnetic beads, with surface conjugated anti-CD3 and anti-CD28 antibodies. In particular instances, the cells of the input composition are not exposed to the agent that inhibits mTOR activity prior to or during the incubation. In some aspects, incubation is performed with an input composition of enriched T cells, e.g., CD4+ and CD8+ cells. In some instances, the input composition is or includes enriched CD4+ T cells. In certain instances, the input composition is or includes enriched CD8+ cells. In some instances, separate compositions of enriched CD4+ T cells and CD8+ T cells, e.g., from the same biological sample, are separately incubated. In some instances, the stimulatory conditions of the incubation may be the same for both compositions. In certain instances, the stimulatory conditions may be different. In certain instances, the incubation of one or more input compositions under stimulatory conditions generates one or more stimulated compositions.

In certain instances, a recombinant receptor is introduced into the cells of the stimulated composition. In certain instances, the recombinant receptor is introduced into the cell by transducing the stimulated composition with a viral vector. In certain instances, the viral vector is or includes a polynucleotide encoding the recombinant receptor. In some instances, the viral vector is a retroviral vector. In certain instances, the viral vector is a lentiviral vector or gammaretroviral vector. In some instances, the stimulated composition includes or contains enriched T cells, e.g., CD4+ and CD8+ cells. In some instances, the stimulated composition is or includes enriched CD4+ T cells. In particular instances, the stimulated composition is or includes enriched CD8+ cells. In some instances, separate compositions of enriched CD4+ T cells and CD8+ T cells, e.g., from the same biological sample, are separately engineered. In particular instances, the recombinant receptor is a chimeric antigen receptor (CAR). In particular instances, the CAR is an anti-CD 19 CAR.

In some instances, a composition of engineered cells, e.g., cells transduced or transfected to express a recombinant receptor, is cultivated the presence of an agent that inhibits mTOR activity, such as any described herein, e.g. Compound 63. In certain instances, the cultivation results in the proliferation and/or expansion of the cells in the composition, such as to produce an output composition that contains engineered T cells. In some aspects, the cells of the composition have not been exposed to, contacted with, and/or incubated with the agent that inhibits mTOR activity, such as any described herein, e.g. Compound 63, prior to the cultivation. In certain instances, the cells were not previously incubated, cultured, stimulated, engineered, transduced, and/or transfected in the presence of the mTOR inhibitor agent, such as any described herein, e.g. Compound 63.

In certain instances, the engineered composition of cells is an engineered composition of enriched CD4+ T cells. In particular instances, the engineered composition is a composition of engineered CD8+ T cells. In some instances, the disclosed methods are used in connection separately cultivating separate engineered compositions of enriched CD4+ T cells and enriched CD8+ T cells in the presence of an agent that inhibits mTOR activity, such as any described herein, e.g. Compound 63.

In some instances, the agent inhibits, reduces, and/or decreases one or more activities associated with mTOR. In some instances, the activity is a kinase activity. In some instances, the activity is an mTORC1 and/or an mTORC2 activity. In some instances, the agent is selected from the group consisting of BEZ235, BGT226, GDC0980, NVP-BEZ235, PF-04691502, PI-103, SAR245409, SF1126, VS5584, or XI,765, pyrazolopyrimidine, Torin l, Torkinib, PP30, Ku-0063794, WAY-600 (Wyeth), WAY-687 (Wyeth), WAY-354 (Wyeth), OSI-027, DS3078a, AZD8055, rapamycin, temsirolimus, everolimus, deforolimus, or AZD8055. In certain instances, the agent is Compound 63, Compound 155, or Compound 246. In some instances, the agent is Compound 63.

In certain instances, the composition of cells is a composition of enriched T cells, enriched CD4+ T cells, and/or enriched CD8+ T cells (herein after also referred to as compositions of enriched T cells, compositions of enriched CD4+ T cells, and compositions of enriched CD8+ T cells, respectively). In certain instances, the composition of enriched T cells, enriched CD4+ T cells, or enriched CD8+ T cells is incubated under stimulatory conditions in the presence of an agent that inhibits mTOR activity, such as any described herein, e.g. Compound 63. In some instances, the composition of enriched T cells, enriched CD4+ T cells, or enriched CD8+ T cells is genetically engineered, e.g., transduced or transfected, to express a recombinant receptor in the presence of an agent that inhibits mTOR activity, such as any described herein, e.g. Compound 63.

In some instances, the disclosed methods are used in association with the isolation, separation, selection, activation or stimulation, transduction, washing, suspension, dilution, concentration, and/or formulation of a single composition of enriched T cells. In some instances, the composition of enriched T cells is a composition of cells that include enriched CD4+ T cells. In certain instances, the composition of enriched CD4+ T cells contains at least 60%, 65%, 70%, 75%, 80%, 85%, 90%, 95%, 98%, 99%, or 99.9% CD4+ T cells. In particular instances, the composition of enriched CD4+ T cells contains 100% CD4+ T cells or contains about 100% CD4+ T cells. In certain instances, the composition of enriched T cells includes or contains less than 20%, less than 10%, less than 5%, less than 1%, less than 0.1%, or less than 0.01% CD8+ T cells, and/or contains no CD8+ T cells, and/or is free or substantially free of CD8+ T cells. In some instances, the populations of cells consist essentially of CD4+ T cells.

In some instances, the composition of enriched T cells is a composition of enriched CD8+ T cells. In certain instances, the composition of enriched CD8+ T cells contains at least 75%, 80%, 85%, 90%, 95%, 98%, 99%, or 99.9% CD8+ T cells, or contains or contains about 100% CD8+ T cells. In certain instances, the composition of enriched CD8+ T cells includes or contains less than 20%, less than 10%, less than 5%, less than 1%, less than 0.1%, or less than 0.01% CD4+ T cells, and/or contains no CD4+ T cells, and/or is free or substantially free of CD4+ T cells. In some instances, the populations of cells consist essentially of CD8+ T cells.

In some instances, the disclosed methods are used in connection with generating two or more separate output compositions of enriched T cells. In some instances, the disclosed methods are separately performed on two or more separate compositions of enriched T cells. In certain instances, the methods may be used in connection with separately activating and/or stimulating two or more compositions of enriched T cells; separately engineering two or more compositions of enriched T cells; and/or separately cultivating two or more compositions of enriched T cells. In certain instances, the methods may also be used in connection with isolating or selecting different cells from a biological sample to generate separate input composition of enriched T cells, such as separate compositions of enriched CD4+ T cells and enriched CD8+ T cells. In particular instances, the disclosed methods may be used in connection with separately harvesting, collecting, and/or formulating separate compositions of enriched T cells after the T cells have been incubated, activated, stimulated, engineered, transduced, transfected, and/or cultivated. In some instances, the two or more separate compositions of enriched T cells include a composition of enriched CD4+ T cells. In certain instances, the two or more separate compositions include CD8+ T cells. In some instances, the two or more separate compositions include a composition of enriched CD4+ T cells and a composition of enriched CD8+ T cells.

In particular instances, the compositions of enriched T cells may be collected, formulated for cryoprotection, cryofrozen, and/or stored below 0°C, below -20°C, or at or below -70°C or -80°C prior to, during, or after any stage or step of the process for generating output compositions of enriched T cells expressing recombinant receptors. In some instances, the cells may be stored for an amount of time under 1, 2, 3, 4, 5, 6, 7, 8, 9, or 10 days, or an amount of time under 1, 2, 3, 4, 5, 6, 7, 8 weeks, or for an amount of time at least 1, 2, 3, 4, 5, 6, 7, or 8 weeks, or for more than 8 weeks. After storage, the compositions of enriched T cells may be thawed and the processing may be resumed from the same point in the process. In some instances, input compositions of enriched T cells are cryofrozen and stored prior to further processing, e.g., incubation under stimulating conditions. In particular instances, cultivated and/or formulated compositions of enriched T cells are cryofrozen and stored prior to being administered to as subject, e.g., as an autologous cell therapy.

In certain instances, separate cell compositions of enriched T cells are combined into a single composition. For example, in some instances, a composition of enriched CD4+ T cells is combined with a composition of enriched CD8+ T cells into a single composition of enriched CD4+ and CD8+ T cells. In certain instances, the separate compositions originated, e.g., were initially isolated, selected, and/or enriched, from the same biological sample, such as the same biological sample obtained, collected, and/or taken from a single subject. In some instances, the separate compositions are separately processed for one or more steps or stages of a process for generating output compositions, e.g., a process in connection with the disclosed methods. In some instances, the separate compositions may be combined into a single composition prior to, during, or subsequent to any step or stage of the process for generating output compositions. Thus in some instances, separate input, stimulated, engineered, cultivated, formulated, and/or harvested compositions of enriched T cells from the same biological sample are combined into a single composition and, in certain instances, are further processed as a single composition. In certain instances, separate output compositions of enriched cells are combined into a single output composition prior to administering the cells to a subject.

In certain instances, at any stage or step in the process, a portion of the cells may be sampled or collected, e.g., cells may be taken from the composition of enriched T cells while the composition remains in the closed system, such as during the isolation, incubation, engineering, cultivation, and/or formulation. In certain instances, such cells may be analyzed for makers, features, or characteristics including but not limited to viability, apoptosis, activation, stimulation, growth, and/or exhaustion, In some instances, the cells are sampled or collected by an automated process while the composition of enriched T cells remains in the closed system. In some instances, the analysis of sampled or collected cells is automated. In particular instances, the analysis is performed in a closed system under sterile conditions.

Also disclosed are cells and compositions prepared by the methods, including pharmaceutical compositions and formulations, and kits, systems, and devices for carrying out the methods. Also disclosed are methods for use of the cells and compositions prepared by the methods, including therapeutic methods, such as methods for adoptive cell therapy, and pharmaceutical compositions for administration to subjects.

### A. Samples and Cell Preparations

In particular instances, the disclosed methods are used in connection with isolating, selecting, and/or enriching cells from a biological sample to generate one or more input compositions of enriched cells, e.g., T cells. In some instances, the disclosed methods include isolation of cells or compositions thereof from biological samples, such as those obtained from or derived from a subject, such as one having a particular disease or condition or in need of a cell therapy or to which cell therapy will be administered. In some aspects, the subject is a human, such as a subject who is a patient in need of a particular therapeutic intervention, such as the adoptive cell therapy for which cells are being isolated, processed, and/or engineered. Accordingly, the cells in some instances are primary cells, e.g., primary human cells. The samples include tissue, fluid, and other samples taken directly from the subject. The biological sample can be a sample obtained directly from a biological source or a sample that is processed. Biological samples include, but are not limited to, body fluids, such as blood, plasma, serum, cerebrospinal fluid, synovial fluid, urine and sweat, tissue and organ samples, including processed samples derived therefrom.

In some aspects, the sample is blood or a blood-derived sample, or is or is derived from an apheresis or leukapheresis product. Exemplary samples include whole blood, peripheral blood mononuclear cells (PBMCs), leukocytes, bone marrow, thymus, tissue biopsy, tumor, leukemia, lymphoma, lymph node, gut associated lymphoid tissue, mucosa associated lymphoid tissue, spleen, other lymphoid tissues, liver, lung, stomach, intestine, colon, kidney, pancreas, breast, bone, prostate, cervix, testes, ovaries, tonsil, or other organ, and/or cells derived therefrom. Samples include, in the context of cell therapy, e.g., adoptive cell therapy, samples from autologous and allogeneic sources.

In some examples, cells from the circulating blood of a subject are obtained, e.g., by apheresis or leukapheresis. The samples, in some aspects, contain lymphocytes, including T cells, monocytes, granulocytes, B cells, other nucleated white blood cells, red blood cells, and/or platelets, and in some aspects contains cells other than red blood cells and platelets.

In some instances, the blood cells collected from the subject are washed, e.g., to remove the plasma fraction and to place the cells in an appropriate buffer or media for subsequent processing steps. In some instances, the cells are washed with phosphate buffered saline (PBS). In some instances, the wash solution lacks calcium and/or magnesium and/or many or all divalent cations. In some aspects, a washing step is accomplished a semi-automated "flow-through" centrifuge (for example, the Cobe 2991 cell processor, Baxter) according to the manufacturer's instructions. In some aspects, a washing step is accomplished by tangential flow filtration (TFF) according to the manufacturer's instructions. In some instances, the cells are resuspended in a variety of biocompatible buffers after washing, such as, for example, Ca⁺⁺/Mg⁺⁺ free PBS. In certain instances, components of a blood cell sample are removed and the cells directly resuspended in culture media.

In some instances, the preparation methods include steps for freezing, e.g., cryopreserving, the cells, either before or after isolation, selection and/or enrichment and/or incubation for transduction and engineering, and/or after cultivation and/or harvesting of the engineered cells. In some instances, the freeze and subsequent thaw step removes granulocytes and, to some extent, monocytes in the cell population. In some instances, the cells are suspended in a freezing solution, e.g., following a washing step to remove plasma and platelets. Any of a variety of known freezing solutions and parameters in some aspects may be used. In some instances, the cells are frozen, e.g., cryofrozen or cryopreserved, in media and/or solution with a final concentration of or of about 12.5%, 12.0%, 11.5%, 11.0%, 10.5%, 10.0%, 9.5%, 9.0%, 8.5%, 8.0%, 7.5%, 7.0%, 6.5%, 6.0%, 5.5%, or 5.0% DMSO, or between 1% and 15%, between 6% and 12%, between 5% and 10%, or between 6% and 8% DMSO. In particular instances, the cells are frozen, e.g., cryofrozen or cryopreserved, in media and/or solution with a final concentration of or of about 5.0%, 4.5%, 4.0%, 3.5%, 3.0%, 2.5%, 2.0%, 1.5%, 1.25%, 1.0%, 0.75%, 0.5%, or 0.25% HSA, or between 0.1% and -5%, between 0.25% and 4%, between 0.5% and 2%, or between 1% and 2% HSA. One example involves using PBS containing 20% DMSO and 8% human serum albumin (HSA), or other suitable cell freezing media. This is then diluted 1:1 with media so that the final concentration of DMSO and HSA are 10% and 4%, respectively. The cells are generally then frozen to or to about -80° C at a rate of or of about 1° per minute and stored in the vapor phase of a liquid nitrogen storage tank.

In some instances, isolation of the cells or populations includes one or more preparation and/or non-affinity based cell separation steps. In some examples, cells are washed, centrifuged, and/or incubated in the presence of one or more reagents, for example, to remove unwanted components, enrich for desired components, lyse or remove cells sensitive to particular reagents. In some examples, cells are separated based on one or more property, such as density, adherent properties, size, sensitivity and/or resistance to particular components. In some instances, the methods include density-based cell separation methods, such as the preparation of white blood cells from peripheral blood by lysing the red blood cells and centrifugation through a Percoll or Ficoll gradient.

In some instances, at least a portion of the selection step includes incubation of cells with a selection reagent. The incubation with a selection reagent or reagents, e.g., as part of selection methods which may be performed using one or more selection reagents for selection of one or more different cell types based on the expression or presence in or on the cell of one or more specific molecules, such as surface markers, e.g., surface proteins, intracellular markers, or nucleic acid. In some instances, any known method using a selection reagent or reagents for separation based on such markers may be used. In some instances, the selection reagent or reagents result in a separation that is affinity- or immunoaffinity-based separation. For example, the selection in some aspects includes incubation with a reagent or reagents for separation of cells and cell populations based on the cells' expression or expression level of one or more markers, typically cell surface markers, for example, by incubation with an antibody or binding partner that specifically binds to such markers, followed generally by washing steps and separation of cells having bound the antibody or binding partner, from those cells having not bound to the antibody or binding partner.

In some aspects of such processes, a volume of cells is mixed with an amount of a desired affinity-based selection reagent. The immunoaffinity-based selection can be carried out using any system or method that results in a favorable energetic interaction between the cells being separated and the molecule specifically binding to the marker on the cell, e.g., the antibody or other binding partner on the solid surface, e.g., particle. In some instances, methods are carried out using particles such as beads, e.g. magnetic beads, that are coated with a selection agent (e.g. antibody) specific to the marker of the cells. The particles (e.g. beads) can be incubated or mixed with cells in a container, such as a tube or bag, while shaking or mixing, with a constant cell density-to-particle (e.g., bead) ratio to aid in promoting energetically favored interactions. In other cases, the methods include selection of cells in which all or a portion of the selection is carried out in the internal cavity of a centrifugal chamber, for example, under centrifugal rotation. In some instances, incubation of cells with selection reagents, such as immunoaffinity-based selection reagents, is performed in a centrifugal chamber. In certain instances, the isolation or separation is carried out using a system, device, or apparatus described in International Patent Application, Publication Number WO2009/072003, or US 20110003380 A1. In one example, the system is a system as described in International Publication Number WO2016/073602.

In some instances, by conducting such selection steps or portions thereof (e.g., incubation with antibody-coated particles, e.g., magnetic beads) in the cavity of a centrifugal chamber, the user is able to control certain parameters, such as volume of various solutions, addition of solution during processing and timing thereof, which can provide advantages compared to other available methods. For example, the ability to decrease the liquid volume in the cavity during the incubation can increase the concentration of the particles (e.g. bead reagent) used in the selection, and thus the chemical potential of the solution, without affecting the total number of cells in the cavity. This in turn can enhance the pairwise interactions between the cells being processed and the particles used for selection. In some instances, carrying out the incubation step in the chamber, e.g., when associated with the systems, circuitry, and control as described herein, permits the user to effect agitation of the solution at desired time(s) during the incubation, which also can improve the interaction.

In some instances, at least a portion of the selection step is performed in a centrifugal chamber, which includes incubation of cells with a selection reagent. In some aspects of such processes, a volume of cells is mixed with an amount of a desired affinity-based selection reagent that is far less than is normally employed when performing similar selections in a tube or container for selection of the same number of cells and/or volume of cells according to manufacturer's instructions. In some instances, an amount of selection reagent or reagents that is/are no more than 5%, no more than 10%, no more than 15%, no more than 20%, no more than 25%, no more than 50%, no more than 60%, no more than 70% or no more than 80% of the amount of the same selection reagent(s) employed for selection of cells in a tube or containerbased incubation for the same number of cells and/or the same volume of cells according to manufacturer's instructions is employed.

In some instances, for selection, e.g., immunoaffinity-based selection of the cells, the cells are incubated in the cavity of the chamber in a composition that also contains the selection buffer with a selection reagent, such as a molecule that specifically binds to a surface marker on a cell that it desired to enrich and/or deplete, but not on other cells in the composition, such as an antibody, which optionally is coupled to a scaffold such as a polymer or surface, e.g., bead, e.g., magnetic bead, such as magnetic beads coupled to monoclonal antibodies specific for CD4 and CD8. In some instances, as described, the selection reagent is added to cells in the cavity of the chamber in an amount that is substantially less than (e.g. is no more than 5%, 10%, 20%, 30%, 40%, 50%, 60%, 70% or 80% of the amount) as compared to the amount of the selection reagent that is typically used or would be necessary to achieve about the same or similar efficiency of selection of the same number of cells or the same volume of cells when selection is performed in a tube with shaking or rotation. In some instances, the incubation is performed with the addition of a selection buffer to the cells and selection reagent to achieve a target volume with incubation of the reagent of, for example, 10 mL to 200 mL, such as at least or about at least 10 mL, 20 mL, 30 mL, 40 mL, 50 mL, 60 mL, 70 mL, 80 mL, 90 mL, 100 mL, 150 mL or 200 mL. In some instances, the selection buffer and selection reagent are pre-mixed before addition to the cells. In some instances, the selection buffer and selection reagent are separately added to the cells. In some instances, the selection incubation is carried out with periodic gentle mixing condition, which can aid in promoting energetically favored interactions and thereby permit the use of less overall selection reagent while achieving a high selection efficiency.

In some instances, the total duration of the incubation with the selection reagent is from or from about 5 minutes to 6 hours, such as 30 minutes to 3 hours, for example, at least or about at least 30 minutes, 60 minutes, 120 minutes or 180 minutes.

In some instances, the incubation generally is carried out under mixing conditions, such as in the presence of spinning, generally at relatively low force or speed, such as speed lower than that used to pellet the cells, such as from or from about 600 rpm to 1700 rpm (e.g. at or about or at least 600 rpm, 1000 rpm, or 1500 rpm or 1700 rpm), such as at an RCF at the sample or wall of the chamber or other container of from or from about 80g to 100g (e.g. at or about or at least 80 g, 85 g, 90 g, 95 g, or 100 g). In some instances, the spin is carried out using repeated intervals of a spin at such low speed followed by a rest period, such as a spin and/or rest for 1, 2, 3, 4, 5, 6, 7, 8, 9, or 10 seconds, such as a spin at approximately 1 or 2 seconds followed by a rest for approximately 5, 6, 7, or 8 seconds.

In some instances, such process is carried out within the entirely closed system to which the chamber is integral. In some instances, this process (and in some aspects also one or more additional step, such as a previous wash step washing a sample containing the cells, such as an apheresis sample) is carried out in an automated fashion, such that the cells, reagent, and other components are drawn into and pushed out of the chamber at appropriate times and centrifugation effected, so as to complete the wash and binding step in a single closed system using an automated program.

In some instances, after the incubation and/or mixing of the cells and selection reagent and/or reagents, the incubated cells are subjected to a separation to select for cells based on the presence or absence of the particular reagent or reagents. In some instances, the separation is performed in the same closed system in which the incubation of cells with the selection reagent was performed. In some instances, after incubation with the selection reagents, incubated cells, including cells in which the selection reagent has bound are transferred into a system for immunoaffinity-based separation of the cells. In some instances, the system for immunoaffinity-based separation is or contains a magnetic separation column.

Such separation steps can be based on positive selection, in which the cells having bound the reagents, e.g. antibody or binding partner, are retained for further use, and/or negative selection, in which the cells having not bound to the reagent, e.g., antibody or binding partner, are retained. In some examples, both fractions are retained for further use. In some aspects, negative selection can be particularly useful where no antibody is available that specifically identifies a cell type in a heterogeneous population, such that separation is best carried out based on markers expressed by cells other than the desired population.

In some instances, the process steps further include negative and/or positive selection of the incubated cells, such as using a system or apparatus that can perform an affinity-based selection. In some instances, isolation is carried out by enrichment for a particular cell population by positive selection, or depletion of a particular cell population, by negative selection. In some instances, positive or negative selection is accomplished by incubating cells with one or more antibodies or other binding agent that specifically bind to one or more surface markers expressed or expressed (marker+) at a relatively higher level (marker^{high}) on the positively or negatively selected cells, respectively.

The separation need not result in 100 % enrichment or removal of a particular cell population or cells expressing a particular marker. For example, positive selection of or enrichment for cells of a particular type, such as those expressing a marker, refers to increasing the number or percentage of such cells, but need not result in a complete absence of cells not expressing the marker. Likewise, negative selection, removal, or depletion of cells of a particular type, such as those expressing a marker, refers to decreasing the number or percentage of such cells, but need not result in a complete removal of all such cells.

In some examples, multiple rounds of separation steps are carried out, where the positively or negatively selected fraction from one step is subjected to another separation step, such as a subsequent positive or negative selection. In some examples, a single separation step can deplete cells expressing multiple markers simultaneously, such as by incubating cells with a plurality of antibodies or binding partners, each specific for a marker targeted for negative selection. Likewise, multiple cell types can simultaneously be positively selected by incubating cells with a plurality of antibodies or binding partners expressed on the various cell types.

For example, in some aspects, specific subpopulations of T cells, such as cells positive or expressing high levels of one or more surface markers, e.g., CD28+, CD62L+, CCR7+, CD27+, CD127+, CD4+, CD8+, CD45RA+, and/or CD45RO+ T cells, are isolated by positive or negative selection techniques. In some instances, such cells are selected by incubation with one or more antibody or binding partner that specifically binds to such markers. In some instances, the antibody or binding partner can be conjugated, such as directly or indirectly, to a solid support or matrix to effect selection, such as a magnetic bead or paramagnetic bead. For example, CD3+, CD28+ T cells can be positively selected using CD3/CD28 conjugated magnetic beads (e.g., DYNABEADS^{®} M-450 CD3/CD28 T Cell Expander, and/or ExpACT^{®} beads).

In some instances, T cells are separated from a PBMC sample by negative selection of markers expressed on non-T cells, such as B cells, monocytes, or other white blood cells, such as CD14. In some aspects, a CD4+ or CD8+ selection step is used to separate CD4+ helper and CD8+ cytotoxic T cells. Such CD4+ and CD8+ populations can be further sorted into subpopulations by positive or negative selection for markers expressed or expressed to a relatively higher degree on one or more naive, memory, and/or effector T cell subpopulations.

In some instances, CD8+ T cells are further enriched for or depleted of naive, central memory, effector memory, and/or central memory stem cells, such as by positive or negative selection based on surface antigens associated with the respective subpopulation. In some instances, enrichment for central memory T (TCM) cells is carried out to increase efficacy, such as to improve long-term survival, expansion, and/or engraftment following administration, which in some aspects is particularly robust in such sub-populations. See Terakura et al., (2012) Blood. 1:72-82; Wang et al. (2012) J Immunother. 35(9):689-701. In some instances, combining TCM-enriched CD8+ T cells and CD4+ T cells further enhances efficacy.

In instances, memory T cells are present in both CD62L+ and CD62L- subsets of CD8+ peripheral blood lymphocytes. PBMC can be enriched for or depleted of CD62L-CD8+ and/or CD62L+CD8+ fractions, such as using anti-CD8 and anti-CD62L antibodies.

In some instances, the enrichment for central memory T (TCM) cells is based on positive or high surface expression of CD45RO, CD62L, CCR7, CD28, CD3, and/or CD 127; in some aspects, it is based on negative selection for cells expressing or highly expressing CD45RA and/or granzyme B. In some aspects, isolation of a CD8+ population enriched for TCM cells is carried out by depletion of cells expressing CD4, CD14, CD45RA, and positive selection or enrichment for cells expressing CD62L. In one aspect, enrichment for central memory T (TCM) cells is carried out starting with a negative fraction of cells selected based on CD4 expression, which is subjected to a negative selection based on expression of CD14 and CD45RA, and a positive selection based on CD62L. Such selections in some aspects are carried out simultaneously and in other aspects are carried out sequentially, in either order. In some aspects, the same CD4 expression-based selection step used in preparing the CD8+ T cell population or subpopulation, also is used to generate the CD4+ T cell population or subpopulation, such that both the positive and negative fractions from the CD4-based separation are retained and used in subsequent steps of the methods, optionally following one or more further positive or negative selection steps. In some instances, the selection for the CD4+ T cell population and the selection for the CD8+ T cell population are carried out simultaneously. In some instances, the CD4+ T cell population and the selection for the CD8+ T cell population are carried out sequentially, in either order. In some instances, methods for selecting cells can include those as described in published U.S. App. No. US20170037369. In some instances, the selected CD4+ T cell population and the selected CD8+ T cell population may be combined subsequent to the selecting. In some aspects, the selected CD4+ T cell population and the selected CD8+ T cell population may be combined in a bioreactor bag as described herein.

In particular instances, a biological sample, e.g., a sample of PBMCs or other white blood cells, are subjected to selection of CD4+ T cells, where both the negative and positive fractions are retained. In certain instances, CD8+ T cells are selected from the negative fraction. In some instances, a biological sample is subjected to selection of CD8+ T cells, where both the negative and positive fractions are retained. In certain instances, CD4+ T cells are selected from the negative fraction.

In a particular example, a sample of PBMCs or other white blood cell sample is subjected to selection of CD4+ T cells, where both the negative and positive fractions are retained. The negative fraction then is subjected to negative selection based on expression of CD14 and CD45RA or CD19, and positive selection based on a marker characteristic of central memory T cells, such as CD62L or CCR7, where the positive and negative selections are carried out in either order.

CD4+ T helper cells may be sorted into naive, central memory, and effector cells by identifying cell populations that have cell surface antigens. CD4+ lymphocytes can be obtained by standard methods. In some instances, naive CD4+ T lymphocytes are CD45RO-, CD45RA+, CD62L+, or CD4+ T cells. In some instances, central memory CD4+ T cells are CD62L+ and CD45RO+. In some instances, effector CD4+ T cells are CD62L- and CD45RO-.

In one example, to enrich for CD4+ T cells by negative selection, a monoclonal antibody cocktail typically includes antibodies to CD14, CD20, CD11b, CD16, HLA-DR, and CD8. In some instances, the antibody or binding partner is bound to a solid support or matrix, such as a magnetic bead or paramagnetic bead, to allow for separation of cells for positive and/or negative selection. For example, in some instances, the cells and cell populations are separated or isolated using immunomagnetic (or affinitymagnetic) separation techniques (reviewed in Methods in Molecular Medicine, vol. 58: Metastasis Research Protocols, Vol. 2: Cell Behavior In Vitro and In Vivo, p 17-25 Edited by: S. A. Brooks and U. Schumacher © Humana Press Inc., Totowa, NJ).

In some aspects, the incubated sample or composition of cells to be separated is incubated with a selection reagent containing small, magnetizable or magnetically responsive material, such as magnetically responsive particles or microparticles, such as paramagnetic beads (e.g., such as Dynalbeads or MACS^{®} beads). The magnetically responsive material, e.g., particle, generally is directly or indirectly attached to a binding partner, e.g., an antibody, that specifically binds to a molecule, e.g., surface marker, present on the cell, cells, or population of cells that it is desired to separate, e.g., that it is desired to negatively or positively select.

In some instances, the magnetic particle or bead comprises a magnetically responsive material bound to a specific binding member, such as an antibody or other binding partner. Many well-known magnetically responsive materials for use in magnetic separation methods are known, e.g., those described in Molday, U.S. Pat. No. 4,452,773, and in European Patent Specification EP 452342 B. Colloidal sized particles, such as those described in Owen U.S. Pat. No. 4,795,698, and Liberti et al., U.S. Pat. No. 5,200,084 also may be used.

The incubation generally is carried out under conditions whereby the antibodies or binding partners, or molecules, such as secondary antibodies or other reagents, which specifically bind to such antibodies or binding partners, which are attached to the magnetic particle or bead, specifically bind to cell surface molecules if present on cells within the sample.

In certain instances, the magnetically responsive particles are coated in primary antibodies or other binding partners, secondary antibodies, lectins, enzymes, or streptavidin. In certain instances, the magnetic particles are attached to cells via a coating of primary antibodies specific for one or more markers. In certain instances, the cells, rather than the beads, are labeled with a primary antibody or binding partner, and then cell-type specific secondary antibody- or other binding partner (e.g., streptavidin)-coated magnetic particles, are added. In certain instances, streptavidin-coated magnetic particles are used in conjunction with biotinylated primary or secondary antibodies.

In some aspects, separation is achieved in a procedure in which the sample is placed in a magnetic field, and those cells having magnetically responsive or magnetizable particles attached thereto will be attracted to the magnet and separated from the unlabeled cells. For positive selection, cells that are attracted to the magnet are retained; for negative selection, cells that are not attracted (unlabeled cells) are retained. In some aspects, a combination of positive and negative selection is performed during the same selection step, where the positive and negative fractions are retained and further processed or subject to further separation steps.

In some instances, the affinity-based selection is via magnetic-activated cell sorting (MACS) (Miltenyi Biotech, Auburn, CA). Magnetic Activated Cell Sorting (MACS), e.g., CliniMACS systems are capable of high-purity selection of cells having magnetized particles attached thereto. In certain instances, MACS operates in a mode wherein the non-target and target species are sequentially eluted after the application of the external magnetic field. That is, the cells attached to magnetized particles are held in place while the unattached species are eluted. Then, after this first elution step is completed, the species that were trapped in the magnetic field and were prevented from being eluted are freed in some manner such that they can be eluted and recovered. In certain instances, the non-target cells are labelled and depleted from the heterogeneous population of cells.

In some instances, the magnetically responsive particles are left attached to the cells that are to be subsequently incubated, cultured and/or engineered; in some aspects, the particles are left attached to the cells for administration to a patient. In some instances, the magnetizable or magnetically responsive particles are removed from the cells. Methods for removing magnetizable particles from cells are known and include, e.g., the use of competing non-labeled antibodies, magnetizable particles or antibodies conjugated to cleavable linkers, etc. In some instances, the magnetizable particles are biodegradable.

In some instances, the isolation and/or selection results in one or more input compositions of enriched T cells, e.g., CD3+ T cells, CD4+ T cells, and/or CD8+ T cells. In some instances, two or more separate input composition are isolated, selected, enriched, or obtained from a single biological sample. In some instances, separate input compositions are isolated, selected, enriched, and/or obtained from separate biological samples collected, taken, and/or obtained from the same subject.

In certain instances, the one or more input compositions is or includes a composition of enriched T cells that includes at least 60%, at least 65%, at least 70%, at least 75%, at least 80%, at least 85%, at least 90%, at least 95%, at least 98%, at least 99%, at least 99.5%, at least 99.9%, or at or at about 100% CD3+ T cells. In particular instances, the input composition of enriched T cells consists essentially of CD3+ T cells.

In certain instances, the one or more input compositions is or includes a composition of enriched CD4+ T cells that includes at least 60%, at least 65%, at least 70%, at least 75%, at least 80%, at least 85%, at least 90%, at least 95%, at least 98%, at least 99%, at least 99.5%, at least 99.9%, or at or at about 100% CD4+ T cells. In certain instances, the input composition of CD4+ T cells includes less than 40%, less than 35%, less than 30%, less than 25%, less than 20%, less than 15%, less than 10%, less than 5%, less than 1%, less than 0.1%, or less than 0.01% CD8+ T cells, and/or contains no CD8+ T cells, and/or is free or substantially free of CD8+ T cells. In some instances, the composition of enriched T cells consists essentially of CD4+ T cells.

In certain instances, the one or more compositions is or includes a composition of CD8+ T cells that is or includes at least 60%, at least 65%, at least 70%, at least 75%, at least 80%, at least 85%, at least 90%, at least 95%, at least 98%, at least 99%, at least 99.5%, at least 99.9%, or at or at about 100% CD8+ T cells. In certain instances, the composition of CD8+ T cells contains less than 40%, less than 35%, less than 30%, less than 25%, less than 20%, less than 15%, less than 10%, less than 5%, less than 1%, less than 0.1%, or less than 0.01% CD4+ T cells, and/or contains no CD4+ T cells, and/or is free of or substantially free of CD4+ T cells. In some instances, the composition of enriched T cells consists essentially of CD8+ T cells.

In some instances, the one or more input compositions of enriched T cells are frozen, e.g., cryopreserved and/or cryofrozen, after isolation, selection and/or enrichment. In some instances, the one or more input compositions of frozen e.g., cryopreserved and/or cryofrozen, prior to any steps of incubating, activating, stimulating, engineering, transducing, transfecting, cultivating, expanding, harvesting, and/or formulating the composition of cells. In particular instances, the one or more cryofrozen input compositions are stored, e.g., at or at about -80°C.

### B. Activation and Stimulation of Cells

In some instances, the disclosed methods are used in connection with incubating cells under stimulating conditions. In some instances, the stimulating conditions include conditions that activate or stimulate, and/or are capable of activing or stimulating a signal in the cell, e.g., a CD4+ T cell, such as a signal generated from a TCR and/or a coreceptor. In some instances, the stimulating conditions include one or more steps of culturing, cultivating, incubating, activating, propagating the cells with and/or in the presence of a stimulatory reagent, e.g., a reagent that activates or stimulates, and/or is capable of activing or stimulating a signal in the cell. In some instances, the stimulatory reagent stimulates and/or activates a TCR and/or a coreceptor. In particular instances, the stimulatory reagent is a reagent described in Section I-B-1.

In certain instances, one or more compositions of enriched T cells are incubated under stimulating conditions prior to genetically engineering the cells, e.g., transfecting and/or transducing the cell such as by a technique provided in Section I-C. In particular instances, one or more compositions of enriched T cells are incubated under stimulating conditions after the one or more compositions have been isolated, selected, enriched, or obtained from a biological sample. In particular instances, the one or more compositions are input compositions. In particular instances, the one or more input compositions have been previously cryofrozen and stored, and are thawed prior to the incubation.

In certain instances, the one or more compositions of enriched T cells are or include two separate compositions, e.g., separate input compositions, of enriched T cells. In particular instances, two separate compositions of enriched T cells, e.g., two separate compositions of enriched T cells selected, isolated, and/or enriched from the same biological sample, are separately incubated under stimulating conditions. In certain instances, the two separate compositions include a composition of enriched CD4+ T cells. In particular instances, the two separate compositions include a composition of enriched CD8+ T cells. In some instances, two separate compositions of enriched CD4+ T cells and enriched CD8+ T cells are separately incubated under stimulating conditions. In some instances, a single composition of enriched T cells is incubated under stimulating conditions. In certain instances, the single composition is a composition of enriched CD4+ T cells. In some instances, the single composition is a composition of enriched CD4+ and CD8+ T cells that have been combined from separate compositions prior to the incubation.

In some instances, the composition of enriched CD4+ T cells that is incubated under stimulating conditions includes at least 60%, at least 65%, at least 70%, at least 75%, at least 80%, at least 85%, at least 90%, at least 95%, at least 98%, at least 99%, at least 99.5%, at least 99.9%, or at or at about 100% CD4+ T cells. In certain instances, the composition of enriched CD4+ T cells that is incubated under stimulating conditions includes less than 40%, less than 35%, less than 30%, less than 25%, less than 20%, less than 15%, less than 10%, less than 5%, less than 1%, less than 0.1%, or less than 0.01% CD8+ T cells, and/or contains no CD8+ T cells, and/or is free or substantially free of CD8+ T cells.

In some instances, the composition of enriched CD8+ T cells that is incubated under stimulating conditions includes at least 60%, at least 65%, at least 70%, at least 75%, at least 80%, at least 85%, at least 90%, at least 95%, at least 98%, at least 99%, at least 99.5%, at least 99.9%, or at or at about 100% CD8+ T cells. In certain instances, the composition of enriched CD8+ T cells that is incubated under stimulating conditions includes less than 40%, less than 35%, less than 30%, less than 25%, less than 20%, less than 15%, less than 10%, less than 5%, less than 1%, less than 0.1%, or less than 0.01% CD4+ T cells, and/or contains no CD4+ T cells, and/or is free or substantially free of CD4+ T cells.

In some instances, separate compositions of enriched CD4+ and CD8+ T cells are combined into a single composition and are incubated under stimulating conditions. In certain instances, separate stimulated compositions of enriched CD4+ and enriched CD8+ T cells are combined into a single composition after the incubation has been performed and/or completed.

In certain instances, one or more compositions of enriched T cells are incubated under stimulating conditions prior to genetically engineering the cells, e.g., transfecting and/or transducing the cell such as by a technique provided in Section I-C. In particular instances, one or more compositions of enriched T cells are incubated under stimulating conditions after the one or more compositions have been isolated, selected, enriched, or obtained from a biological sample. In particular instances, the one or more compositions are input compositions. In some instances, the one or more input compositions have been previously cryofrozen and stored, and are thawed prior to the incubation.

In some instances, the incubation under stimulating conditions can include culture, cultivation, stimulation, activation, propagation, including by incubation in the presence of stimulating conditions, for example, conditions designed to induce proliferation, expansion, activation, and/or survival of cells in the population, to mimic antigen exposure, and/or to prime the cells for genetic engineering, such as for the introduction of a recombinant antigen receptor. In particular instances, the stimulating conditions can include one or more of particular media, temperature, oxygen content, carbon dioxide content, time, agents, e.g., nutrients, amino acids, antibiotics, ions, and/or stimulatory factors, such as cytokines, chemokines, antigens, binding partners, fusion proteins, recombinant soluble receptors, and any other agents designed to activate the cells.

In some aspects, the stimulation and/or incubation under stimulating conditions is carried out in accordance with techniques such as those described in US Patent No. 6,040,1 77 to Riddell et al., Klebanoff et al.(2012) J Immunother. 35(9): 651-660, Terakuraet al. (2012) Blood. 1:72-82, and/or Wang et al. (2012) J Immunother. 35(9):689-701.

In some instances, the cells, e.g., T cells, compositions of cells, and/or cell populations, such as CD4⁺ and CD8⁺ T cells or compositions, populations, or subpopulations thereof, are expanded by adding to the culture-initiating composition feeder cells, such as non-dividing peripheral blood mononuclear cells (PBMC), (e.g., such that the resulting population of cells contains at least about 5, 10, 20, or 40 or more PBMC feeder cells for each T lymphocyte in the initial population to be expanded); and incubating the culture (e.g. for a time sufficient to expand the numbers of T cells). In some aspects, the non-dividing feeder cells can comprise gamma- irradiated PBMC feeder cells. In some instances, the PBMC are irradiated with gamma rays in the range of about 3000 to 3600 rads to prevent cell division. In some aspects, the feeder cells are added to culture medium prior to the addition of the populations of T cells.

In some instances, the stimulating conditions include temperature suitable for the growth of human T lymphocytes, for example, at least about 25 degrees Celsius, generally at least about 30 degrees, and generally at or about 37 degrees Celsius. In some instances, a temperature shift is effected during culture, such as from 37 degrees Celsius to 35 degrees Celsius. Optionally, the incubation may further comprise adding non-dividing EBVtransformed lymphoblastoid cells (LCL) as feeder cells. LCL can be irradiated with gamma rays in the range of about 6000 to 10,000 rads. The LCL feeder cells in some aspects is provided in any suitable amount, such as a ratio of LCL feeder cells to initial T lymphocytes of at least about 10:1.

In instances, populations of CD4⁺ and CD8⁺ that are antigen specific can be obtained by stimulating naive or antigen specific T lymphocytes with antigen. For example, antigenspecific T cell lines or clones can be generated to cytomegalovirus antigens by isolating T cells from infected subjects and stimulating the cells in vitro with the same antigen. Naive T cells may also be used.

In particular instances, the stimulating conditions include incubating, culturing, and/or cultivating the cells with a stimulatory reagent. In particular instances, the stimulatory reagent is a reagent described in Section I-B-1. In certain instances, the stimulatory reagent contains or includes a bead. In certain instances, the start and or initiation of the incubation, culturing, and/or cultivating cells under stimulating conditions occurs when the cells are come into contact with and/or are incubated with the stimulatory reagent. In particular instances, the cells are incubated prior to, during, and/or subsequent to genetically engineering the cells, e.g., introducing a recombinant polynucleotide into the cell such as by transduction or transfection.

In some instances, the composition of enriched T cells are incubated at a ratio of stimulatory reagent and/or beads to cells at or at about 3:1, 2.5:1, 2:1, 1.5:1, 1.25:1, 1.2:1, 1.1:1, 1:1, 0.9:1, 0.8:1, 0.75:1, 0.67:1, 0.5:1, 0.3:1, or 0.2:1. In particular instances, the ratio of stimulatory reagent and/or beads to cells is between 2.5:1 and 0.2:1, between 2:1 and 0.5:1, between 1.5:1 and 0.75:1, between 1.25:1 and 0.8:1, between 1.1:1 and 0.9:1. In particular instances, the ratio of stimulatory reagent to cells is about 1:1 or is 1:1.

In particular instances, the cells are not incubated, contacted, and/or exposed to an agent that inhibits mTOR activity, e.g., an agent described in Section II, prior to and/or during the incubation under stimulatory conditions.

In certain instances, at least a portion of the incubation is performed in the presence of an agent that inhibits mTOR activity, e.g., an agent described in Section II. In some instances, all of and/or the entire incubation is performed in the presence of an agent that inhibits mTOR activity.

In particular instances, the cells are incubated with the stimulatory reagent in the presence of an agent that inhibits mTOR activity. In certain instances, the agent that inhibits mTOR activity is an agent described in Section II. In some instances, the agent that inhibits mTOR activity also inhibits the activity of an additional kinase. In certain instances, the agent that inhibits mTOR activity also inhibits phosphoinositiol-3 kinase (PI3K) activity. In certain instances, the agent selectively inhibits mTOR activity, e.g., does not detectably inhibit PI3K activity, and/or does not inhibit PI3K activity to the same extent as mTOR activity, at concentrations that are sufficient to inhibit mTOR activity. In some instances, the agent that inhibits mTOR activity inhibits kinase activity. In certain instances, the agent that inhibits mTOR activity inhibits mTORC1 and/or mTORC2 activity. In some instances, the agent that inhibits mTOR activity inhibits mTORC1 and mTORC2 activity.

In some instances, the agents include, but are not limited to, PI-103, SF1126, BGT226, XI,765, PF-04691502, NVP-BEZ235, a pyrazolopyrimidine, Torin l, Torkinib (PP242), PP30, Ku-0063794, WAY-600 (Wyeth), WAY-687 (Wyeth), WAY-354 (Wyeth), AZD8055, rapamycin (sirolimus), temsirolimus (CC1779), everolimus (RAD001), deforolimus (AP23573), AZD8055 (AstraZeneca), and OSI-027 (OSI). In some instances, the agent that inhibits mTOR activity has or includes a formula that is provided in Section II, e.g., Formula (I), Formula (II), or Formula (III). In some instances, the agent is Compound 155, Compound 246, or Compound 63.

In certain instances, the cells are incubated in the presence of an agent that inhibits mTOR activity at a concentration that inhibits, reduces, and/or decreases mTOR activity. In some instances, concentration inhibits, reduced, and/or decreases one or more activities of mTOR by about or at least 25%, 30%, 40%, 50%, 60%, 70%, 80%, 85%, 90%, 95%, 97%, 98%, 99%, or 99.9%. In some instances, the concentration of the agent does not prevent primary T cells from proliferating and/or expanding. In some instances, the cells are incubated in the presence of between 1 nM and 1 µM, between 1 nM and 100 nM, between 50 nM and 200 nM, between 100 nM and 250 nM, between 200 nM and 500 nM, between 500 nM and 1 µM, between 1 µM and 10 µM, or between 5 µM and 50 µM of the agent that inhibits mTOR activity. In certain instances, the cells are incubated in the presence of, of about, or of at least 0.1 nM, 0.5 nM, 1 nM, 5 nM, 10 nM, 25 nM, 50 nM, 100 nM, 150 nM, 200 nM, 250 nM, 500 nM, 1 µM, 5 µM, 10 µM, 25 µM, 50 µM, or 100 µM of the agent that inhibits mTOR activity.

In some instances, the cells are incubated in the presence of Compound 155. In certain instances, the cells are incubated in the presence of between 1 nM and 1 µM, between 1 nM and 100 nM, between 50 nM and 200 nM, between 100 nM and 250 nM, or between 200 nM and 500 nM of Compound 155. In particular instances, the cells are incubated in the presence of, of about, or of at least 10 nM, 25 nM, 50 nM, 100 nM, 150 nM, 200 nM, 250 nM, or 500 nM of Compound 155.

In certain instances, the cells are incubated in the presence of Compound 246. In certain instances, the cells are incubated in the presence of between 1 nM and 1 µM, between 1 nM and 100 nM, between 50 nM and 200 nM, between 100 nM and 250 nM, or between 200 nM and 500 nM of Compound 155. In certain instances, the cells are incubated in the presence of, of about, or of at least 10 nM, 25 nM, 50 nM, 100 nM, 150 nM, 200 nM, 250 nM, or 500 nM of Compound 246.

In particular instances, the cells are incubated in the presence of Compound 63. In certain instances, the cells are incubated in the presence of between 1 nM and 1 µM, between 1 nM and 100 nM, between 50 nM and 200 nM, between 100 nM and 250 nM, or between 200 nM and 500 nM of Compound 63. In some instances, the cells are incubated in the presence of, of about, or of at least 10 nM, 25 nM, 50 nM, 100 nM, 150 nM, 200 nM, 250 nM, or 500 nM of Compound 63.

In some instances, the compositions or cells are incubated in the presence of stimulating conditions or a stimulatory agent. Such conditions include those designed to induce proliferation, expansion, activation, and/or survival of cells in the population, to mimic antigen exposure, and/or to prime the cells for genetic engineering, such as for the introduction of a recombinant antigen receptor. Exemplary stimulatory reagents are described below.

In some instances, the conditions for stimulation and/or activation can include one or more of particular media, temperature, oxygen content, carbon dioxide content, time, agents, e.g., nutrients, amino acids, antibiotics, ions, and/or stimulatory factors, such as cytokines, chemokines, antigens, binding partners, fusion proteins, recombinant soluble receptors, and any other agents designed to activate the cells.

In some aspects, incubation is carried out in accordance with techniques such as those described in US Patent No. 6,040,1 77 to Riddell et al., Klebanoff et al.(2012) J Immunother. 35(9): 651-660, Terakura et al. (2012) Blood.1:72-82, and/or Wang et al. (2012) J Immunother. 35(9):689-701.

In some instances, at least a portion of the incubation in the presence of one or more stimulating conditions or a stimulatory agents is carried out in the internal cavity of a centrifugal chamber, for example, under centrifugal rotation, such as described in International Publication Number WO2016/073602. In some instances, at least a portion of the incubation performed in a centrifugal chamber includes mixing with a reagent or reagents to induce stimulation and/or activation. In some instances, cells, such as selected cells, are mixed with a stimulating condition or stimulatory agent in the centrifugal chamber. In some aspects of such processes, a volume of cells is mixed with an amount of one or more stimulating conditions or agents that is far less than is normally employed when performing similar stimulations in a cell culture plate or other system.

In some instances, the stimulating agent is added to cells in the cavity of the chamber in an amount that is substantially less than (e.g. is no more than 5%, 10%, 20%, 30%, 40%, 50%, 60%, 70% or 80% of the amount) as compared to the amount of the stimulating agent that is typically used or would be necessary to achieve about the same or similar efficiency of selection of the same number of cells or the same volume of cells when selection is performed without mixing in a centrifugal chamber, e.g. in a tube or bag with periodic shaking or rotation. In some instances, the incubation is performed with the addition of an incubation buffer to the cells and stimulating agent to achieve a target volume with incubation of the reagent of, for example, 10 mL to 200 mL, such as at least or about at least or about or 10 mL, 20 mL, 30 mL, 40 mL, 50 mL, 60 mL, 70 mL, 80 mL, 90 mL, 100 mL, 150 mL or 200 mL. In some instances, the incubation buffer and stimulating agent are pre-mixed before addition to the cells. In some instances, the incubation buffer and stimulating agent are separately added to the cells. In some instances, the stimulating incubation is carried out with periodic gentle mixing condition, which can aid in promoting energetically favored interactions and thereby permit the use of less overall stimulating agent while achieving stimulating and activation of cells.

In some instances, the incubation generally is carried out under mixing conditions, such as in the presence of spinning, generally at relatively low force or speed, such as speed lower than that used to pellet the cells, such as from or from about 600 rpm to 1700 rpm (e.g. at or about or at least 600 rpm, 1000 rpm, or 1500 rpm or 1700 rpm), such as at an RCF at the sample or wall of the chamber or other container of from or from about 80g to 100g (e.g. at or about or at least 80 g, 85 g, 90 g, 95 g, or 100 g). In some instances, the spin is carried out using repeated intervals of a spin at such low speed followed by a rest period, such as a spin and/or rest for 1, 2, 3, 4, 5, 6, 7, 8, 9, or 10 seconds, such as a spin at approximately 1 or 2 seconds followed by a rest for approximately 5, 6, 7, or 8 seconds.

In some instances, the total duration of the incubation, e.g. with the stimulating agent, is between or between about 1 hour and 96 hours, 1 hour and 72 hours, 1 hour and 48 hours, 4 hours and 36 hours, 8 hours and 30 hours or 12 hours and 24 hours, such as at least or about at least 6 hours, 12 hours, 18 hours, 24 hours, 36 hours or 72 hours. In some instances, the further incubation is for a time between or about between 1 hour and 48 hours, 4 hours and 36 hours, 8 hours and 30 hours or 12 hours and 24 hours, inclusive.

In particular instances, the stimulating conditions include incubating, culturing, and/or cultivating a composition of enriched T cells with and/or in the presence of one or more cytokines. In particular instances, the one or more cytokines are recombinant cytokines. In some instances, the one or more cytokines are human recombinant cytokines. In certain instances, the one or more cytokines bind to and/or are capable of binding to receptors that are expressed by and/or are endogenous to T cells. In particular instances, the one or more cytokines is or includes a member of the 4-alpha-helix bundle family of cytokines. In some instances, members of the 4-alpha-helix bundle family of cytokines include, but are not limited to, interleukin-2 (IL-2), interleukin-4 (IL-4), interleukin-7 (IL-7), interleukin-9 (IL-9), interleukin 12 (IL-12), interleukin 15 (IL-15), granulocyte colony-stimulating factor (G-CSF), and granulocyte-macrophage colony-stimulating factor (GM-CSF).

In some instances, the stimulation results in activation and/or proliferation of the cells, for example, prior to transduction.

### 1. Stimulatory Reagents

In some instances, incubating a composition of cells, e.g., input cells, under stimulating conditions is or includes incubating and/or contacting the composition of enriched cells with a stimulatory reagent that is capable of activating and/or expanding T cells. In some instances, the stimulatory reagent is capable of stimulating and/or activating one or more signals in the cells. In some instances, the one or more signals are mediated by a receptor. In particular instances, the one or more signals are or are associated with a change in signal transduction and/or a level or amount of secondary messengers, e.g., cAMP and/or intracellular calcium, a change in the amount, cellular localization, confirmation, phosphorylation, ubiquitination, and/or truncation of one or more cellular proteins, and/or a change in a cellular activity, e.g., transcription, translation, protein degradation, cellular morphology, activation state, and/or cell division. In particular instances, the stimulatory reagent activates and/or is capable of activating one or more intracellular signaling domains of one or more components of a TCR complex and/or one or more intracellular signaling domains of one or more costimulatory molecules.

In certain instances, the stimulatory reagent contains a particle, e.g., a bead, that is conjugated or linked to one or more agents, e.g., biomolecules, that are capable of activating and/or expanding cells, e.g., T cells. In some instances, the one or more agents are bound to a bead. In some instances, the bead is biocompatible, i.e., composed of a material that is suitable for biological use. In some instances, the beads are non-toxic to cultured cells, e.g., cultured T cells. In some instances, the beads may be any particles which are capable of attaching agents in a manner that permits an interaction between the agent and a cell.

In some instances, a stimulatory reagent contains one or more agents that are capable of activating and/or expanding cells, e.g., T cells, that are bound to or otherwise attached to a bead, for example to the surface of the bead. In certain instances, the bead is a non-cell particle. In particular instances, the bead may include a colloidal particle, a microsphere, nanoparticle, a magnetic bead, or the like. In some instances the beads are agarose beads. In certain instances, the beads are sepharose beads.

In particular instances, the stimulatory reagent contains beads that are monodisperse. In certain instances, beads that are monodisperse comprise size dispersions having a diameter standard deviation of less than 5% from each other.

In some instances, the bead contains one or more agents, such as an agent that is coupled, conjugated, or linked (directly or indirectly) to the surface of the bead. In some instances, an agent as contemplated herein can include, but is not limited to, RNA, DNA, proteins (e.g., enzymes), antigens, polyclonal antibodies, monoclonal antibodies, antibody fragments, carbohydrates, lipids, lectins, or any other biomolecule with an affinity for a desired target. In some instances, the desired target is a T cell receptor and/or a component of a T cell receptor. In certain instances, the desired target is CD3. In certain instances, the desired target is a T cell costimulatory molecule, e.g., CD28, CD137 (4-1-BB), OX40, or ICOS. The one or more agents may be attached directly or indirectly to the bead by a variety of methods known and available in the art. The attachment may be covalent, noncovalent, electrostatic, or hydrophobic and may be accomplished by a variety of attachment means, including for example, a chemical means, a mechanical means, or an enzymatic means. In some instances, a biomolecule (e.g., a biotinylated anti-CD3 antibody) may be attached indirectly to the bead via another biomolecule (e.g., anti-biotin antibody) that is directly attached to the bead.

In some instances, the stimulatory reagent contains a bead and one or more agents that directly interact with a macromolecule on the surface of a cell. In certain instances, the bead (e.g., a paramagnetic bead) interacts with a cell via one or more agents (e.g., an antibody) specific for one or more macromolecules on the cell (e.g., one or more cell surface proteins). In certain instances, the bead (e.g., a paramagnetic bead) is labeled with a first agent described herein, such as a primary antibody (e.g., an anti-biotin antibody) or other biomolecule, and then a second agent, such as a secondary antibody (e.g., a biotinylated anti-CD3 antibody) or other second biomolecule (e.g., streptavidin), is added, whereby the secondary antibody or other second biomolecule specifically binds to such primary antibodies or other biomolecule on the particle.

In some instances, the stimulatory reagent contains one or more agents (e.g. antibody) that is attached to a bead (e.g., a paramagnetic bead) and specifically binds to one or more of the following macromolecules on a cell (e.g., a T cell): CD2, CD3, CD4, CD5, CD8, CD25, CD27, CD28, CD29, CD31, CD44, CD45RA, CD45RO, CD54 (ICAM-1), CD127, MHCI, MHCII, CTLA-4, ICOS, PD-1, OX40, CD27L (CD70), 4-1BB (CD137), 4-1BBL, CD30L, LIGHT, IL-2R, IL-12R, IL-1R, IL-15R; IFN-gammaR, TNF-alphaR, IL-4R, IL- 10R, CD18/CD11a (LFA-1), CD62L (L-selectin), CD29/CD49d (VLA-4), Notch ligand (e.g. Deltalike 1/4, Jagged 1/2, etc.), CCR1, CCR2, CCR3, CCR4, CCR5, CCR7, and CXCR3 or fragment thereof including the corresponding ligands to these macromolecules or fragments thereof. In some instances, an agent (e.g. antibody) attached to the bead specifically binds to one or more of the following macromolecules on a cell (e.g. a T cell): CD28, CD62L, CCR7, CD27, CD127, CD3, CD4, CD8, CD45RA, and/or CD45RO.

In some instances, one or more of the agents attached to the bead is an antibody. The antibody can include a polyclonal antibody, monoclonal antibody (including full length antibodies which have an immunoglobulin Fc region), antibody compositions with polyepitopic specificity, multispecific antibodies (e.g., bispecific antibodies, diabodies, and single-chain molecules, as well as antibody fragments (e.g., Fab, F(ab')2, and Fv). In some instances, the stimulatory reagent is an antibody fragment (including antigen-binding fragment), e.g., a Fab, Fab'-SH, Fv, scFv, or (Fab')2 fragment. It will be appreciated that constant regions of any isotype can be used for the antibodies contemplated herein, including IgG, IgM, IgA, IgD, and IgE constant regions, and that such constant regions can be obtained from any human or animal species (e.g., murine species). In some instances, the agent is an antibody that binds to and/or recognizes one or more components of a T cell receptor. In particular instances, the agent is an anti-CD3 antibody. In certain instances, the agent is an antibody that binds to and/or recognizes a co-receptor. In some instances, the stimulatory reagent comprises an anti-CD28 antibody. In some instances, the bead has a diameter of greater than about 0.001 µm, greater than about 0.01 µm, greater than about 0.1 µm, greater than about 1.0 µm, greater than about 10 µm, greater than about 50 µm, greater than about 100 µm or greater than about 1000 µm and no more than about 1500µm. In some instances, the bead has a diameter of about 1.0 µm to about 500 µm, about 1.0 µm to about 150 µm, about 1.0 µm to about 30 µm, about 1.0 µm to about 10 µm, about 1.0 µm to about 5.0 µm, about 2.0 µm to about 5.0 µm, or about 3.0 µm to about 5.0 µm. In some instances, the bead has a diameter of about 3 µm to about 5µm. In some instances, the bead has a diameter of at least or at least about or about 0.001 µm, 0.01 µm, 0.1µm, 0.5µm, 1.0 µm, 1.5 µm, 2.0 µm, 2.5 µm, 3.0 µm, 3.5 µm, 4.0 µm, 4.5 µm, 5.0 µm, 5.5 µm, 6.0 µm, 6.5 µm, 7.0 µm, 7.5 µm, 8.0 µm, 8.5 µm, 9.0 µm, 9.5 µm, 10 µm, 12 µm, 14 µm, 16 µm, 18 µm or 20 µm. In certain instances, the bead has a diameter of or about 4.5 µm. In certain instances, the bead has a diameter of or about 2.8 µm.

In some instances, the beads have a density of greater than 0.001 g/cm³, greater than 0.01 g/cm³, greater than 0.05 g/cm³, greater than 0.1 g/cm³, greater than 0.5 g/cm³, greater than 0.6 g/cm³, greater than 0.7 g/cm³, greater than 0.8 g/cm³, greater than 0.9 g/cm³, greater than 1 g/cm³, greater than 1.1 g/cm³, greater than 1.2 g/cm³, greater than 1.3 g/cm³, greater than 1.4 g/cm³, greater than 1.5 g/cm³, greater than 2 g/cm³, greater than 3 g/cm³, greater than 4 g/cm³, or greater than 5g/cm³. In some instances, the beads have a density of between about 0.001 g/cm³ and about 100 g/cm³, about 0.01 g/cm³ and about 50 g/cm³, about 0.1 g/cm³ and about 10 g/cm³, about 0.1 g/cm³ and about .5 g/cm³, about 0.5 g/cm³ and about 1 g/cm³, about 0.5 g/cm³ and about 1.5 g/cm³, about 1 g/cm³ and about 1.5 g/cm³, about 1 g/cm³ and about 2 g/cm³, or about 1 g/cm³ and about 5 g/cm³. In some instances, the beads have a density of about 0.5 g/cm³, about 0.6 g/cm³, about 0.7 g/cm³, about 0.8 g/cm³, about 0.9 g/cm³, about 1.0 g/cm³, about 1.1 g/cm³, about 1.2 g/cm³, about 1.3 g/cm³, about 1.4 g/cm³, about 1.5 g/cm³, about 1.6 g/cm³, about 1.7 g/cm³, about 1.8 g/cm³, about 1.9 g/cm³, or about 2.0 g/cm³. In certain instances, the beads have a density of about 1.6 g/cm³. In particular instances, the beads or particles have a density of about 1.5 g/cm³. In certain instances, the particles have a density of about 1.3 g/cm³.

In certain instances, a plurality of the beads has a uniform density. In certain instances, a uniform density comprises a density standard deviation of less than 10%, less than 5%, or less than 1% of the mean bead density.

In some instances, the beads have a surface area of between about 0.001 m² per each gram of particles (m²/g) to about 1,000 m²/g, about .010 m²/g to about 100 m²/g, about 0.1 m²/g to about 10 m²/g, about 0.1 m²/g to about 1 m²/g, about 1 m²/g to about 10 m²/g, about 10 m²/g to about 100 m²/g, about 0.5 m²/g to about 20 m²/g, about 0.5 m²/g to about 5 m²/g, or about 1 m²/g to about 4 m²/g. In some instances, the particles or beads have a surface area of about 1 m²/g to about 4 m²/g.

In some instances, the bead contains at least one material at or near the bead surface that can be coupled, linked, or conjugated to an agent. In some instances, the bead is surface functionalized, i.e. comprises functional groups that are capable of forming a covalent bond with a binding molecule, e.g., a polynucleotide or a polypeptide. In particular instances, the bead comprises surface-exposed carboxyl, amino, hydroxyl, tosyl, epoxy, and/or chloromethyl groups. In particular instances, the beads comprise surface exposed agarose and/or sepharose. In certain instances, the bead surface comprises attached stimulatory reagents that can bind or attach binding molecules. In particular instances, the biomolecules are polypeptides. In some instances, the beads comprise surface exposed protein A, protein G, or biotin.

In some instances, the bead reacts in a magnetic field. In some instances, the bead is a magnetic bead. In some instances, the magnetic bead is paramagnetic. In particular instances, the magnetic bead is superparamagnetic. In certain instances, the beads do not display any magnetic properties unless they are exposed to a magnetic field.

In particular instances, the bead comprises a magnetic core, a paramagnetic core, or a superparamagnetic core. In some instances, the magnetic core contains a metal. In some instances, the metal can be, but is not limited to, iron, nickel, copper, cobalt, gadolinium, manganese, tantalum, zinc, zirconium or any combinations thereof. In certain instances, the magnetic core comprises metal oxides (e.g., iron oxides), ferrites (e.g., manganese ferrites, cobalt ferrites, nickel ferrites, etc.), hematite and metal alloys (e.g., CoTaZn). In some instances, the magnetic core comprises one or more of a ferrite, a metal, a metal alloy, an iron oxide, or chromium dioxide. In some instances, the magnetic core comprises elemental iron or a compound thereof. In some instances, the magnetic core comprises one or more of magnetite (Fe304), maghemite (γFe2O3), or greigite (Fe3S4). In some instances, the inner core comprises an iron oxide (e.g., Fe₃O₄).

In certain instances, the bead contains a magnetic, paramagnetic, and/or superparamagnetic core that is covered by a surface functionalized coat or coating. In some instances, the coat can contain a material that can include, but is not limited to, a polymer, a polysaccharide, a silica, a fatty acid, a protein, a carbon, agarose, sepharose, or a combination thereof. In some instances, the polymer can be a polyethylene glycol, poly (lactic-co-glycolic acid), polyglutaraldehyde, polyurethane, polystyrene, or a polyvinyl alcohol. In certain instances, the outer coat or coating comprises polystyrene. In particular instances, the outer coating is surface functionalized.

In some instances, the stimulatory reagent comprises a bead that contains a metal oxide core (e.g., an iron oxide core) and a coat, wherein the metal oxide core comprises at least one polysaccharide (e.g., dextran), and wherein the coat comprises at least one polysaccharide (e.g., amino dextran), at least one polymer (e.g., polyurethane) and silica. In some instances the metal oxide core is a colloidal iron oxide core. In certain instances, the one or more agents include an antibody or antigen-binding fragment thereof. In particular instances, the one or more agents include an anti-CD3 antibody and an anti-CD28 antibody. In some instances, the stimulatory reagent comprises an anti-CD3 antibody, anti-CD28 antibody, and an anti-biotin antibody. In some instances, the stimulatory reagent comprises an anti-biotin antibody. In some instances, the bead has a diameter of about 3 µm to about 10 µm. In some instances, the bead has a diameter of about 3 µm to about 5 µm. In certain instances, the bead has a diameter of about 3.5 µm.

In some instances, the stimulatory reagent comprises one or more agents that are attached to a bead comprising a metal oxide core (e.g., an iron oxide inner core) and a coat (e.g., a protective coat), wherein the coat comprises polystyrene. In certain instances, the beads are monodisperse, paramagnetic (e.g., superparamagnetic) beads comprising a paramagnetic (e.g., superparamagnetic) iron core, e.g., a core comprising magnetite (Fe₃O₄) and/or maghemite (γFe₂O₃) c and a polystyrene coat or coating. In some instances, the bead is non-porous. In some instances, the beads contain a functionalized surface to which the one or more agents are attached. In certain instances, the one or more agents are covalently bound to the beads at the surface. In some instances, the one or more agents include an antibody or antigen-binding fragment thereof. In some instances, the one or more agents include an anti-CD3 antibody and an anti-CD28 antibody. In some instances, the one or more agents include an anti-CD3 antibody and/or an anti-CD28 antibody, and an antibody or antigen fragment thereof capable of binding to a labeled antibody (e.g., biotinylated antibody), such as a labeled anti-CD3 or anti-CD28 antibody. In certain instances, the beads have a density of about 1.5 g/cm³ and a surface area of about 1 m²/g to about 4 m²/g. In particular instances; the beads are monodisperse superparamagnetic beads that have a diameter of about 4.5 µm and a density of about 1.5 g/cm³. In some instances, the beads the beads are monodisperse superparamagnetic beads that have a mean diameter of about 2.8 µm and a density of about 1.3 g/cm³.

### C. Engineering Cells

In some instances, the methods disclosed herein are used in association with engineering one or more compositions of T cells. In certain instances, the engineering is or includes the introduction of a polynucleotide, e.g., a polynucleotide encoding a recombinant protein. In particular instances, the recombinant proteins are recombinant receptors, such as any described in Section II. Introduction of the nucleic acid molecules encoding the recombinant protein, such as recombinant receptor, in the cell may be carried out using any of a number of known vectors. Such vectors include viral and non-viral systems, including lentiviral and gammaretroviral systems, as well as transposon-based systems such as PiggyBac or Sleeping Beauty-based gene transfer systems. Exemplary methods include those for transfer of nucleic acids encoding the receptors, including via viral, e.g., retroviral or lentiviral, transduction, transposons, and electroporation. In some instances, the engineering produces one or more engineered compositions of enriched T cells.

In certain instances, one or more compositions of T cells are engineered, e.g., transduced or transfected, prior to cultivating the cells, e.g., under conditions that promote proliferation and/or expansion, such as by a method provided in Section I-D. In particular instances, one or more compositions of enriched T cells are engineered after the one or more compositions have been stimulated, activated, and/or incubated under stimulating conditions. In particular instances, the one or more compositions are stimulated compositions. In particular instances, the one or more stimulated compositions have been previously cryofrozen and stored, and are thawed prior to engineering.

In certain instances, the one or more compositions of stimulated T cells are or include two separate stimulated compositions of enriched T cells. In particular instances, two separate compositions of enriched T cells, e.g., two separate compositions of enriched T cells that have been selected, isolated, and/or enriched from the same biological sample, are separately engineered. In certain instances, the two separate compositions include a composition of enriched CD4+ T cells. In particular instances, the two separate compositions include a composition of enriched CD8+ T cells. In some instances, two separate compositions of enriched CD4+ T cells and enriched CD8+ T cells are genetically engineered separately. In some instances, a single composition of enriched T cells is genetically engineered. In certain instances, the single composition is a composition of enriched CD4+ T cells. In some instances, the single composition is a composition of enriched CD4+ and CD8+ T cells that have been combined from separate compositions prior to the engineering.

In some instances, the composition of enriched CD4+ T cells that is engineered, e.g., transduced or transfected, includes at least 60%, at least 65%, at least 70%, at least 75%, at least 80%, at least 85%, at least 90%, at least 95%, at least 98%, at least 99%, at least 99.5%, at least 99.9%, or at or at about 100% CD4+ T cells. In certain instances, the composition of enriched CD4+ T cells that is engineered includes less than 40%, less than 35%, less than 30%, less than 25%, less than 20%, less than 15%, less than 10%, less than 5%, less than 1%, less than 0.1%, or less than 0.01% CD8+ T cells, and/or contains no CD8+ T cells, and/or is free or substantially free of CD8+ T cells.

In some instances, the composition of enriched CD8+ T cells that is engineered, e.g., transduced or transfected, includes at least 60%, at least 65%, at least 70%, at least 75%, at least 80%, at least 85%, at least 90%, at least 95%, at least 98%, at least 99%, at least 99.5%, at least 99.9%, or at or at about 100% CD8+ T cells. In certain instances, the composition of enriched CD8+ T cells that is incubated under stimulating conditions includes less than 40%, less than 35%, less than 30%, less than 25%, less than 20%, less than 15%, less than 10%, less than 5%, less than 1%, less than 0.1%, or less than 0.01% CD4+ T cells, and/or contains no CD4+ T cells, and/or is free or substantially free of CD4+ T cells.

In some instances, separate compositions of enriched CD4+ and CD8+ T cells are combined into a single composition and are genetically engineered, e.g., transduced or transfected. In certain instances, separate engineered compositions of enriched CD4+ and enriched CD8+ T cells are combined into a single composition after the genetic engineering has been performed and/or completed.

In some instances, gene transfer is accomplished by first stimulating the cell, such as by combining it with a stimulus that induces a response such as proliferation, survival, and/or activation, e.g., as measured by expression of a cytokine or activation marker, followed by transduction of the activated cells, and expansion in culture to numbers sufficient for clinical applications. In certain instances, the gene transfer is accomplished by first incubating the cells under stimulating conditions, such as by any of the methods described in Section I-B.

In some instances, methods for genetic engineering are carried out by contacting one or more cells of a composition with a nucleic acid molecule encoding the recombinant protein, e.g. recombinant receptor. In some instances, the contacting can be effected with centrifugation, such as spinoculation (e.g. centrifugal inoculation). Such methods include any of those as described in International Publication Number WO2016/073602. Exemplary centrifugal chambers include those produced and sold by Biosafe SA, including those for use with the Sepax^{®} and Sepax^{®} 2 system, including an A-200/F and A-200 centrifugal chambers and various kits for use with such systems. Exemplary chambers, systems, and processing instrumentation and cabinets are described, for example, in US Patent No. 6,123,655, US Patent No. 6,733,433 and Published U.S. Patent Application, Publication No.: US 2008/0171951, and published international patent application, publication no. WO 00/38762. Exemplary kits for use with such systems include, but are not limited to, single-use kits sold by BioSafe SA under product names CS-430.1, CS-490.1, CS-600.1 or CS-900.2.

In some instances, the cells are not incubated, contacted, and/or exposed to an agent that inhibits mTOR activity, such as an agent described herein, e.g. in Section II, prior to and/or during the engineering.

In particular instances, at least a portion of the engineering is performed in the presence of an agent that inhibits mTOR activity, such as an agent described herein e.g., an agent described in Section II. In some instances, all of and/or the engineering step is performed in the presence of an agent that inhibits mTOR activity.

In particular instances, the cells are engineered, e.g., transduced or transfected, in the presence of an agent that inhibits mTOR activity. In certain instances, the agent that inhibits mTOR activity is an agent described herein, such as in Section II, e.g. Compound 63. In some instances, the agent that inhibits mTOR activity also inhibits the activity of an additional kinase. In certain instances, the agent that inhibits mTOR activity also inhibits phosphoinositiol-3 kinase (PI3K) activity. In certain instances, the agent selectively inhibits mTOR activity, e.g., does not measurably inhibit PI3K activity, and/or does not inhibit PI3K activity to the same extent as mTOR activity, at concentrations that are sufficient to inhibit mTOR activity. In some instances, the agent that inhibits mTOR activity inhibits kinase activity. In certain instances, the agent that inhibits mTOR activity inhibits mTORC1 and/or mTORC2 activity. In some instances, the agent that inhibits mTOR activity inhibits mTORC1 and mTORC2 activity.

In some instances, the agents include, but are not limited to, PI-103, SF1126, BGT226, XL765, PF-04691502, NVP-BEZ235, a pyrazolopyrimidine, Torin l, Torkinib (PP242), PP30, Ku-0063794, WAY-600 (Wyeth), WAY-687 (Wyeth), WAY-354 (Wyeth), AZD8055, rapamycin (sirolimus), temsirolimus (CC1779), everolimus (RAD001), deforolimus (AP23573), AZD8055 (AstraZeneca), and OSI-027 (OSI). In some instances, the agent that inhibits mTOR activity has or includes a formula that is provided in Section II, e.g., Formula (I), Formula (II), or Formula (III). In some instances, the agent is Compound 155, Compound 246, or Compound 63. In some instances, the agent is Compound 63.

In certain instances, the cells are engineered in the presence of an agent that inhibits mTOR activity at a concentration that inhibits, reduces, and/or decreases mTOR activity. In some instances, concentration inhibits, reduced, and/or decreases one or more activities of mTOR by about or at least 25%, 30%, 40%, 50%, 60%, 70%, 80%, 85%, 90%, 95%, 97%, 98%, 99%, or 99.9%. In some instances, the concentration of the agent does not prevent primary T cells from proliferating and/or expanding. In some instances, the cells are engineered in the presence of between 1 nM and 1 µM, between 1 nM and 100 nM, between 50 nM and 200 nM, between 100 nM and 250 nM, between 200 nM and 500 nM, between 500 nM and 1 µM, between 1 µM and 10 µM, or between 5 µM and 50 µM of the agent that inhibits mTOR activity. In certain instances, the cells are engineered in the presence of, of about, or of at least 0.1 nM, 0.5 nM, 1 nM, 5 nM, 10 nM, 25 nM, 50 nM, 100 nM, 150 nM, 200 nM, 250 nM, 500 nM, 1 µM, 5 µM, 10 µM, 25 µM, 50 µM, or 100 µM of the agent that inhibits mTOR activity.

In some instances, the cells are engineered in the presence of Compound 155. In certain instances, the cells are engineered in the presence of between 1 nM and 1 µM, between 1 nM and 100 nM, between 50 nM and 200 nM, between 100 nM and 250 nM, or between 200 nM and 500 nM of Compound 155. In particular instances, the cells are engineered in the presence of, of about, or of at least 10 nM, 25 nM, 50 nM, 100 nM, 150 nM, 200 nM, 250 nM, or 500 nM of Compound 155.

In certain instances, the cells are engineered in the presence of Compound 246. In certain instances, the cells are engineered in the presence of between 1 nM and 1 µM, between 1 nM and 100 nM, between 50 nM and 200 nM, between 100 nM and 250 nM, or between 200 nM and 500 nM of Compound 155. In certain instances, the cells are engineered in the presence of, of about, or of at least 10 nM, 25 nM, 50 nM, 100 nM, 150 nM, 200 nM, 250 nM, or 500 nM of Compound 246.

In particular instances, the cells are engineered in the presence of Compound 63. In certain instances, the cells are engineered in the presence of between 1 nM and 1 µM, between 1 nM and 100 nM, between 50 nM and 200 nM, between 100 nM and 250 nM, or between 200 nM and 500 nM of Compound 63. In some instances, the cells are engineered in the presence of, of about, or of at least 10 nM, 25 nM, 50 nM, 100 nM, 150 nM, 200 nM, 250 nM, or 500 nM of Compound 63.

In some instances, the contacting can be effected with centrifugation, such as spinoculation (e.g., centrifugal inoculation). In some instances, the composition containing cells, viral particles and reagent can be rotated, generally at relatively low force or speed, such as speed lower than that used to pellet the cells, such as from or from about 600 rpm to 1700 rpm (e.g., at or about or at least 600 rpm, 1000 rpm, or 1500 rpm or 1700 rpm). In some instances, the rotation is carried at a force, e.g., a relative centrifugal force, of from or from about 100 g to 3200 g (e.g., at or about or at least at or about 100 g, 200 g, 300 g, 400 g, 500 g, 1000 g, 1500 g, 2000 g, 2500 g, 3000 g or 3200 g), as measured for example at an internal or external wall of the chamber or cavity. The term "relative centrifugal force" or RCF is generally understood to be the effective force imparted on an object or substance (such as a cell, sample, or pellet and/or a point in the chamber or other container being rotated), relative to the earth's gravitational force, at a particular point in space as compared to the axis of rotation. The value may be determined using well-known formulas, taking into account the gravitational force, rotation speed and the radius of rotation (distance from the axis of rotation and the object, substance, or particle at which RCF is being measured).

In some instances, the introducing is carried out by contacting one or more cells of a composition with a nucleic acid molecule encoding the recombinant protein, e.g. recombinant receptor. In some instances, the contacting can be effected with centrifugation, such as spinoculation (e.g. centrifugal inoculation). Such methods include any of those as described in International Publication Number WO2016/073602. Exemplary centrifugal chambers include those produced and sold by Biosafe SA, including those for use with the Sepax^{®} and Sepax^{®} 2 system, including an A-200/F and A-200 centrifugal chambers and various kits for use with such systems. Exemplary chambers, systems, and processing instrumentation and cabinets are described, for example, in US Patent No. 6,123,655, US Patent No. 6,733,433 and Published U.S. Patent Application, Publication No.: US 2008/0171951, and published international patent application, publication no. WO 00/38762. Exemplary kits for use with such systems include, but are not limited to, single-use kits sold by BioSafe SA under product names CS-430.1, CS-490.1, CS-600.1 or CS-900.2.

In some instances, the system is included with and/or placed into association with other instrumentation, including instrumentation to operate, automate, control and/or monitor aspects of the transduction step and one or more various other processing steps performed in the system, e.g. one or more processing steps that can be carried out with or in connection with the centrifugal chamber system as described herein or in International Publication Number WO2016/073602. This instrumentation in some instances is contained within a cabinet. In some instances, the instrumentation includes a cabinet, which includes a housing containing control circuitry, a centrifuge, a cover, motors, pumps, sensors, displays, and a user interface. An exemplary device is described in US Patent No. 6,123,655, US Patent No. 6,733,433 and US 2008/0171951.

In some instances, the system comprises a series of containers, e.g., bags, tubing, stopcocks, clamps, connectors, and a centrifuge chamber. In some instances, the containers, such as bags, include one or more containers, such as bags, containing the cells to be transduced and the viral vector particles, in the same container or separate containers, such as the same bag or separate bags. In some instances, the system further includes one or more containers, such as bags, containing medium, such as diluent and/or wash solution, which is pulled into the chamber and/or other components to dilute, resuspend, and/or wash components and/or compositions during the methods. The containers can be connected at one or more positions in the system, such as at a position corresponding to an input line, diluent line, wash line, waste line and/or output line.

In some instances, the chamber is associated with a centrifuge, which is capable of effecting rotation of the chamber, such as around its axis of rotation. Rotation may occur before, during, and/or after the incubation in connection with transduction of the cells and/or in one or more of the other processing steps. Thus, in some instances, one or more of the various processing steps is carried out under rotation, e.g., at a particular force. The chamber is typically capable of vertical or generally vertical rotation, such that the chamber sits vertically during centrifugation and the side wall and axis are vertical or generally vertical, with the end wall(s) horizontal or generally horizontal.

In some instances, the composition containing cells, the vector, e.g., viral particles, and reagent can be rotated, generally at relatively low force or speed, such as speed lower than that used to pellet the cells, such as from or from about 600 rpm to 1700 rpm (e.g. at or about or at least 600 rpm, 1000 rpm, or 1500 rpm or 1700 rpm). In some instances, the rotation is carried at a force, e.g., a relative centrifugal force, of from or from about 100 g to 3200 g (e.g. at or about or at least at or about 100 g, 200 g, 300 g, 400 g, 500 g, 1000 g, 1500 g, 2000 g, 2500 g, 3000 g or 3200 g), as measured for example at an internal or external wall of the chamber or cavity. The term "relative centrifugal force" or RCF is generally understood to be the effective force imparted on an object or substance (such as a cell, sample, or pellet and/or a point in the chamber or other container being rotated), relative to the earth's gravitational force, at a particular point in space as compared to the axis of rotation. The value may be determined using well-known formulas, taking into account the gravitational force, rotation speed and the radius of rotation (distance from the axis of rotation and the object, substance, or particle at which RCF is being measured).

In some instances, during at least a part of the genetic engineering, e.g. transduction, and/or subsequent to the genetic engineering the cells are transferred to the bioreactor bag assembly for culture of the genetically engineered cells, such as for cultivation or expansion of the cells, as described above.

Also disclosed are one or more polynucleotides (e.g., nucleic acid molecules) encoding recombinant receptors, vectors for genetically engineering cells to express such receptors and methods for producing the engineered cells. In some instances, the vector contains the nucleic acid encoding the recombinant receptor. In particular instances, the vector is a viral vector a non-viral vector. In some cases, the vector is a viral vector, such as a retroviral vector, e.g., a lentiviral vector or a gammaretroviral vector.

In some instances, the vectors include viral vectors, e.g., retroviral or lentiviral, non-viral vectors or transposons, e.g. *Sleeping Beauty* transposon system, vectors derived from simian virus 40 (SV40), adenoviruses, adeno-associated virus (AAV), lentiviral vectors or retroviral vectors, such as gamma-retroviral vectors, retroviral vector derived from the Moloney murine leukemia virus (MoMI,V), myeloproliferative sarcoma virus (MPSV), murine embryonic stem cell virus (MESV), murine stem cell virus (MSCV), spleen focus forming virus (SFFV) or adeno-associated virus (AAV).

In some instances, the viral vector or the non-viral DNA contains a nucleic acid that encodes a heterologous recombinant protein. In some instances, the heterologous recombinant molecule is or includes a recombinant receptor, *e.g.,* an antigen receptor, SB-transposons, *e.g.,* for gene silencing, capsid-enclosed transposons, homologous double stranded nucleic acid, *e.g.,* for genomic recombination or reporter genes (*e.g*., fluorescent proteins, such as GFP) or luciferase).

### 1. Viral Vector Particles

In some instances, recombinant nucleic acids are transferred into cells using recombinant infectious virus particles, such as, e.g., vectors derived from simian virus 40 (SV40), adenoviruses, adeno-associated virus (AAV). In some instances, recombinant nucleic acids are transferred into T cells using recombinant lentiviral vectors or retroviral vectors, such as gamma-retroviral vectors (see, e.g., Koste et al. (2014) Gene Therapy 2014 Apr 3. doi: 10.1038/gt.2014.25; Carlens et al. (2000) Exp Hematol 28(10): 1137-46; Alonso-Camino et al. (2013) Mol Ther Nucl Acids 2, e93; Park et al., Trends Biotechnol. 2011 November 29(11): 550-557).

In some instances, the retroviral vector has a long terminal repeat sequence (LTR), e.g., a retroviral vector derived from the Moloney murine leukemia virus (MoMI,V), myeloproliferative sarcoma virus (MPSV), murine embryonic stem cell virus (MESV), murine stem cell virus (MSCV), spleen focus forming virus (SFFV), or adeno-associated virus (AAV). Most retroviral vectors are derived from murine retroviruses. In some instances, the retroviruses include those derived from any avian or mammalian cell source. The retroviruses typically are amphotropic, meaning that they are capable of infecting host cells of several species, including humans. In one instance, the gene to be expressed replaces the retroviral gag, pol and/or env sequences. A number of illustrative retroviral systems have been described (e.g., U.S. Pat. Nos. 5,219,740; 6,207,453; 5,219,740; Miller and Rosman (1989) BioTechniques 7:980-990; Miller, A. D. (1990) Human Gene Therapy 1:5-14; Scarpa et al. (1991) Virology 180:849-852; Burns et al. (1993) Proc. Natl. Acad. Sci. USA 90:8033-8037; and Boris-Lawrie and Temin (1993) Cur. Opin. Genet. Develop. 3:102-109.

Methods of lentiviral transduction are known. Exemplary methods are described in, e.g., Wang et al. (2012) J. Immunother. 35(9): 689-701; Cooper et al. (2003) Blood. 101:1637-1644; Verhoeyen et al. (2009) Methods Mol Biol. 506: 97-114; and Cavalieri et al. (2003) Blood. 102(2): 497-505.

In some instances, the viral vector particles contain a genome derived from a retroviral genome based vector, such as derived from a lentiviral genome based vector. In some aspects of the disclosed viral vectors, the heterologous nucleic acid encoding a recombinant receptor, such as an antigen receptor, such as a CAR, is contained and/or located between the 5' LTR and 3' LTR sequences of the vector genome.

In some instances, the viral vector genome is a lentivirus genome, such as an HIV-1 genome or an SIV genome. For example, lentiviral vectors have been generated by multiply attenuating virulence genes, for example, the genes env, vif, vpu and nef can be deleted, making the vector safer for therapeutic purposes. Lentiviral vectors are known. See Naldini et al., (1996 and 1998); Zufferey et al., (1997); Dull et al., 1998, U.S. Pat. Nos. 6,013,516; and 5,994,136). In some instances, these viral vectors are plasmid-based or virus-based, and are configured to carry the essential sequences for incorporating foreign nucleic acid, for selection, and for transfer of the nucleic acid into a host cell. Known lentiviruses can be readily obtained from depositories or collections such as the American Type Culture Collection ("ATCC"; 10801 University Blvd., Manassas, Va. 20110-2209), or isolated from known sources using commonly available techniques.

Non-limiting examples of lentiviral vectors include those derived from a lentivirus, such as Human Immunodeficiency Virus 1 (HIV-1), HIV-2, an Simian Immunodeficiency Virus (SIV), Human T-lymphotropic virus 1 (HTLV-1), HTLV-2 or equine infection anemia virus (E1AV). For example, lentiviral vectors have been generated by multiply attenuating the HIV virulence genes, for example, the genes env, vif, vpr, vpu and nef are deleted, making the vector safer for therapeutic purposes. Lentiviral vectors are known in the art, see Naldini et al., (1996 and 1998); Zufferey et al., (1997); Dull et al., 1998, U.S. Pat. Nos. 6,013,516; and 5,994,136). In some instances, these viral vectors are plasmid-based or virus-based, and are configured to carry the essential sequences for incorporating foreign nucleic acid, for selection, and for transfer of the nucleic acid into a host cell. Known lentiviruses can be readily obtained from depositories or collections such as the American Type Culture Collection ("ATCC"; 10801 University Blvd., Manassas, Va. 20110-2209), or isolated from known sources using commonly available techniques.

In some instances, the viral genome vector can contain sequences of the 5' and 3' LTRs of a retrovirus, such as a lentivirus. In some aspects, the viral genome construct may contain sequences from the 5' and 3' LTRs of a lentivirus, and in particular can contain the R and U5 sequences from the 5' LTR of a lentivirus and an inactivated or self-inactivating 3' LTR from a lentivirus. The LTR sequences can be LTR sequences from any lentivirus from any species. For example, they may be LTR sequences from HIV, SIV, FIV or BIV. Typically, the LTR sequences are HIV LTR sequences.

In some instances, the nucleic acid of a viral vector, such as an HIV viral vector, lacks additional transcriptional units. The vector genome can contain an inactivated or self-inactivating 3' LTR (Zufferey et al. J Virol 72: 9873, 1998; Miyoshi et al., J Virol 72:8150, 1998). For example, deletion in the U3 region of the 3' LTR of the nucleic acid used to produce the viral vector RNA can be used to generate self-inactivating (SIN) vectors. This deletion can then be transferred to the 5' LTR of the proviral DNA during reverse transcription. A self-inactivating vector generally has a deletion of the enhancer and promoter sequences from the 3' long terminal repeat (LTR), which is copied over into the 5' LTR during vector integration. In some instances enough sequence can be eliminated, including the removal of a TATA box, to abolish the transcriptional activity of the LTR. This can prevent production of full-length vector RNA in transduced cells. In some aspects, the U3 element of the 3' LTR contains a deletion of its enhancer sequence, the TATA box, Sp1, and NF-kappa B sites. As a result of the self-inactivating 3' LTR, the provirus that is generated following entry and reverse transcription contains an inactivated 5' LTR. This can improve safety by reducing the risk of mobilization of the vector genome and the influence of the LTR on nearby cellular promoters. The self-inactivating 3' LTR can be constructed by any method known in the art. In some instances, this does not affect vector titers or the in vitro or in vivo properties of the vector.

Optionally, the U3 sequence from the lentiviral 5' LTR can be replaced with a promoter sequence in the viral construct, such as a heterologous promoter sequence. This can increase the titer of virus recovered from the packaging cell line. An enhancer sequence can also be included. Any enhancer/promoter combination that increases expression of the viral RNA genome in the packaging cell line may be used. In one example, the CMV enhancer/promoter sequence is used (U.S. Pat. No. 5,385,839 and U.S. Pat. No. 5,168,062).

In certain instances, the risk of insertional mutagenesis can be minimized by constructing the retroviral vector genome, such as lentiviral vector genome, to be integration defective. A variety of approaches can be pursued to produce a non-integrating vector genome. In some instances, a mutation(s) can be engineered into the integrase enzyme component of the pol gene, such that it encodes a protein with an inactive integrase. In some instances, the vector genome itself can be modified to prevent integration by, for example, mutating or deleting one or both attachment sites, or making the 3' LTR-proximal polypurine tract (PPT) non-functional through deletion or modification. In some instances, non-genetic approaches are available; these include pharmacological agents that inhibit one or more functions of integrase. The approaches are not mutually exclusive; that is, more than one of them can be used at a time. For example, both the integrase and attachment sites can be non-functional, or the integrase and PPT site can be non-functional, or the attachment sites and PPT site can be non-functional, or all of them can be non-functional. Such methods and viral vector genomes are known and available (see Philpott and Thrasher, Human Gene Therapy 18:483, 2007; Engelman et al. J Virol 69:2729, 1995; Brown et al J Virol 73:9011 (1999); WO 2009/076524; McWilliams et al., J Virol 77:11150, 2003; Powell and Levin J Virol 70:5288, 1996).

In some instances, the vector contains sequences for propagation in a host cell, such as a prokaryotic host cell. In some instances, the nucleic acid of the viral vector contains one or more origins of replication for propagation in a prokaryotic cell, such as a bacterial cell. In some instances, vectors that include a prokaryotic origin of replication also may contain a gene whose expression confers a detectable or selectable marker such as drug resistance.

The viral vector genome is typically constructed in a plasmid form that can be transfected into a packaging or producer cell line. Any of a variety of known methods can be used to produce retroviral particles whose genome contains an RNA copy of the viral vector genome. In some instances, at least two components are involved in making a virus-based gene delivery system: first, packaging plasmids, encompassing the structural proteins as well as the enzymes necessary to generate a viral vector particle, and second, the viral vector itself, i.e., the genetic material to be transferred. Biosafety safeguards can be introduced in the design of one or both of these components.

In some instances, the packaging plasmid can contain all retroviral, such as HIV-1, proteins other than envelope proteins (Naldini et al., 1998). In other instances, viral vectors can lack additional viral genes, such as those that are associated with virulence, e.g., vpr, vif, vpu and nef, and/or Tat, a primary transactivator of HIV. In some instances, lentiviral vectors, such as HIV-based lentiviral vectors, comprise only three genes of the parental virus: gag, pol and rev, which reduces or eliminates the possibility of reconstitution of a wild-type virus through recombination.

In some instances, the viral vector genome is introduced into a packaging cell line that contains all the components necessary to package viral genomic RNA, transcribed from the viral vector genome, into viral particles. Alternatively, the viral vector genome may comprise one or more genes encoding viral components in addition to the one or more sequences, e.g., recombinant nucleic acids, of interest. In some aspects, in order to prevent replication of the genome in the target cell, however, endogenous viral genes required for replication are removed and provided separately in the packaging cell line.

In some instances, a packaging cell line is transfected with one or more plasmid vectors containing the components necessary to generate the particles. In some instances, a packaging cell line is transfected with a plasmid containing the viral vector genome, including the LTRs, the cis-acting packaging sequence and the sequence of interest, i.e. a nucleic acid encoding an antigen receptor, such as a CAR; and one or more helper plasmids encoding the virus enzymatic and/or structural components, such as Gag, pol and/or rev. In some instances, multiple vectors are utilized to separate the various genetic components that generate the retroviral vector particles. In some such instances, providing separate vectors to the packaging cell reduces the chance of recombination events that might otherwise generate replication competent viruses. In some instances, a single plasmid vector having all of the retroviral components can be used.

In some instances, the retroviral vector particle, such as lentiviral vector particle, is pseudotyped to increase the transduction efficiency of host cells. For example, a retroviral vector particle, such as a lentiviral vector particle, in some instances is pseudotyped with a VSV-G glycoprotein, which provides a broad cell host range extending the cell types that can be transduced. In some instances, a packaging cell line is transfected with a plasmid or polynucleotide encoding a non-native envelope glycoprotein, such as to include xenotropic, polytropic or amphotropic envelopes, such as Sindbis virus envelope, GALV or VSV-G.

In some instances, the packaging cell line provides the components, including viral regulatory and structural proteins, that are required in trans for the packaging of the viral genomic RNA into lentiviral vector particles. In some instances, the packaging cell line may be any cell line that is capable of expressing lentiviral proteins and producing functional lentiviral vector particles. In some aspects, suitable packaging cell lines include 293 (ATCC CCL X), 293T, HeLA (ATCC CCL 2), D17 (ATCC CCL 183), MDCK (ATCC CCL 34), BHK (ATCC CCL-10) and Cf2Th (ATCC CRL 1430) cells.

In some instances, the packaging cell line stably expresses the viral protein(s). For example, in some aspects, a packaging cell line containing the gag, pol, rev and/or other structural genes but without the LTR and packaging components can be constructed. In some instances, a packaging cell line can be transiently transfected with nucleic acid molecules encoding one or more viral proteins along with the viral vector genome containing a nucleic acid molecule encoding a heterologous protein, and/or a nucleic acid encoding an envelope glycoprotein.

In some instances, the viral vectors and the packaging and/or helper plasmids are introduced via transfection or infection into the packaging cell line. The packaging cell line produces viral vector particles that contain the viral vector genome. Methods for transfection or infection are well known. Non-limiting examples include calcium phosphate, DEAE-dextran and lipofection methods, electroporation and microinjection.

When a recombinant plasmid and the retroviral LTR and packaging sequences are introduced into a special cell line (e.g., by calcium phosphate precipitation for example), the packaging sequences may permit the RNA transcript of the recombinant plasmid to be packaged into viral particles, which then may be secreted into the culture media. The media containing the recombinant retroviruses in some instances is then collected, optionally concentrated, and used for gene transfer. For example, in some aspects, after cotransfection of the packaging plasmids and the transfer vector to the packaging cell line, the viral vector particles are recovered from the culture media and titered by standard methods used by those of skill in the art.

In some instances, a retroviral vector, such as a lentiviral vector, can be produced in a packaging cell line, such as an exemplary HEK 293T cell line, by introduction of plasmids to allow generation of lentiviral particles. In some instances, a packaging cell is transfected and/or contains a polynucleotide encoding gag and pol, and a polynucleotide encoding a recombinant receptor, such as an antigen receptor, for example, a CAR. In some instances, the packaging cell line is optionally and/or additionally transfected with and/or contains a polynucleotide encoding a rev protein. In some instances, the packaging cell line is optionally and/or additionally transfected with and/or contains a polynucleotide encoding a non-native envelope glycoprotein, such as VSV-G. In some such instances, approximately two days after transfection of cells, *e.g.,* HEK 293T cells, the cell supernatant contains recombinant lentiviral vectors, which can be recovered and titered.

Recovered and/or produced retroviral vector particles can be used to transduce target cells using the methods as described. Once in the target cells, the viral RNA is reversetranscribed, imported into the nucleus and stably integrated into the host genome. One or two days after the integration of the viral RNA, the expression of the recombinant protein, e.g., antigen receptor, such as CAR, can be detected.

In some instances, the disclosed methods involve methods of transducing cells by contacting, e.g., incubating, a cell composition comprising a plurality of cells with a viral particle. In some instances, the cells to be transfected or transduced are or comprise primary cells obtained from a subject, such as cells enriched and/or selected from a subject.

In some instances, the concentration of cells to be transduced of the composition is from or from about 1.0 × 10⁵ cells/mL to 1.0 × 10⁸ cells/mL, such as at least or about at least or about 1.0 × 10⁵ cells/mL, 5 × 10⁵ cells/mL, 1 × 10⁶ cells/mL, 5 × 10⁶ cells/mL, 1 × 10⁷ cells/mL, 5 × 10⁷ cells/mL or 1 × 10⁸ cells/mL.

In some instances, the viral particles are provided at a certain ratio of copies of the viral vector particles or infectious units (IU) thereof, per total number of cells to be transduced (IU/cell). For example, in some instances, the viral particles are present during the contacting at or about or at least at or about 0.5, 1, 2, 3, 4, 5, 10, 15, 20, 30, 40, 50, or 60 IU of the viral vector particles per one of the cells.

In some instances, the titer of viral vector particles is between or between about 1 × 10⁶ IU/mL and 1 × 10⁸ IU/mL, such as between or between about 5 × 10⁶ IU/mL and 5 × 10⁷ IU/mL, such as at least 6 × 10⁶ IU/mL, 7 × 10⁶ IU/mL, 8 × 10⁶ IU/mL, 9 × 10⁶ IU/mL, 1 × 10⁷ IU/mL, 2 × 10⁷ IU/mL, 3 × 10⁷ IU/mL, 4 × 10⁷ IU/mL, or 5 ×10⁷ IU/mL.

In some instances, transduction can be achieved at a multiplicity of infection (MOI) of less than 100, such as generally less than 60, 50, 40, 30, 20, 10, 5 or less.

In some instances, the method involves contacting or incubating, the cells with the viral particles. In some instances, the contacting is for 30 minutes to 72 hours, such as 30 minute to 48 hours, 30 minutes to 24 hours or 1 hour to 24 hours, such as at least or about at least 30 minutes, 1 hour, 2 hours, 6 hours, 12 hours, 24 hours, 36 hours or more.

In some instances, contacting is performed in solution. In some instances, the cells and viral particles are contacted in a volume of from or from about 0.5 mL to 500 mL, such as from or from about 0.5 mL to 200 mL, 0.5 mL to 100 mL, 0.5 mL to 50 mL, 0.5 mL to 10 mL, 0.5 mL to 5 mL, 5 mL to 500 mL, 5 mL to 200 mL, 5 mL to 100 mL, 5 mL to 50 mL, 5 mL to 10 mL, 10 mL to 500 mL, 10 mL to 200 mL, 10 mL to 100 mL, 10 mL to 50 mL, 50 mL to 500 mL, 50 mL to 200 mL, 50 mL to 100 mL, 100 mL to 500 mL, 100 mL to 200 mL or 200 mL to 500 mL.

In certain instances, the input cells are treated, incubated, or contacted with particles that comprise binding molecules that bind to or recognize the recombinant receptor that is encoded by the viral DNA.

In some instances, the incubation of the cells with the viral vector particles results in or produces an output composition comprising cells transduced with the viral vector particles.

### 2. Non-viral vectors

In some instances, recombinant nucleic acids are transferred into T cells via electroporation (*see,* e.g., Chicaybam et al, (2013) PLoS ONE 8(3): e60298 and Van Tedeloo et al. (2000) Gene Therapy 7(16): 1431-1437). In some instances, recombinant nucleic acids are transferred into T cells via transposition (see, e.g., Manuri et al. (2010) Hum Gene Ther 21(4): 427-437; Sharma et al. (2013) Molec Ther Nucl Acids 2, e74; and Huang et al. (2009) Methods Mol Biol 506: 115-126). Other methods of introducing and expressing genetic material in immune cells include calcium phosphate transfection (e.g., as described in Current Protocols in Molecular Biology, John Wiley & Sons, New York. N.Y.), protoplast fusion, cationic liposomemediated transfection; tungsten particle-facilitated microparticle bombardment (Johnston, Nature, 346: 776-777 (1990)); and strontium phosphate DNA co-precipitation (Brash et al., Mol. Cell Biol., 7: 2031-2034 (1987)).

Other approaches and vectors for transfer of the nucleic acids encoding the recombinant products are those described, e.g., in international patent application, Publication No.: WO2014055668, and U.S. Patent No. 7,446,190.

In some instances, the cells, e.g., T cells, may be transfected either during or after expansion e.g. with a T cell receptor (TCR) or a chimeric antigen receptor (CAR). This transfection for the introduction of the gene of the desired receptor can be carried out with any suitable retroviral vector, for example. The genetically modified cell population can then be liberated from the initial stimulus (the CD3/CD28 stimulus, for example) and subsequently be stimulated with a second type of stimulus e.g. via a de novo introduced receptor). This second type of stimulus may include an antigenic stimulus in form of a peptide/MHC molecule, the cognate (cross-linking) ligand of the genetically introduced receptor (e.g. natural ligand of a CAR) or any ligand (such as an antibody) that directly binds within the framework of the new receptor (e.g. by recognizing constant regions within the receptor). See, for example, Cheadle et al, "Chimeric antigen receptors for T-cell based therapy" Methods Mol Biol. 2012; 907:645-66 or Barrett et al., Chimeric Antigen Receptor Therapy for Cancer Annual Review of Medicine Vol. 65: 333-347 (2014).

In some cases, a vector may be used that does not require that the cells, *e.g.,* T cells, are activated. In some such instances, the cells may be selected and/or transduced prior to activation. Thus, the cells may be engineered prior to, or subsequent to culturing of the cells, and in some cases at the same time as or during at least a portion of the culturing.

In some aspects, the cells further are engineered to promote expression of cytokines or other factors. Among additional nucleic acids, e.g., genes for introduction are those to improve the efficacy of therapy, such as by promoting viability and/or function of transferred cells; genes to provide a genetic marker for selection and/or evaluation of the cells, such as to assess *in vivo* survival or localization; genes to improve safety, for example, by making the cell susceptible to negative selection in vivo as described by Lupton S. D. et al., Mol. and Cell Biol., 11:6 (1991); and Riddell et al., Human Gene Therapy 3:319-338 (1992); see also the publications of PCT/US91/08442 and PCT/US94/05601 by Lupton et al. describing the use of bifunctional selectable fusion genes derived from fusing a dominant positive selectable marker with a negative selectable marker. *See,* e.g., Riddell et al., US Patent No. 6,040,177, at columns 14-17.

In some instances, recombinant nucleic acids are transferred into T cells via transposons. Transposons (transposable elements), are mobile segments of DNA that can move from one locus to another within genomes. These elements move via a conservative, "cut-and-paste" mechanism: the transposase catalyzes the excision of the transposon from its original location and promotes its reintegration elsewhere in the genome. Transposase-deficient elements can be mobilized if the transposase is provided by another transposase gene. Thus, transposons can be utilized to incorporate a foreign DNA into a host genome without the use of a viral transduction system. Examples of transposons suitable for use with mammalian cells, e.g., human primary leukocytes, include but are not limited to Sleeping Beauty and Piggybac.

Transposon-based transfection is a two-component system consisting of a transposase and a transposon. In some instances, the system comprises a transposon is engineered to comprise a foreign DNA (also referred herein as cargo DNA), e.g., a gene encoding a recombinant receptor, that is flanked by inverted repeat/direct repeat (IR/DR) sequences that are recognized by an accompanying tranposase. In some instances, a non-viral plasmid encodes a transposase under the control of a promoter. Transfection of the plasmid into a host cell results in a transitory expression of the transposase, thus for an initial period following transfection, the transposase is expressed at sufficient levels to integrate the transposon into the genomic DNA. In some instances, the transposase itself is not integrated into the genomic DNA, and therefor expression of the transposase decreases over time. In some instances, the transposase expression is expressed by the host cell at levels sufficient to integrate a corresponding transposon for less than about 4 hours, less than about 8 hours, less than about 12 hours, less than about 24 hours, less than about 2 days, less than about 3 days, less than about 4 days, less than about 5 days, less than about 6 days, less than about 7 days, less than about 2 weeks, less than about 3 weeks, less than about 4 weeks, less than about 5 weeks, or less than about 8 weeks. In some instances, the cargo DNA that is introduced into the host's genome is not subsequently removed from the host's genome, at least because the host dose not express an endogenous transposase capable of excising the cargo DNA.

Sleeping Beauty (SB) is a synthetic member of the Tc/1-mariner superfamily of transposons, reconstructed from dormant elements harbored in the salmonid fish genome. SB transposon-based transfection is a two-component system consisting of a transposase and a transposon containing inverted repeat/direct repeat (IR/DR) sequences that result in precise integration into a TA dinucleotide. The transposon is designed with an expression cassette of interest flanked by IR/DRs. The SB transposase binds specific binding sites that are located on the IR of the Sleeping beauty transposon. The SB transposase mediates integration of the transposon, a mobile element encoding a cargo sequence flanked on both sides by inverted terminal repeats that harbor binding sites for the catalytic enzyme (SB). Stable expression results when SB inserts gene sequences into vertebrate chromosomes at a TA target dinucleotide through a cut-and-paste mechanism. This system has been used to engineer a variety of vertebrate cell types, including primary human peripheral blood leukocytes. In some instances, the cells are contacted, incubated, and/or treated with an SB transposon comprising a cargo gene, e.g., a gene encoding a recombinant receptor or a CAR, flanked by SB IR sequences. In particular instances, the cells to be transfected are contacted, incubated, and/or treated with a plasmid comprising an SB transposon comprising a cargo gene, e.g., a gene encoding a CAR, flanked by SB IR sequences. In certain instances, the plasmid further comprises a gene encoding an SB transposase that is not flanked by SB IR sequences.

PiggyBac (PB) is another transposon system that can be used to integrate cargo DNA into a host's, e.g., a human's, genomic DNA. The PB transposase recognizes PB transposonspecific inverted terminal repeat sequences (ITRs) located on both ends of the transposon and efficiently moves the contents from the original sites and efficiently integrates them into TTAA chromosomal sites. The PB transposon system enables genes of interest between the two ITRs in the PB vector to be mobilized into target genomes. The PB system has been used to engineer a variety of vertebrate cell types, including primary human cells. In some instances, the cells to be transfected are contacted, incubated, and/or treated with an PB transposon comprising a cargo gene, e.g., a gene encoding a CAR, flanked by PB IR sequences. In particular instances, the cells to be transfected are contacted, incubated, and/or treated with a plasmid comprising a PB transposon comprising a cargo gene, e.g., a gene encoding a CAR, flanked by PB IR sequences. In certain instances, the plasmid further comprises a gene encoding an SB transposase that is not flanked by PB IR sequences.

In some instances, the various elements of the transposon/transposase the employed in the subject methods, e.g., SB or PB vector(s), may be produced by standard methods of restriction enzyme cleavage, ligation and molecular cloning. One protocol for constructing the subject vectors includes the following steps. First, purified nucleic acid fragments containing desired component nucleotide sequences as well as extraneous sequences are cleaved with restriction endonucleases from initial sources, e.g., a vector comprising the transposase gene. Fragments containing the desired nucleotide sequences are then separated from unwanted fragments of different size using conventional separation methods, e.g., by agarose gel electrophoresis. The desired fragments are excised from the gel and ligated together in the appropriate configuration so that a circular nucleic acid or plasmid containing the desired sequences, e.g., sequences corresponding to the various elements of the subject vectors, as described above is produced. Where desired, the circular molecules so constructed are then amplified in a prokaryotic host, e.g., *E. coli.* The procedures of cleavage, plasmid construction, cell transformation and plasmid production involved in these steps are well known to one skilled in the art and the enzymes required for restriction and ligation are available commercially. (See, for example, R. Wu, Ed., Methods in Enzymology, Vol. 68, Academic Press, N.Y. (1979); T. Maniatis, E. F. Fritsch and J. Sambrook, Molecular Cloning: A Laboratory Manual, Cold Spring Harbor Laboratory Press, Cold Spring Harbor, N.Y. (1982); Catalog 1982-83, New England Biolabs, Inc.; Catalog 1982-83, Bethesda Research Laboratories, Inc. An example of how to construct the vectors employed in the subject methods is provided in the Experimental section, infra. The preparation of a representative Sleeping Beauty transposon system is also disclosed in WO 98/40510 and WO 99/25817).

In some instances, transduction with transposons is performed with a plasmid that comprises a transposase gene and a plasmid that comprises a transposon that contains a cargo DNA sequence that is flanked by inverted repeat/direct repeat (IR/DR) sequences that are recognized by the transposase. In certain instances, the cargo DNA sequence encodes a heterologous protein, e.g., a recombinant T cell receptor or a CAR. In some instances, the plasmid comprises transposase and the transposon. In some instances, the transposase is under control of a ubiquitous promoter, or any promoter suitable to drive expression of the transposase in the target cell. Ubiquitous promoters include, but are not limited to, EF1a, CMB, SV40, PGK1, Ubc, human β-actin, CAG, TRE, UAS, Ac5, CaMKIIa, and U6. In some instances, the cargo DNA comprises a selection cassette allowing for the selection of cells with stable integration of the cargo DNA into the genomic DNA. Suitable selection cassettes include, but are not limited to, selection cassettes encoding a kanamycin resistance gene, spectinomycin resistance gene, streptomycin resistance gene, ampicillin resistance gene, carbenicillin resistance gene, hygromycin resistance gene, bleomycin resistance gene, erythromycin resistance gene, and polymyxin B resistance gene.

In some instances, the components for transduction with a transposon, e.g., plasmids comprising an SB transposase and SB transposon, are introduced into the target cell. Any convenient protocol may be employed, where the protocol may provide for in vitro or in vivo introduction of the system components into the target cell, depending on the location of the target cell. For example, where the target cell is an isolated cell, the system may be introduced directly into the cell under cell culture conditions permissive of viability of the target cell, e.g., by using standard transformation techniques. Such techniques include, but are not necessarily limited to: viral infection, transformation, conjugation, protoplast fusion, electroporation, particle gun technology, calcium phosphate precipitation, direct microinjection, viral vector delivery, and the like. The choice of method is generally dependent on the type of cell being transformed and the circumstances under which the transformation is taking place (i.e. in vitro, ex vivo, or in vivo). A general discussion of these methods can be found in Ausubel, et al, Short Protocols in Molecular Biology, 3rd ed., Wiley & Sons,1995.

In some instances, the SB transposon and the SB transposase source are introduced into a target cell of a multicellular organism, e.g., a mammal or a human, under conditions sufficient for excision of the inverted repeat flanked nucleic acid from the vector carrying the transposon and subsequent integration of the excised nucleic acid into the genome of the target cell. Some instances further comprise a step of ensuring that the requisite transposase activity is present in the target cell along with the introduced transposon. Depending on the structure of the transposon vector itself, i.e. whether or not the vector includes a region encoding a product having transposase activity, the method may further include introducing a second vector into the target cell which encodes the requisite transposase activity.

In some instances, the amount of vector nucleic acid comprising the transposon and the amount of vector nucleic acid encoding the transposase that is introduced into the cell is sufficient to provide for the desired excision and insertion of the transposon nucleic acid into the target cell genome. As such, the amount of vector nucleic acid introduced should provide for a sufficient amount of transposase activity and a sufficient copy number of the nucleic acid that is desired to be inserted into the target cell. The amount of vector nucleic acid that is introduced into the target cell varies depending on the efficiency of the particular introduction protocol that is employed, e.g., the particular ex vivo administration protocol that is employed.

Once the vector DNA has entered the target cell in combination with the requisite transposase, the nucleic acid region of the vector that is flanked by inverted repeats, i.e. the vector nucleic acid positioned between the Sleeping Beauty transposase recognized inverted repeats, is excised from the vector via the provided transposase and inserted into the genome of the targeted cell. As such, introduction of the vector DNA into the target cell is followed by subsequent transposase mediated excision and insertion of the exogenous nucleic acid carried by the vector into the genome of the targeted cell. In particular instances, the vector is integrated into the genomes of at least 1%, at least 2%, at least 3%, at least 4%, at least 5%, at least 6% at least 7% at least 8%, at least 9%, at least 10%, at least 15%, or at least 20% of the cells that are transfected with the SB transposon and/or SB transposase. In some instances, integration of the nucleic acid into the target cell genome is stable, i.e., the vector nucleic acid remains present in the target cell genome for more than a transient period of time and is passed on a part of the chromosomal genetic material to the progeny of the target cell.

In certain instances, the transposons are used to integrate nucleic acids, i.e. polynucleotides, of various sizes into the target cell genome. In some instances, the size of DNA that is inserted into a target cell genome using the subject methods ranges from about 0.1 kb to 200 kb, from about 0.5 kb to 100 kb, from about 1.0 kb to about 8.0 kb, from about 1.0 to about 200 kb, from about 1.0 to about 10 kb, from about 10 kb to about 50 kb, from about 50 kb to about 100 kb, or from about 100 kb to about 200 kb. In some instances, the size of DNA that is inserted into a target cell genome using the subject methods ranges from about from about 1.0 kb to about 8.0 kb. In some instances, the size of DNA that is inserted into a target cell genome using the subject methods ranges from about 1.0 to about 200 kb. In particular instances, the size of DNA that is inserted into a target cell genome using the subject methods ranges from about 1.0 kb to about 8.0 kb.

### D. Cultivation and/or Expansion of Cells

In some instances, the disclosed methods include one or more steps for cultivating cells, e.g., cultivating cells under conditions that promote proliferation and/or expansion. In some instances, cells are cultivated under conditions that promote proliferation and/or expansion subsequent to a step of genetically engineering, e.g., introducing a recombinant polypeptide to the cells by transduction or transfection. In particular instances, the cells are cultivated after the cells have been incubated under stimulating conditions and transduced or transfected with a recombinant polynucleotide, e.g., a polynucleotide encoding a recombinant receptor.

In certain instances, the one or more compositions of engineered T cells are or include two separate compositions of enriched T cells. In particular instances, two separate compositions of enriched T cells, e.g., two separate compositions of enriched T cells selected, isolated, and/or enriched from the same biological sample, are separately cultivated under stimulating conditions. In certain instances, the two separate compositions include a composition of enriched CD4+ T cells. In particular instances, the two separate compositions include a composition of enriched CD8+ T cells. In some instances, two separate compositions of enriched CD4+ T cells and enriched CD8+ T cells are separately cultivated, e.g., under conditions that promote proliferation and/or expansion. In some instances, a single composition of enriched T cells is cultivated. In certain instances, the single composition is a composition of enriched CD4+ T cells. In some instances, the single composition is a composition of enriched CD4+ and CD8+ T cells that have been combined from separate compositions prior to the cultivation.

In some instances, the composition of enriched CD4+ T cells that is cultivated, e.g., under conditions that promote proliferation and/or expansion, includes at least 60%, at least 65%, at least 70%, at least 75%, at least 80%, at least 85%, at least 90%, at least 95%, at least 98%, at least 99%, at least 99.5%, at least 99.9%, or at or at about 100% CD4+ T cells. In some instances, the composition includes at least 30%, at least 40%, at least 50%, at least 60%, at least 70%, at least 80%, at least 90%, at least 95%, at least 98%, at least 99%, at least 99.5%, at least 99.9%, or at or at about 100% CD8+ T cells that express the recombinant receptor and/or have been transduced or transfected with the recombinant polynucleotide. In certain instances, the composition of enriched CD4+ T cells that is cultivated includes less than 40%, less than 35%, less than 30%, less than 25%, less than 20%, less than 15%, less than 10%, less than 5%, less than 1%, less than 0.1%, or less than 0.01% CD8+ T cells, and/or contains no CD8+ T cells, and/or is free or substantially free of CD8+ T cells.

In some instances, the composition of enriched CD8+ T cells that is cultivated, e.g., under conditions that promote proliferation and/or expansion, includes at least 60%, at least 65%, at least 70%, at least 75%, at least 80%, at least 85%, at least 90%, at least 95%, at least 98%, at least 99%, at least 99.5%, at least 99.9%, or at or at about 100% CD8+ T cells. In particular instances, the composition includes at least 30%, at least 40%, at least 50%, at least 60%, at least 70%, at least 80%, at least 90%, at least 95%, at least 98%, at least 99%, at least 99.5%, at least 99.9%, or at or at about 100% CD8+ T cells that express the recombinant receptor and/or have been transduced or transfected with the recombinant polynucleotide. In certain instances, the composition of enriched CD8+ T cells that is incubated under stimulating conditions includes less than 40%, less than 35%, less than 30%, less than 25%, less than 20%, less than 15%, less than 10%, less than 5%, less than 1%, less than 0.1%, or less than 0.01% CD4+ T cells, and/or contains no CD4+ T cells, and/or is free or substantially free of CD4+ T cells.

In some instances, separate compositions of enriched CD4+ and CD8+ T cells are combined into a single composition and are cultivated, e.g., under conditions that promote proliferation and/or expansion. In certain instances, separate cultivated compositions of enriched CD4+ and enriched CD8+ T cells are combined into a single composition after the cultivation has been performed and/or completed.

In some instances, a composition of enriched T cells is cultivated under conditions that promote proliferation and/or expansion. In some instances, such conditions may be designed to induce proliferation, expansion, activation, and/or survival of cells in the population. In particular instances, the stimulating conditions can include one or more of particular media, temperature, oxygen content, carbon dioxide content, time, agents, e.g., nutrients, amino acids, antibiotics, ions, and/or stimulatory factors, such as cytokines, chemokines, antigens, binding partners, fusion proteins, recombinant soluble receptors, and any other agents designed to promote growth, division, and/or expansion of the cells.

In particular instances, the cells are cultivated in the presence of one or more cytokines. In particular instances, the one or more cytokines are recombinant cytokines. In some instances, the one or more cytokines are human recombinant cytokines. In certain instances, the one or more cytokines bind to and/or are capable of binding to receptors that are expressed by and/or are endogenous to T cells. In particular instances, the one or more cytokines is or includes a member of the 4-alpha-helix bundle family of cytokines. In some instances, members of the 4-alpha-helix bundle family of cytokines include, but are not limited to, interleukin-2 (IL-2), interleukin-4 (IL-4), interleukin-7 (IL-7), interleukin-9 (IL-9), interleukin 12 (IL-12), interleukin 15 (IL-15), granulocyte colony-stimulating factor (G-CSF), and granulocyte-macrophage colony-stimulating factor (GM-CSF).

In particular instances, the cells are not incubated, contacted, and/or exposed to an agent that inhibits mTOR activity, such as an agent described herein, e.g. in Section II, prior to the cultivation, e.g., a cultivation under conditions that promote proliferation and/or expansion.

In certain instances, at least a portion of the cultivation is performed in the presence of an agent that inhibits mTOR activity, such as an agent described herein, e.g. in Section II. In some instances, all of and/or the entire cultivation is performed in the presence of an agent that inhibits mTOR activity.

In particular instances, the cells are cultivated in the presence of an agent that inhibits mTOR activity. In certain instances, the agent that inhibits mTOR activity is an agent described herein, such as in Section II. In some instances, the agent that inhibits mTOR activity also inhibits the activity of an additional kinase. In certain instances, the agent that inhibits mTOR activity also inhibits phosphoinositiol-3 kinase (PI3K) activity. In certain instances, the agent selectively inhibits mTOR activity, e.g., does not detectably inhibit PI3K activity, and/or does not inhibit PI3K activity to the same extent as mTOR activity, at concentrations that are sufficient to inhibit mTOR activity. In some instances, the agent that inhibits mTOR activity inhibits kinase activity. In certain instances, the agent that inhibits mTOR activity inhibits mTORC1 and/or mTORC2 activity. In some instances, the agent that inhibits mTOR activity inhibits mTORC1 and mTORC2 kinase activity.

In some instances, the cells are cultivated with an agents selected from PI-103, SF1126, BGT226, XL765, PF-04691502, NVP-BEZ235, a pyrazolopyrimidine, Torin l, Torkinib (PP242), PP30, Ku-0063794, WAY-600 (Wyeth), WAY-687 (Wyeth), WAY-354 (Wyeth), AZD8055, rapamycin (sirolimus), temsirolimus (CC1779), everolimus (RAD001), deforolimus (AP23573), AZD8055 (AstraZeneca), and OSI-027 (OSI). In some instances, the cells are cultivated with an agent that has or includes a formula that is provided in Section II, e.g., Formula (I), Formula (II), or Formula (III). In some instances, the agent is Compound 155, Compound 246, or Compound 63.

In certain instances, the cells are cultivated in the presence of an agent that inhibits mTOR activity at a concentration that inhibits, reduces, and/or decreases mTOR activity. In some instances, concentration inhibits, reduced, and/or decreases one or more activities of mTOR by about or at least 25%, 30%, 40%, 50%, 60%, 70%, 80%, 85%, 90%, 95%, 97%, 98%, 99%, or 99.9%. In some instances, the concentration of the agent does not prevent primary T cells from proliferating and/or expanding. In some instances, the cells are cultivated in the presence of between 1 nM and 1 µM, between 1 nM and 100 nM, between 50 nM and 250 nM, between 100 nM and 250 nM, between 200 nM and 500 nM, between 50 nM and 250 nM, between 100 nM and 500 nM, between 500 nM and 1 µM, between 1 µM and 10 µM, or between 5 µM and 50 µM of the agent that inhibits mTOR activity. In certain instances, the cells are cultivated in the presence of, of about, or of at least 0.1 nM, 0.5 nM, 1 nM, 5 nM, 10 nM, 25 nM, 50 nM, 100 nM, 150 nM, 200 nM, 250 nM, 500 nM, 1 µM, 5 µM, 10 µM, 25 µM, 50 µM, or 100 µM of the agent that inhibits mTOR activity.

In some instances, the cells are cultivated in the presence of Compound 155. In certain instances, the cells are cultivated in the presence of between 1 nM and 1 µM, between 1 nM and 100 nM, between 50 nM and 200 nM, between 100 nM and 250 nM, or between 200 nM and 500 nM of Compound 155. In particular instances, the cells are cultivated in the presence of, of about, or of at least 10 nM, 25 nM, 50 nM, 100 nM, 150 nM, 200 nM, 250 nM, or 500 nM of Compound 155.

In certain instances, the cells are cultivated in the presence of Compound 246. In certain instances, the cells are cultivated in the presence of between 1 nM and 1 µM, between 1 nM and 100 nM, between 50 nM and 200 nM, between 100 nM and 250 nM, or between 200 nM and 500 nM of Compound 155. In certain instances, the cells are cultivated in the presence of, of about, or of at least 10 nM, 25 nM, 50 nM, 100 nM, 150 nM, 200 nM, 250 nM, or 500 nM of Compound 246.

In particular instances, the cells are cultivated in the presence of Compound 63. In certain instances, the cells are cultivated in the presence of between 1 nM and 1 µM, between 1 nM and 100 nM, between 50 nM and 200 nM, between 100 nM and 250 nM, or between 200 nM and 500 nM of Compound 63. In some instances, the cells are cultivated in the presence of, of about, or of at least 10 nM, 25 nM, 50 nM, 100 nM, 150 nM, 200 nM, 250 nM, or 500 nM of Compound 63.

In some instances, the cultivation is performed under conditions that generally include a temperature suitable for the growth of primary immune cells, such as human T lymphocytes, for example, at least about 25 degrees Celsius, generally at least about 30 degrees, and generally at or about 37 degrees Celsius. In some instances, the composition of enriched T cells is incubated at a temperature of 25 to 38°C, such as 30 to 37°C, for example at or about 37 °C ± 2 °C. In some instances, the incubation is carried out for a time period until the culture, e.g. cultivation or expansion, results in a desired or threshold density, number or dose of cells. In some instances, the incubation is greater than or greater than about or is for about or 24 hours, 48 hours, 72 hours, 96 hours, 5 days, 6 days, 7 days, 8 days, 9 days or more.

In particular instances, the cultivation is performed in a closed system. In certain instances, the cultivation is performed in a closed system under sterile conditions. In particular instances, the cultivation is performed in the same closed system as one or more steps of the disclosed systems. In some instances the composition of enriched T cells is removed from a closed system and placed in and/or connected to a bioreactor for the cultivation. Examples of suitable bioreactors for the cultivation include, but are not limited to, GE Xuri W25, GE Xuri W5, Sartorius BioSTAT RM 20 | 50, Finesse SmartRocker Bioreactor Systems, and Pall XRS Bioreactor Systems. In some instances, the bioreactor is used to perfuse and/or mix the cells during at least a portion of the cultivation step.

In some instances, the mixing is or includes rocking and/or motioning. In some cases, the bioreactor can be subject to motioning or rocking, which, in some aspects, can increase oxygen transfer. Motioning the bioreactor may include, but is not limited to rotating along a horizontal axis, rotating along a vertical axis, a rocking motion along a tilted or inclined horizontal axis of the bioreactor or any combination thereof. In some instances, at least a portion of the incubation is carried out with rocking. The rocking speed and rocking angle may be adjusted to achieve a desired agitation. In some instances the rock angle is 20°, 19°, 18°, 17°, 16°, 15°, 14°, 13°, 12°, 11°, 10°, 9°, 8°, 7°, 6°, 5°, 4°, 3°, 2° or 1°. In certain instances, the rock angle is between 6-16°. In other instances, the rock angle is between 7-16°. In other instances, the rock angle is between 8-12°. In some instances, the rock rate is 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40 rpm. In some instances, the rock rate is between 4 and 12 rpm, such as between 4 and 6 rpm, inclusive.

In some instances, the bioreactor maintains the temperature at or near 37°C and CO2 levels at or near 5% with a steady air flow at, at about, or at least 0.01 L/min, 0.05 L/min, 0.1 L/min, 0.2 L/min, 0.3 L/min, 0.4 L/min, 0.5 L/min, 1.0 L/min, 1.5 L/min, or 2.0 L/min or greater than 2.0 L/min. In certain instances, at least a portion of the cultivation is performed with perfusion, such as with a rate of 290 ml/day, 580 ml/day, and/or 1160 ml/day, e.g., depending on the timing in relation to the start of the cultivation and/or density of the cultivated cells. In some instances, at least a portion of the cell culture expansion is performed with a rocking motion, such as at an angle of between 5° and 10°, such as 6°, at a constant rocking speed, such as a speed of between 5 and 15 RPM, such as 6 RMP or 10 RPM.

### E. Formulating the Cells

In some instances, the disclosed methods for manufacturing, generating or producing a cell therapy and/or engineered cells may include formulation of cells, such as formulation of genetically engineered cells resulting from the disclosed processing steps prior to or after the incubating, engineering, and cultivating, and/or one or more other processing steps as described. In some instances, the disclosed methods associated with formulation of cells include processing transduced cells, such as cells transduced and/or expanded using the processing steps described above, in a closed system. In some instances, the dose of cells comprising cells engineered with a recombinant antigen receptor, e.g. CAR or TCR, is described as a composition or formulation, such as a pharmaceutical composition or formulation. Such compositions can be used in accord with the disclosed methods, such as in the prevention or treatment of diseases, conditions, and disorders, or in detection, diagnostic, and prognostic methods.

In some cases, the cells are processed in one or more steps (e.g. carried out in the centrifugal chamber and/or closed system) for manufacturing, generating or producing a cell therapy and/or engineered cells may include formulation of cells, such as formulation of genetically engineered cells resulting from the disclosed transduction processing steps prior to or after the culturing, e.g. cultivation and expansion, and/or one or more other processing steps as described. In some cases, the cells can be formulated in an amount for dosage administration, such as for a single unit dosage administration or multiple dosage administration. In some instances, the disclosed methods associated with formulation of cells include processing transduced cells, such as cells transduced and/or expanded using the processing steps described above, in a closed system.

In certain instances, one or more compositions of enriched T cells are formulated. In particular instances, one or more compositions of enriched T cells are formulated after the one or more compositions have been engineered and/or cultivated. In particular instances, the one or more compositions are input compositions. In some instances, the one or more input compositions have been previously cryofrozen and stored, and are thawed prior to the incubation.

In some instances, T cells, such as CD4+ and/or CD8+ T cells, generated by one or more of the processing steps are formulated. In some aspects, a plurality of compositions are separately manufactured, produced or generated, each containing a different population and/or sub-types of cells from the subject, such as for administration separately or independently, optionally within a certain period of time. For example, separate formulations of engineered cells containing different populations or sub-types of cells can include CD8⁺ and CD4⁺ T cells, respectively, and/or CD8+- and CD4+-enriched populations, respectively, e.g., CD4+ and/or CD8+ T cells each individually including cells genetically engineered to express the recombinant receptor. In some instances, at least one composition is formulated with comprises CD4+ T cells genetically engineered to express the recombinant receptor. In some instances, at least one composition is formulated with CD8+ T cells genetically engineered to express the recombinant receptor. In some instances, the administration of the dose comprises administration of a first composition comprising a dose of CD8+ T cells or a dose of CD4+ T cells and administration of a second composition comprising the other of the dose of CD4+ T cells and the CD8+ T cells. In some instances, a first composition comprising a dose of CD8+ T cells or a dose of CD4+ T cells is administered prior to the second composition comprising the other of the dose of CD4+ T cells and the CD8+ T cells. In some instances, the administration of the dose comprises administration of a composition comprising both of a dose of CD8+ T cells and a dose of CD4+ T cells.

In certain instances, the one or more compositions of enriched T cells are or include two separate compositions, e.g., separate engineered and/or cultivated compositions, of enriched T cells. In particular instances, two separate compositions of enriched T cells, e.g., two separate compositions of enriched T cells selected, isolated, and/or enriched from the same biological sample, are separately formulated. In certain instances, the two separate compositions include a composition of enriched CD4+ T cells. In particular instances, the two separate compositions include a composition of enriched CD8+ T cells. In some instances, two separate compositions of enriched CD4+ T cells and enriched CD8+ T cells are separately formulated. In some instances, a single composition of enriched T cells is formulated. In certain instances, the single composition is a composition of enriched CD4+ T cells. In some instances, the single composition is a composition of enriched CD4+ and CD8+ T cells that have been combined from separate compositions prior to the formulation.

In some instances, separate compositions of enriched CD4+ and CD8+ T cells are combined into a single composition and are formulated. In certain instances, separate formulated compositions of enriched CD4+ and enriched CD8+ T cells are combined into a single composition after the formulation has been performed and/or completed.

In some instances, the composition of enriched CD4+ T cells that is formulated, includes at least 60%, at least 65%, at least 70%, at least 75%, at least 80%, at least 85%, at least 90%, at least 95%, at least 98%, at least 99%, at least 99.5%, at least 99.9%, or at or at about 100% CD4+ T cells. In some instances, the composition includes at least 30%, at least 40%, at least 50%, at least 60%, at least 70%, at least 80%, at least 90%, at least 95%, at least 98%, at least 99%, at least 99.5%, at least 99.9%, or at or at about 100% CD4+ T cells that express a recombinant receptor and/or have been transduced or transfected with the recombinant polynucleotide. In certain instances, the composition of enriched CD4+ T cells that is formulated includes less than 40%, less than 35%, less than 30%, less than 25%, less than 20%, less than 15%, less than 10%, less than 5%, less than 1%, less than 0.1%, or less than 0.01% CD8+ T cells, and/or contains no CD8+ T cells, and/or is free or substantially free of CD8+ T cells.

In some instances, the composition of enriched CD8+ T cells that is formulated, e.g., under conditions that promote proliferation and/or expansion, includes at least 60%, at least 65%, at least 70%, at least 75%, at least 80%, at least 85%, at least 90%, at least 95%, at least 98%, at least 99%, at least 99.5%, at least 99.9%, or at or at about 100% CD8+ T cells. In certain instances, the composition includes at least 30%, at least 40%, at least 50%, at least 60%, at least 70%, at least 80%, at least 90%, at least 95%, at least 98%, at least 99%, at least 99.5%, at least 99.9%, or at or at about 100% CD8+ T cells that express the recombinant receptor and/or have been transduced or transfected with the recombinant polynucleotide. In certain instances, the composition of enriched CD8+ T cells that is incubated under stimulating conditions includes less than 40%, less than 35%, less than 30%, less than 25%, less than 20%, less than 15%, less than 10%, less than 5%, less than 1%, less than 0.1%, or less than 0.01% CD4+ T cells, and/or contains no CD4+ T cells, and/or is free or substantially free of CD4+ T cells.

In certain instances, the formulated cells are output cells. In some instances, a formulated composition of enriched T cells is an output composition of enriched T cells. In particular instances, the formulated CD4+ T cells and/or formulated CD8+ T cells are the output CD4+ and/or CD8+ T cells. In particular instances, a formulated composition of enriched CD4+ T cells is an output composition of enriched CD4+ T cells. In some instances, a formulated composition of enriched CD8+ T cells is an output composition of enriched CD8+ T cells.

In some instances, cells can be formulated into a container, such as a bag or vial.

In some instances, the cells are formulated in a pharmaceutically acceptable buffer, which may, in some aspects, include a pharmaceutically acceptable carrier or excipient. In some instances, the processing includes exchange of a medium into a medium or formulation buffer that is pharmaceutically acceptable or desired for administration to a subject. In some instances, the processing steps can involve washing the transduced and/or expanded cells to replace the cells in a pharmaceutically acceptable buffer that can include one or more optional pharmaceutically acceptable carriers or excipients. Exemplary of such pharmaceutical forms, including pharmaceutically acceptable carriers or excipients, can be any described below in conjunction with forms acceptable for administering the cells and compositions to a subject. The pharmaceutical composition in some instances contains the cells in amounts effective to treat or prevent the disease or condition, such as a therapeutically effective or prophylactically effective amount.

A "pharmaceutically acceptable carrier" refers to an ingredient in a pharmaceutical formulation, other than an active ingredient, which is nontoxic to a subject. A pharmaceutically acceptable carrier includes, but is not limited to, a buffer, excipient, stabilizer, or preservative.

In some aspects, the choice of carrier is determined in part by the particular cell and/or by the method of administration. Accordingly, there are a variety of suitable formulations. For example, the pharmaceutical composition can contain preservatives. Suitable preservatives may include, for example, methylparaben, propylparaben, sodium benzoate, and benzalkonium chloride. In some aspects, a mixture of two or more preservatives is used. The preservative or mixtures thereof are typically present in an amount of about 0.0001% to about 2% by weight of the total composition. Carriers are described, e.g., by Remington's Pharmaceutical Sciences 16th edition, Osol, A. Ed. (1980). Pharmaceutically acceptable carriers are generally nontoxic to recipients at the dosages and concentrations employed, and include, but are not limited to: buffers such as phosphate, citrate, and other organic acids; antioxidants including ascorbic acid and methionine; preservatives (such as octadecyldimethylbenzyl ammonium chloride; hexamethonium chloride; benzalkonium chloride; benzethonium chloride; phenol, butyl or benzyl alcohol; alkyl parabens such as methyl or propyl paraben; catechol; resorcinol; cyclohexanol; 3-pentanol; and m-cresol); low molecular weight (less than about 10 residues) polypeptides; proteins, such as serum albumin, gelatin, or immunoglobulins; hydrophilic polymers such as polyvinylpyrrolidone; amino acids such as glycine, glutamine, asparagine, histidine, arginine, or lysine; monosaccharides, disaccharides, and other carbohydrates including glucose, mannose, or dextrins; chelating agents such as EDTA; sugars such as sucrose, mannitol, trehalose or sorbitol; salt-forming counter-ions such as sodium; metal complexes (e.g. Zn-protein complexes); and/or non-ionic surfactants such as polyethylene glycol (PEG).

Buffering agents in some aspects are included in the compositions. Suitable buffering agents include, for example, citric acid, sodium citrate, phosphoric acid, potassium phosphate, and various other acids and salts. In some aspects, a mixture of two or more buffering agents is used. The buffering agent or mixtures thereof are typically present in an amount of about 0.001% to about 4% by weight of the total composition. Methods for preparing administrable pharmaceutical compositions are known. Exemplary methods are described in more detail in, for example, Remington: The Science and Practice of Pharmacy, Lippincott Williams & Wilkins; 21st ed. (May 1, 2005).

The formulations can include aqueous solutions. The formulation or composition may also contain more than one active ingredient useful for the particular indication, disease, or condition being treated with the cells, preferably those with activities complementary to the cells, where the respective activities do not adversely affect one another. Such active ingredients are suitably present in combination in amounts that are effective for the purpose intended. Thus, in some instances, the pharmaceutical composition further includes other pharmaceutically active agents or drugs, such as chemotherapeutic agents, e.g., asparaginase, busulfan, carboplatin, cisplatin, daunorubicin, doxorubicin, fluorouracil, gemcitabine, hydroxyurea, methotrexate, paclitaxel, rituximab, vinblastine, and/or vincristine.

Compositions in some instances are described as sterile liquid preparations, e.g., isotonic aqueous solutions, suspensions, emulsions, dispersions, or viscous compositions, which may in some aspects be buffered to a selected pH. Liquid compositions can comprise carriers, which can be a solvent or dispersing medium containing, for example, water, saline, phosphate buffered saline, polyol (for example, glycerol, propylene glycol, liquid polyethylene glycol) and suitable mixtures thereof. Sterile injectable solutions can be prepared by incorporating the cells in a solvent, such as in admixture with a suitable carrier, diluent, or excipient such as sterile water, physiological saline, glucose, dextrose, or the like. The compositions can contain auxiliary substances such as wetting, dispersing, or emulsifying agents (e.g., methylcellulose), pH buffering agents, gelling or viscosity enhancing additives, preservatives, flavoring agents, and/or colors, depending upon the route of administration and the preparation desired. Standard texts may in some aspects be consulted to prepare suitable preparations.

Various additives which enhance the stability and sterility of the compositions, including antimicrobial preservatives, antioxidants, chelating agents, and buffers, can be added. Prevention of the action of microorganisms can be ensured by various antibacterial and antifungal agents, for example, parabens, chlorobutanol, phenol, and sorbic acid. Prolonged absorption of the injectable pharmaceutical form can be brought about by the use of agents delaying absorption, for example, aluminum monostearate and gelatin.

In some instances, the formulation buffer contains a cryopreservative. In some instances, the cell are formulated with a cyropreservative solution that contains 1.0% to 30% DMSO solution, such as a 5% to 20% DMSO solution or a 5% to 10% DMSO solution. In some instances, the cryopreservation solution is or contains, for example, PBS containing 20% DMSO and 8% human serum albumin (HSA), or other suitable cell freezing media. In some instances, the cryopreservative solution is or contains, for example, at least or about 7.5% DMSO. In some instances, the processing steps can involve washing the transduced and/or expanded cells to replace the cells in a cryopreservative solution. In some instances, the cells are frozen, e.g., cryofrozen or cryopreserved, in media and/or solution with a final concentration of or of about 12.5%, 12.0%, 11.5%, 11.0%, 10.5%, 10.0%, 9.5%, 9.0%, 8.5%, 8.0%, 7.5%, 7.0%, 6.5%, 6.0%, 5.5%, or 5.0% DMSO, or between 1% and 15%, between 6% and 12%, between 5% and 10%, or between 6% and 8% DMSO. In particular instances, the cells are frozen, e.g., cryofrozen or cryopreserved, in media and/or solution with a final concentration of or of about 5.0%, 4.5%, 4.0%, 3.5%, 3.0%, 2.5%, 2.0%, 1.5%, 1.25%, 1.0%, 0.75%, 0.5%, or 0.25% HSA, or between 0.1% and 5%, between 0.25% and 4%, between 0.5% and 2%, or between 1% and 2% HSA.

In some instances, the formulation is carried out using one or more processing step including washing, diluting or concentrating the cells, such as the cultured or expanded cells. In some instances, the processing can include dilution or concentration of the cells to a desired concentration or number, such as unit dose form compositions including the number of cells for administration in a given dose or fraction thereof. In some instances, the processing steps can include a volume-reduction to thereby increase the concentration of cells as desired. In some instances, the processing steps can include a volume-addition to thereby decrease the concentration of cells as desired. In some instances, the processing includes adding a volume of a formulation buffer to transduced and/or expanded cells. In some instances, the volume of formulation buffer is from or from about 10 mL to 1000 mL, such as at least or about at least or about or 50 mL, 100 mL, 200 mL, 300 mL, 400 mL, 500 mL, 600 mL, 700 mL, 800 mL, 900 mL or 1000 mL.

In some instances, such processing steps for formulating a cell composition is carried out in a closed system. Exemplary of such processing steps can be performed using a centrifugal chamber in conjunction with one or more systems or kits associated with a cell processing system, such as a centrifugal chamber produced and sold by Biosafe SA, including those for use with the Sepax^{®} or Sepax 2^{®} cell processing systems. An exemplary system and process is described in International Publication Number WO2016/073602. In some instances, the method includes effecting expression from the internal cavity of the centrifugal chamber a formulated composition, which is the resulting composition of cells formulated in a formulation buffer, such as pharmaceutically acceptable buffer, in any of the above instances as described. In some instances, the expression of the formulated composition is to a container, such as the vials of the biomedical material vessels described herein, that is operably linked as part of a closed system with the centrifugal chamber. In some instances, the biomedical material vessels are configured for integration and or operable connection and/or is integrated or operably connected, to a closed system or device that carries out one or more processing steps. In some instances, the biomedical material vessel is connected to a system at an output line or output position. In some cases, the closed system is connected to the vial of the biomedical material vessel at the inlet tube. Exemplary close systems for use with the biomedical material vessels described herein include the Sepax^{®} and Sepax^{®} 2 system.

In some instances, the closed system, such as associated with a centrifugal chamber or cell processing system, includes a multi-port output kit containing a multi-way tubing manifold associated at each end of a tubing line with a port to which one or a plurality of containers can be connected for expression of the formulated composition. In some aspects, a desired number or plurality of vials, can be sterilely connected to one or more, generally two or more, such as at least 3, 4, 5, 6, 7, 8 or more of the ports of the multi-port output. For example, in some instances, one or more containers, e.g., biomedical material vessels, can be attached to the ports, or to fewer than all of the ports. Thus, in some instances, the system can effect expression of the output composition into a plurality of vials of the biomedical material vessels.

In some aspects, cells can be expressed to the one or more of the plurality of output containers, e.g., vials, in an amount for dosage administration, such as for a single unit dosage administration or multiple dosage administration. For example, in some instances, the vials, may each contain the number of cells for administration in a given dose or fraction thereof. Thus, each vial, in some aspects, may contain a single unit dose for administration or may contain a fraction of a desired dose such that more than one of the plurality of vials, such as two of the vials, or 3 of the vials, together constitute a dose for administration.

Thus, the containers, e.g. bags or vials, generally contain the cells to be administered, e.g., one or more unit doses thereof. The unit dose may be an amount or number of the cells to be administered to the subject or twice the number (or more) of the cells to be administered. It may be the lowest dose or lowest possible dose of the cells that would be administered to the subject.

In some instances, each of the containers, e.g. bags or vials, individually comprises a unit dose of the cells. Thus in some instances, each of the containers comprises the same or approximately or substantially the same number of cells. In some instances, each unit dose contains at least or about at least 1 × 10⁶, 2 × 10⁶, 5 × 10⁶, 1 × 10⁷, 5 × 10⁷, or 1 × 10⁸ engineered cells, total cells, T cells, or PBMCs. In some instances, the volume of the formulated cell composition in each container, e.g. bag or vial, is 10 mL to 100 mL, such as at least or about at least 20 mL, 30 mL, 40 mL, 50 mL, 60 mL, 70 mL, 80 mL, 90 mL or 100 mL. In some instances, the cells in the container, e.g. bag or vials, can be cryopreserved. In some instances, the container, e.g. vials, can be stored in liquid nitrogen until further use.

In some instances, such cells produced by the method, or a composition comprising such cells, are administered to a subject for treating a disease or condition.

### F. Exemplary Features of the Output Composition

In particular instances, the disclosed methods are used in connection with a process that produces or generates one or more output compositions of enriched T cells from one or more input compositions and/or from a single biological sample. In certain instances, the one or more output compositions contain cells that express a recombinant receptor, e.g., a TCR or a CAR. In some instances, the process involves an incubation, engineering, and/or cultivation of cells in the presence of an agent that inhibits mTOR activity, such as any as described, e.g. Compound 63. In particular instances, the cells of the output compositions are suitable for administration to a subject as a therapy, e.g., an autologous cell therapy.

In some instances, the cells of the output composition are engineered to express a recombinant receptor by the methods disclosed herein, such as described above. In certain instances, the cells of the output composition are engineered to express a chimeric antigen receptor (CAR), e.g., an anti-CD19 CAR.

In some instances, the one or more output composition is a composition of enriched CD4+ and CD8+ T cells. In certain instances, the one or more output compositions include a composition of enriched CD4+ T cells. In particular instances, the one or more output compositions include a composition of enriched CD8+ T cells. In some instances, the one or more output compositions includes an output composition of enriched CD4+ T cells and an output composition of enriched CD8+ T cells.

In some instances, an output composition of enriched CD4+ T cells includes at least 60%, at least 65%, at least 70%, at least 75%, at least 80%, at least 85%, at least 90%, at least 95%, at least 98%, at least 99%, at least 99.5%, at least 99.9%, or at or at about 100% CD4+ T cells. In certain instances, the output composition includes at least 30%, at least 40%, at least 50%, at least 60%, at least 70%, at least 80%, at least 90%, at least 95%, at least 98%, at least 99%, at least 99.5%, at least 99.9%, or at or at about 100% CD4+ T cells that express the recombinant receptor and/or have been transduced or transfected with the recombinant polynucleotide. In certain instances, the output composition of enriched CD4+ T cells includes less than 40%, less than 35%, less than 30%, less than 25%, less than 20%, less than 15%, less than 10%, less than 5%, less than 1%, less than 0.1%, or less than 0.01% CD8+ T cells, and/or contains no CD8+ T cells, and/or is free or substantially free of CD8+ T cells.

In some instances, an output composition of enriched CD8+ T cells includes at least 60%, at least 65%, at least 70%, at least 75%, at least 80%, at least 85%, at least 90%, at least 95%, at least 98%, at least 99%, at least 99.5%, at least 99.9%, or at or at about 100% CD8+ T cells. In particular instances, the composition includes at least 30%, at least 40%, at least 50%, at least 60%, at least 70%, at least 80%, at least 90%, at least 95%, at least 98%, at least 99%, at least 99.5%, at least 99.9%, or at or at about 100% CD8+ T cells that express the recombinant receptor and/or have been transduced or transfected with the recombinant polynucleotide. In certain instances, the output composition of enriched CD8+ T cells includes less than 40%, less than 35%, less than 30%, less than 25%, less than 20%, less than 15%, less than 10%, less than 5%, less than 1%, less than 0.1%, or less than 0.01% CD4+ T cells, and/or contains no CD4+ T cells, and/or is free or substantially free of CD4+ T cells.

In certain instances, the process associated with the disclosed methods is compared to an exemplary and/or alternative process. In some instances, the alternative and/or exemplary process is similar or the same as the process associated with the disclosed methods, with the exception that that compositions of cells (e.g., input cells, stimulated cells, and/or engineered cells that included enriched CD4+ T cells, enriched CD8+ T cells, and/or enriched CD4+ and CD8+ T cells) are not incubated, engineered, and/or cultivated in the presence of an mTOR inhibitor, e.g. Compound 63. In some instances, the output cells of the exemplary alternative process are not previously cultivated, e.g., to expand engineered T cells, in the presence of an agent that inhibits mTOR activity, e.g. Compound 63. In some instances, the output cells produced by the exemplary, alternative process are engineered to express the same recombinant receptor as the cells of the out composition produced in association with the disclosed methods.

In some instances, one or more genes are differentially expressed by the cells of the output composition as compared to expression in output cells of an exemplary alternative process, e.g., a process whereby the cells are not cultivated in the presence of an mTOR inhibitor, e.g. Compound 63. In some instances, one or more genes are down regulated in cells of the output composition as compared to output cells produced by an exemplary alternative process. In some instances, one or more genes associated with metabolic stress response, T cell activation, Th1 and Th2 activation pathway, cell cycle arrest, glucocorticoid biosynthesis, hematopoiesis from pluripotent stem cells, T cell activation, lymphocyte differentiation, leukocyte migration, cysteine-type endopeptidase inhibitor activity, cell cycle progression, response to nutrient levels, and growth factor receptor signaling are downregulated in cells from the output composition as compared to output cells produced by the exemplary alternative process.

In certain instances, one or more genes are upregulated in cells of the output composition as compared to output cells produced by an exemplary alternative process, such as a process not carried out in the presence of an agent that inhibits mTOR, e.g. Compound 63. In some instances, one or more genes associated with growth factor receptor signaling, fatty acid oxidation, common-gamma cytokine receptor signaling pathway, protein deglycosylation, T cell activation, cell-cycle progression lymphocyte differentiation, Th1 and Th2 activation pathway, sterol homeostasis, hematopoiesis from pluripotent stem cells, apoptotic process, T cell activation, and RAR activation, and ion-mediated signaling are upregulated in cells from the output composition as compared to output cells produced by the exemplary alternative process.

In some instances, the output composition contains cells, e.g., CD4+ and/or CD8+ T cells engineered to express a recombinant receptor, that have the same or greater response to stimulation by an antigen, e.g., an antigen that is bound by and/or recognized by the recombinant receptor, as compared to output cells produced by an exemplary, alternative process, e.g., a process where cells are not cultivated in the presence of an mTOR inhibitor, e.g. Compound 63. In some instances, cells of the output composition have the same or greater, and/or are capable of producing the same or greater increase in glycolytic metabolism, mTOR activity, cytokine production, cytolytic activity, expansion and/or proliferation in response to stimulation by the antigen as compared to output cells produced by the exemplary, alternative process.

In some instances, the output cells have a similar response with respect to a parameter, attribute, and/or activity as output cells produced by an exemplary/alternative process (e.g. a process where the cells are not incubated, engineered, and/or cultivated in the presence of an mTOR inhibitor, e.g. Compound 63). In some instances, a measurement of the similar response of the output cells is not statistically different from a measurement of the response by output cells produced by the exemplary, alternative process. In some instances, the measurement of the similar response of the output cells is within 25%, 20%, 10%, 5%, or 1% of the measurement of the response by output cells produced by the exemplary, alternative process.

In certain instances, changes in cellular metabolism, e.g., the rate glycolytic metabolism, may function as a driver and/or a regulator of immune cell function. In some instances, the cells of the output composition have a similar increase in the rate of glycolytic metabolism by antigen stimulation as output cells produced by an exemplary, alternative process (e.g., a process where the cells are not cultivated in the presence of an mTOR inhibitor, e.g. Compound 63). In some instances, the cells of the output composition have, have about, or have at least a 10%, 20%, 25%, 30%, 40%, 50%, 60%, 70%, 75%, 80%, 90%, 100%, 125%, 150%, 1-fold, 2-fold, 3-fold, 4-fold, or 5-fold increase in the rate of glycolytic metabolism in response to stimulation by an antigen. In certain instances, the increase in glycolytic metabolism in response to antigen stimulation is at least 5%, 10%, 15%, 20%, 25%, 50%, or 100% greater than the increase in the rate of glycolytic metabolism by antigen stimulation in output cells produced by the exemplary, alternative process. In certain instances, glycolytic metabolism may be measured by any known means, including by extracellular measurement of oxygen consumption and acid production (ECAR).

In particular instances, the cells of the output composition have a similar increase of mTOR activity by antigen stimulation as output cells produced by an exemplary, alternative process (e.g., a process where the cells are not cultivated in the presence of an mTOR inhibitor, e.g. Compound 63). In some instances, the cells of the output composition have, have about, or have at least a 25%, 50%, 75%, 100%, 125%, 150%, 1-fold, 2-fold, 3-fold, 4-fold, or 5-fold increase in mTOR activity in response to stimulation by an antigen. In certain instances, the increased mTOR activity in response to antigen stimulation is at least 25%, 50%, 75%, or 100% greater than the increase in the mTOR activity by antigen stimulation in output cells produced by the exemplary, alternative process.

In certain instances, the cells of the output composition have a similar cytokine production in response to antigen-stimulation as output cells produced by an exemplary, alternative process (e.g., a process where the cells are not cultivated in the presence of an mTOR inhibitor, e.g. Compound 63). In some instances, the cells of the output composition have a similar production of a cytokine, e.g., TNF-alpha, IFN-gamma, and/or IL-2, in response to antigen-stimulation as output cells produced by the exemplary, alternative process. In some instances, the cells of the output composition have, have about, or have at least a 50%, 60%, 70%, 75%, 80%, 90%, 100%, 125%, 150%, 1-fold, 2-fold, 3-fold, 4-fold, or 5-fold increase in the production of one or more cytokines in response to stimulation by an antigen compared to an alternative process not carried out in the presence of an agent that inhibits mTOR, e.g. Compound 63. In certain instances, the increased mTOR activity in response to antigen stimulation is at least 5%, 10%, 15%, 20%, 25%, 50%, or 100% greater than the production of the one or more cytokines following antigen stimulation in output cells produced by the exemplary, alternative process. In some instances, the production of a cytokine may be measured or assessed by standard known techniques, including but not limited to ELISA and/or antibody based detection methods.

In particular instances, the cells of the output composition have a similar portion, percentage, and/or amount of cells that produce one or more cytokines in response to antigen-stimulation as the portion, percentage, and/or amount of the output cells produced by an exemplary, alternative process (e.g., a process where the cells are not cultivated in the presence of an mTOR inhibitor, e.g. Compound 63). In certain instances, about or at least 10%, 20%, 25%, 30%, 40%, 50%, 60%, 70%, 75%, 80%, 90%, 95%, or 100% of the cells of the output composition produce the one or more cytokines, e.g., TNF-alpha, IFN-gamma, and/or IL-2, in response to antigen-stimulation. In particular instances, the portion, percentage, and/or amount of cells of the output composition that produce the one or more cytokines is about or at least 5%, 10%, 15%, 20%, 25%, 30%, 35%, 40%, 45%, 50%, 75%, 100%, 125%, 150%, or 1-fold, 2-fold, 3-fold, greater than the portion, percentage, and/or amount of output cells produced by the exemplary, alternative process that produce the one or more cytokines. In certain instances, the portion, percentage, and/or amount of the cells that produce a cytokine may be measured or assessed by any known or standard technique, including intracellular cytokine staining (ICS) assays.

In particular instances, the cells of the output composition have a similar cytolytic activity towards cells expressing an antigen bound by and/or recognized by the recombinant receptor (e.g., target cells) as output cells produced by an exemplary, alternative process (e.g., a process where the cells are not incubated, engineered, and/or cultivated in the presence of an mTOR inhibitor, e.g. Compound 63). In some instances, when the cells of the output composition are exposed to the cells that express the antigen, e.g., the target cells, the cells of the output composition kill, kill about, or kill at least 25%, 30%, 35%, 40%, 45%, 50%, 55%, 60%, 70%, 75%, 80%, 85%, 90%, 95%, or 100% of cells that express the antigen. In certain instances, the cells of the output composition kill at least 25%, 50%, 75%, 100%, 150%, or 1-fold, 2-fold, 3-fold, 4-fold, or 5-fold greater amount of cells that express the antigen, e.g., target cells, than output cells produced by an exemplary alternative process under similar or the same conditions.

In particular instances, the cells of the output composition have a lower, reduced, and/or decreased portion, percentage, and/or amount of cells that express one or more markers of exhaustion as compared to the portion, percentage, and/or amount of the output cells produced by an exemplary, alternative process (e.g., a process where the cells are not cultivated in the presence of an mTOR inhibitor, e.g. Compound 63). In certain instances, less than or about 40%, 35%, 30%, 25%, 20%, 15%, 10%, 5%, 1%, or 0.1% of the cells of the output composition express one or more markers of exhaustion. In certain instances, the portion, percentage, and/or amount of cells that express one or more markers of exhaustion in the output composition is or is at least 10%, 20%, 30%, 40%, 50%, 60%, 70%, 80%, or 90% less than the portion, percentage, and/or amount of cells that express the one or more markers in an output composition produced by the exemplary, alternative process. In particular instances, the one or more markers of exhaustion is or includes CTLA-4, FOXP3, PD-1, TIGIT, LAB-3, 2B4, BTLA, TIM3, VISTA, and/or CD96.

In various instances, the cells of the output composition have a lower, reduced, and/or decreased portion, percentage, and/or amount of cells that are differentiated as compared to the portion, percentage, and/or amount of the output cells produced by an exemplary, alternative process (e.g., a process where the cells are not cultivated in the presence of an mTOR inhibitor, e.g. Compound 63). In certain instances, the cells of the output composition are less differentiated than cells produced by alternative methods. In particular instances, the less differentiated cells of the output composition have or include a greater capacity for stimulation, activation, expansion, cytokine response, cytolytic activity, or anti-tumor activity than more differentiated cells produced by an exemplary, alternative process.

In some instances, the disclosed methods produce an output composition of cells that are increased in the number or percentage of memory-like T cells, such as a less-differentiated, long-lived population T cells such as long-lived memory T cells. In some instances, such memory T cells are central memory T cells (T_{CM}) or T memory stem cells (T_{SCM}) cells. In some instances, the memory T cells are T_{SCM} cells. In some instances, the cells of the output composition have an increased or greater number or percentage of cells that have a memory-like phenotype, such as long-lived memory T cells. In some instances, the number or percentage of memory-like T cells, such as long-lived memory T cells or memory stem cells (T_{SCM}), in the composition is increased at least 2-fold, 3-fold, 4-fold, 5-fold, 6-fold, 7-fold, 8-fold, 9-fold or 10-fold compared to the number or percentage of the corresponding population of output cells produced by an exemplary, alternative process (e.g., a process where the cells are not cultivated in the presence of an mTOR inhibitor, e.g. Compound 63).

In certain instances, the cells of the output composition are administered to a subject. In some instances, the cells of the output composition are administered to treat a disease or condition. In some instances, the disease or condition is cancer. In some instances, the cells the output compositions are administered to the subject, and the subject experiences a reduction in cancer cells and/or tumor volume. In some instances, the subject has, has about, or has at least a 25%, 50%, 60%, 70%, 75%, 80%, 85%, 90%, 95%, 97%, 98%, 99%, 100% reduction of the amount of cancer cells and/or tumor reduction following administration of the cells of the output composition, e.g., as compared to the amount of cancer cells and/or the tumor volume in the subject prior to the administration. In some instances, administration of cells of the output composition results in an increased reduction of tumor volume and/or the amount of cancer cells in the subject as compared to the reduction of tumor volume and/or the amount of cancer cells in the subject following administration of output cells produced by an exemplary alternative process (e.g. a process where the cells are not cultivated in the presence of an mTOR inhibitor, e.g. Compound 63). In particular instances, administration of cells of the output composition results in an increase in the reduction of tumor volume and/or the amount of cancer cells in the subject of, of about, or of at least 5%, 10%, 20%, 30%, 40%, 50%, 60%, 70%, 80%, 90%, 100%, 125%, 150%, 1-fold, 2-fold, 3-fold, 4-fold, of 5-fold, as compared to the reduction of tumor volume and/or the amount of cancer cells in the subject following administration of output cells produced by the exemplary alternative process.

In particular instances, the cells of the output compositions, e.g., engineered cells expressing a recombinant receptor, are detectable in a subject, e.g., detectable in biological samples such as serum samples obtained from a subject, following administration. In certain instances, the cells of the output composition, are detectable in subjects at or at least at 1, 2, 3, 4, 5, 6, 7, or 8 weeks following, or at or at least 3, 6, 12, 18, 24, 30, or 36 months, or at or at least 1, 2, 3, 4, 5, or more years following the administration of the cells of the output composition. In some instances, administration of cells of the output composition results in an increased or enhanced persistence *in vivo* following administration as compared to the output cells produced by an exemplary alternative process (e.g. a process where the cells are not cultivated in the presence of an mTOR inhibitor, e.g. Compound 63). In particular instances, administration of cells of the output composition are detectable in a subject for, for about, or for at least 1, 2, 3, 4, 5, 6, 7, or 8 weeks, or for, for about, or for at least 3, 6, 12, 18, 24, 30, or 36 months, or for, for about, or for at least 1, 2, 3, or more years longer than output cells produced by an exemplary alternative process.

In some instances, administering the cells of the output composition to a subject, e.g., a subject with a disease or condition such as a cancer, improves the probability and/or likelihood of survival. For example, in some instances, the cells of the output composition are administered to a subject with disease or condition, the probability and/or likelihood of survival over, over about, or over at least 2, 3, 4, 5, 6, 7, 8, 9, 10, 11, or 12 months, or over 1, 2, 3, 4, 5, 10, or more than 10 years is at least 30%, 40%, 50%, 60%, 70%, 80%, 90%, or 100%. In certain instances, the administration with the cells of the output composition provides at least a 10%, 20%, 25%, 30%, 40%, 50%, 60%, 70%, 80%, 90%, 100%, 125%, 150%, or at least a 1-fold, 2-fold, 3-fold, 4-fold, or 5-fold greater probability and/or likelihood of survival than administration with output cells of an exemplary alternative process (e.g. a process where the cells are not incubated, engineered, and/or cultivated in the presence of an mTOR inhibitor, e.g. Compound 63).

### II. AGENTS THAT INHIBIT MTOR ACTIVITY

In some instances, one or more steps of the disclosed methods are carried out in the presence of an agent that inhibits mTOR activity. mTOR is also known as mammalian target of rapamycin, mechanistic target of rapamycin, FK506-binding protein 12-rapamycin complex-associated protein 1, FKBP12-rapamycin complex-associated protein, Rapamycin and FKBP12 target 1, Rapamycin target protein 1, FRAP, FRAP1, FRAP2, RAFT1, and RAPT1. In some aspects, the human mTOR protein corresponds to Uniprot No.: P42345. In some instances, the amino acid sequence of the human mTOR protein is set forth in SEQ ID NO: 34. In some instances, an agent that inhibits mTOR activity inhibits, reduces, and/or decreases, and/or is capable of inhibiting, reducing, and/or decreasing at least one activity of mTOR. In particular instances, an agent that inhibits mTOR activity inhibits, reduces, and/or decreases, and/or is capable of inhibiting, reducing, and/or decreasing an mTOR kinase activity.

In some aspects, mTOR is a conserved threonine and serine protein kinase and belongs to the family of phosphatidylinositol-3-kinase-related kinases (PIKKs). mTOR is a protein kinase that phosphorylates threonine and serine residues in its substrates. In certain aspects, mTOR serves as the catalytic subunits of two multi-protein complexes termed as the mTOR complex 1 (mTORC1) and complex 2 (mTORC2). In particular aspects, mTORC1 and mTORC2 function independently from each other, despite that fact that, in certain aspect, both mTORC1 and mTORC2 are involved in the phosphoinositol-3 kinase (PI3K) and Akt signaling pathway. In some instances, an agent that inhibits mTOR activity inhibits, reduces, and/or decreases, and/or is capable of inhibiting, reducing, and/or decreasing an mTORC1 activity, e.g., an mTORC1 kinase activity, and/or an mTORC2 activity.

In some aspects, mTORC1 is a protein complex with five components: mTOR, which is the catalytic subunit of the complex; regulatory-associated protein of mTOR (Raptor); mammalian lethal with Sec13 protein 8 (mLST8, also known as GβL); proline-rich AKT substrate 40 kDa (PRAS40); and DEP-domain-containing mTOR-interacting protein (Deptor). In some instances, the agent that inhibits mTOR activity prevents the formation of and/or destabilizes the mTORC1 complex.

In certain aspects, mTORC2 comprises six different proteins, several of which are common to mTORC1 and mTORC2: mTOR; rapamycin-insensitive companion of mTOR (Rictor); mammalian stress-activated protein kinase interacting protein (mSIN1); protein observed with Rictor-1 (Protor-1); mLST8; and Deptor. In particular instances, the agent that inhibits activity prevents the formation of and/or destabilizes the mTORC2 complex.

In some instances, the agent that inhibits mTOR activity is a compound, a small molecule, e.g., small organic molecule, a polynucleotide, an oligonucleotide, an siRNA, a polypeptide, or a fragment, isoform, variant, analog, or derivative thereof that inhibits, reduces, prevents, and/or is capable of inhibiting, reducing, or preventing, one or more activities of mTOR. In some instances, the agent is a small molecule. In particular instances, the agent is a small molecule with a molecular weight of less than 10 kD, less than 9 kD, less than 8 kD, less than 7 kD, less than 6 kD, less than 5 kD, less than 4 kD, less than 3 kD, less than 2 kD, less than 1 kD, less than 0.5 kD, or less than 0.1 kD. In some instances, the agent is a small molecule that is or contains nucleic acids, peptides, polypeptides, peptidomimetics, peptoids, carbohydrates, lipids, components thereof or other organic or inorganic molecules. Libraries of chemical and/or biological mixtures, such as fungal, bacterial, or algal extracts, are known in the art and can be screened with any of the assays of the invention. Examples of methods for the synthesis of molecular libraries can be found in: (Carell et al, 1994a; Carell et al, 1994b; Cho et al , 1993; DeWitt et al, 1993; Gallop et al, 1994; Zuckermann et al, 1994).

In particular instances, the agent that inhibits mTOR activity specifically and/or selectively inhibits at least one mTOR activity. In various instances, that agent that inhibits mTOR activity inhibits at least one activity of an mTOR protein, such as, for example, the serine/threonine protein kinase activity on at least one of its substrates, e.g., p70S6 kinase 1, 4E-BP1, Akt, and eEF2. In some instances, the agent that inhibits mTOR activity binds directly to and inhibits, and/or is capable of binding directly to and inhibiting, mTORCl, mTORC2, or both mTORCl and mTORC2. In some instances, inhibition of mTOR activity by the agent is irreversible. In certain instances, inhibition of mTOR activity by the agent is reversible.

In certain instances, the agent that inhibits mTOR activity has an IC₅₀ of less than 500 µM, less than 200 µM, less than 100 µM, less than 50 µM, less than 10 µM, less than 5 µM, less than 1 µM, less than 500 nM, less than 200 nM, less than 100 nM, less than 50 nM, less than 10 nM, less than 5 nM, less than 1 nM, or less than 500 pM. In certain instances, the agent that inhibits mTOR activity has an IC₅₀ of between 1 nM and 500 µM, between 1 nM and 500 nM, between 1 µM and 500 µM, between 10 µM and 100 µM, between 100 nM and 1 µM, between 250 nM and 750 nM, between 50 nM and 200 nM, or between 400 nM and between 600 nM. In some instances, IC₅₀ determinations can be accomplished using any known standard and/or conventional techniques. For example, in some instances, an IC₅₀ can be determined by measuring the mTOR activity in the presence of a range of concentrations of the inhibitor under study. The experimentally obtained values of enzyme activity then are plotted against the inhibitor concentrations used. The concentration of the inhibitor that shows 50% enzyme activity (as compared to the activity in the absence of any inhibitor) is taken as the "IC₅₀" value. Analogously, other inhibitory concentrations can be defined through appropriate determinations of activity. In some instances, the IC₅₀ is measured in a cell free assay. In particular instances, the IC₅₀ is measured in a cell culture assay. In certain instances, the cell culture is a T cell culture, e.g., a primary T cell culture.

In some instances, inhibition of mTORCl and/or mTORC2 activity can be determined by a reduction in signal transduction downstream of mTORC1 and/or mTORC2. A wide variety of readouts can be utilized to establish a reduction of the output of such signaling pathway. For example, in some instances, non-limiting exemplary readouts include for mTORC2 activity include (1) a decrease in phosphorylation of Akt at residues, including but not limited to S473 and T308 and/or (2) a decrease in activation of Akt as evidenced, for example, by a reduction of phosphorylation of Akt substrates including but not limited to Fox01 and Fox03a T24/32, GSK3-beta S21/9, and TSC2 T1462. In certain instances, non-limiting exemplary readouts of mTORC1 activity include a decrease in phosphorylation of signaling molecules downstream of mTORC1, including but not limited to ribosomal S6 S240/244, S6K1 T389, and 4EBP1 T37/46. In certain instances, an exemplary readout of mTORC1 and/or mTORC2 inhibition is the inhibition of proliferation of cancerous cells.

Measuring, detecting, and/or assessing proteins with site-specific phosphorylation can be performed by any known means, including, but not limited to, antibody staining techniques and immunoassays, enzyme-linked immunosorbent assay (ELISA), enzyme immunoassay (EIA), radioimmunoassay (RIA), surface plasmon resonance (SPR), Western Blot, or protein array.

In some instances, the agent that inhibits mTOR activity may also inhibit kinases that are structurally similar to mTOR and/or have the same or substantially similar activities as mTOR (a pan-inhibitor). In certain instances, the agent that inhibits mTOR activity also inhibits phosphoinositol-3 kinase activity (PI3K). In certain instances, the agent that inhibits mTOR activity inhibits mTORC1 activity, mTORC2 activity, and PI3K activity. In particular instances, the agent that inhibits mTOR activity inhibits the kinase activity of mTORC1, mTORC2, and PI3K. In particular aspects, a wide variety of readouts can be utilized to establish a reduction of the PI3K activity. In some instances, such readouts include, but are not limited to, (1) a decrease in phosphorylation of Akt at residues, including but not limited to S473 and T308 and/or (2) a decrease in activation of Akt as evidenced, for example, by a reduction of phosphorylation of Akt substrates including but not limited to Fox01 and Fox03a T24/32, GSK3-beta S21/9, and TSC2 T1462, and/or (3) a reduction of the amount, level, or concentration of phosphatidylinositol (3,4,5) trisphosphates (PIP3).

In some instances, the agent that inhibits mTOR activity, e.g., mTORC1 and/or mTORC2 kinase activity also inhibits PI3K. In some instances, the agent that inhibits mTOR activity inhibits the activity of PI3K, mTORCl, and mTORC2 with an IC₅₀ (concentration that inhibits 50% of the activity) of less than 500 µM, less than 200 µM, less than 100 µM, less than 50 µM, less than 10 µM, less than 5 µM, less than 1 µM, less than 500 nM, less than 200 nM, less than 100 nM, less than 50 nM, less than 10 nM, less than 5 nM, less than 1 nM, or less than 500 pM. In certain instances, the agent that inhibits mTOR activity inhibits the activity of PI3K, mTORCl, and mTORC2 with an IC₅₀ of between 1 nM and 500 µM, between 1 nM and 500 nM, between 1 µM and 500 µM, between 1 nM and 1 µM, between 10 µM and 100 µM, between 100 nM and 1 µM, between 250 nM and 750 nM, between 50 nM and 200 nM, or between 400 nM and between 600 nM. In some instances, the IC₅₀ is measured in a cell free assay. In particular instances, the IC₅₀ is measured in a cell culture assay. In certain instances, the cell culture is a T cell culture, e.g., a primary T cell culture. In certain instances, such agents include, but are not limited to, PI-103, SF1126 (Semafore), BGT226 (Novartis), XI,765 (Exelixis), PF-04691502, and NVP-BEZ235 (Novartis). In certain instances, the agent that inhibits mTOR activity is PI-103, SF1126, BGT226, XI,765, PF-04691502, and NVP-BEZ235.

In some instances, the agent that inhibits mTOR activity does not inhibit PI3K activity. In certain instances, the agent does not detectably reduce, inhibit, or decrease PI3K activity at the IC₅₀ for mTOR activity. In particular instances, the agent that inhibits mTOR activity has an IC₅₀ for PI3K activity that is at least 50%, at least 60%, at least 70%, at least 75%, at least 80%, at least 85%, at least 90%, at least 100%, at least 150%, at least 1-fold, at least 2-fold, at least 3-fold, at least 4-fold, at least 5-fold, at least 10-fold, at least 50-fold, or at least 100-fold greater than the IC₅₀ for an mTOR activity. In some instances, the agent that inhibits mTOR activity inhibits, e.g., selectively inhibits, mTORC1 and mTORC2 kinase activity relative to PI3K activity. In certain instances, the inhibitor of mTOR activity is a pyrazolopyrimidine, Torin l, Torkinib (PP242), PP30, Ku-0063794, WAY-600 (Wyeth), WAY-687 (Wyeth), WAY-354 (Wyeth), or AZD8055.

In particular instances, the agent that inhibits mTOR activity selectively inhibits mTORC1 with an IC₅₀ of less than 500 µM, less than 200 µM, less than 100 µM, less than 50 µM, less than 10 µM, less than 5 µM, less than 1 µM, less than 500 nM, less than 200 nM, less than 100 nM, less than 50 nM, less than 10 nM, less than 5 nM, less than 1 nM, or less than 500 pM. In certain instances, the agent that inhibits mTOR activity inhibits the activity of mTORCl with an IC₅₀ of between 1 nM and 500 µM, between 1 nM and 500 nM, between 1 µM and 500 µM, between 10 µM and 100 µM, between 100 nM and 1 µM, between 250 nM and 750 nM, between 50 nM and 200 nM, or between 400 nM and between 600 nM. In some instances, the IC₅₀ is measured in a cell free assay. In particular instances, the IC₅₀ is measured in a cell culture assay. In certain instances, the cell culture is a T cell culture, e.g., a primary T cell culture.

In some instances, the agent that inhibits mTOR activity is selectively inhibits mTORC1 activity relative to mTORC2 and/or PI3K activity. In certain instances, the agent that inhibits mTOR activity has an IC₅₀ for PI3K activity that is at least 50%, at least 60%, at least 70%, at least 75%, at least 80%, at least 85%, at least 90%, at least 100%, at least 150%, at least 1-fold, at least 2-fold, at least 3-fold, at least 4-fold, at least 5-fold, at least 10-fold, at least 50-fold, or at least 100-fold greater than the IC₅₀ for an mTORC1 activity. In some instances, the agent is rapamycin (sirolimus). In particular instances, the agent is a rapalog.

In certain instances, the rapalog is binds to and/or is capable of binding to the mTOR FRB domain (FKBP rapamycin binding domain), is structurally related to rapamycin, and/or retains the mTORC1 inhibiting properties of rapamycin. In some instances, the rapalog is an ester, ether, oxime, hydrazone, and/or a hydroxylamine of rapamycin, and/or is a compounds in which functional groups on the rapamycin core structure have been modified, for example, by reduction or oxidation. Pharmaceutically acceptable salts of such compounds are also considered to be rapamycin derivatives. Illustrative examples of rapalogs suitable for use in the methods contemplated herein include, without limitation, temsirolimus (CC1779), everolimus (RAD001), deforolimus (AP23573), AZD8055 (AstraZeneca), and OSI-027 (OSI).

In some instances, the agent is a molecule that is described in PCT Pub. Nos.: WO2008/051493; WO2008/051494; or WO2010/062571; and/or U.S. Patent Nos.: 7,981,893; 8,372,976; 7,968,556; 8,383,634; 8,110,578; or 8,492,381.

In certain instances, the agent that inhibits mTOR activity has or includes the formula set forth in Formula (I): 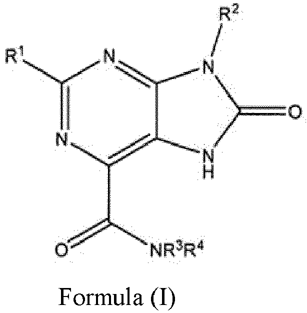
wherein R¹ is substituted or unsubstituted C₁₋₈alkyl, substituted or unsubstituted aryl, substituted or unsubstituted heteroaryl, substituted or unsubstituted cycloalkyl, or substituted or unsubstituted heterocycloalkyl,
R² is substituted or unsubstituted C₁₋₈alkyl, substituted or unsubstituted aryl, substituted or unsubstituted heteroaryl, substituted or unsubstituted cycloalkyl, or substituted or unsubstituted heterocycloalkyl, and
R³ and R⁴ are independently H or C₁₋₈ alkyl.

In some instances, the agent that inhibits mTOR is or contains a compound of Formula (I), or a pharmaceutically acceptable salt or solvate thereof. In some instances, the agent that inhibits mTOR is or contains a compound of formula (I) wherein R¹ is substituted aryl, substituted or unsubstituted heteroaryl, such as substituted phenyl. In certain instances, the agent that inhibits mTOR activity is or contains a compound of formula (I) are those wherein R² is substituted or unsubstituted aryl, such as substituted or unsubstituted phenyl. In particular instances, the agent that inhibits mTOR is or contains a compound of formula (I) wherein groups that are substituted are substituted with one or more halogen; C₁₋₈ alkyl; C₂₋₈ alkenyl; C₂₋₈ alkynyl; hydroxyl; C₁₋₈ alkoxyl; amino; nitro; thiol; thioether; imine; cyano; amido; phosphonato; phosphine; carboxyl; thiocarbonyl; sulfonyl; sulfonamide; ketone; aldehyde; ester; carbonyl; haloalkyl; B(OH)₂; carbocyclic cycloalkyl, heterocycloalkyl, monocyclic or fused or non-fused polycyclic aryl or heteroaryl; amino; O-lower alkyl; O-aryl, aryl; aryl-lower alkyl; CO₂CH₃; CONH₂; OCH₂CONH₂; NH₂; SO₂NH₂; OCHF₂; CF₃; or OCF₃ groups, wherein each of these groups is optionally substituted.

In some instances, the agent that has or includes the formula set forth in Formula (I) is Compound 63. In particular instances, the agent that inhibits mTOR activity is Compound 63. In some aspects, Compound 63 is 2-(3-Hydroxyphenyl)-9-(2-isopropylphenyl)-8-oxo-8,9-dihydro-7H-purine-6-carboxamide. In some aspects, the agent that inhibits mTOR activity is 2-(3-hydroxyphenyl)-9-(2-isopropylphenyl)-8-oxo-8,9-dihydro-7H-purine-6-carboxamide, or a pharmaceutically acceptable salt or solvate thereof. In particular aspects, Compound 63 has the formula:

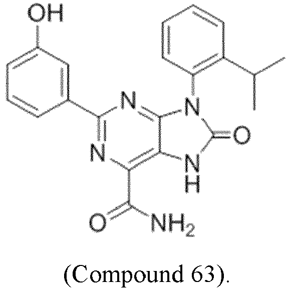

In particular instances, the agent that inhibits mTOR activity has or includes the formula set forth in Formula (II): 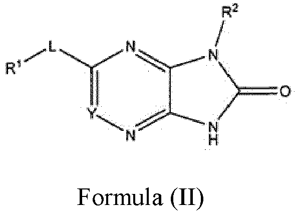
wherein L is a direct bond, NH or O,
Y is N or CR³,
wherein R¹ is H, substituted or unsubstituted C₁₋₈alkyl, substituted or unsubstituted C₂₋₈ alkenyl, substituted or unsubstituted aryl, substituted or unsubstituted heteroaryl, substituted or unsubstituted cycloalkyl or substituted or unsubstituted heterocycloalkyl,
R² is H, substituted or unsubstituted C₁₋₈alkyl, substituted or unsubstituted aryl, substituted or unsubstituted heteroaryl, substituted or unsubstituted cycloalkyl, or substituted or unsubstituted heterocycloalkyl,
R³ is H, substituted or unsubstituted C₁₋₈alkyl, substituted or unsubstituted aryl, substituted or unsubstituted heteroaryl, substituted or unsubstituted cycloalkyl, substituted or unsubstituted heterocycloalkyl, -NHR⁴ or -N(R⁴)₂, and
R⁴ is at each occurrence independently substituted or unsubstituted C₁₋₈alkyl, substituted or unsubstituted aryl, substituted or unsubstituted heteroaryl, substituted or unsubstituted cycloalkyl, or substituted or unsubstituted heterocycloalkyl.

In some instances, the agent that inhibits mTOR is or contains a compound of Formula (II), or a pharmaceutically acceptable salt or solvate thereof. In certain instances, the agent that inhibits mTOR activity has or incudes the formula set forth in Formula (II) wherein R¹ is substituted aryl, such as substituted phenyl. In particular instances, the agent that inhibits mTOR activity has or incudes the formula set forth in Formula (II) wherein Y is CH. In some instances, the agent that inhibits mTOR activity has or incudes the formula set forth in Formula (II) wherein L is a direct bond. In particular instances, the agent that inhibits mTOR activity has or incudes the formula set forth in Formula (II) wherein R¹ is substituted or unsubstituted aryl and R² is Ci-s alkyl substituted with one or more substituents selected from alkoxy, amino, hydroxy, cycloalkyl, or heterocycloalkyl.

In certain instances, the agent that inhibits mTOR activity has or incudes the formula set forth in Formula (II) wherein the groups that are "substituted or unsubstituted," when substituted, they may be substituted with one or more of any substituent. Examples of substituents are those found in the exemplary compounds and instances disclosed herein, as well as halo *(e.g.,* chloro, iodo, bromo, or fluoro); C₁₋₈ alkyl; C₂₋₈ alkenyl; C₂₋₈ alkynyl; hydroxyl; C₁₋₈ alkoxyl; amino; nitro; thiol; thioether; imine; cyano; amido; phosphonato; phosphine; carboxyl; carbamoyl; carbamate; acetal; urea; thiocarbonyl; sulfonyl; sulfonamide; sulfinyl; ketone; aldehyde; ester; acetyl; acetoxy; oxygen (=O); haloalkyl *(e.g.,* trifluoromethyl); substituted aminoacyl and aminoalkyl; carbocyclic cycloalkyl, which may be monocyclic or fused or nonfused polycyclic *(e.g*., cyclopropyl, cyclobutyl, cyclopentyl, or cyclohexyl), or a heterocycloalkyl, which may be monocyclic or fused or non-fused polycyclic *(e.g.,* pyrrolidinyl, piperidinyl, piperazinyl, morpholinyl, furanyl, or thiazinyl); carbocyclic or heterocyclic, monocyclic or fused or nonfused polycyclic aryl *(e.g.,* phenyl, naphthyl, pyrrolyl, indolyl, furanyl, thienyl, imidazolyl, oxazolyl, isoxazolyl, thiazolyl, triazolyl, tetrazolyl, pyrazolyl, pyridinyl, quinolinyl, isoquinolinyl, acridinyl, pyrazinyl, pyridazinyl, pyrimidinyl, benzimidazolyl, benzothienyl, or benzofuranyl); amino (primary, secondary, or tertiary); -O-lower alkyl; -0-aryl; aryl; aryl-lower alkyl; CO₂CH₃; CONH₂; OCH₂CONH₂; NH₂; N(C₁₋₄alkyl)₂; NHC(O)C₁₋₄alkyl;SO₂NH₂; SO₂C₁₋₄alkyl; OCHF₂; CF₃; OCF₃; and such moieties may also be optionally substituted by a fused-ring structure or bridge, for example -OCH₂0- or -0-lower alkylene-0-. These substituents may optionally be further substituted with a substituent selected from such groups.

In particular instances, the agent that has or includes the formula set forth in Formula (II) is Compound 155. In particular instances, the agent that inhibits mTOR activity is Compound 155. In some aspects, Compound 155 is 6-(4-(2H-1,2,4-Triazol-3-yl)phenyl)-1-(2-(tetrahydro-2H-pyran-4-yl)ethyl)-1H-imidazo [4,5-b]pyrazine-2(3H)-one. In some aspects, the agent that inhibits mTOR activity is 6-(4-(2H-1,2,4-triazol-3-yl)phenyl)-1-(2-(tetrahydro-2H-pyran-4-yl)ethyl)-1H-imidazo [4,5-b]pyrazine-2(3H)-one, or a pharmaceutically acceptable salt or solvate thereof. In particular aspects, Compound 155 has the formula:

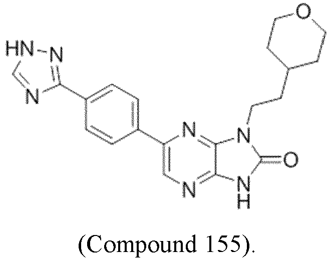

In particular instances, the agent that inhibits mTOR activity has or includes the formula set forth in Formula (III): 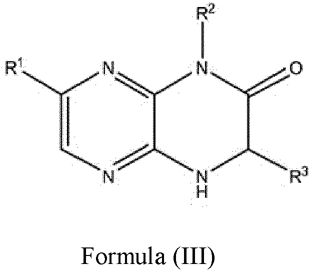
wherein R¹ is substituted or unsubstituted C₁₋₈ alkyl, substituted or unsubstituted aryl, substituted or unsubstituted cycloalkyl, substituted or unsubstituted heterocyclyl, or substituted or unsubstituted heterocyclylalkyl,
R² is H, substituted or unsubstituted C₁₋₈ alkyl, substituted or unsubstituted cycloalkyl, substituted or unsubstituted heterocyclyl, substituted or unsubstituted heterocyclylalkyl, substituted or unsubstituted aralkyl, or substituted or unsubstituted cycloalkylalkyl, and
R³ is H, or a substituted or unsubstituted Ci-s alkyl.

In some instances, the agent that inhibits mTOR is or contains a compound of Formula (III), or a pharmaceutically acceptable salt or solvate thereof. In some instances, the agent that inhibits mTOR activity has or includes a formula set forth in Formula (III) wherein R¹ is substituted or unsubstituted aryl or substituted or unsubstituted heteroaryl, such as for example, R¹ is phenyl, pyridyl, pyrimidyl, benzimidazolyl, 1H-pyrrolo[2,3-b ]pyridyl, indazolyl, indolyl, lH-imidazo[ 4,5-b]pyridyl, lH-imidazo[4,5-b]pyridin-2(3H)-onyl, 3H-imidazo[4,5-b]pyridyl, or pyrazolyl, each optionally substituted. In particular instances, the agent that inhibits mTOR activity has or includes a formula set forth in Formula (III) wherein R¹ is phenyl substituted with one or more substituents independently selected from the group consisting of substituted or unsubstituted C₁₋₈ alkyl (for example, methyl), substituted or unsubstituted heterocyclyl (for example, a substituted or unsubstituted triazolyl or pyrazolyl), aminocarbonyl, halogen (for example, fluorine), cyano, hydroxyalkyl and hydroxy. In other instances, R1 is pyridyl substituted with one or more substituents independently selected from the group consisting of substituted or unsubstituted C₁₋₈ alkyl (for example, methyl), substituted or unsubstituted heterocyclyl (for example, a substituted or unsubstituted triazolyl), halogen, aminocarbonyl, cyano, hydroxyalkyl (for example, hydroxypropyl), -OR, and -NR2, wherein each R is independently H, or a substituted or unsubstituted C₁₋₄ alkyl. In some instances, R¹ is 1H-pyrrolo[2,3-b]pyridyl or benzimidazolyl, optionally substituted with one or more substituents independently selected from the group consisting of substituted or unsubstituted Ci-s alkyl, and -NR₂, wherein R is independently H, or a substituted or unsubstituted C₁₋₄ alkyl.

In some instances, the agent that inhibits mTOR activity has or includes a formula set forth in Formula (III) wherein R¹ is 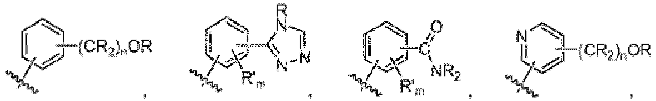 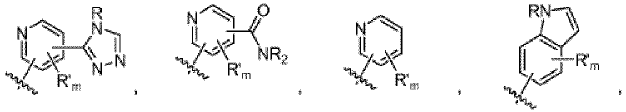 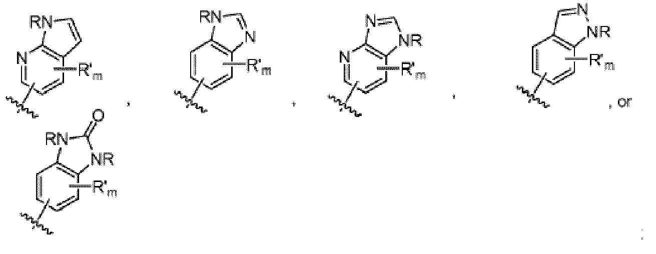 wherein R is at each occurrence independently H, or a substituted or unsubstituted C₁₋₄ alkyl (for example, methyl); R¹ is at each occurrence independently a substituted or unsubstituted C1-4 alkyl (for example, methyl), halogen (for example, fluoro ), cyano, -OR, or -NR₂; m is 0-3; and n is 0-3. It will be understood that any of the substituents R' may be attached to any suitable atom of any of the rings in the fused ring systems.

In some instances of compounds of formula (III), R² is H, substituted or unsubstituted C₁₋₈ alkyl, substituted or unsubstituted cycloalkyl, substituted or unsubstituted heterocyclyl, substituted or unsubstituted C₁₋₄ alkyl-heterocyclyl, substituted or unsubstituted C₁₋₄ alkyl-aryl, or substituted or unsubstituted C₁₋₄ alkyl-cycloalkyl. For example, R² is H, methyl, ethyl, n-propyl, isopropyl, n-butyl, sec-butyl, isobutyl, tert-butyl, n-pentyl, isopentyl, cyclopentyl, cyclohexyl, tetrahydrofuranyl, tetrahydropyranyl, (C₁₋₄ alkyl)-phenyl, (C₁₋₄ alkyl)-cyclopropyl, (C₁₋₄ alkyl)-cyclobutyl, (C₁₋₄ alkyl)-cyclopentyl, (C₁₋₄ alkyl)-cyclohexyl, (C₁₋₄ alkyl)-pyrrolidyl, (C₁₋₄ alkyl)-piperidyl, (C₁₋₄ alkyl)-piperazinyl, (C₁₋₄ alkyl)-morpholinyl, (C₁₋₄ alkyl)-tetrahydrofuranyl, or (C₁₋₄ alkyl)-tetrahydropyranyl, each optionally substituted.

In certain instances, R² is H, C1-4 alkyl, (C₁₋₄ alkyl)(OR), 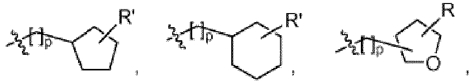 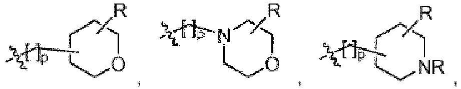 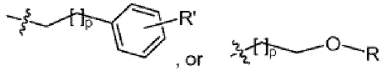 wherein R is at each occurrence independently H, or a substituted or unsubstituted C₁₋₈ alkyl, R' is at each occurrence independently H, -OR, cyano, or a substituted or unsubstituted C₁₋₈ alkyl, and p is 0-3.

In particular instances, the agent that has or includes the formula set forth in Formula (III) is Compound 246. In particular instances, the agent that inhibits mTOR activity is Compound 246. In some aspects, Compound 246 is 7-(6-(2-hydroxypropan-2-yl)pyridin-3-yl)-1-((1r,4r)-4-methoxycyclohexyl)-3,4-dihydropyrazino[2,3-b]pyrazin-2(1H)-one. In some aspects, the agent that inhibits mTOR activity is 7-(6-(2-hydroxypropan-2-yl)pyridin-3-yl)-1-((1r,4r)-4-methoxycyclohexyl)-3,4-dihydropyrazino[2,3-b]pyrazin-2(1H)-one, or a pharmaceutically acceptable salt or solvate thereof. In particular aspects, Compound 246 has the formula:

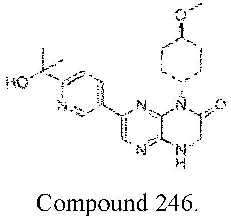

### III. METHODS FOR LONG TERM STIMULATION

Disclosed herein is a long term stimulation method (also referred to herein as a method for long term stimulation) that is useful, inter alia, for assessing phenotypes, characteristics, or activities of a cell composition, e.g., a cell composition. In some instances, long-term stimulation method is or includes incubating a cell composition, e.g., an input composition containing cells expressing a recombinant receptor such as a CAR, under conditions to stimulate a recombinant receptor-dependent activity (e.g., CAR-dependent activity) in the cells. In such instances, a recombinant receptor-dependent activity is an activity that is specific to stimulation of the recombinant receptor, e.g. CAR, such as via the presence of an antigen or other agent recognized by the antigen binding domain of the recombinant receptor, e.g. CAR, or that specifically stimulates the recombinant receptor. In some aspects, the cell composition. e.g, the input composition, contains T cells expressing a recombinant receptor (e.g., a CAR) comprising an extracellular antigen-binding domain that specifically binds or recognizes an antigen. In some aspects, the incubation results in a T cell composition, e.g., an output compositions, containing cells that exhibit features of chronically stimulated cells or of cells having prolonged exposure to antigen.

In certain instances, the conditions to stimulate a recombinant receptor activity, e.g., a CAR dependent activity, includes incubating the cell composition, e.g., the input composition, with a binding molecule, such as a binding molecule that binds, e.g., specifically binds, to the antigen binding domain of the recombinant receptor, e.g., the CAR. In certain instances, the binding molecule is attached to a support. In particular instances, the support is a solid support, such as the surface of a cell culture plate or dish, a well in a microplate, or the surface of a particle or bead. In some aspects, the incubation takes place or is carried out in the microplate a cell culture plate or dish, or well in a microplate with surface attached binding molecules. In some aspects, the incubation takes place or is carried out in the presence of a plurality of particles or beads that contain binding molecules. In certain aspects, the binding molecules are surface attached to the beads or particles. In some instances, the incubation is performed or carried out in vitro or ex vivo.

In various instances, the cells, e.g., cells of an input composition, are incubated with the binding molecules in the presence of a media without additional agents that promote cell division, growth, expansion, or activation. In some instances, the cells are incubated with the particles for an extended amount of time. e.g., 14 days, without any additional manipulations, e.g., media changes, bead replacement, or splitting or replating the cells. In certain aspects, the

In some aspects, the long-term stimulation methods includes incubating an input composition, e.g., a cell composition containing a recombinant receptor-expressing cell composition in the presence of a binding molecule that binds or recognizes the recombinant receptor. In some aspects, the length of time chosen for the incubation is a time at which one or more functions or activities of cells of the composition exhibits features of chronically stimulated cells or cells having prolonged exposure to antigen at the termination or end of the incubation. In some instances, the binding molecule is an antigen (such as a recombinant antigen or fragment thereof) that is bound by or is recognized by the recombinant receptor. In certain instances, the binding molecule is an anti-ID that binds to or recognizes the recombinant receptor. In some instances, such features may include evidence of decreased viability, activity, or persistence or increased exhaustion or differentiation.

In certain instances, the long term stimulation methods disclosed herein are useful, inter alia, to identify cell compositions that may have desirable features when administered *in vivo,* such as a maintained or extended persistence, viability, or activity. In some instances, the assay is performed on two or more different cell compositions to identify differences that may enhance or prolong persistence, activity, or viability, or decrease exhaustion or differentiation. In some instances, such differences may include, but are not limited to, aspects of the manufacturing process, such the presence of agents during one or more steps or procedures of the engineering process, e.g., agents that inhibit mTOR kinase activity.

In particular instances, the long term stimulation method is performed in two or more cell compositions to identify agents that increase or maintain viability, activity, or persistence, or increase or maintain expression of markers, e.g., biomarkers, indicative of increased viability, activity, or persistence. In certain instances, the long term stimulation method is performed in two or more cell compositions to identify differences in cell compositions that decrease or prevent exhaustion or differentiation (e.g., such as differentiation to a senescent state), or decrease expression of markers indicative of increased exhaustion or differentiation. In some instances, the binding molecule is conjugated or attached to a solid surface, such as a surface of a cell culture plate or dish. In particular instances, the binding molecules are conjugated or attached to particles, e.g., beads.

In certain instances, the methods for long term stimulation are or include steps for incubating the cells in the presence of particles, e.g., beads, containing a binding molecule, e.g., a binding molecule that binds to or recognizes the recombinant receptor.

In some instances, the binding molecule is an antigen, e.g., a recombinant antigen of fragment thereof that is recognized or bound by the recombinant receptor. In certain instances, the antigen is a polypeptide, or a portion of a polypeptide, that is associated with a disease, e.g., a cancer. In some instances, the antigen is a polypeptide, or a variant or fragment of a polypeptide that is expressed on the surface of a cell that is associated with a disease, for example, a cancer cell and/or a tumor cell.

In some instances, the antigen is or includes αvβ6 integrin (avb6 integrin), B cell maturation antigen (BCMA), B7-H3, B7-H6, carbonic anhydrase 9 (CA9, also known as CAIX or G250), a cancer-testis antigen, cancer/testis antigen 1B (CTAG, also known as NY-ESO-1 and LAGE-2), carcinoembryonic antigen (CEA), a cyclin, cyclin A2, C-C Motif Chemokine Ligand 1 (CCL-1), CD19, CD20, CD22, CD23, CD24, CD30, CD33, CD38, CD44, CD44v6, CD44v7/8, CD123, CD133, CD138, CD171, chondroitin sulfate proteoglycan 4 (CSPG4), epidermal growth factor protein (EGFR), type III epidermal growth factor receptor mutation (EGFR vIII), epithelial glycoprotein 2 (EPG-2), epithelial glycoprotein 40 (EPG-40), ephrinB2, ephrin receptor A2 (EPHa2), estrogen receptor, Fc receptor like 5 (FCRL5; also known as Fc receptor homolog 5 or FCRH5), fetal acetylcholine receptor (fetal AchR), a folate binding protein (FBP), folate receptor alpha, ganglioside GD2, O-acetylated GD2 (OGD2), ganglioside GD3, glycoprotein 100 (gp100), glypican-3 (GPC3), G Protein Coupled Receptor 5D (GPRC5D), Her2/neu (receptor tyrosine kinase erb-B2), Her3 (erb-B3), Her4 (erb-B4), erbB dimers, Human high molecular weight-melanoma-associated antigen (HMW-MAA), hepatitis B surface antigen, Human leukocyte antigen A1 (HLA-A1), Human leukocyte antigen A2 (HLA-A2), IL-22 receptor alpha(IL-22Rα), IL-13 receptor alpha 2 (IL-13Rα2), kinase insert domain receptor (kdr), kappa light chain, L1 cell adhesion molecule (L1-CAM), CE7 epitope of L1-CAM, Leucine Rich Repeat Containing 8 Family Member A (LRRC8A), Lewis Y, Melanoma-associated antigen (MAGE)-A1, MAGE-A3, MAGE-A6, MAGE-A10, mesothelin (MSLN), c-Met, murine cytomegalovirus (CMV), mucin 1 (MUC1), MUC16, natural killer group 2 member D (NKG2D) ligands, melan A (MART-1), neural cell adhesion molecule (NCAM), oncofetal antigen, Preferentially expressed antigen of melanoma (PRAME), progesterone receptor, a prostate specific antigen, prostate stem cell antigen (PSCA), prostate specific membrane antigen (PSMA), Receptor Tyrosine Kinase Like Orphan Receptor 1 (ROR1), survivin, Trophoblast glycoprotein (TPBG also known as 5T4), tumor-associated glycoprotein 72 (TAG72), Tyrosinase related protein 1 (TRP1, also known as TYRP1 or gp75), Tyrosinase related protein 2 (TRP2, also known as dopachrome tautomerase, dopachrome delta-isomerase or DCT), vascular endothelial growth factor receptor (VEGFR), vascular endothelial growth factor receptor 2 (VEGFR2), Wilms Tumor 1 (WT-1), a pathogen-specific or pathogen-expressed antigen, or an antigen associated with a universal tag, and/or biotinylated molecules, and/or molecules expressed by HIV, HCV, HBV or other pathogens. Antigens targeted by the receptors in some instances include antigens associated with a B cell malignancy, such as any of a number of known B cell marker. In some instances, the antigen is or includes CD20, CD19, CD22, ROR1, CD45, CD21, CD5, CD33, Igkappa, Iglambda, CD79a, CD79b or CD30. In some instances, the antigen is or includes recombinant BCMA, CD19, CD22, or ROR1.

In some instances, the anti-ID is an anti-idiotype antibody or antigen-binding fragments that specifically recognizes a target antibody or antigen-binding fragment (e.g. scFv) that is part of the extracellular antigen-binding domain of the recombinant receptor. In some instances, the antigen-binding domain of the recombinant receptor contains an antibody or antigen-binding fragment (e.g. scFv) that binds to a target antigen, such as a target antigen associated with or expressed on a cell or tissue of a disease or condition, e.g. cancer. In some instances, the anti-ID is an anti-idiotype antibody or antigen-binding fragment thereof that specifically recognizes a target antibody or antigen-binding fragment that binds αvβ6 integrin (avb6 integrin), B cell maturation antigen (BCMA), B7-H3, B7-H6, carbonic anhydrase 9 (CA9, also known as CAIX or G250), a cancer-testis antigen, cancer/testis antigen 1B (CTAG, also known as NY-ESO-1 and LAGE-2), carcinoembryonic antigen (CEA), a cyclin, cyclin A2, C-C Motif Chemokine Ligand 1 (CCL-1), CD19, CD20, CD22, CD23, CD24, CD30, CD33, CD38, CD44, CD44v6, CD44v7/8, CD123, CD133, CD138, CD 171, chondroitin sulfate proteoglycan 4 (CSPG4), epidermal growth factor protein (EGFR), type III epidermal growth factor receptor mutation (EGFR vIII), epithelial glycoprotein 2 (EPG-2), epithelial glycoprotein 40 (EPG-40), ephrinB2, ephrin receptor A2 (EPHa2), estrogen receptor, Fc receptor like 5 (FCRL5; also known as Fc receptor homolog 5 or FCRH5), fetal acetylcholine receptor (fetal AchR), a folate binding protein (FBP), folate receptor alpha, ganglioside GD2, O-acetylated GD2 (OGD2), ganglioside GD3, glycoprotein 100 (gp100), glypican-3 (GPC3), G Protein Coupled Receptor 5D (GPRC5D), Her2/neu (receptor tyrosine kinase erb-B2), Her3 (erb-B3), Her4 (erb-B4), erbB dimers, Human high molecular weight-melanoma-associated antigen (HMW-MAA), hepatitis B surface antigen, Human leukocyte antigen A1 (HLA-A1), Human leukocyte antigen A2 (HLA-A2), IL-22 receptor alpha(IL-22Rα), IL-13 receptor alpha 2 (IL-13Rα2), kinase insert domain receptor (kdr), kappa light chain, L1 cell adhesion molecule (L1-CAM), CE7 epitope of L1-CAM, Leucine Rich Repeat Containing 8 Family Member A (LRRC8A), Lewis Y, Melanoma-associated antigen (MAGE)-A1, MAGE-A3, MAGE-A6, MAGE-A10, mesothelin (MSLN), c-Met, murine cytomegalovirus (CMV), mucin 1 (MUC1), MUC16, natural killer group 2 member D (NKG2D) ligands, melan A (MART-1), neural cell adhesion molecule (NCAM), oncofetal antigen, Preferentially expressed antigen of melanoma (PRAME), progesterone receptor, a prostate specific antigen, prostate stem cell antigen (PSCA), prostate specific membrane antigen (PSMA), Receptor Tyrosine Kinase Like Orphan Receptor 1 (ROR1), survivin, Trophoblast glycoprotein (TPBG also known as 5T4), tumor-associated glycoprotein 72 (TAG72), Tyrosinase related protein 1 (TRP1, also known as TYRP1 or gp75), Tyrosinase related protein 2 (TRP2, also known as dopachrome tautomerase, dopachrome delta-isomerase or DCT), vascular endothelial growth factor receptor (VEGFR), vascular endothelial growth factor receptor 2 (VEGFR2), Wilms Tumor 1 (WT-1), a pathogen-specific or pathogen-expressed antigen, or an antigen associated with a universal tag, and/or biotinylated molecules, and/or molecules expressed by HIV, HCV, HBV or other pathogens. Antigens targeted by the receptors in some instances include antigens associated with a B cell malignancy, such as any of a number of known B cell marker. In some instances, the antigen is or includes CD20, CD19, CD22, ROR1, CD45, CD21, CD5, CD33, Igkappa, Iglambda, CD79a, CD79b or CD30. In some instances, the anti-ID binds to the antigen-binding domain of an anti-CD19 CAR.

In some instances, the particle (e.g., bead) reacts in a magnetic field. In some instances, the particle is a magnetic particle (e.g., a magnetic bead). In some instances, the magnetic particle is paramagnetic. In particular instances, the magnetic particle is superparamagnetic. In certain instances, the particles, e.g., beads, do not display any magnetic properties unless they are exposed to a magnetic field. In some instances, the particles or beads have a diameter of between or between about 1 µm and 10 µm, inclusive. In particular instances, the particles, e.g., beads, have a mean diameter of or of about 2.8 µm. In some instances, the particles, e.g., beads, have a diameter of or of about 4.8 µm.

In particular instances, the cells of the input composition of cells are incubated with the binding molecule, e.g., particles or beads that contain the binding molecule, for, for about, or for at least10 days, 11 days, 12 days, 13 days, 14 days, 15 days, 16 days, 17 days, 18 days, 19 days, 20 days, or 21 days. In various instances, the cells or compositions of cells are incubated with the binding molecule, e.g., particles or beads that contain the binding molecule, for between or between about 10 days and 21 days, 12 days and 18 days, or 14 days and 16 days, inclusive. In certain instances, the cells are incubated with the binding molecule, e.g., particles or beads that contain the binding molecule, for, for about, or for at least 14 days. In particular instances, the cells of the cell composition are incubated with the binding molecule, e.g., particles or beads that contain the binding molecule, at temperatures greater than room temperature. In some instances, the incubation is performed at a temperature greater than about 25 °C, such as generally greater than or greater than about 32 °C, 35 °C or 37 °C. In some instances, the treatment, contacting, or incubation is performed at a temperature of at or about 37 °C ± 2 °C, such as at a temperature of at or about 37 °C.

In some instances, the cells are incubated with the binding molecule, e.g., with particles or beads containing the binding molecules in the presence of a media without additional agents that promote cell, e.g., T cell, division, growth, expansion, or activation. In some instances, the cells are incubated with the binding molecules in the absence of any recombinant cytokines. In certain instances, the incubation is performed continuously without interruption. In certain instances, the incubation takes place under static conditions. In particular instances, the incubation takes place without any perfusion, mixing, rocking, or shaking. In some aspects, the binding molecules are present for the entire duration of the incubation. In certain instances, the binding moleculesare not changed or replaced during the incubation. Particular instances contemplate that since, in some aspects, the media does not contain any recombinant cytokines, and cytokines present in the media during the incubation would have been produced by the cells, e.g., in response to an interaction between the recombinant receptor of the cell and the binding molecule of the particle.

In some instances, the amount of binding molecule, e.g., the amount of particles or beads containing binding molecules, is sufficient to provide between or between about 1 binding molecule and 10¹² binding molecules per cell, such as between or between about 10² binding molecules and 10¹⁰ binding molecules, 10³ binding molecules and 10⁸ binding molecules, 10⁴ binding molecules and 10⁶ binding molecules, 1 binding molecule to 10² binding molecules, between 10² binding molecules and 10³ binding molecules, 10³ binding molecules and 10⁴ binding molecules, 10⁴ binding molecules and 10⁵ binding molecules, 10⁵ binding molecules and 10⁶ binding molecules, 10⁵ binding molecules and 10⁶ binding molecules, 10⁶ binding molecules and 10⁷ binding molecules, 10⁷ binding molecules and 10⁸ binding molecules, or 10⁹ binding molecules and 10¹⁰ binding molecules, inclusive. In some instances, the amount of binding molecules, e.g., the amount of particles or beads containing binding molecules, is an amount to sufficient provide between 10⁴ binding molecules and about 10⁶ binding molecules, inclusive, for each cell. In some instances, the amount of particles, e.g., beads, contains about 10⁵ binding molecules for each cell.

In some instances, the input composition is incubated with the particles, e.g., beads, containing a binding molecule at a ratio of total cells to particles, e.g., beads, from between or between about 10:1 and 1:10, 5:1 and 1:5, 3:1 and 1:3, or 2:1 and 1:2, each inclusive. In particular instances, the ratio is or is between or between about 1:0.2 and 1:5, inclusive. In some instances, the ratio of total cells of the input composition to particles, e.g., beads, is about 1:1.

In some instances, the assays may be used to compare different cells or cell compositions. For example, in some instances, two or more cell compositions that each contain cells expressing the same recombinant receptor, e.g., a same CAR, may be compared by incubating the cells with the same binding molecule, e.g., particles or beads containing the same binding molecule, that binds to or recognizes the recombinant receptor. In certain instances, the two or more cell compositions are generated in the presence of different agents, e.g., agents that inhibit mTOR kinase activity. In particular instances, the cell compositions may be compared to a control or reference cell composition. In some aspects, control or reference cell compositions may include, but are not limited to, cell compositions that do not undergo any incubation, cell compositions that are not incubated in the presence of particles, e.g., beads, containing a binding molecule, cell compositions that do not contain cells expressing the recombinant receptor, cell compositions that are generated from a different engineering process, and/or cell compositions that are engineered in the presence of a vehicle or control compound.

In particular instances, two or more cell compositions that each contain cells expressing the different recombinants receptor, e.g., different CARs, may be compared by incubating the cells with binding molecules, such as with particles, e.g., beads, containing different binding molecules, that bind to or recognizes the different recombinant receptors.

In some instances, the cells are assessed at different time points during the incubation. For example, in some aspects, a phenotype, characteristic, or activity of cells from one or more cell compositions are assessed at an intermediate time point, such as a time point that occurs at, at about, or at least a 5%, 10%, 20%, 25%, 30%, 40%, 50%, 60%, 70%, 75%, 80%, 90%, completion of the incubation. In certain instances, the cells are assessed once, twice, three times, four times, five times, six times, seven times, eight times, nine times, ten times or more than ten times during the incubation. In certain instances, the cells are assessed following different intervals during the incubation, such as interval of, of about, or of at least 6 hours, 12 hours, 18 hours, 24 hours, 36 hours, 48 hours, 1 day, 2 days, 3 days, 4 days, 5 days, 6 days, 7 days, or 8 days. In some instances, the cells are assessed every day. In some instances, the cells are assessed every three days. In certain instances, the cells are assessed every 7 days.

In certain instances, the cells, e.g., cells of the output composition, are assessed for an activity, e.g., an antigen simulated activity, a phenotype, or a characteristic. In some instances, antigen stimulated activity is assessed in cells of cell compositions during or after the method for long stimulation, e.g., during or after the incubation with particles, e.g., beads, containing binding molecules. In particular instances, results of the assessment are compared to an assessment of the same or a similar activity measured in cells from a different cell composition, e.g., a control or reference cell composition.

In some instances, the activity is an antigen-stimulated activity. Particular instances contemplate that antigen-stimulated activity of cells, such as T cells expressing a recombinant receptor, can be assessed by any of a number of known suitable techniques. In some instances, the production of one or more cytokines is measured, detected, and/or quantified by intracellular cytokine staining. In some aspects, intracellular cytokine staining (ICS) by flow cytometry is a technique well-suited for studying cytokine production at the single-cell level. In certain aspects, ICS is useful to detect the production and accumulation of cytokines within the endoplasmic reticulum after cell stimulation, such as with an cell expressing the antigen or a particle, e.g., a bead particle, with a conjugated antigen, allowing for the identification of cell populations that are positive or negative for production of a particular cytokine or for the separation of high producing and low producing cells based on a threshold. ICS can also be used in combination with other flow cytometry protocols for immunophenotyping using cell surface markers, e.g., CD4 or CD8, to access cytokine production in a particular subgroup of cells. Other single-cell techniques for measuring or detecting cytokine production include, but are not limited to ELISPOT, limiting dilution, and T cell cloning.

In some instances, the antigen-stimulated activity is the production of one or more cytokines. Cytokines that may be produced in response to antigen stimulation may include, but are not limited to, IL-1, IL-1β, IL-2, sIL-2Ra, IL-3, IL-5, IL-6, IL-7, IL-8, IL-10, IL-12, IL-13, IL 27, IL-33, IL-35, TNF, TNF alpha, CXCL2, CCL2, CCL3, CCL5, CCL17, CCL24, PGD2, LTB4, interferon gamma (IFN-γ), granulocyte macrophage colony stimulating factor (GM-CSF), macrophage inflammatory protein (MIP)-1a, MIP-1b, Flt-3L, fracktalkine, and/or IL-5. In some instances, the one or more cytokines are or include one or more of IL-2, IFN-gamma, or TNF-alpha. In some instances, cytokine secretion is assessed by measuring, detecting, or quantifying the amount or concentration of extracellular cytokines following a co-culture with antigen expressing cells or following an incubation of particles, e.g., beads, containing attached antigen or antigen fragments.

In particular instances, the antigen-stimulated activity is cytolytic (cytotoxic) activity. In some instances, cytolytic activity is assessed by exposing, incubating, and/or contacting the cells of the composition, e.g., cells expressing the recombinant receptor, with a target cell that expresses the antigen or an epitope that is recognized by the recombinant receptor. The cytolytic activity can be measured by directly or indirectly measuring the target cell number over time. For example, the target cells may be incubated with a detectable marker prior to being incubated with recombinant receptor expressing cells, such a marker that is detectable when the target cell is lysed, or a detectable marker that is only detectable in viable target cells. These readouts provide direct or indirect of target cell number and/or target cell death, and can be measured at different time points during the assay. A reduction of target cell number and/or an increase of target cell death indicate the cytolytic activity of the cells. Suitable methods for performing cytolytic assays are known in the art, and include, but are not limited to chromium-51 release assays, non-radioactive chromium assays, flow cytometric assays that use fluorescent dyes such as carboxyfluorescein succinimidyl ester (CFSE), PKH-2, and PKH-26.

In certain instances, the cells, e.g., cells expressing the recombinant receptor, of the cell composition are assessed for one or more characteristics or phenotypes during or after the assay, e.g., during or after an incubation with particles, e.g., beads, containing a binding receptor. In some instances, the one or more characteristics or phenotypes are or relate to one or more of activation, exhaustion, or differentiation. In certain instances, the one or more phenotypes or characteristics are assessed by measuring, detecting, or quantifying the presence, absence, amount, or level of one or more markers, e.g., biomarkers.

In some instances, the expression of a marker, e.g., a marker that is positively or negatively associated with activation, exhaustion, or differentiation, is or includes assessing, measuring, determining, and/or quantifying a level, amount, or concentration of a marker in the sample. In certain instances, the marker is a gene product, e.g., any biomolecule that is assembled, generated, and/or synthesized with information encoded by a gene, and may include polynucleotides and/or polypeptides. In certain instances, the level, amount, or concentration of the marker may be transformed (e.g., normalized) or directly analyzed (e.g., raw). In some instances, the marker is a protein. In certain instances, the marker is a polynucleotide, e.g., an mRNA or a protein, that is encoded by the gene.

In particular instances, the amount or level of a marker that is a polynucleotide may be assessed, measured, determined, and/or quantified by any suitable known means. For example, in some instances, the amount or level of a polynucleotide marker can be assessed, measured, determined, and/or quantified by polymerase chain reaction (PCR), including reverse transcriptase (rt) PCR, droplet digital PCR, real-time and quantitative PCR methods (including, e.g., TAQMAN^{®}, molecular beacon, LIGHTUP^{™}, SCORPION^{™}, SIMPLEPROBES^{®}; see, e.g., U.S. Pat. Nos.5,538,848; 5,925,517; 6,174,670; 6,329,144; 6,326,145 and 6,635,427); northern blotting; Southern blotting, e.g., of reverse transcription products and derivatives; array based methods, including blotted arrays, microarrays, or in situ-synthesized arrays; and sequencing, e.g., sequencing by synthesis, pyrosequencing, dideoxy sequencing, or sequencing by ligation, or any other methods known in the art, such as discussed in Shendure et al., Nat. Rev. Genet. 5:335-44 (2004) or Nowrousian, Euk. Cell 9(9): 1300-1310 (2010), including such specific platforms as HELICOS^{®}, ROCHE^{®} 454, ILLUMINA^{®}/SOLEXA^{®}, ABI SOLiD^{®}, and POLONATOR^{®} sequencing. In particular instances, the levels of nucleic acid gene products are measured by qRT-PCR. In some instances, the qRT-PCR uses three nucleic acid sets for each gene, where the three nucleic acids comprise a primer pair together with a probe that binds between the regions of a target nucleic acid where the primers bind- known commercially as a TAQMAN^{®} assay.

In particular instances, the expression of two or more polynucleotide markers are measured or assessed simultaneously. In certain instances, a multiplex PCR, e.g., a multiplex rt-PCR assessing, measuring, determining, and/or quantifying the level, amount, or concentration of two or more gene products. In some instances, microarrays (e.g., AFFYMETRIX^{®}, AGILENT^{®} and ILLUMINA^{®}-style arrays) are used for assessing, measuring, determining, and/or quantifying the level, amount, or concentration of two or more gene products. In some instances, microarrays are used for assessing, measuring, determining, and/or quantifying the level, amount, or concentration of a cDNA polynucleotide that is derived from an RNA gene product.

In some instances, the expression of one or more polynucleotide markers is determined by sequencing a marker mRNA or a cDNA polynucleotide that is derived from the marker mRNA. In some instances, the sequencing is performed by a non-Sanger sequencing method and/or a next generation sequencing (NGS) technique. Examples of Next Generation Sequencing techniques include, but are not limited to Massively Parallel Signature Sequencing (MPSS), Polony sequencing, pyrosequencing, Reversible dye-terminator sequencing, SOLiD sequencing, Ion semiconductor sequencing, DNA nanoball sequencing, Helioscope single molecule sequencing, Single molecule real time (SMRT) sequencing, Single molecule real time (RNAP) sequencing, and Nanopore DNA sequencing.

In particular instances, the marker is a protein or fragment thereof. In certain instances, one or more protein markers are measured by any suitable means known in the art. Suitable methods for assessing, measuring, determining, and/or quantifying the level, amount, or concentration or more or more protein markers include, but are not limited to, detection with immunoassays, nucleic acid-based or protein-based aptamer techniques, HPLC (high precision liquid chromatography), peptide sequencing (such as Edman degradation sequencing or mass spectrometry (such as MS/MS), optionally coupled to HPLC), and microarray adaptations of any of the foregoing (including nucleic acid, antibody or protein-protein (i.e., non- antibody) arrays). In some instances, the immunoassay is or includes methods or assays that detect proteins based on an immunological reaction, e.g., by detecting the binding of an antibody or antigen binding antibody fragment to a gene product. Immunoassays include, but are not limited to, quantitative immunocytochemistry or immunohistochemistry, ELISA (including direct, indirect, sandwich, competitive, multiple and portable ELISAs (see, e.g., U.S. Patent No. 7,510,687), western blotting (including one, two or higher dimensional blotting or other chromatographic means, optionally including peptide sequencing), enzyme immunoassay (EIA), RIA (radioimmunoassay), and SPR (surface plasmon resonance).

In some instances, the protein marker is measured, detected, or quantified by flow cytometry. In some aspects, flow cytometry is a laser- or impedance-based, biophysical technology employed in marker detection by suspending cells in a stream of fluid and passing them through an electronic detection apparatus. Markers present on cells may be labeled, such as with a fluorescence tagged antibody for detection by flow cytometry. In some aspects, flow cytometry is employed to measure, detect, or quantify the presence, absence, amount, or level of a marker present in a population of cells. In some aspects the population of cells may be the total or total viable cells of the cell composition or from a subset of cells from the cell composition, e.g., cells positive for the recombinant receptor, CD4+ T cells, or CD8+ T cells.

In particular instances, the marker is positively associated or correlated with activation or an activation-like state. In some instances, the marker is or includes CD25, CD26, CD27, CD28, CD30, CD71, CD154, CD40L, CD127, LAG3, or Ki67. In some instances, the marker is positively associated or correlated with exhaustion or a condition related to exhaustion. In particular instances, the marker is or includes one or more of CTLA-4, FOXP3, PD-1, TIGIT, LAB-3, 2B4, BTLA, TIM3, VISTA, or CD96. In some instances, the biomarker is associated with differentiation of a T cell. In particular instances, the marker is or includes one or more of CD25, CD45RO, CD56, KLRG1, CD95 and/or one or more of CD45RA, CD27, CD28, CD62L, and CCR7.In some instances, cells, e.g., cells of the output composition, are assessed for cells that are surface positive for a T cell activation marker selected from the group consisting of CD45RA, CD27, CD28, CD62L, and CCR7; and/or that are surface negative for a marker selected from the group consisting of CD25, CD45RO, CD56, KLRG1; and/or have low expression of CD95; and/or are negative for intracellular expression of a cytokine selected from the group consisting of IL-2, IFN-γ, IL-4, IL-10. In some the output composition is assessed for cells that are CD45RA+, CD27+, CCR7+, and/or CD45RO-.

In some instances, the cells of an output cell composition display features of cells that have undergone prolonged or chronic stimulation following the long term stimulation method, e.g., following an incubation with the particles, e.g., beads, containing a binding molecule. In some instances, the cells expressing the recombinant receptor of a cell composition, e.g., a reference or control cell composition, display features of cells that have undergone prolonged or chronic stimulation following the assay, e.g., following an incubation with the particles, e.g., beads, containing a binding molecule.

### IV. RECOMBINANT RECEPTORS

In some instances, the cells that are treated, processed, engineered, and/or produced by the methods disclosed herein contain or express, or are engineered to contain or express, a recombinant protein, such as a recombinant receptor, e.g., a chimeric antigen receptor (CAR), or a T cell receptor (TCR). In certain instances, the methods disclosed herein produce and/or a capable of producing cells, or populations or compositions containing and/or enriched for cells, that are engineered to express or contain a recombinant protein. In some instances, CD4+ T cells, or populations or compositions of CD4+ T cells, are treated, processed, engineered, and/or produced

In some instances, the cells include one or more nucleic acids introduced via genetic engineering, and thereby express recombinant or genetically engineered products of such nucleic acids. In some instances, gene transfer is accomplished by first stimulating the cells, such as by combining it with a stimulus that induces a response such as proliferation, survival, and/or activation, e.g., as measured by expression of a cytokine or activation marker, followed by transduction of the activated cells, and expansion in culture to numbers sufficient for clinical applications.

The cells generally express recombinant receptors, such as antigen receptors including functional non-TCR antigen receptors, e.g., chimeric antigen receptors (CARs), and other antigen-binding receptors such as transgenic T cell receptors (TCRs). Also among the receptors are other chimeric receptors

### A. Chimeric Antigen Receptors

In some instances of the disclosed methods and uses, chimeric receptors, such as a chimeric antigen receptors, contain one or more domains that combine a ligand-binding domain (e.g. antibody or antibody fragment) that provides specificity for a desired antigen (e.g., tumor antigen) with intracellular signaling domains. In some instances, the intracellular signaling domain is an activating intracellular domain portion, such as a T cell activating domain, providing a primary activation signal. In some instances, the intracellular signaling domain contains or additionally contains a costimulatory signaling domain to facilitate effector functions. In some instances, chimeric receptors when genetically engineered into immune cells can modulate T cell activity, and, in some cases, can modulate T cell differentiation or homeostasis, thereby resulting in genetically engineered cells with improved longevity, survival and/or persistence *in vivo,* such as for use in adoptive cell therapy methods.

Exemplary antigen receptors, including CARs, and methods for engineering and introducing such receptors into cells, include those described, for example, in international patent application publication numbers WO200014257, WO2013126726, WO2012/129514, WO2014031687, WO2013/166321, WO2013/071154, WO2013/123061 U.S. patent application publication numbers US2002131960, US2013287748, US20130149337, U.S. Patent Nos.: 6,451,995, 7,446,190, 8,252,592, , 8,339,645, 8,398,282, 7,446,179, 6,410,319, 7,070,995, 7,265,209, 7,354,762, 7,446,191, 8,324,353, and 8,479,118, and European patent application number EP2537416,and/or those described by Sadelain et al., Cancer Discov. 2013 April; 3(4): 388-398; Davila et al. (2013) PLoS ONE 8(4): e61338; Turtle et al., Curr. Opin. Immunol., 2012 October; 24(5): 633-39; Wu et al., Cancer, 2012 March 18(2): 160-75. In some aspects, the antigen receptors include a CAR as described in U.S. Patent No.: 7,446,190, and those described in International Patent Application Publication No.: WO/2014055668 A1. Examples of the CARs include CARs as disclosed in any of the aforementioned publications, such as WO2014031687, US 8,339,645, US 7,446,179, US 2013/0149337, U.S. Patent No.: 7,446,190, US Patent No.: 8,389,282, Kochenderfer et al., 2013, Nature Reviews Clinical Oncology, 10, 267-276 (2013); Wang et al. (2012) J. Immunother. 35(9): 689-701; and Brentjens et al., Sci Transl Med. 2013 5(177). See also WO2014031687, US 8,339,645, US 7,446,179, US 2013/0149337, U.S. Patent No.: 7,446,190, and US Patent No.: 8,389,282.

The chimeric receptors, such as CARs, generally include an extracellular antigen binding domain, such as a portion of an antibody molecule, generally a variable heavy (VH) chain region and/or variable light (VL) chain region of the antibody, e.g., an scFv antibody fragment.

In some instances, the antigen targeted by the receptor is a polypeptide. In some instances, it is a carbohydrate or other molecule. In some instances, the antigen is selectively expressed or overexpressed on cells of the disease or condition, e.g., the tumor or pathogenic cells, as compared to normal or non-targeted cells or tissues. In other instances, the antigen is expressed on normal cells and/or is expressed on the engineered cells.

In some instances, the antigen is or includes αvβ6 integrin (avb6 integrin), B cell maturation antigen (BCMA), B7-H3, B7-H6, carbonic anhydrase 9 (CA9, also known as CAIX or G250), a cancer-testis antigen, cancer/testis antigen 1B (CTAG, also known as NY-ESO-1 and LAGE-2), carcinoembryonic antigen (CEA), a cyclin, cyclin A2, C-C Motif Chemokine Ligand 1 (CCL-1), CD19, CD20, CD22, CD23, CD24, CD30, CD33, CD38, CD44, CD44v6, CD44v7/8, CD123, CD133, CD138, CD171, chondroitin sulfate proteoglycan 4 (CSPG4), epidermal growth factor protein (EGFR), type III epidermal growth factor receptor mutation (EGFR vIII), epithelial glycoprotein 2 (EPG-2), epithelial glycoprotein 40 (EPG-40), ephrinB2, ephrin receptor A2 (EPHa2), estrogen receptor, Fc receptor like 5 (FCRLS; also known as Fc receptor homolog 5 or FCRH5), fetal acetylcholine receptor (fetal AchR), a folate binding protein (FBP), folate receptor alpha, ganglioside GD2, O-acetylated GD2 (OGD2), ganglioside GD3, glycoprotein 100 (gp100), glypican-3 (GPC3), G Protein Coupled Receptor 5D (GPRCSD), Her2/neu (receptor tyrosine kinase erb-B2), Her3 (erb-B3), Her4 (erb-B4), erbB dimers, Human high molecular weight-melanoma-associated antigen (HMW-MAA), hepatitis B surface antigen, Human leukocyte antigen A1 (HLA-A1), Human leukocyte antigen A2 (HLA-A2), IL-22 receptor alpha(IL-22Rα), IL-13 receptor alpha 2 (IL-13Rα2), kinase insert domain receptor (kdr), kappa light chain, L1 cell adhesion molecule (L1-CAM), CE7 epitope of L1-CAM, Leucine Rich Repeat Containing 8 Family Member A (LRRC8A), Lewis Y, Melanoma-associated antigen (MAGE)-A1, MAGE-A3, MAGE-A6, MAGE-A10, mesothelin (MSLN), c-Met, murine cytomegalovirus (CMV), mucin 1 (MUC1), MUC16, natural killer group 2 member D (NKG2D) ligands, melan A (MART-1), neural cell adhesion molecule (NCAM), oncofetal antigen, Preferentially expressed antigen of melanoma (PRAME), progesterone receptor, a prostate specific antigen, prostate stem cell antigen (PSCA), prostate specific membrane antigen (PSMA), Receptor Tyrosine Kinase Like Orphan Receptor 1 (ROR1), survivin, Trophoblast glycoprotein (TPBG also known as ST4), tumor-associated glycoprotein 72 (TAG72), Tyrosinase related protein 1 (TRP1, also known as TYRP1 or gp75), Tyrosinase related protein 2 (TRP2, also known as dopachrome tautomerase, dopachrome delta-isomerase or DCT), vascular endothelial growth factor receptor (VEGFR), vascular endothelial growth factor receptor 2 (VEGFR2), Wilms Tumor 1 (WT-1), a pathogen-specific or pathogen-expressed antigen, or an antigen associated with a universal tag, and/or biotinylated molecules, and/or molecules expressed by HIV, HCV, HBV or other pathogens. Antigens targeted by the receptors in some instances include antigens associated with a B cell malignancy, such as any of a number of known B cell marker. In some instances, the antigen is or includes CD20, CD19, CD22, ROR1, CD45, CD21, CD5, CD33, Igkappa, Iglambda, CD79a, CD79b or CD30.

In some instances, the antigen is or includes a pathogen-specific or pathogen-expressed antigen. In some instances, the antigen is a viral antigen (such as a viral antigen from HIV, HCV, HBV, etc.), bacterial antigens, and/or parasitic antigens. Antigens targeted by the receptors in some instances include antigens associated with a B cell malignancy, such as any of a number of known B cell marker. In some instances, the antigen targeted by the receptor is CD20, CD19, CD22, ROR1, CD45, CD21, CD5, CD33, Igkappa, Iglambda, CD79a, CD79b or CD30.

In some instances, the antigen or antigen binding domain is CD19. In some instances, the scFv contains a VH and a VL derived from an antibody or an antibody fragment specific to CD19. In some instances, the antibody or antibody fragment that binds CD19 is a mouse derived antibody such as FMC63 and SJ25C1. In some instances, the antibody or antibody fragment is a human antibody, e.g., as described in U.S. Patent Publication No. US 2016/0152723.

The term "antibody" herein is used in the broadest sense and includes polyclonal and monoclonal antibodies, including intact antibodies and functional (antigen-binding) antibody fragments, including fragment antigen binding (Fab) fragments, F(ab')₂ fragments, Fab' fragments, Fv fragments, recombinant IgG (rIgG) fragments, heavy chain variable (V_{H}) regions capable of specifically binding the antigen, single chain antibody fragments, including single chain variable fragments (scFv), and single domain antibodies (e.g., sdAb, sdFv, nanobody) fragments. The term encompasses genetically engineered and/or otherwise modified forms of immunoglobulins, such as intrabodies, peptibodies, chimeric antibodies, fully human antibodies, humanized antibodies, and heteroconjugate antibodies, multispecific, e.g., bispecific or trispecific, antibodies, diabodies, triabodies, and tetrabodies, tandem di-scFv, tandem tri-scFv. Unless otherwise stated, the term "antibody" should be understood to encompass functional antibody fragments thereof also referred to herein as "antigen-binding fragments." The term also encompasses intact or full-length antibodies, including antibodies of any class or sub-class, including IgG and sub-classes thereof, IgM, IgE, IgA, and IgD.

The terms "complementarity determining region," and "CDR," synonymous with "hypervariable region" or "HVR," are known in the art to refer to non-contiguous sequences of amino acids within antibody variable regions, which confer antigen specificity and/or binding affinity. In general, there are three CDRs in each heavy chain variable region (CDR-H1, CDR-H2, CDR-H3) and three CDRs in each light chain variable region (CDR-L1, CDR-L2, CDR-L3). "Framework regions" and "FR" are known in the art to refer to the non-CDR portions of the variable regions of the heavy and light chains. In general, there are four FRs in each full-length heavy chain variable region (FR-H1, FR-H2, FR-H3, and FR-H4), and four FRs in each full-length light chain variable region (FR-L1, FR-L2, FR-L3, and FR-L4).

The precise amino acid sequence boundaries of a given CDR or FR can be readily determined using any of a number of well-known schemes, including those described by Kabat et al. (1991), "Sequences of Proteins of Immunological Interest," 5th Ed. Public Health Service, National Institutes of Health, Bethesda, MD ("Kabat" numbering scheme); Al-Lazikani et al., (1997) JMB 273,927-948 ("Chothia" numbering scheme); MacCallum et al., J. Mol. Biol. 262:732-745 (1996), "Antibody-antigen interactions: Contact analysis and binding site topography," J. Mol. Biol. 262, 732-745." ("Contact" numbering scheme); Lefranc MP et al., "IMGT unique numbering for immunoglobulin and T cell receptor variable domains and Ig superfamily V-like domains," Dev Comp Immunol, 2003 Jan;27(1):55-77 ("IMGT" numbering scheme); Honegger A and Plückthun A, "Yet another numbering scheme for immunoglobulin variable domains: an automatic modeling and analysis tool," J Mol Biol, 2001 Jun 8;309(3):657-70, ("Aho" numbering scheme); and Martin et al., "Modeling antibody hypervariable loops: a combined algorithm," PNAS, 1989, 86(23):9268-9272, ("AbM" numbering scheme).

The boundaries of a given CDR or FR may vary depending on the scheme used for identification. For example, the Kabat scheme is based on structural alignments, while the Chothia scheme is based on structural information. Numbering for both the Kabat and Chothia schemes is based upon the most common antibody region sequence lengths, with insertions accommodated by insertion letters, for example, "30a," and deletions appearing in some antibodies. The two schemes place certain insertions and deletions ("indels") at different positions, resulting in differential numbering. The Contact scheme is based on analysis of complex crystal structures and is similar in many respects to the Chothia numbering scheme. The AbM scheme is a compromise between Kabat and Chothia definitions based on that used by Oxford Molecular's AbM antibody modeling software.

**Table 1,** below, lists exemplary position boundaries of CDR-L1, CDR-L2, CDR-L3 and CDR-H1, CDR-H2, CDR-H3 as identified by Kabat, Chothia, AbM, and Contact schemes, respectively. For CDR-H1, residue numbering is listed using both the Kabat and Chothia numbering schemes. FRs are located between CDRs, for example, with FR-L1 located before CDR-L1, FR-L2 located between CDR-L1 and CDR-L2, FR-L3 located between CDR-L2 and CDR-L3 and so forth. It is noted that because the shown Kabat numbering scheme places insertions at H35A and H35B, the end of the Chothia CDR-H1 loop when numbered using the shown Kabat numbering convention varies between H32 and H34, depending on the length of the loop.

**Table 1. Boundaries of CDRs according to various numbering schemes.**

| **CDR** | **Kabat** | **Chothia** | **AbM** | **Contact** |
|---|---|---|---|---|
| CDR-L1 | L24--L34 | L24--L34 | L24--L34 | L30--L36 |
| CDR-L2 | L50--L56 | L50--L56 | L50--L56 | L46--L55 |
| CDR-L3 | L89--L97 | L89--L97 | L89--L97 | L89--L96 |
| CDR-H1 (Kabat Numbering¹) | H31--H35B | H26--H32..34 | H26--H3 5B | H3 0--H3 5B |
| CDR-H1 (Chothia Numbering²) | H31--H35 | H26--H32 | H26--H35 | H30--H35 |
| CDR-H2 | H50--H65 | H52--H56 | H50--H58 | H47--H58 |
| CDR-H3 | H95--H102 | H95--H102 | H95--H102 | H93-H101 |

| | | | | |
|---|---|---|---|---|
| 1 - Kabat et al. (1991), "Sequences of Proteins of Immunological Interest," 5th Ed. Public Health Service, National Institutes of Health, Bethesda, MD 2 - Al-Lazikani et al., (1997) JMB 273,927-948 | | | | |

Thus, unless otherwise specified, a "CDR" or "complementary determining region," or individual specified CDRs (*e*.*g*., CDR-H1, CDR-H2, CDR-H3), of a given antibody or region thereof, such as a variable region thereof, should be understood to encompass a (or the specific) complementary determining region as defined by any of the aforementioned schemes, or other known schemes. For example, where it is stated that a particular CDR (*e*.*g*., a CDR-H3) contains the amino acid sequence of a corresponding CDR in a given V_{H} or V_{L} region amino acid sequence, it is understood that such a CDR has a sequence of the corresponding CDR (*e*.*g*., CDR-H3) within the variable region, as defined by any of the aforementioned schemes, or other known schemes. In some instances, specific CDR sequences are specified. Exemplary CDR sequences of disclosed antibodies are described using various numbering schemes, although it is understood that an antibody can include CDRs as described according to any of the other aforementioned numbering schemes or other numbering schemes known to a skilled artisan.

Likewise, unless otherwise specified, a FR or individual specified FR(s) (*e*.*g*., FR-H1, FR-H2, FR-H3, FR-H4), of a given antibody or region thereof, such as a variable region thereof, should be understood to encompass a (or the specific) framework region as defined by any of the known schemes. In some instances, the scheme for identification of a particular CDR, FR, or FRs or CDRs is specified, such as the CDR as defined by the Kabat, Chothia, AbM or Contact method, or other known schemes. In other cases, the particular amino acid sequence of a CDR or FR is given.

The term "variable region" or "variable domain" refers to the domain of an antibody heavy or light chain that is involved in binding the antibody to antigen. The variable regions of the heavy chain and light chain (V_{H} and V_{L}, respectively) of a native antibody generally have similar structures, with each domain comprising four conserved framework regions (FRs) and three CDRs. (See, *e.g.,* Kindt et al. Kuby Immunology, 6th ed., W.H. Freeman and Co., page 91 (2007). A single V_{H} or V_{L} domain may be sufficient to confer antigen-binding specificity. Furthermore, antibodies that bind a particular antigen may be isolated using a V_{H} or V_{L} domain from an antibody that binds the antigen to screen a library of complementary V_{L} or V_{H} domains, respectively. See, *e.g.,* Portolano et al., J. Immunol. 150:880-887 (1993); Clarkson et al., Nature 352:624-628 (1991).

Among the antibodies included in the disclosed CARs are antibody fragments. An "antibody fragment" or "antigen-binding fragment" refers to a molecule other than an intact antibody that comprises a portion of an intact antibody that binds the antigen to which the intact antibody binds. Examples of antibody fragments include but are not limited to Fv, Fab, Fab', Fab'-SH, F(ab')₂; diabodies; linear antibodies; heavy chain variable (V_{H}) regions, single-chain antibody molecules such as scFvs and single-domain antibodies comprising only the V_{H} region; and multispecific antibodies formed from antibody fragments. In some instances, the antigen-binding domain in the disclosed CARs is or comprises an antibody fragment comprising a variable heavy chain (V_{H}) and a variable light chain (V_{L}) region. In particular instances, the antibodies are single-chain antibody fragments comprising a heavy chain variable (V_{H}) region and/or a light chain variable (V_{L}) region, such as scFvs.

In some instances, the scFv is derived from FMC63. FMC63 generally refers to a mouse monoclonal IgG1 antibody raised against Nalm-1 and -16 cells expressing CD19 of human origin (Ling, N. R., et al. (1987). Leucocyte typing III. 302). In some instances, the FMC63 antibody comprises CDRH1 and H2 set forth in SEQ ID NOS: 39 and 40, respectively, and CDRH3 set forth in SEQ ID NO: 41 or 55 and CDRL1 set forth in SEQ ID NO: 36 and CDR L2 set forth in SEQ ID NO: 37 or 56 and CDR L3 set forth in SEQ ID NO: 38 or 35. In some instances, the FMC63 antibody comprises the heavy chain variable region (V_{H}) comprising the amino acid sequence of SEQ ID NO: 42 and the light chain variable region (V_{L}) comprising the amino acid sequence of SEQ ID NO: 43.

In some instances, the scFv comprises a variable light chain containing the CDRL1 sequence of SEQ ID NO:36, a CDRL2 sequence of SEQ ID NO:37, and a CDRL3 sequence of SEQ ID NO:38 and/or a variable heavy chain containing a CDRH1 sequence of SEQ ID NO:39, a CDRH2 sequence of SEQ ID NO:40, and a CDRH3 sequence of SEQ ID NO:41. In some instances, the scFv comprises a variable heavy chain region set forth in SEQ ID NO:42 and a variable light chain region set forth in SEQ ID NO:43. In some instances, the variable heavy and variable light chains are connected by a linker. In some instances, the linker is set forth in SEQ ID NO:57. In some instances, the scFv comprises, in order, a V_{H}, a linker, and a V_{L}. In some instances, the scFv comprises, in order, a V_{L}, a linker, and a V_{H}. In some instances, the scFv is encoded by a sequence of nucleotides set forth in SEQ ID NO:58 or a sequence that exhibits at least 85%, 86%, 87%, 88%, 89%, 90%, 91%, 92%, 93%, 94%, 95%, 96%, 97%, 98%, or 99% sequence identity to SEQ ID NO:58. In some instances, the scFv comprises the sequence of amino acids set forth in SEQ ID NO:44 or a sequence that exhibits at least 85%, 86%, 87%, 88%, 89%, 90%, 91%, 92%, 93%, 94%, 95%, 96%, 97%, 98%, or 99% sequence identity to SEQ ID NO:44.

In some instances the scFv is derived from SJ25C1. SJ25C1 is a mouse monoclonal IgG1 antibody raised against Nalm-1 and -16 cells expressing CD19 of human origin (Ling, N. R., et al. (1987). Leucocyte typing III. 302). In some instances, the SJ25C1 antibody comprises CDRH1, H2 and H3 set forth in SEQ ID NOS: 48-50, respectively, and CDRL1, L2 and L3 sequences set forth in SEQ ID NOS: 45-47, respectively. In some instances, the SJ25C1 antibody comprises the heavy chain variable region (V_{H}) comprising the amino acid sequence of SEQ ID NO: 51 and the light chain variable region (V_{L}) comprising the amino acid sequence of SEQ ID NO: 52.

In some instances, the scFv comprises a variable light chain containing the CDRL1 sequence of SEQ ID NO:45, a CDRL2 sequence of SEQ ID NO: 46, and a CDRL3 sequence of SEQ ID NO:47 and/or a variable heavy chain containing a CDRH1 sequence of SEQ ID NO:48, a CDRH2 sequence of SEQ ID NO:49, and a CDRH3 sequence of SEQ ID NO:50. In some instances, the scFv comprises a variable heavy chain region set forth in SEQ ID NO:51 and a variable light chain region set forth in SEQ ID NO:52. In some instances, the variable heavy and variable light chain are connected by a linker. In some instances, the linker is set forth in SEQ ID NO:53. In some instances, the scFv comprises, in order, a V_{H}, a linker, and a V_{L}. In some instances, the scFv comprises, in order, a V_{L}, a linker, and a V_{H}. In some instances, the scFv comprises the sequence of amino acids set forth in SEQ ID NO:54 or a sequence that exhibits at least 85%, 86%, 87%, 88%, 89%, 90%, 91%, 92%, 93%, 94%, 95%, 96%, 97%, 98%, or 99% sequence identity to SEQ ID NO:54.

In some instances, the antigen or antigen binding domain is BCMA. In some instances, the scFv contains a VH and a VL derived from an antibody or an antibody fragment specific to BCMA. In some instances, the antibody or antibody fragment that binds BCMA is or contains a VH and a VL from an antibody or antibody fragment set forth in International Patent Applications, Publication Number WO 2016/090327 and WO 2016/090320.

In some instances, the antigen or antigen binding domain is GPRCSD. In some instances, the scFv contains a VH and a VL derived from an antibody or an antibody fragment specific to GPRCSD. In some instances, the antibody or antibody fragment that binds GPRCSD is or contains a VH and a VL from an antibody or antibody fragment set forth in International Patent Applications, Publication Number WO 2016/090329 and WO 2016/090312.

In some instances, the antigen is CD20. In some instances, the scFv contains a VH and a VL derived from an antibody or an antibody fragment specific to CD20. In some instances, the antibody or antibody fragment that binds CD20 is an antibody that is or is derived from Rituximab, such as is Rituximab scFv.

In some instances, the antigen is CD22. In some instances, the scFv contains a VH and a VL derived from an antibody or an antibody fragment specific to CD22. In some instances, the antibody or antibody fragment that binds CD22 is an antibody that is or is derived from m971, such as is m971 scFv.

In some instances, the chimeric antigen receptor includes an extracellular portion containing an antibody or antibody fragment. In some aspects, the chimeric antigen receptor includes an extracellular portion containing the antibody or fragment and an intracellular signaling domain. In some instances, the antibody or fragment includes an scFv.

In some instances, the antibody portion of the recombinant receptor, e.g., CAR, further includes at least a portion of an immunoglobulin constant region, such as a hinge region, e.g., an IgG4 hinge region, and/or a CH1/CL and/or Fc region. In some instances, the constant region or portion is of a human IgG, such as IgG4 or IgG1. In some aspects, the portion of the constant region serves as a spacer region between the antigen-recognition component, e.g., scFv, and transmembrane domain. The spacer can be of a length that provides for increased responsiveness of the cell following antigen binding, as compared to in the absence of the spacer. Exemplary spacers include, but are not limited to, those described in Hudecek et al. (2013) Clin. Cancer Res., 19:3153, international patent application publication number WO2014031687, U.S. Patent No. 8,822,647 or published app. No. US2014/0271635.

In some instances, the constant region or portion is of a human IgG, such as IgG4 or IgG1. In some instances, the spacer has the sequence ESKYGPPCPPCP (set forth in SEQ ID NO: 1), and is encoded by the sequence set forth in SEQ ID NO: 2. In some instances, the spacer has the sequence set forth in SEQ ID NO: 3. In some instances, the spacer has the sequence set forth in SEQ ID NO: 4. In some instances, the constant region or portion is of IgD. In some instances, the spacer has the sequence set forth in SEQ ID NO: 5. In some instances, the spacer has a sequence of amino acids that exhibits at least 85%, 86%, 87%, 88%, 89%, 90%, 91%, 92%, 93%, 94%, 95%, 96%, 97%, 98%, 99% or more sequence identity to any of SEQ ID NOS: 1, 3, 4 or 5. In some instances, the spacer has the sequence set forth in SEQ ID NOS: 25-33. In some instances, the spacer has a sequence of amino acids that exhibits at least 85%, 86%, 87%, 88%, 89%, 90%, 91%, 92%, 93%, 94%, 95%, 96%, 97%, 98%, 99% or more sequence identity to any of SEQ ID NOS: 25-33.

In some instances, the antigen receptor comprises an intracellular domain linked directly or indirectly to the extracellular domain. In some instances, the chimeric antigen receptor includes a transmembrane domain linking the extracellular domain and the intracellular signaling domain. In some instances, the intracellular signaling domain comprises an ITAM. For example, in some aspects, the antigen recognition domain (e.g. extracellular domain) generally is linked to one or more intracellular signaling components, such as signaling components that mimic activation through an antigen receptor complex, such as a TCR complex, in the case of a CAR, and/or signal via another cell surface receptor. In some instances, the chimeric receptor comprises a transmembrane domain linked or fused between the extracellular domain (e.g. scFv) and intracellular signaling domain. Thus, in some instances, the antigen-binding component (e.g., antibody) is linked to one or more transmembrane and intracellular signaling domains.

In one instances, a transmembrane domain that naturally is associated with one of the domains in the receptor, e.g., CAR, is used. In some instances, the transmembrane domain is selected or modified by amino acid substitution to avoid binding of such domains to the transmembrane domains of the same or different surface membrane proteins to minimize interactions with other members of the receptor complex.

The transmembrane domain in some instances is derived either from a natural or from a synthetic source. Where the source is natural, the domain in some aspects is derived from any membrane-bound or transmembrane protein. Transmembrane regions include those derived from (i.e. comprise at least the transmembrane region(s) of) the alpha, beta or zeta chain of the T-cell receptor, CD28, CD3 epsilon, CD45, CD4, CD5, CD8, CD9, CD16, CD22, CD33, CD37, CD64, CD80, CD86, CD134, CD137, CD154. Alternatively the transmembrane domain in some instances is synthetic. In some aspects, the synthetic transmembrane domain comprises predominantly hydrophobic residues such as leucine and valine. In some aspects, a triplet of phenylalanine, tryptophan and valine will be found at each end of a synthetic transmembrane domain. In some instances, the linkage is by linkers, spacers, and/or transmembrane domain(s). In some aspects, the transmembrane domain contains a transmembrane portion of CD28.

In some instances, the extracellular domain and transmembrane domain can be linked directly or indirectly. In some instances, the extracellular domain and transmembrane are linked by a spacer, such as any described herein. In some instances, the receptor contains extracellular portion of the molecule from which the transmembrane domain is derived, such as a CD28 extracellular portion.

Among the intracellular signaling domains are those that mimic or approximate a signal through a natural antigen receptor, a signal through such a receptor in combination with a costimulatory receptor, and/or a signal through a costimulatory receptor alone. In some instances, a short oligo- or polypeptide linker, for example, a linker of between 2 and 10 amino acids in length, such as one containing glycines and serines, e.g., glycine-serine doublet, is present and forms a linkage between the transmembrane domain and the cytoplasmic signaling domain of the CAR.

T cell activation is in some aspects described as being mediated by two classes of cytoplasmic signaling sequences: those that initiate antigen-dependent primary activation through the TCR (primary cytoplasmic signaling sequences), and those that act in an antigen-independent manner to provide a secondary or co-stimulatory signal (secondary cytoplasmic signaling sequences). In some aspects, the CAR includes one or both of such signaling components.

The receptor, e.g., the CAR, generally includes at least one intracellular signaling component or components. In some aspects, the CAR includes a primary cytoplasmic signaling sequence that regulates primary activation of the TCR complex. Primary cytoplasmic signaling sequences that act in a stimulatory manner may contain signaling motifs which are known as immunoreceptor tyrosine-based activation motifs or ITAMs. Examples of ITAM containing primary cytoplasmic signaling sequences include those derived from CD3 zeta chain, FcR gamma, CD3 gamma, CD3 delta and CD3 epsilon. In some instances, cytoplasmic signaling molecule(s) in the CAR contain(s) a cytoplasmic signaling domain, portion thereof, or sequence derived from CD3 zeta.

In some instances, the receptor includes an intracellular component of a TCR complex, such as a TCR CD3 chain that mediates T-cell activation and cytotoxicity, e.g., CD3 zeta chain. Thus, in some aspects, the antigen-binding portion is linked to one or more cell signaling modules. In some instances, cell signaling modules include CD3 transmembrane domain, CD3 intracellular signaling domains, and/or other CD3 transmembrane domains. In some instances, the receptor, e.g., CAR, further includes a portion of one or more additional molecules such as Fc receptor γ, CD8, CD4, CD25, or CD16. For example, in some aspects, the CAR or other chimeric receptor includes a chimeric molecule between CD3-zeta (CD3-ζ) or Fc receptor γ and CD8, CD4, CD25 or CD16.

In some instances, upon ligation of the CAR or other chimeric receptor, the cytoplasmic domain or intracellular signaling domain of the receptor activates at least one of the normal effector functions or responses of the immune cell, e.g., T cell engineered to express the CAR. For example, in some contexts, the CAR induces a function of a T cell such as cytolytic activity or T-helper activity, such as secretion of cytokines or other factors. In some instances, a truncated portion of an intracellular signaling domain of an antigen receptor component or costimulatory molecule is used in place of an intact immunostimulatory chain, for example, if it transduces the effector function signal. In some instances, the intracellular signaling domain or domains include the cytoplasmic sequences of the T cell receptor (TCR), and in some aspects also those of co-receptors that in the natural context act in concert with such receptors to initiate signal transduction following antigen receptor engagement.

In the context of a natural TCR, full activation generally requires not only signaling through the TCR, but also a costimulatory signal. Thus, in some instances, to promote full activation, a component for generating secondary or co-stimulatory signal is also included in the CAR. In other instances, the CAR does not include a component for generating a costimulatory signal. In some aspects, an additional CAR is expressed in the same cell and provides the component for generating the secondary or costimulatory signal.

In some instances, the chimeric antigen receptor contains an intracellular domain of a T cell costimulatory molecule. In some instances, the CAR includes a signaling domain and/or transmembrane portion of a costimulatory receptor, such as CD28, 4-1BB, OX40, DAP10, and ICOS. In some aspects, the same CAR includes both the activating and costimulatory components. In some instances, the chimeric antigen receptor contains an intracellular domain derived from a T cell costimulatory molecule or a functional variant thereof, such as between the transmembrane domain and intracellular signaling domain. In some aspects, the T cell costimulatory molecule is CD28 or 41BB.

In some instances, the activating domain is included within one CAR, whereas the costimulatory component is provided by another CAR recognizing another antigen. In some instances, the CARs include activating or stimulatory CARs, costimulatory CARs, both expressed on the same cell (see WO2014/055668). In some aspects, the cells include one or more stimulatory or activating CAR and/or a costimulatory CAR. In some instances, the cells further include inhibitory CARs (iCARs, see Fedorov et al., Sci. Transl. Medicine, 5(215) (December, 2013), such as a CAR recognizing an antigen other than the one associated with and/or specific for the disease or condition whereby an activating signal delivered through the disease-targeting CAR is diminished or inhibited by binding of the inhibitory CAR to its ligand, e.g., to reduce off-target effects.

In some instances, the two receptors induce, respectively, an activating and an inhibitory signal to the cell, such that ligation of one of the receptor to its antigen activates the cell or induces a response, but ligation of the second inhibitory receptor to its antigen induces a signal that suppresses or dampens that response. Examples are combinations of activating CARs and inhibitory CARs (iCARs). Such a strategy may be used, for example, to reduce the likelihood of off-target effects in the context in which the activating CAR binds an antigen expressed in a disease or condition but which is also expressed on normal cells, and the inhibitory receptor binds to a separate antigen which is expressed on the normal cells but not cells of the disease or condition.

In some aspects, the chimeric receptor is or includes an inhibitory CAR (e.g. iCAR) and includes intracellular components that dampen or suppress an immune response, such as an ITAM- and/or co stimulatory-promoted response in the cell. Exemplary of such intracellular signaling components are those found on immune checkpoint molecules, including PD-1, CTLA4, LAG3, BTLA, OX2R, TIM-3, TIGIT, LAIR-1, PGE2 receptors, EP2/4 Adenosine receptors including A2AR. In some aspects, the engineered cell includes an inhibitory CAR including a signaling domain of or derived from such an inhibitory molecule, such that it serves to dampen the response of the cell, for example, that induced by an activating and/or costimulatory CAR.

In certain instances, the intracellular signaling domain comprises a CD28 transmembrane and signaling domain linked to a CD3 (e.g., CD3-zeta) intracellular domain. In some instances, the intracellular signaling domain comprises a chimeric CD28 and CD137 (4-1BB, TNFRSF9) co-stimulatory domains, linked to a CD3 zeta intracellular domain.

In some instances, the CAR encompasses one or more, e.g., two or more, costimulatory domains and an activation domain, e.g., primary activation domain, in the cytoplasmic portion. Exemplary CARs include intracellular components of CD3-zeta, CD28, and 4-1BB.

In some instances, the antigen receptor further includes a marker and/or cells expressing the CAR or other antigen receptor further includes a surrogate marker, such as a cell surface marker, which may be used to confirm transduction or engineering of the cell to express the receptor. In some aspects, the marker includes all or part (e.g., truncated form) of CD34, a NGFR, or epidermal growth factor receptor, such as truncated version of such a cell surface receptor (e.g., tEGFR). In some instances, the nucleic acid encoding the marker is operably linked to a polynucleotide encoding for a linker sequence, such as a cleavable linker sequence, e.g., T2A. For example, a marker, and optionally a linker sequence, can be any as disclosed in published patent application No. WO2014031687. For example, the marker can be a truncated EGFR (tEGFR) that is, optionally, linked to a linker sequence, such as a T2A cleavable linker sequence.

An exemplary polypeptide for a truncated EGFR (e.g. tEGFR) comprises the sequence of amino acids set forth in SEQ ID NO: 7 or 16 or a sequence of amino acids that exhibits at least 85%, 86%, 87%, 88%, 89%, 90%, 91%, 92%, 93%, 94%, 95%, 96%, 97%, 98%, 99% or more sequence identity to SEQ ID NO: 7 or 16. An exemplary T2A linker sequence comprises the sequence of amino acids set forth in SEQ ID NO: 6 or 17 or a sequence of amino acids that exhibits at least 85%, 86%, 87%, 88%, 89%, 90%, 91%, 92%, 93%, 94%, 95%, 96%, 97%, 98%, 99% or more sequence identity to SEQ ID NO: 6 or 17.

In some instances, the marker is a molecule, e.g., cell surface protein, not naturally found on T cells or not naturally found on the surface of T cells, or a portion thereof. In some instances, the molecule is a non-self molecule, e.g., non-self protein, i.e., one that is not recognized as "self' by the immune system of the host into which the cells will be adoptively transferred.

In some instances, the marker serves no therapeutic function and/or produces no effect other than to be used as a marker for genetic engineering, e.g., for selecting cells successfully engineered. In other instances, the marker may be a therapeutic molecule or molecule otherwise exerting some desired effect, such as a ligand for a cell to be encountered in vivo, such as a costimulatory or immune checkpoint molecule to enhance and/or dampen responses of the cells upon adoptive transfer and encounter with ligand.

In some cases, CARs are referred to as first, second, and/or third generation CARs. In some aspects, a first generation CAR is one that solely provides a CD3-chain induced signal upon antigen binding; in some aspects, a second-generation CARs is one that provides such a signal and costimulatory signal, such as one including an intracellular signaling domain from a costimulatory receptor such as CD28 or CD137; in some aspects, a third generation CAR is one that includes multiple costimulatory domains of different costimulatory receptors.

For example, in some instances, the CAR contains an antibody, e.g., an antibody fragment, such as an scFv, specific to an antigen including any as described, a transmembrane domain that is or contains a transmembrane portion of CD28 or a functional variant thereof, and an intracellular signaling domain containing a signaling portion of CD28 or functional variant thereof and a signaling portion of CD3 zeta or functional variant thereof. In some instances, the CAR contains an antibody, e.g., antibody fragment, such as an scFv, specific to an antigen including any as described, a transmembrane domain that is or contains a transmembrane portion of CD28 or a functional variant thereof, and an intracellular signaling domain containing a signaling portion of a 4-1BB or functional variant thereof and a signaling portion of CD3 zeta or functional variant thereof. In some such instances, the receptor further includes a spacer containing a portion of an Ig molecule, such as a human Ig molecule, such as an Ig hinge, e.g. an IgG4 hinge, such as a hinge-only spacer.

In some instances, the transmembrane domain of the recombinant receptor, e.g., the CAR, is or includes a transmembrane domain of human CD28 (e.g. Accession No. P01747.1) or variant thereof, such as a transmembrane domain that comprises the sequence of amino acids set forth in SEQ ID NO: 8 or a sequence of amino acids that exhibits at least 85%, 86%, 87%, 88%, 89%, 90%, 91%, 92%, 93%, 94%, 95%, 96%, 97%, 98%, 99% or more sequence identity to SEQ ID NO: 8; in some instances, the transmembrane-domain containing portion of the recombinant receptor comprises the sequence of amino acids set forth in SEQ ID NO: 9 or a sequence of amino acids having at least at or about 85%, 86%, 87%, 88%, 89%, 90%, 91%, 92%, 93%, 94%, 95%, 96%, 97%, 98%, 99% or more sequence identity thereto.

In some instances, the intracellular signaling component(s) of the recombinant receptor, *e.g.* the CAR, contains an intracellular costimulatory signaling domain of human CD28 or a functional variant or portion thereof, such as a domain with an LL to GG substitution at positions 186-187 of a native CD28 protein. For example, the intracellular signaling domain can comprise the sequence of amino acids set forth in SEQ ID NO: 10 or 11 or a sequence of amino acids that exhibits at least 85%, 86%, 87%, 88%, 89%, 90%, 91%, 92%, 93%, 94%, 95%, 96%, 97%, 98%, 99% or more sequence identity to SEQ ID NO: 10 or 11. In some instances, the intracellular domain comprises an intracellular costimulatory signaling domain of 4-1BB (e.g. (Accession No. Q07011.1) or functional variant or portion thereof, such as the sequence of amino acids set forth in SEQ ID NO: 12 or a sequence of amino acids that exhibits at least 85%, 86%, 87%, 88%, 89%, 90%, 91%, 92%, 93%, 94%, 95%, 96%, 97%, 98%, 99% or more sequence identity to SEQ ID NO: 12.

In some instances, the intracellular signaling domain of the recombinant receptor, *e.g.* the CAR, comprises a human CD3 zeta stimulatory signaling domain or functional variant thereof, such as an 112 AA cytoplasmic domain of isoform 3 of human CD3ζ (Accession No.: P20963.2) or a CD3 zeta signaling domain as described in U.S. Patent No.: 7,446,190 or U.S. Patent No. 8,911,993. For example, in some instances, the intracellular signaling domain comprises the sequence of amino acids as set forth in SEQ ID NO: 13, 14 or 15 or a sequence of amino acids that exhibits at least 85%, 86%, 87%, 88%, 89%, 90%, 91%, 92%, 93%, 94%, 95%, 96%, 97%, 98%, 99% or more sequence identity to SEQ ID NO: 13, 14 or 15.

In some aspects, the spacer contains only a hinge region of an IgG, such as only a hinge of IgG4 or IgG1, such as the hinge only spacer set forth in SEQ ID NO: 1. In other instances, the spacer is or contains an Ig hinge, e.g., an IgG4-derived hinge, optionally linked to a CH2 and/or CH3 domains. In some instances, the spacer is an Ig hinge, e.g., an IgG4 hinge, linked to CH2 and CH3 domains, such as set forth in SEQ ID NO: 4. In some instances, the spacer is an Ig hinge, e.g., an IgG4 hinge, linked to a CH3 domain only, such as set forth in SEQ ID NO: 3. In some instances, the spacer is or comprises a glycine-serine rich sequence or other flexible linker such as known flexible linkers.

For example, in some instances, the CAR includes an antibody such as an antibody fragment, including scFvs, a spacer, such as a spacer containing a portion of an immunoglobulin molecule, such as a hinge region and/or one or more constant regions of a heavy chain molecule, such as an Ig-hinge containing spacer, a transmembrane domain containing all or a portion of a CD28-derived transmembrane domain, a CD28-derived intracellular signaling domain, and a CD3 zeta signaling domain. In some instances, the CAR includes an antibody or fragment, such as scFv, a spacer such as any of the Ig-hinge containing spacers, a CD28-derived transmembrane domain, a 4-1BB-derived intracellular signaling domain, and a CD3 zeta-derived signaling domain.

Exemplary surrogate markers can include truncated forms of cell surface polypeptides, such as truncated forms that are non-functional and to not transduce or are not capable of transducing a signal or a signal ordinarily transduced by the full-length form of the cell surface polypeptide, and/or do not or are not capable of internalizing. Exemplary truncated cell surface polypeptides including truncated forms of growth factors or other receptors such as a truncated human epidermal growth factor receptor 2 (tHER2), a truncated epidermal growth factor receptor (tEGFR, exemplary tEGFR sequence set forth in 7 or 16) or a prostate-specific membrane antigen (PSMA) or modified form thereof. tEGFR may contain an epitope recognized by the antibody cetuximab (Erbitux^{®}) or other therapeutic anti-EGFR antibody or binding molecule, which can be used to identify or select cells that have been engineered to express the tEGFR construct and an encoded exogenous protein, and/or to eliminate or separate cells expressing the encoded exogenous protein. See U.S. Patent No. 8,802,374 and Liu et al., Nature Biotech. 2016 April; 34(4): 430-434). In some aspects, the marker, *e*.*g*. surrogate marker, includes all or part (*e*.*g*., truncated form) of CD34, a NGFR, a CD19 or a truncated CD19, e.g., a truncated non-human CD19, or epidermal growth factor receptor (*e.g.,* tEGFR). In some instances, the marker is or comprises a fluorescent protein, such as green fluorescent protein (GFP), enhanced green fluorescent protein (EGFP), such as super-fold GFP (sfGFP), red fluorescent protein (RFP), such as tdTomato, mCherry, mStrawberry, AsRed2, DsRed or DsRed2, cyan fluorescent protein (CFP), blue green fluorescent protein (BFP), enhanced blue fluorescent protein (EBFP), and yellow fluorescent protein (YFP), and variants thereof, including species variants, monomeric variants, and codon-optimized and/or enhanced variants of the fluorescent proteins. In some instances, the marker is or comprises an enzyme, such as a luciferase, the lacZ gene from *E. coli,* alkaline phosphatase, secreted embryonic alkaline phosphatase (SEAP), chloramphenicol acetyl transferase (CAT). Exemplary light-emitting reporter genes include luciferase (luc), β-galactosidase, chloramphenicol acetyltransferase (CAT), β-glucuronidase (GUS) or variants thereof.

In some instances, the marker is a selection marker. In some instances, the selection marker is or comprises a polypeptide that confers resistance to exogenous agents or drugs. In some instances, the selection marker is an antibiotic resistance gene. In some instances, the selection marker is an antibiotic resistance gene confers antibiotic resistance to a mammalian cell. In some instances, the selection marker is or comprises a Puromycin resistance gene, a Hygromycin resistance gene, a Blasticidin resistance gene, a Neomycin resistance gene, a Geneticin resistance gene or a Zeocin resistance gene or a modified form thereof.

In some instances, the nucleic acid encoding the marker is operably linked to a polynucleotide encoding for a linker sequence, such as a cleavable linker sequence, e.g., a T2A. For example, a marker, and optionally a linker sequence, can be any as disclosed in PCT Pub. No. WO2014031687.

In some instances, nucleic acid molecules encoding such CAR constructs further includes a sequence encoding a T2A ribosomal skip element and/or a tEGFR sequence, e.g., downstream of the sequence encoding the CAR. In some instances, the sequence encodes a T2A ribosomal skip element set forth in SEQ ID NO: 6 or 17, or a sequence of amino acids that exhibits at least 85%, 86%, 87%, 88%, 89%, 90%, 91%, 92%, 93%, 94%, 95%, 96%, 97%, 98%, 99% or more sequence identity to SEQ ID NO: 6 or 17. In some instances, T cells expressing an antigen receptor (e.g. CAR) can also be generated to express a truncated EGFR (EGFRt) as a non-immunogenic selection epitope (e.g. by introduction of a construct encoding the CAR and EGFRt separated by a T2A ribosome switch to express two proteins from the same construct), which then can be used as a marker to detect such cells (see e.g. U.S. Patent No. 8,802,374). In some instances, the sequence encodes an tEGFR sequence set forth in SEQ ID NO: 7 or 16, or a sequence of amino acids that exhibits at least 85%, 86%, 87%, 88%, 89%, 90%, 91%, 92%, 93%, 94%, 95%, 96%, 97%, 98%, 99% or more sequence identity to SEQ ID NO: 7 or 16. In some cases, the peptide, such as T2A, can cause the ribosome to skip (ribosome skipping) synthesis of a peptide bond at the C-terminus of a 2A element, leading to separation between the end of the 2A sequence and the next peptide downstream (*see,* for example, de Felipe. Genetic Vaccines and Ther. 2:13 (2004) and deFelipe et al. Traffic 5:616-626 (2004)). Many 2A elements are known. Examples of 2A sequences that can be used in the methods and nucleic acids disclosed herein, without limitation, 2A sequences from the foot-and-mouth disease virus (F2A, *e.g.,* SEQ ID NO: 21), equine rhinitis A virus (E2A, *e.g.,* SEQ ID NO: 20), Thosea asigna virus (T2A, *e.g.,* SEQ ID NO: 6 or 17), and porcine teschovirus-1 (P2A, *e.g.,* SEQ ID NO: 18 or 19) as described in U.S. Patent Publication No. 20070116690.

In some cases, the nucleic acid sequence encoding the recombinant receptor, e.g., chimeric antigen receptor (CAR) contains a signal sequence that encodes a signal peptide. Nonlimiting exemplary examples of signal peptides include, for example, the GMCSFR alpha chain signal peptide set forth in SEQ ID NO: 62 and encoded by the nucleotide sequence set forth in SEQ ID NO: 61, the CD8 alpha signal peptide set forth in SEQ ID NO: 60, or the CD33 signal peptide set forth in SEQ ID NO:59.

The recombinant receptors, such as CARs, expressed by the cells administered to the subject generally recognize or specifically bind to a molecule that is expressed in, associated with, and/or specific for the disease or condition or cells thereof being treated. Upon specific binding to the molecule, e.g., antigen, the receptor generally delivers an immunostimulatory signal, such as an ITAM-transduced signal, into the cell, thereby promoting an immune response targeted to the disease or condition. For example, in some instances, the cells express a CAR that specifically binds to an antigen expressed by a cell or tissue of the disease or condition or associated with the disease or condition.

### B. TCRs

In some instances, engineered cells, such as T cells, are disclosed that express a T cell receptor (TCR) or antigen-binding portion thereof that recognizes an peptide epitope or T cell epitope of a target polypeptide, such as an antigen of a tumor, viral or autoimmune protein.

In some instances, a "T cell receptor" or "TCR" is a molecule that contains a variable α and β chains (also known as TCRα and TCRβ, respectively) or a variable γ and δ chains (also known as TCRα and TCRβ, respectively), or antigen-binding portions thereof, and which is capable of specifically binding to a peptide bound to an MHC molecule. In some instances, the TCR is in the αβ form. Typically, TCRs that exist in αβ and γδ forms are generally structurally similar, but T cells expressing them may have distinct anatomical locations or functions. A TCR can be found on the surface of a cell or in soluble form. Generally, a TCR is found on the surface of T cells (or T lymphocytes) where it is generally responsible for recognizing antigens bound to major histocompatibility complex (MHC) molecules.

Unless otherwise stated, the term "TCR" should be understood to encompass full TCRs as well as antigen-binding portions or antigen-binding fragments thereof. In some instances, the TCR is an intact or full-length TCR, including TCRs in the αβ form or γδ form. In some instances, the TCR is an antigen-binding portion that is less than a full-length TCR but that binds to a specific peptide bound in an MHC molecule, such as binds to an MHC-peptide complex. In some cases, an antigen-binding portion or fragment of a TCR can contain only a portion of the structural domains of a full-length or intact TCR, but yet is able to bind the peptide epitope, such as MHC-peptide complex, to which the full TCR binds. In some cases, an antigen-binding portion contains the variable domains of a TCR, such as variable α chain and variable β chain of a TCR, sufficient to form a binding site for binding to a specific MHC-peptide complex. Generally, the variable chains of a TCR contain complementarity determining regions involved in recognition of the peptide, MHC and/or MHC-peptide complex.

In some instances, the variable domains of the TCR contain hypervariable loops, or complementarity determining regions (CDRs), which generally are the primary contributors to antigen recognition and binding capabilities and specificity. In some instances, a CDR of a TCR or combination thereof forms all or substantially all of the antigen-binding site of a given TCR molecule. The various CDRs within a variable region of a TCR chain generally are separated by framework regions (FRs), which generally display less variability among TCR molecules as compared to the CDRs (see, e.g., Jores et al., Proc. Nat'l Acad. Sci. U.S.A. 87:9138, 1990; Chothia et al., EMBO J. 7:3745, 1988; see also Lefranc et al., Dev. Comp. Immunol. 27:55, 2003). In some instances, CDR3 is the main CDR responsible for antigen binding or specificity, or is the most important among the three CDRs on a given TCR variable region for antigen recognition, and/or for interaction with the processed peptide portion of the peptide-MHC complex. In some contexts, the CDR1 of the alpha chain can interact with the N-terminal part of certain antigenic peptides. In some contexts, CDR1 of the beta chain can interact with the C-terminal part of the peptide. In some contexts, CDR2 contributes most strongly to or is the primary CDR responsible for the interaction with or recognition of the MHC portion of the MHC-peptide complex. In some instances, the variable region of the β-chain can contain a further hypervariable region (CDR4 or HVR4), which generally is involved in superantigen binding and not antigen recognition (Kotb (1995) Clinical Microbiology Reviews, 8:411-426).

In some instances, a TCR also can contain a constant domain, a transmembrane domain and/or a short cytoplasmic tail (see, e.g., Janeway et al., Immunobiology: The Immune System in Health and Disease, 3rd Ed., Current Biology Publications, p. 4:33, 1997). In some aspects, each chain of the TCR can possess one N-terminal immunoglobulin variable domain, one immunoglobulin constant domain, a transmembrane region, and a short cytoplasmic tail at the C-terminal end. In some instances, a TCR is associated with invariant proteins of the CD3 complex involved in mediating signal transduction.

In some instances, a TCR chain contains one or more constant domain. For example, the extracellular portion of a given TCR chain (e.g., α-chain or β-chain) can contain two immunoglobulin-like domains, such as a variable domain (e.g., Vα or Vβ; typically amino acids 1 to 116 based on Kabat numbering Kabat et al., "Sequences of Proteins of Immunological Interest, US Dept. Health and Human Services, Public Health Service National Institutes of Health, 1991, 5th ed.) and a constant domain (e.g., α-chain constant domain or Cα, typically positions 117 to 259 of the chain based on Kabat numbering or β chain constant domain or Cβ, typically positions 117 to 295 of the chain based on Kabat) adjacent to the cell membrane. For example, in some cases, the extracellular portion of the TCR formed by the two chains contains two membrane-proximal constant domains, and two membrane-distal variable domains, which variable domains each contain CDRs. The constant domain of the TCR may contain short connecting sequences in which a cysteine residue forms a disulfide bond, thereby linking the two chains of the TCR. In some instances, a TCR may have an additional cysteine residue in each of the α and β chains, such that the TCR contains two disulfide bonds in the constant domains.

In some instances, the TCR chains contain a transmembrane domain. In some instances, the transmembrane domain is positively charged. In some cases, the TCR chain contains a cytoplasmic tail. In some cases, the structure allows the TCR to associate with other molecules like CD3 and subunits thereof. For example, a TCR containing constant domains with a transmembrane region may anchor the protein in the cell membrane and associate with invariant subunits of the CD3 signaling apparatus or complex. The intracellular tails of CD3 signaling subunits (e.g. CD3γ, CD3δ, CD3ε and CD3ζ chains) contain one or more immunoreceptor tyrosine-based activation motif or ITAM that are involved in the signaling capacity of the TCR complex.

In some instances, the TCR may be a heterodimer of two chains α and β (or optionally γ and δ) or it may be a single chain TCR construct. In some instances, the TCR is a heterodimer containing two separate chains (α and β chains or γ and δ chains) that are linked, such as by a disulfide bond or disulfide bonds.

In some instances, the TCR can be generated from a known TCR sequence(s), such as sequences of Vα,β chains, for which a substantially full-length coding sequence is readily available. Methods for obtaining full-length TCR sequences, including V chain sequences, from cell sources are well known. In some instances, nucleic acids encoding the TCR can be obtained from a variety of sources, such as by polymerase chain reaction (PCR) amplification of TCR-encoding nucleic acids within or isolated from a given cell or cells, or synthesis of publicly available TCR DNA sequences.

In some instances, the TCR is obtained from a biological source, such as from cells such as from a T cell (e.g. cytotoxic T cell), T-cell hybridomas or other publicly available source. In some instances, the T-cells can be obtained from *in vivo* isolated cells. In some instances, the TCR is a thymically selected TCR. In some instances, the TCR is a neoepitope-restricted TCR. In some instances, the T- cells can be a cultured T-cell hybridoma or clone. In some instances, the TCR or antigen-binding portion thereof or antigen-binding fragment thereof can be synthetically generated from knowledge of the sequence of the TCR.

In some instances, the TCR is generated from a TCR identified or selected from screening a library of candidate TCRs against a target polypeptide antigen, or target T cell epitope thereof. TCR libraries can be generated by amplification of the repertoire of Vα and Vβ from T cells isolated from a subject, including cells present in PBMCs, spleen or other lymphoid organ. In some cases, T cells can be amplified from tumor-infiltrating lymphocytes (TILs). In some instances, TCR libraries can be generated from CD4+ or CD8+ T cells. In some instances, the TCRs can be amplified from a T cell source of a normal of healthy subject, i.e. normal TCR libraries. In some instances, the TCRs can be amplified from a T cell source of a diseased subject, i.e. diseased TCR libraries. In some instances, degenerate primers are used to amplify the gene repertoire of Vα and Vβ, such as by RT-PCR in samples, such as T cells, obtained from humans. In some instances, scTv libraries can be assembled from naïve Vα and Vβ libraries in which the amplified products are cloned or assembled to be separated by a linker. Depending on the source of the subject and cells, the libraries can be HLA allele-specific. Alternatively, in some instances, TCR libraries can be generated by mutagenesis or diversification of a parent or scaffold TCR molecule. In some aspects, the TCRs are subjected to directed evolution, such as by mutagenesis, e.g., of the α or β chain. In some aspects, particular residues within CDRs of the TCR are altered. In some instances, selected TCRs can be modified by affinity maturation. In some instances, antigen-specific T cells may be selected, such as by screening to assess CTL activity against the peptide. In some aspects, TCRs, e.g. present on the antigen-specific T cells, may be selected, such as by binding activity, e.g., particular affinity or avidity for the antigen.

In some instances, the TCR or antigen-binding portion thereof is one that has been modified or engineered. In some instances, directed evolution methods are used to generate TCRs with altered properties, such as with higher affinity for a specific MHC-peptide complex. In some instances, directed evolution is achieved by display methods including, but not limited to, yeast display (Holler et al. (2003) Nat Immunol, 4, 55-62; Holler et al. (2000) Proc Natl Acad Sci USA, 97, 5387-92), phage display (Li et al. (2005) Nat Biotechnol, 23, 349-54), or T cell display (Chervin et al. (2008) J Immunol Methods, 339, 175-84). In some instances, display approaches involve engineering, or modifying, a known, parent or reference TCR. For example, in some cases, a wild-type TCR can be used as a template for producing mutagenized TCRs in which in one or more residues of the CDRs are mutated, and mutants with an desired altered property, such as higher affinity for a desired target antigen, are selected.

In some instances, peptides of a target polypeptide for use in producing or generating a TCR of interest are known or can be readily identified. In some instances, peptides suitable for use in generating TCRs or antigen-binding portions can be determined based on the presence of an HLA-restricted motif in a target polypeptide of interest, such as a target polypeptide described below. In some instances, peptides are identified using available computer prediction models. In some instances, for predicting MHC class I binding sites, such models include, but are not limited to, ProPred1 (Singh and Raghava (2001) Bioinformatics 17(12):1236-1237, and SYFPEITHI (see Schuler et al. (2007) Immunoinformatics Methods in Molecular Biology, 409(1): 75-93 2007). In some instances, the MHC-restricted epitope is HLA-A0201, which is expressed in approximately 39-46% of all Caucasians and therefore, represents a suitable choice of MHC antigen for use preparing a TCR or other MHC-peptide binding molecule.

HLA-A0201-binding motifs and the cleavage sites for proteasomes and immune-proteasomes using computer prediction models are known. For predicting MHC class I binding sites, such models include, but are not limited to, ProPred1 (described in more detail in Singh and Raghava, ProPred: prediction of HLA-DR binding sites. BIOINFORMATICS 17(12):1236-1237 2001), and SYFPEITHI (see Schuler et al. SYFPEITHI, Database for Searching and T-Cell Epitope Prediction. in Immunoinformatics Methods in Molecular Biology, vol 409(1): 75-93 2007).

In some instances, the TCR or antigen binding portion thereof may be a recombinantly produced natural protein or mutated form thereof in which one or more property, such as binding characteristic, has been altered. In some instances, a TCR may be derived from one of various animal species, such as human, mouse, rat, or other mammal. A TCR may be cell-bound or in soluble form. In some instances, for purposes of the disclosed methods, the TCR is in cell-bound form expressed on the surface of a cell.

In some instances, the TCR is a full-length TCR. In some instances, the TCR is an antigen-binding portion. In some instances, the TCR is a dimeric TCR (dTCR). In some instances, the TCR is a single-chain TCR (sc-TCR). In some instances, a dTCR or scTCR have the structures as described in WO 03/020763, WO 04/033685, WO2011/044186.

In some instances, the TCR contains a sequence corresponding to the transmembrane sequence. In some instances, the TCR does contain a sequence corresponding to cytoplasmic sequences. In some instances, the TCR is capable of forming a TCR complex with CD3. In some instances, any of the TCRs, including a dTCR or scTCR, can be linked to signaling domains that yield an active TCR on the surface of a T cell. In some instances, the TCR is expressed on the surface of cells.

In some instances a dTCR contains a first polypeptide wherein a sequence corresponding to a TCR α chain variable region sequence is fused to the N terminus of a sequence corresponding to a TCR α chain constant region extracellular sequence, and a second polypeptide wherein a sequence corresponding to a TCR β chain variable region sequence is fused to the N terminus a sequence corresponding to a TCR β chain constant region extracellular sequence, the first and second polypeptides being linked by a disulfide bond. In some instances, the bond can correspond to the native inter-chain disulfide bond present in native dimeric αβ TCRs. In some instances, the interchain disulfide bonds are not present in a native TCR. For example, in some instances, one or more cysteines can be incorporated into the constant region extracellular sequences of dTCR polypeptide pair. In some cases, both a native and a non-native disulfide bond may be desirable. In some instances, the TCR contains a transmembrane sequence to anchor to the membrane.

In some instances, a dTCR contains a TCR α chain containing a variable α domain, a constant α domain and a first dimerization motif attached to the C-terminus of the constant α domain, and a TCR β chain comprising a variable β domain, a constant β domain and a first dimerization motif attached to the C-terminus of the constant β domain, wherein the first and second dimerization motifs easily interact to form a covalent bond between an amino acid in the first dimerization motif and an amino acid in the second dimerization motif linking the TCR α chain and TCR β chain together.

In some instances, the TCR is a scTCR. Typically, a scTCR can be generated using methods known, See e.g., Soo Hoo, W. F. et al. PNAS (USA) 89, 4759 (1992); Wülfing, C. and Plückthun, A., J. Mol. Biol. 242, 655 (1994); Kurucz, I. et al. PNAS (USA) 90 3830 (1993); International published PCT Nos. WO 96/13593, WO 96/18105, WO99/60120, WO99/18129, WO 03/020763, WO2011/044186; and Schlueter, C. J. et al. J. Mol. Biol. 256, 859 (1996). In some instances, a scTCR contains an introduced non-native disulfide interchain bond to facilitate the association of the TCR chains (see e.g. International published PCT No. WO 03/020763). In some instances, a scTCR is a non-disulfide linked truncated TCR in which heterologous leucine zippers fused to the C-termini thereof facilitate chain association (see e.g. International published PCT No. WO99/60120). In some instances, a scTCR contain a TCRα variable domain covalently linked to a TCRβ variable domain via a peptide linker (see e.g., International published PCT No. WO99/18129).

In some instances, a scTCR contains a first segment constituted by an amino acid sequence corresponding to a TCR α chain variable region, a second segment constituted by an amino acid sequence corresponding to a TCR β chain variable region sequence fused to the N terminus of an amino acid sequence corresponding to a TCR β chain constant domain extracellular sequence, and a linker sequence linking the C terminus of the first segment to the N terminus of the second segment.

In some instances, a scTCR contains a first segment constituted by an α chain variable region sequence fused to the N terminus of an α chain extracellular constant domain sequence, and a second segment constituted by a β chain variable region sequence fused to the N terminus of a sequence β chain extracellular constant and transmembrane sequence, and, optionally, a linker sequence linking the C terminus of the first segment to the N terminus of the second segment.

In some instances, a scTCR contains a first segment constituted by a TCR β chain variable region sequence fused to the N terminus of a β chain extracellular constant domain sequence, and a second segment constituted by an α chain variable region sequence fused to the N terminus of a sequence α chain extracellular constant and transmembrane sequence, and, optionally, a linker sequence linking the C terminus of the first segment to the N terminus of the second segment.

In some instances, the linker of a scTCRs that links the first and second TCR segments can be any linker capable of forming a single polypeptide strand, while retaining TCR binding specificity. In some instances, the linker sequence may, for example, have the formula - P-AA-P- wherein P is proline and AA represents an amino acid sequence wherein the amino acids are glycine and serine. In some instances, the first and second segments are paired so that the variable region sequences thereof are orientated for such binding. Hence, in some cases, the linker has a sufficient length to span the distance between the C terminus of the first segment and the N terminus of the second segment, or vice versa, but is not too long to block or reduces bonding of the scTCR to the target ligand. In some instances, the linker can contain from 10 to 45 amino acids or from about 10 to about 45 amino acids, such as 10 to 30 amino acids or 26 to 41 amino acids residues, for example 29, 30, 31 or 32 amino acids. In some instances, the linker has the formula -PGGG-(SGGGG)5-P- wherein P is proline, G is glycine and S is serine (SEQ ID NO:28). In some instances, the linker has the sequence GSADDAKKDAAKKDGKS (SEQ ID NO:29).

In some instances, the scTCR contains a covalent disulfide bond linking a residue of the immunoglobulin region of the constant domain of the α chain to a residue of the immunoglobulin region of the constant domain of the β chain. In some instances, the interchain disulfide bond in a native TCR is not present. For example, in some instances, one or more cysteines can be incorporated into the constant region extracellular sequences of the first and second segments of the scTCR polypeptide. In some cases, both a native and a non-native disulfide bond may be desirable.

In some instances of a dTCR or scTCR containing introduced interchain disulfide bonds, the native disulfide bonds are not present. In some instances, the one or more of the native cysteines forming a native interchain disulfide bonds are substituted to another residue, such as to a serine or alanine. In some instances, an introduced disulfide bond can be formed by mutating non-cysteine residues on the first and second segments to cysteine. Exemplary non-native disulfide bonds of a TCR are described in published International PCT No. WO2006/000830.

In some instances, the TCR or antigen-binding fragment thereof exhibits an affinity with an equilibrium binding constant for a target antigen of between or between about 10-5 and 10-12 M and all individual values and ranges therein. In some instances, the target antigen is an MHC-peptide complex or ligand.

In some instances, nucleic acid or nucleic acids encoding a TCR, such as α and β chains, can be amplified by PCR, cloning or other suitable means and cloned into a suitable expression vector or vectors. The expression vector can be any suitable recombinant expression vector, and can be used to transform or transfect any suitable host. Suitable vectors include those designed for propagation and expansion or for expression or both, such as plasmids and viruses.

In some instances, the vector can a vector of the pUC series (Fermentas Life Sciences), the pBluescript series (Stratagene, LaJolla, Calif.), the pET series (Novagen, Madison, Wis.), the pGEX series (Pharmacia Biotech, Uppsala, Sweden), or the pEX series (Clontech, Palo Alto, Calif.). In some cases, bacteriophage vectors, such as λG10, λGT11, λZapII (Stratagene), λEMBL4, and λNM1149, also can be used. In some instances, plant expression vectors can be used and include pBI01, pBI101.2, pBI101.3, pBI121 and pBIN19 (Clontech). In some instances, animal expression vectors include pEUK-Cl, pMAM and pMAMneo (Clontech). In some instances, a viral vector is used, such as a retroviral vector.

In some instances, the recombinant expression vectors can be prepared using standard recombinant DNA techniques. In some instances, vectors can contain regulatory sequences, such as transcription and translation initiation and termination codons, which are specific to the type of host (e.g., bacterium, fungus, plant, or animal) into which the vector is to be introduced, as appropriate and taking into consideration whether the vector is DNA- or RNA-based. In some instances, the vector can contain a nonnative promoter operably linked to the nucleotide sequence encoding the TCR or antigen-binding portion (or other MHC-peptide binding molecule). In some instances, the promoter can be a non-viral promoter or a viral promoter, such as a cytomegalovirus (CMV) promoter, an SV40 promoter, an RSV promoter, and a promoter found in the long-terminal repeat of the murine stem cell virus. Other known promoters also are contemplated.

In some instances, to generate a vector encoding a TCR, the α and β chains are PCR amplified from total cDNA isolated from a T cell clone expressing the TCR of interest and cloned into an expression vector. In some instances, the α and β chains are cloned into the same vector. In some instances, the α and β chains are cloned into different vectors. In some instances, the generated α and β chains are incorporated into a retroviral, e.g. lentiviral, vector.

### V. COMPOSITIONS, FORMULATIONS, AND METHODS OF ADMINISTRATION

In some instances, output compositions of enriched T cells produced by the methods disclosed herein, such as described in Section I, are administered as a cell therapy, e.g., an adoptive cell therapy. In particular instances, one or more cell compositions, e.g., output cell compositions described herein are administered as a cell therapy. In some instances, adoptive T cell therapy methods are described, e.g., in US Patent Application Publication No. 2003/0170238 to Gruenberg et al; US Patent No. 4,690,915 to Rosenberg; Rosenberg (2011) Nat Rev Clin Oncol. 8(10):577-85). See, e.g., Themeli et al. (2013) Nat Biotechnol. 31(10): 928-933; Tsukahara et al. (2013) Biochem Biophys Res Commun 438(1): 84-9; Davila et al. (2013) PLoS ONE 8(4): e61338.

In certain instances, the methods disclosed herein produce a single output composition of enriched T cells from input cells isolated, selected and/or enriched from a single biological sample that is administered as a cell therapy. In particular instances, the single output composition is a composition of enriched CD4+ T cells. In certain instances, the single output composition is a composition of enriched CD4+ and CD8+ T cells. In some instances, the methods disclosed herein produce two or more output compositions from a single source, e.g., a biological sample and/or input compositions isolated, selected, or enriched from a biological sample, that are administered to a subject. In some instances, the two or more output compositions are administered to the subject separately. In certain instances, the two or more output compositions are combined into a single composition and administered to the subject. In certain instances, the two or more output compositions include an output composition of enriched CD4+ T cells. In particular instances, the two or more output compositions include an output composition of enriched CD8 + T cells.

In some instances, an output composition of enriched CD4+ T cells that is administered to a subject includes at least 60%, at least 65%, at least 70%, at least 75%, at least 80%, at least 85%, at least 90%, at least 95%, at least 98%, at least 99%, at least 99.5%, at least 99.9%, or at or at about 100% CD4+ T cells. In certain instances, the output composition includes at least 30%, at least 40%, at least 50%, at least 60%, at least 70%, at least 80%, at least 90%, at least 95%, at least 98%, at least 99%, at least 99.5%, at least 99.9%, or at or at about 100% CD4+ T cells that express the recombinant receptor and/or have been transduced or transfected with the recombinant polynucleotide. In certain instances, the output composition of enriched CD4+ T cells that is administered to the subject includes less than 40%, less than 35%, less than 30%, less than 25%, less than 20%, less than 15%, less than 10%, less than 5%, less than 1%, less than 0.1%, or less than 0.01% CD8+ T cells, and/or contains no CD8+ T cells, and/or is free or substantially free of CD8+ T cells.

In some instances, an output composition of enriched CD8+ T cells that is administered to a subject includes at least 60%, at least 65%, at least 70%, at least 75%, at least 80%, at least 85%, at least 90%, at least 95%, at least 98%, at least 99%, at least 99.5%, at least 99.9%, or at or at about 100% CD8+ T cells. In particular instances, the composition includes at least 30%, at least 40%, at least 50%, at least 60%, at least 70%, at least 80%, at least 90%, at least 95%, at least 98%, at least 99%, at least 99.5%, at least 99.9%, or at or at about 100% CD8+ T cells that express the recombinant receptor and/or have been transduced or transfected with the recombinant polynucleotide. In certain instances, the output composition of enriched CD8+ T cells that is administered to the subject includes less than 40%, less than 35%, less than 30%, less than 25%, less than 20%, less than 15%, less than 10%, less than 5%, less than 1%, less than 0.1%, or less than 0.01% CD4+ T cells, and/or contains no CD4+ T cells, and/or is free or substantially free of CD4+ T cells.

The disease or condition that is treated can be any in which expression of an antigen is associated with and/or involved in the etiology of a disease condition or disorder, e.g. causes, exacerbates or otherwise is involved in such disease, condition, or disorder. Exemplary diseases and conditions can include diseases or conditions associated with malignancy or transformation of cells (e.g. cancer), autoimmune or inflammatory disease, or an infectious disease, e.g. caused by a bacterial, viral or other pathogen. Exemplary antigens, which include antigens associated with various diseases and conditions that can be treated, are described above. In particular instances, the chimeric antigen receptor or transgenic TCR specifically binds to an antigen associated with the disease or condition.

Among the diseases, conditions, and disorders are tumors, including solid tumors, hematologic malignancies, and melanomas, and including localized and metastatic tumors, infectious diseases, such as infection with a virus or other pathogen, e.g., HIV, HCV, HBV, CMV, HPV, and parasitic disease, and autoimmune and inflammatory diseases. In some instances, the disease, disorder or condition is a tumor, cancer, malignancy, neoplasm, or other proliferative disease or disorder. Such diseases include but are not limited to leukemia, lymphoma, e.g., acute myeloid (or myelogenous) leukemia (AML), chronic myeloid (or myelogenous) leukemia (CML), acute lymphocytic (or lymphoblastic) leukemia (ALL), chronic lymphocytic leukemia (CLL), hairy cell leukemia (HCL), small lymphocytic lymphoma (SLL), Mantle cell lymphoma (MCL), Marginal zone lymphoma, Burkitt lymphoma, Hodgkin lymphoma (HL), non-Hodgkin lymphoma (NHL), Anaplastic large cell lymphoma (ALCL), follicular lymphoma, refractory follicular lymphoma, diffuse large B-cell lymphoma (DLBCL) and multiple myeloma (MM). In some instances, disease or condition is a B cell malignancy selected from among acute lymphoblastic leukemia (ALL), adult ALL, chronic lymphoblastic leukemia (CLL), non-Hodgkin lymphoma (NHL), and Diffuse Large B-Cell Lymphoma (DLBCL). In some instances, the disease or condition is NHL and the NHL is selected from the group consisting of aggressive NHL, diffuse large B cell lymphoma (DLBCL), NOS (de novo and transformed from indolent), primary mediastinal large B cell lymphoma (PMBCL), T cell/histocyte-rich large B cell lymphoma (TCHRBCL), Burkitt's lymphoma, mantle cell lymphoma (MCL), and/or follicular lymphoma (FL), optionally, follicular lymphoma Grade 3B (FL3B). In some aspects, the recombinant receptor, such as a CAR, specifically binds to an antigen associated with the disease or condition or expressed in cells of the environment of a lesion associated with the B cell malignancy. Antigens targeted by the receptors in some instances include antigens associated with a B cell malignancy, such as any of a number of known B cell marker. In some instances, the antigen targeted by the receptor is CD20, CD19, CD22, ROR1, CD45, CD21, CD5, CD33, Igkappa, Iglambda, CD79a, CD79b or CD30, or combinations thereof.

In some instances, the disease or condition is a myeloma, such as a multiple myeloma. In some aspects, the recombinant receptor, such as a CAR, specifically binds to an antigen associated with the disease or condition or expressed in cells of the environment of a lesion associated with the multiple myeloma. Antigens targeted by the receptors in some instances include antigens associated with multiple myeloma. In some aspects, the antigen, e.g., the second or additional antigen, such as the disease-specific antigen and/or related antigen, is expressed on multiple myeloma, such as B cell maturation antigen (BCMA), G protein-coupled receptor class C group 5 member D (GPRC5D), CD38 (cyclic ADP ribose hydrolase), CD138 (syndecan-1, syndecan, SYN-1), CS-1 (CS1, CD2 subset 1, CRACC, SLAMF7, CD319, and 19A24), BAFF-R, TACI and/or FcRH5. Other exemplary multiple myeloma antigens include CD56, TIM-3, CD33, CD123, CD44, CD20, CD40, CD74, CD200, EGFR, β2-Microglobulin, HM1.24, IGF-1R, IL-6R, TRAIL-R1, and the activin receptor type IIA (ActRIIA). See Benson and Byrd, J. Clin. Oncol. (2012) 30(16): 2013-15; Tao and Anderson, Bone Marrow Research (2011):924058; Chu et al., Leukemia (2013) 28(4):917-27; Garfall et al., Discov Med. (2014) 17(91):37-46. In some instances, the antigens include those present on lymphoid tumors, myeloma, AIDS-associated lymphoma, and/or post-transplant lymphoproliferations, such as CD38. Antibodies or antigen-binding fragments directed against such antigens are known and include, for example, those described in U.S. Patent No. 8,153,765; 8,603477, 8,008,450; U.S. Pub. No. US20120189622 or US20100260748; and/or International PCT Publication Nos. WO2006099875, WO2009080829 or WO2012092612 or WO2014210064. In some instances, such antibodies or antigen-binding fragments thereof (e.g. scFv) are contained in multispecific antibodies, multispecific chimeric receptors, such as multispecific CARs, and/or multispecific cells.

In some instances, the disease or condition is an infectious disease or condition, such as, but not limited to, viral, retroviral, bacterial, and protozoal infections, immunodeficiency, Cytomegalovirus (CMV), Epstein-Barr virus (EBV), adenovirus, BK polyomavirus. In some instances, the disease or condition is an autoimmune or inflammatory disease or condition, such as arthritis, e.g., rheumatoid arthritis (RA), Type I diabetes, systemic lupus erythematosus (SLE), inflammatory bowel disease, psoriasis, scleroderma, autoimmune thyroid disease, Grave's disease, Crohn's disease, multiple sclerosis, asthma, and/or a disease or condition associated with transplant.

In some instances, the antigen associated with the disease or disorder is selected from among αvβ6 integrin (avb6 integrin), B cell maturation antigen (BCMA), B7-H6, carbonic anhydrase 9 (CA9, also known as CAIX or G250), a cancer-testis antigen, cancer/testis antigen 1B (CTAG, also known as NY-ESO-1 and LAGE-2), carcinoembryonic antigen (CEA), a cyclin, cyclin A2, C-C Motif Chemokine Ligand 1 (CCL-1), CD19, CD20, CD22, CD23, CD24, CD30, CD33, CD38, CD44, CD44v6, CD44v7/8, CD123, CD138, CD171, epidermal growth factor protein (EGFR), truncated epidermal growth factor protein (tEGFR), type III epidermal growth factor receptor mutation (EGFR vIII), epithelial glycoprotein 2 (EPG-2), epithelial glycoprotein 40 (EPG-40), ephrinB2, ephrine receptor A2 (EPHa2), estrogen receptor, Fc receptor like 5 (FCRL5; also known as Fc receptor homolog 5 or FCRH5), fetal acetylcholine receptor (fetal AchR), a folate binding protein (FBP), folate receptor alpha, fetal acetylcholine receptor, ganglioside GD2, O-acetylated GD2 (OGD2), ganglioside GD3, glycoprotein 100 (gp100), Her2/neu (receptor tyrosine kinase erbB2), Her3 (erb-B3), Her4 (erb-B4), erbB dimers, Human high molecular weight-melanoma-associated antigen (HMW-MAA), hepatitis B surface antigen, Human leukocyte antigen A1 (HLA-AI), Human leukocyte antigen A2 (HLA-A2), IL-22 receptor alpha(IL-22Ra), IL-13 receptor alpha 2 (IL-13Ra2), kinase insert domain receptor (kdr), kappa light chain, L1 cell adhesion molecule (L1CAM), CE7 epitope of L1-CAM, Leucine Rich Repeat Containing 8 Family Member A (LRRC8A), Lewis Y, Melanoma-associated antigen (MAGE)-A1, MAGE-A3, MAGE-A6, mesothelin, c-Met, murine cytomegalovirus (CMV), mucin 1 (MUC1), MUC16, natural killer group 2 member D (NKG2D) ligands, melan A (MART-1), neural cell adhesion molecule (NCAM), oncofetal antigen, Preferentially expressed antigen of melanoma (PRAME), progesterone receptor, a prostate specific antigen, prostate stem cell antigen (PSCA), prostate specific membrane antigen (PSMA), Receptor Tyrosine Kinase Like Orphan Receptor 1 (ROR1), survivin, Trophoblast glycoprotein (TPBG also known as 5T4), tumor-associated glycoprotein 72 (TAG72), vascular endothelial growth factor receptor (VEGFR), vascular endothelial growth factor receptor 2 (VEGFR2), Wilms Tumor 1 (WT-1), a pathogen-specific antigen, or an antigen associated with a universal tag, and/or biotinylated molecules, and/or molecules expressed by HIV, HCV, HBV or other pathogens. Antigens targeted by the receptors in some instances include antigens associated with a B cell malignancy, such as any of a number of known B cell marker. In some instances, the antigen targeted by the receptor is CD20, CD19, CD22, ROR1, CD45, CD21, CD5, CD33, Igkappa, Iglambda, CD79a, CD79b or CD30. In some instances, the antigen is a pathogen-specific antigen. In some instances, the antigen is a viral antigen (such as a viral antigen from HIV, HCV, HBV, etc.), bacterial antigens, and/or parasitic antigens.

In some instances, the cell therapy, e.g., adoptive T cell therapy, is carried out by autologous transfer, in which the cells are isolated and/or otherwise prepared from the subject who is to receive the cell therapy, or from a sample derived from such a subject. Thus, in some aspects, the cells are derived from a subject, e.g., patient, in need of a treatment and the cells, following isolation and processing are administered to the same subject.

In some instances, the cell therapy, e.g., adoptive T cell therapy, is carried out by allogeneic transfer, in which the cells are isolated and/or otherwise prepared from a subject other than a subject who is to receive or who ultimately receives the cell therapy, e.g., a first subject. In such instances, the cells then are administered to a different subject, e.g., a second subject, of the same species. In some instances, the first and second subjects are genetically identical. In some instances, the first and second subjects are genetically similar. In some instances, the second subject expresses the same HLA class or supertype as the first subject.

The cells, e.g., engineered cells generated by a method disclosed in Section I, can be administered by any suitable means. In particular instances, cells from two or more separate output compositions, e.g., compositions of enriched T cells produced by the methods described in Section-I, are combined into a single composition of cells to be administered. In certain instances, the cells from separate output compositions are each administered separately to the subject. In certain instances, CD4+ T cells are administered separately from CD8+ T cells.

In some instances the cells may be administered by bolus infusion, by injection, e.g., intravenous or subcutaneous injections, intraocular injection, periocular injection, subretinal injection, intravitreal injection, trans-septal injection, subscleral injection, intrachoroidal injection, intracameral injection, subconjectval injection, subconjuntival injection, sub-Tenon's injection, retrobulbar injection, peribulbar injection, or posterior juxtascleral delivery. In some instances, they are administered by parenteral, intrapulmonary, and intranasal, and, if desired for local treatment, intralesional administration. Parenteral infusions include intramuscular, intravenous, intraarterial, intraperitoneal, or subcutaneous administration. In some instances, a given dose is administered by a single bolus administration of the cells. In some instances, it is administered by multiple bolus administrations of the cells, for example, over a period of no more than 3 days, or by continuous infusion administration of the cells. In some instances, administration of the cell dose or any additional therapies, e.g., the lymphodepleting therapy, intervention therapy and/or combination therapy, is carried out via outpatient delivery.

For the prevention or treatment of disease, the appropriate dosage may depend on the type of disease to be treated, the type of cells or recombinant receptors, the severity and course of the disease, whether the cells are administered for preventive or therapeutic purposes, previous therapy, the subject's clinical history and response to the cells, and the discretion of the attending physician. The compositions and cells are in some instances suitably administered to the subject at one time or over a series of treatments.

In some instances, the cells are administered as part of a combination treatment, such as simultaneously with or sequentially with, in any order, another therapeutic intervention, such as an antibody or engineered cell or receptor or agent, such as a cytotoxic or therapeutic agent. The cells in some instances are co-administered with one or more additional therapeutic agents or in connection with another therapeutic intervention, either simultaneously or sequentially in any order. In some contexts, the cells are co-administered with another therapy sufficiently close in time such that the cell populations enhance the effect of one or more additional therapeutic agents, or vice versa. In some instances, the cells are administered prior to the one or more additional therapeutic agents. In some instances, the cells are administered after the one or more additional therapeutic agents. In some instances, the one or more additional agents include a cytokine, such as IL-2, for example, to enhance persistence. In some instances, the methods comprise administration of a chemotherapeutic agent.

In some instances, the methods comprise administration of a chemotherapeutic agent, e.g., a conditioning chemotherapeutic agent, for example, to reduce tumor burden prior to the administration.

Preconditioning subjects with immunodepleting (e.g., lymphodepleting) therapies in some aspects can improve the effects of adoptive cell therapy (ACT).

Thus, in some instances, the methods include administering a preconditioning agent, such as a lymphodepleting or chemotherapeutic agent, such as cyclophosphamide, fludarabine, or combinations thereof, to a subject prior to the initiation of the cell therapy. For example, the subject may be administered a preconditioning agent at least 2 days prior, such as at least 3, 4, 5, 6, or 7 days prior, to the initiation of the cell therapy. In some instances, the subject is administered a preconditioning agent no more than 7 days prior, such as no more than 6, 5, 4, 3, or 2 days prior, to the initiation of the cell therapy.

In some instances, the subject is preconditioned with cyclophosphamide at a dose between or between about 20 mg/kg and 100 mg/kg, such as between or between about 40 mg/kg and 80 mg/kg. In some aspects, the subject is preconditioned with or with about 60 mg/kg of cyclophosphamide. In some instances, the cyclophosphamide can be administered in a single dose or can be administered in a plurality of doses, such as given daily, every other day or every three days. In some instances, the cyclophosphamide is administered once daily for one or two days. In some instances, where the lymphodepleting agent comprises cyclophosphamide, the subject is administered cyclophosphamide at a dose between or between about 100 mg/m² and 500 mg/m², such as between or between about 200 mg/m² and 400 mg/m², or 250 mg/m² and 350 mg/m², inclusive. In some instances, the subject is administered about 300 mg/m² of cyclophosphamide. In some instances, the cyclophosphamide can be administered in a single dose or can be administered in a plurality of doses, such as given daily, every other day or every three days. In some instances, cyclophosphamide is administered daily, such as for 1-5 days, for example, for 3 to 5 days. In some instances, the subject is administered about 300 mg/m² of cyclophosphamide, daily for 3 days, prior to initiation of the cell therapy.

In some instances, where the lymphodepleting agent comprises fludarabine, the subject is administered fludarabine at a dose between or between about 1 mg/m² and 100 mg/m², such as between or between about 10 mg/m² and 75 mg/m², 15 mg/m² and 50 mg/m², 20 mg/m² and 40 mg/m², or 24 mg/m² and 35 mg/m², inclusive. In some instances, the subject is administered about 30 mg/m² of fludarabine. In some instances, the fludarabine can be administered in a single dose or can be administered in a plurality of doses, such as given daily, every other day or every three days. In some instances, fludarabine is administered daily, such as for 1-5 days, for example, for 3 to 5 days. In some instances, the subject is administered about 30 mg/m² of fludarabine, daily for 3 days, prior to initiation of the cell therapy.

In some instances, the lymphodepleting agent comprises a combination of agents, such as a combination of cyclophosphamide and fludarabine. Thus, the combination of agents may include cyclophosphamide at any dose or administration schedule, such as those described above, and fludarabine at any dose or administration schedule, such as those described above. For example, in some aspects, the subject is administered 60 mg/kg (~2 g/m²) of cyclophosphamide and 3 to 5 doses of 25 mg/m² fludarabine prior to the first or subsequent dose.

Following administration of the cells, the biological activity of the engineered cell populations in some instances is measured, e.g., by any of a number of known methods. Parameters to assess include specific binding of an engineered or natural T cell or other immune cell to antigen, in vivo, e.g., by imaging, or ex vivo, e.g., by ELISA or flow cytometry. In certain instances, the ability of the engineered cells to destroy target cells can be measured using any suitable known methods, such as cytotoxicity assays described in, for example, Kochenderfer et al., J. Immunotherapy, 32(7): 689-702 (2009), and Herman et al. J. Immunological Methods, 285(1): 25-40 (2004). In certain instances, the biological activity of the cells is measured by assaying expression and/or secretion of one or more cytokines, such as CD107a, IFNγ, IL-2, and TNF. In some aspects the biological activity is measured by assessing clinical outcome, such as reduction in tumor burden or load.

In certain instances, the engineered cells are further modified in any number of ways, such that their therapeutic or prophylactic efficacy is increased. For example, the engineered CAR or TCR expressed by the population can be conjugated either directly or indirectly through a linker to a targeting moiety. The practice of conjugating compounds, e.g., the CAR or TCR, to targeting moieties is known. See, for instance, Wadwa et al., J. Drug Targeting 3: 1 1 1 (1995), and U.S. Patent 5,087,616.

In some instances, a dose of cells is administered to subjects in accord with the disclosed methods, and/or with the disclosed articles of manufacture or compositions. In some instances, the size or timing of the doses is determined as a function of the particular disease or condition in the subject. In some cases, the size or timing of the doses for a particular disease in view of the provided description may be empirically determined.

In some instances, the dose of cells comprises between at or about 2 × 10⁵ of the cells/kg and at or about 2 × 10⁶ of the cells/kg, such as between at or about 4 × 10⁵ of the cells/kg and at or about 1 × 10⁶ of the cells/kg or between at or about 6 × 10⁵ of the cells/kg and at or about 8 × 10⁵ of the cells/kg. In some instances, the dose of cells comprises no more than 2 × 10⁵ of the cells (e.g. antigen-expressing, such as CAR-expressing cells) per kilogram body weight of the subject (cells/kg), such as no more than at or about 3 × 10⁵ cells/kg, no more than at or about 4 × 10⁵ cells/kg, no more than at or about 5 × 10⁵ cells/kg, no more than at or about 6 × 10⁵ cells/kg, no more than at or about 7 × 10⁵ cells/kg, no more than at or about 8 × 10⁵ cells/kg, no more than at or about 9 × 10⁵ cells/kg, no more than at or about 1 × 10⁶ cells/kg, or no more than at or about 2 × 10⁶ cells/kg. In some instances, the dose of cells comprises at least or at least about or at or about 2 × 10⁵ of the cells (e.g. antigen-expressing, such as CAR-expressing cells) per kilogram body weight of the subject (cells/kg), such as at least or at least about or at or about 3 × 10⁵ cells/kg, at least or at least about or at or about 4 × 10⁵ cells/kg, at least or at least about or at or about 5 × 10⁵ cells/kg, at least or at least about or at or about 6 × 10⁵ cells/kg, at least or at least about or at or about 7 × 10⁵ cells/kg, at least or at least about or at or about 8 × 10⁵ cells/kg, at least or at least about or at or about 9 × 10⁵ cells/kg, at least or at least about or at or about 1 × 10⁶ cells/kg, or at least or at least about or at or about 2 × 10⁶ cells/kg.

In certain instances, the cells, or individual populations of sub-types of cells, are administered to the subject at a range of about one million to about 100 billion cells and/or that amount of cells per kilogram of body weight, such as, e.g., 1 million to about 50 billion cells (e.g., about 5 million cells, about 25 million cells, about 500 million cells, about 1 billion cells, about 5 billion cells, about 20 billion cells, about 30 billion cells, about 40 billion cells, or a range defined by any two of the foregoing values), such as about 10 million to about 100 billion cells (e.g., about 20 million cells, about 30 million cells, about 40 million cells, about 60 million cells, about 70 million cells, about 80 million cells, about 90 million cells, about 10 billion cells, about 25 billion cells, about 50 billion cells, about 75 billion cells, about 90 billion cells, or a range defined by any two of the foregoing values), and in some cases about 100 million cells to about 50 billion cells (e.g., about 120 million cells, about 250 million cells, about 350 million cells, about 450 million cells, about 650 million cells, about 800 million cells, about 900 million cells, about 3 billion cells, about 30 billion cells, about 45 billion cells) or any value in between these ranges and/or per kilogram of body weight. Dosages may vary depending on attributes particular to the disease or disorder and/or patient and/or other treatments.

In some instances, the dose of cells is a flat dose of cells or fixed dose of cells such that the dose of cells is not tied to or based on the body surface area or weight of a subject.

In some instances, for example, where the subject is a human, the dose includes fewer than about 1 × 10⁸ total recombinant receptor (e.g., CAR)-expressing cells, T cells, or peripheral blood mononuclear cells (PBMCs), e.g., in the range of about 1 × 10⁶ to 1 × 10⁸ such cells, such as 2 × 10⁶, 5 × 10⁶, 1 × 10⁷, 5 × 10⁷, or 1 × 10⁸ or total such cells, or the range between any two of the foregoing values. In some instances, where the subject is a human, the dose includes between about 1 × 10⁶ and 3 × 10⁸ total recombinant receptor (e.g., CAR)-expressing cells, e.g., in the range of about 1 × 10⁷ to 2 × 10⁸ such cells, such as 1 × 10⁷, 5 × 10⁷, 1 × 10⁸ or 1.5 × 10⁸ total such cells, or the range between any two of the foregoing values. In some instances, the patient is administered multiple doses, and each of the doses or the total dose can be within any of the foregoing values. In some instances, the dose of cells comprises the administration of from or from about 1 × 10⁵ to 5 × 10⁸ total recombinant receptor-expressing T cells or total T cells, 1 × 10⁵ to 1 × 10⁸ total recombinant receptor-expressing T cells or total T cells, from or from about 5 × 10⁵ to 1 × 10⁷ total recombinant receptor-expressing T cells or total T cells, or from or from about 1 × 10⁶ to 1 × 10⁷ total recombinant receptor-expressing T cells or total T cells, each inclusive.

In some instances, the T cells of the dose include CD4+ T cells, CD8+ T cells or CD4+ and CD8+ T cells.

In some instances, for example, where the subject is human, the CD8+ T cells of the dose, including in a dose including CD4+ and CD8+ T cells, includes between about 1 × 10⁶ and 1 × 10⁸ total recombinant receptor (e.g., CAR)-expressing CD8+cells, e.g., in the range of about 5 × 10⁶ to 1 × 10⁸ such cells, such cells 1 × 10⁷, 2.5 × 10⁷, 5 x 10⁷, 7.5 × 10⁷ or 1 × 10⁸ total such cells, or the range between any two of the foregoing values. In some instances, the patient is administered multiple doses, and each of the doses or the total dose can be within any of the foregoing values. In some instances, the dose of cells comprises the administration of from or from about 1 × 10⁷ to 0.75 × 10⁸ total recombinant receptor-expressing CD8+ T cells, 1 × 10⁷ to 2.5 × 10⁷ total recombinant receptor-expressing CD8+ T cells, from or from about 1 × 10⁷ to 0.75 × 10⁸ total recombinant receptor-expressing CD8+ T cells, each inclusive. In some instances, the dose of cells comprises the administration of or about 1 × 10', 2.5 × 10⁷, 5 × 10⁷ 7.5 × 10⁷ or 1 × 10⁸ total recombinant receptor-expressing CD8+ T cells.

In some instances, the dose of cells, e.g., recombinant receptor-expressing T cells, is administered to the subject as a single dose or is administered only one time within a period of two weeks, one month, three months, six months, 1 year or more.

In the context of adoptive cell therapy, administration of a given "dose" encompasses administration of the given amount or number of cells as a single composition and/or single uninterrupted administration, e.g., as a single injection or continuous infusion, and also encompasses administration of the given amount or number of cells as a split dose or as a plurality of compositions, provided in multiple individual compositions or infusions, over a specified period of time, such as over no more than 3 days. Thus, in some contexts, the dose is a single or continuous administration of the specified number of cells, given or initiated at a single point in time. In some contexts, however, the dose is administered in multiple injections or infusions over a period of no more than three days, such as once a day for three days or for two days or by multiple infusions over a single day period.

Thus, in some aspects, the cells of the dose are administered in a single pharmaceutical composition. In some instances, the cells of the dose are administered in a plurality of compositions, collectively containing the cells of the dose.

In some instances, the term "split dose" refers to a dose that is split so that it is administered over more than one day. This type of dosing is encompassed by the present methods and is considered to be a single dose.

Thus, the dose of cells may be administered as a split dose, e.g., a split dose administered over time. For example, in some instances, the dose may be administered to the subject over 2 days or over 3 days. Exemplary methods for split dosing include administering 25% of the dose on the first day and administering the remaining 75% of the dose on the second day. In other instances, 33% of the dose may be administered on the first day and the remaining 67% administered on the second day. In some aspects, 10% of the dose is administered on the first day, 30% of the dose is administered on the second day, and 60% of the dose is administered on the third day. In some instances, the split dose is not spread over more than 3 days.

In some instances, cells of the dose may be administered by administration of a plurality of compositions or solutions, such as a first and a second, optionally more, each containing some cells of the dose. In some aspects, the plurality of compositions, each containing a different population and/or sub-types of cells, are administered separately or independently, optionally within a certain period of time. For example, the populations or sub-types of cells can include CD8⁺ and CD4⁺ T cells, respectively, and/or CD8+- and CD4+-enriched populations, respectively, e.g., CD4+ and/or CD8+ T cells each individually including cells genetically engineered to express the recombinant receptor. In some instances, the administration of the dose comprises administration of a first composition comprising a dose of CD8+ T cells or a dose of CD4+ T cells and administration of a second composition comprising the other of the dose of CD4+ T cells and the CD8+ T cells.

In some instances, the administration of the composition or dose, e.g., administration of the plurality of cell compositions, involves administration of the cell compositions separately. In some instances, the cell compositions are separate output compositions produced by the methods described in Section I. In some aspects, the separate administrations are carried out simultaneously, or sequentially, in any order. In some instances, the dose comprises a first composition and a second composition, and the first composition and second composition are administered 0 to 12 hours apart, 0 to 6 hours apart or 0 to 2 hours apart. In some instances, the initiation of administration of the first composition and the initiation of administration of the second composition are carried out no more than 2 hours, no more than 1 hour, or no more than 30 minutes apart, no more than 15 minutes, no more than 10 minutes or no more than 5 minutes apart. In some instances, the initiation and/or completion of administration of the first composition and the completion and/or initiation of administration of the second composition are carried out no more than 2 hours, no more than 1 hour, or no more than 30 minutes apart, no more than 15 minutes, no more than 10 minutes or no more than 5 minutes apart.

In some composition, the first composition, e.g., first composition of the dose, comprises CD4+ T cells. In some composition, the first composition, e.g., first composition of the dose, comprises CD8+ T cells. In some instances, the first composition is administered prior to the second composition.

In some instances, the dose or composition of cells includes a defined or target ratio of CD4+ T cells expressing a recombinant receptor to CD8+ T cells expressing a recombinant receptor and/or of CD4+ T cells to CD8+ T cells, which ratio optionally is approximately 1:1 or is between approximately 1:3 and approximately 3:1, such as approximately 1:1. In some aspects, the administration of a composition or dose with the target or desired ratio of different cell populations (such as CD4+:CD8+ ratio or CAR+CD4+:CAR+CD8+ ratio, e.g., 1:1) involves the administration of a cell composition containing one of the populations and then administration of a separate cell composition comprising the other of the populations, where the administration is at or approximately at the target or desired ratio. In some aspects, administration of a dose or composition of cells at a defined ratio leads to improved expansion, persistence and/or antitumor activity of the T cell therapy.

In some instances, the subject receives multiple doses, e.g., two or more doses or multiple consecutive doses, of the cells. In some instances, two doses are administered to a subject. In some instances, the subject receives the consecutive dose, e.g., second dose, is administered approximately 4, 5, 6, 7, 8, 9, 10, 11, 12, 13, 14, 15, 16, 17, 18, 19, 20 or 21 days after the first dose. In some instances, multiple consecutive doses are administered following the first dose, such that an additional dose or doses are administered following administration of the consecutive dose. In some aspects, the number of cells administered to the subject in the additional dose is the same as or similar to the first dose and/or consecutive dose. In some instances, the additional dose or doses are larger than prior doses.

In some aspects, the size of the first and/or consecutive dose is determined based on one or more criteria such as response of the subject to prior treatment, e.g. chemotherapy, disease burden in the subject, such as tumor load, bulk, size, or degree, extent, or type of metastasis, stage, and/or likelihood or incidence of the subject developing toxic outcomes, e.g., CRS, macrophage activation syndrome, tumor lysis syndrome, neurotoxicity, and/or a host immune response against the cells and/or recombinant receptors being administered.

In some aspects, the time between the administration of the first dose and the administration of the consecutive dose is about 9 to about 35 days, about 14 to about 28 days, or 15 to 27 days. In some instances, the administration of the consecutive dose is at a time point more than about 14 days after and less than about 28 days after the administration of the first dose. In some aspects, the time between the first and consecutive dose is about 21 days. In some instances, an additional dose or doses, e.g. consecutive doses, are administered following administration of the consecutive dose. In some aspects, the additional consecutive dose or doses are administered at least about 14 and less than about 28 days following administration of a prior dose. In some instances, the additional dose is administered less than about 14 days following the prior dose, for example, 4, 5, 6, 7, 8, 9, 10, 11, 12, or 13 days after the prior dose. In some instances, no dose is administered less than about 14 days following the prior dose and/or no dose is administered more than about 28 days after the prior dose.

In some instances, the dose of cells, e.g., recombinant receptor-expressing cells, comprises two doses (e.g., a double dose), comprising a first dose of the T cells and a consecutive dose of the T cells, wherein one or both of the first dose and the second dose comprises administration of the split dose of T cells.

In some instances, the dose of cells is generally large enough to be effective in reducing disease burden.

In some instances, the cells are administered at a desired dosage, which in some aspects includes a desired dose or number of cells or cell type(s) and/or a desired ratio of cell types. Thus, the dosage of cells in some instances is based on a total number of cells (or number per kg body weight) and a desired ratio of the individual populations or sub-types, such as the CD4+ to CD8+ ratio. In some instances, the dosage of cells is based on a desired total number (or number per kg of body weight) of cells in the individual populations or of individual cell types. In some instances, the dosage is based on a combination of such features, such as a desired number of total cells, desired ratio, and desired total number of cells in the individual populations.

In some instances, the populations or sub-types of cells, such as CD8⁺ and CD4⁺ T cells, are administered at or within a tolerated difference of a desired dose of total cells, such as a desired dose of T cells. In some aspects, the desired dose is a desired number of cells or a desired number of cells per unit of body weight of the subject to whom the cells are administered, e.g., cells/kg. In some aspects, the desired dose is at or above a minimum number of cells or minimum number of cells per unit of body weight. In some aspects, among the total cells, administered at the desired dose, the individual populations or sub-types are present at or near a desired output ratio (such as CD4⁺ to CD8⁺ ratio), e.g., within a certain tolerated difference or error of such a ratio.

In some instances, the cells are administered at or within a tolerated difference of a desired dose of one or more of the individual populations or sub-types of cells, such as a desired dose of CD4+ T cells and/or a desired dose of CD8+ T cells. In some aspects, the desired dose is a desired number of cells of the sub-type or population, or a desired number of such cells per unit of body weight of the subject to whom the cells are administered, e.g., cells/kg. In some aspects, the desired dose is at or above a minimum number of cells of the population or sub-type, or minimum number of cells of the population or sub-type per unit of body weight.

Thus, in some instances, the dosage is based on a desired fixed dose of total cells and a desired ratio, and/or based on a desired fixed dose of one or more, e.g., each, of the individual sub-types or sub-populations. Thus, in some instances, the dosage is based on a desired fixed or minimum dose of T cells and a desired ratio of CD4⁺ to CD8⁺ T cells, and/or is based on a desired fixed or minimum dose of CD4⁺ and/or CD8⁺ T cells.

In some instances, the cells are administered at or within a tolerated range of a desired output ratio of multiple cell populations or sub-types, such as CD4+ and CD8+ T cells or sub-types. In some aspects, the desired ratio can be a specific ratio or can be a range of ratios. for example, in some instances, the desired ratio (e.g., ratio of CD4⁺ to CD8⁺ T cells) is between at or about 5:1 and at or about 5:1 (or greater than about 1:5 and less than about 5:1), or between at or about 1:3 and at or about 3:1 (or greater than about 1:3 and less than about 3:1), such as between at or about 2:1 and at or about 1:5 (or greater than about 1:5 and less than about 2:1, such as at or about 5:1, 4.5:1, 4:1, 3.5:1, 3:1, 2.5:1, 2:1, 1.9:1, 1.8:1, 1.7:1, 1.6:1, 1.5:1, 1.4:1, 1.3:1, 1.2:1, 1.1:1, 1:1, 1:1.1, 1:1.2, 1:1.3, 1:1.4, 1:1.5, 1:1.6, 1:1.7, 1:1.8, 1:1.9: 1:2, 1:2.5, 1:3, 1:3.5, 1:4, 1:4.5, or 1:5. In some aspects, the tolerated difference is within about 1%, about 2%, about 3%, about 4% about 5%, about 10%, about 15%, about 20%, about 25%, about 30%, about 35%, about 40%, about 45%, about 50% of the desired ratio, including any value in between these ranges. In certain instances, the compositions of enriched CD4+ T cells and enriched CD8+ T cells are combined at the desired ratio and administered to the subject as a single cell composition. In particular instances, the compositions of enriched CD4+ T cells and enriched CD8+ T cells are administered as separate compositions at the desired ratio.

In particular instances, the numbers and/or concentrations of cells refer to the number of recombinant receptor (e.g., CAR)-expressing cells. In other instances, the numbers and/or concentrations of cells refer to the number or concentration of all cells, T cells, or peripheral blood mononuclear cells (PBMCs) administered.

In some aspects, the size of the dose is determined based on one or more criteria such as response of the subject to prior treatment, e.g. chemotherapy, disease burden in the subject, such as tumor load, bulk, size, or degree, extent, or type of metastasis, stage, and/or likelihood or incidence of the subject developing toxic outcomes, e.g., CRS, macrophage activation syndrome, tumor lysis syndrome, neurotoxicity, and/or a host immune response against the cells and/or recombinant receptors being administered.

In some instances, the methods also include administering one or more additional doses of cells expressing a chimeric antigen receptor (CAR) and/or lymphodepleting therapy, and/or one or more steps of the methods are repeated. In some instances, the one or more additional dose is the same as the initial dose. In some instances, the one or more additional dose is different from the initial dose, e.g., higher, such as 2-fold, 3-fold, 4-fold, 5-fold, 6-fold, 7-fold, 8-fold, 9-fold or 10-fold or more higher than the initial dose, or lower, such as e.g., higher, such as 2-fold, 3-fold, 4-fold, 5-fold, 6-fold, 7-fold, 8-fold, 9-fold or 10-fold or more lower than the initial dose. In some instances, administration of one or more additional doses is determined based on response of the subject to the initial treatment or any prior treatment, disease burden in the subject, such as tumor load, bulk, size, or degree, extent, or type of metastasis, stage, and/or likelihood or incidence of the subject developing toxic outcomes, e.g., CRS, macrophage activation syndrome, tumor lysis syndrome, neurotoxicity, and/or a host immune response against the cells and/or recombinant receptors being administered.

### VI. ARTICLES OF MANUFACTURE

Also disclosed are articles of manufacture and kits containing engineered cells expressing a recombinant receptor produced by the methods disclosed herein, such as the methods described herein, such as in Section I, e.g. the output compositions of cells, and optionally instructions for use, for example, instructions for administering the engineered cells to a subject, such as by methods described herein, such as in Section III.

In some instances, disclosed herein are articles of manufacture and/or kits that include a composition comprising a therapeutically effective amount of any of the engineered cells described herein, and instructions for administering, to a subject for treating a disease or condition. In some instances, the instructions can specify some or all of the elements of the methods for administrating the cells that are disclosed herein. In some instances, the instructions specify particular instructions for administration of the cells for cell therapy, e.g., doses, timing, selection and/or identification of subjects for administration and conditions for administration. In some instances, the articles of manufacture and/or kits further comprise an agent for lymphodepleting therapy, and optionally further includes instructions for administering the lymphodepleting therapy. In some instances, the instructions can be included as a label or package insert accompanying the compositions for administration.

In some instances, the article of manufacture may have a container, optionally a vial, containing a composition of enriched CD4+ T cells expressing a recombinant receptor. In some instances, the article of manufacture or kit comprises optionally comprises a second container, optionally a second vial, containing a composition of enriched CD8+ T cells expressing a recombinant receptor. In some instances, a cryoprotectant is included with the cells. In some aspects the container is a vial or a bag. In some instances, the container contains a composition of enriched CD4+ and CD8+ T cells.

In some instances, the composition of enriched CD4+ T cells within the container includes at least 60%, at least 65%, at least 70%, at least 75%, at least 80%, at least 85%, at least 90%, at least 95%, at least 98%, at least 99%, at least 99.5%, at least 99.9%, or at or at about 100% CD4+ T cells. In certain instances, the composition of the container includes at least 30%, at least 40%, at least 50%, at least 60%, at least 70%, at least 80%, at least 90%, at least 95%, at least 98%, at least 99%, at least 99.5%, at least 99.9%, or at or at about 100% CD4+ T cells that express the recombinant receptor and/or have been transduced or transfected with the recombinant polynucleotide. In certain instances, the composition of enriched CD4+ T cells within the container includes less than 40%, less than 35%, less than 30%, less than 25%, less than 20%, less than 15%, less than 10%, less than 5%, less than 1%, less than 0.1%, or less than 0.01% CD8+ T cells, and/or contains no CD8+ T cells, and/or is free or substantially free of CD8+ T cells.

In some instances, the composition of enriched CD8+ T cells within the container includes at least 60%, at least 65%, at least 70%, at least 75%, at least 80%, at least 85%, at least 90%, at least 95%, at least 98%, at least 99%, at least 99.5%, at least 99.9%, or at or at about 100% CD8+ T cells. In particular instances, the composition with the container at least 30%, at least 40%, at least 50%, at least 60%, at least 70%, at least 80%, at least 90%, at least 95%, at least 98%, at least 99%, at least 99.5%, at least 99.9%, or at or at about 100% CD8+ T cells that express the recombinant receptor and/or have been transduced or transfected with the recombinant polynucleotide. In certain instances, the output composition of enriched CD8+ T cells that is administered to the subject includes less than 40%, less than 35%, less than 30%, less than 25%, less than 20%, less than 15%, less than 10%, less than 5%, less than 1%, less than 0.1%, or less than 0.01% CD4+ T cells, and/or contains no CD4+ T cells, and/or is free or substantially free of CD4+ T cells.

In some instances, the instructions specify the dose of cells to be administered. For example, in some instances, the dose specified in the instructions include a total recombinant receptor (e.g., CAR)-expressing cells between about 1 × 10⁶ and 3 × 10⁸, e.g., in the range of about 1 × 10⁷ to 2 × 10⁸ such cells, such as 1 × 10⁷, 5 × 10⁷, 1 × 10⁸ or 1.5 × 10⁸ total such cells, or the range between any two of the foregoing values. In some instances, the patient is administered multiple doses, and each of the doses or the total dose can be within any of the foregoing values.

In some instances, the container such as the vial comprises greater than or greater than about 10 × 10⁶ T cells or recombinant receptor-expressing T cells, greater than or greater than about 15 × 10⁶ T cells or recombinant receptor-expressing T cells, greater than or greater than about 25 × 10⁶ T cells or recombinant receptor-expressing T cell. In some aspects, the vial comprises between about 10 million cells per ml and about 70 million cells per ml, between about 10 million cells per ml and about 50 million cells per ml, between about 10 million cells per ml and about 25 million cells per ml, between about 10 million cells per ml and about 15 million cells per ml, 15 million cells per ml and about 70 million cells per ml, between about 15 million cells per ml and about 50 million cells per ml, between about 15 million cells per ml and about 25 million cells per ml, between about 25 million cells per ml and about 70 million cells per ml, between about 25 million cells per ml and about 50 million cells per ml, and between about 50 million cells per ml and about 70 million cells per ml.

In some instances, the plurality of vials or plurality of cells or unit dose of cells specified for administration, collectively, comprises a dose of cells comprising from or from about 1 × 10⁵ to 5 × 10⁸ total recombinant receptor-expressing T cells or total T cells, 1 × 10⁵ to 1 × 10⁸ total recombinant receptor-expressing T cells or total T cells, from or from about 5 × 10⁵ to 1 × 10⁷ total recombinant receptor-expressing T cells or total T cells, or from or from about 1 × 10⁶ to 1 × 10⁷ total recombinant receptor-expressing T cells or total T cells, each inclusive. In some aspects, the article comprises one or more unit dose of the CD4+ and CD8+ T cells or of the CD4+receptor+ T cells and CD8+receptor+ T cells, wherein the unit dose comprises between at or about 1 × 10⁷ and at or about 2 × 10⁸ recombinant receptor-expressing T cells, between at or about 5 × 10⁷ and at or about 1.5 × 10⁸ recombinant receptor-expressing T cells, at or about 5 × 10⁷ recombinant receptor-expressing T cells, at or about 1 × 10⁸ recombinant receptor-expressing T cells, or at or about 1.5 × 10⁸ recombinant receptor-expressing T cells, optionally wherein the information in the article specifies administration of one or of a plurality of unit doses and/or a volume corresponding to such one or plurality of unit doses. In some cases, the article comprises one or more unit doses of the CD8+ T cells, wherein the dose comprises between at or about 5 × 10⁶ and at or about 1 × 10⁸ recombinant receptor-expressing CD8+ T cells, the dose comprises between at or about 1 × 10⁷ and at or about 0.75 × 10⁸ recombinant receptor-expressing CD8+ T cells, the dose comprises at or about 2.5 × 10⁷ recombinant receptor-expressing CD8+ T cells, or the dose comprises at or about 5 × 10⁷ recombinant receptor-expressing CD8+ T cells, or the dose comprises at or about 0.75 × 10⁸ recombinant receptor-expressing CD8+ T cells, optionally wherein the information in the article specifies administration of one or of a plurality of unit doses and/or a volume corresponding to such one or plurality of unit doses. In some instances, the cells in the article, collectively, comprise a dose of cells comprising no more than 1 × 10⁸ total recombinant receptor-expressing T cells or total T cells, no more than 1 × 10⁷ total recombinant receptor-expressing T cells or total T cells, no more than 0.5 × 10⁷ total recombinant receptor-expressing T cells or total T cells, no more than 1 × 10⁶ total recombinant receptor-expressing T cells or total T cells, no more than 0.5 × 10⁶ total recombinant receptor-expressing T cells or total T cells.

In some instances, the instructions can specify dosage regimen and timing of the administration. For example, in some instances, the instructions can specify administering to the subject multiple doses, e.g., two or more doses, of the cells. In some instances, the instructions specify the timing of the multiple doses, e.g., the second dose being administered approximately 4, 5, 6, 7, 8, 9, 10, 11, 12, 13, 14, 15, 16, 17, 18, 19, 20 or 21 days after the first dose; and/or the dosage amount in each dose.

In some instances, the article of manufacture or kit comprises a composition of enriched CD4+ T cells expressing a recombinant receptor, and instructions for administering, to a subject having a disease or condition, all or a portion of the composition of enriched CD4+ T cells and further administering CD8+ T cells expressing a recombinant receptor. In some instances, the instructions specify administering the CD4+ T cells prior to administering the CD8+ T cells. In some cases, the instructions specify administering the CD8+ T cells prior to administering the CD4+ T cells. In some instances, the article of manufacture or kit comprises a plurality of CD8+ T cells expressing a recombinant receptor, and instructions for administering, to a subject having a disease or condition, all or a portion of the plurality of CD8+ T cells and CD4+ T cells expressing a recombinant receptor. In some instances, the instructions specify dosage regimen and timing of the administration of the cells.

In some aspects, the instructions specify administering all or a portion of the CD4+ T cells and the all or a portion of the CD8+ T cells 0 to 12 hours apart, 0 to 6 hours apart or 0 to 2 hours apart. In some cases, the instructions specify administering the CD4+ T cells and the CD8+ T cells no more than 2 hours, no more than 1 hour, no more than 30 minutes, no more than 15 minutes, no more than 10 minutes or no more than 5 minutes apart.

In some instances, the instructions specify the dose or number of cells or cell type(s) and/or a ratio of cell types, e.g., individual populations or sub-types, such as the CD4+ to CD8+ ratio. In some instances, the populations or sub-types of cells, such as CD8⁺ and CD4⁺ T cells. For example, in some instances, the instructions specify that the cells are administered at or within a tolerated range of an output ratio of multiple cell populations or sub-types, such as CD4+ and CD8+ T cells or sub-types, of between at or about 5:1 and at or about 5:1 (or greater than about 1:5 and less than about 5:1), or between at or about 1:3 and at or about 3:1 (or greater than about 1:3 and less than about 3:1), such as between at or about 2:1 and at or about 1:5 (or greater than about 1:5 and less than about 2:1, such as at or about 5:1, 4.5:1, 4:1, 3.5:1, 3:1, 2.5:1, 2:1, 1.9:1, 1.8:1, 1.7:1, 1.6:1, 1.5:1, 1.4:1, 1.3:1, 1.2:1, 1.1:1, 1:1, 1:1.1, 1:1.2, 1:1.3, 1:1.4, 1:1.5, 1:1.6, 1:1.7, 1:1.8, 1:1.9: 1:2, 1:2.5, 1:3, 1:3.5, 1:4, 1:4.5, or 1:5. In certain instances, the instructions specify that the compositions of enriched CD4+ T cells and enriched CD8+ T cells are combined at the desired ratio and administered to the subject as a single cell composition. In particular instances, the instructions specify the compositions of enriched CD4+ T cells and enriched CD8+ T cells are administered as separate compositions at the desired ratio. In some aspects, the tolerated difference is within about 1%, about 2%, about 3%, about 4% about 5%, about 10%, about 15%, about 20%, about 25%, about 30%, about 35%, about 40%, about 45%, about 50% of the desired ratio, including any value in between these ranges.

### VII. DEFINITIONS

Unless defined otherwise, all terms of art, notations and other technical and scientific terms or terminology used herein are intended to have the same meaning as is commonly understood by one of ordinary skill in the art to which the claimed subject matter pertains. In some cases, terms with commonly understood meanings are defined herein for clarity and/or for ready reference, and the inclusion of such definitions herein should not necessarily be construed to represent a substantial difference over what is generally understood in the art.

The terms "polypeptide" and "protein" are used interchangeably to refer to a polymer of amino acid residues, and are not limited to a minimum length. Polypeptides, including the provided receptors and other polypeptides, e.g., linkers or peptides, may include amino acid residues including natural and/or non-natural amino acid residues. The terms also include post-expression modifications of the polypeptide, for example, glycosylation, sialylation, acetylation, and phosphorylation. In some aspects, the polypeptides may contain modifications with respect to a native or natural sequence, as long as the protein maintains the desired activity. These modifications may be deliberate, as through site-directed mutagenesis, or may be accidental, such as through mutations of hosts which produce the proteins or errors due to PCR amplification.

As used herein, a "subject" is a mammal, such as a human or other animal, and typically is human. In some embodiments, the subject, e.g., patient, to whom the agent or agents, cells, cell populations, or compositions are administered, is a mammal, typically a primate, such as a human. In some embodiments, the primate is a monkey or an ape. The subject can be male or female and can be any suitable age, including infant, juvenile, adolescent, adult, and geriatric subjects. In some embodiments, the subject is a non-primate mammal, such as a rodent.

As used herein, "treatment" (and grammatical variations thereof such as "treat" or "treating") refers to complete or partial amelioration or reduction of a disease or condition or disorder, or a symptom, adverse effect or outcome, or phenotype associated therewith. Desirable effects of treatment include, but are not limited to, preventing occurrence or recurrence of disease, alleviation of symptoms, diminishment of any direct or indirect pathological consequences of the disease, preventing metastasis, decreasing the rate of disease progression, amelioration or palliation of the disease state, and remission or improved prognosis. The terms do not imply complete curing of a disease or complete elimination of any symptom or effect(s) on all symptoms or outcomes.

As used herein, "delaying development of a disease" means to defer, hinder, slow, retard, stabilize, suppress and/or postpone development of the disease (such as cancer). This delay can be of varying lengths of time, depending on the history of the disease and/or individual being treated. In some embodiments, sufficient or significant delay can, in effect, encompass prevention, in that the individual does not develop the disease. For example, a late stage cancer, such as development of metastasis, may be delayed.

"Preventing," as used herein, includes providing prophylaxis with respect to the occurrence or recurrence of a disease in a subject that may be predisposed to the disease but has not yet been diagnosed with the disease. In some embodiments, the provided cells and compositions are used to delay development of a disease or to slow the progression of a disease.

As used herein, to "suppress" a function or activity is to reduce the function or activity when compared to otherwise same conditions except for a condition or parameter of interest, or alternatively, as compared to another condition. For example, cells that suppress tumor growth reduce the rate of growth of the tumor compared to the rate of growth of the tumor in the absence of the cells.

An "effective amount" of an agent, e.g., a pharmaceutical formulation, cells, or composition, in the context of administration, refers to an amount effective, at dosages/amounts and for periods of time necessary, to achieve a desired result, such as a therapeutic or prophylactic result.

A "therapeutically effective amount" of an agent, e.g., a pharmaceutical formulation or cells, refers to an amount effective, at dosages and for periods of time necessary, to achieve a desired therapeutic result, such as for treatment of a disease, condition, or disorder, and/or pharmacokinetic or pharmacodynamic effect of the treatment. The therapeutically effective amount may vary according to factors such as the disease state, age, sex, and weight of the subject, and the populations of cells administered. In some embodiments, the provided methods involve administering the cells and/or compositions at effective amounts, e.g., therapeutically effective amounts.

A "prophylactically effective amount" refers to an amount effective, at dosages and for periods of time necessary, to achieve the desired prophylactic result. Typically but not necessarily, since a prophylactic dose is used in subjects prior to or at an earlier stage of disease, the prophylactically effective amount will be less than the therapeutically effective amount. In the context of lower tumor burden, the prophylactically effective amount in some aspects will be higher than the therapeutically effective amount.

The term "about" as used herein refers to the usual error range for the respective value readily known to the skilled person in this technical field. Reference to "about" a value or parameter herein includes (and describes) embodiments that are directed to that value or parameter per se.

As used herein, the singular forms "a," "an," and "the" include plural referents unless the context clearly dictates otherwise. For example, "a" or "an" means "at least one" or "one or more."

Throughout this disclosure, various aspects of the claimed subject matter are presented in a range format. It should be understood that the description in range format is merely for convenience and brevity and should not be construed as an inflexible limitation on the scope of the claimed subject matter. Accordingly, the description of a range should be considered to have specifically disclosed all the possible sub-ranges as well as individual numerical values within that range. For example, where a range of values is provided, it is understood that each intervening value, between the upper and lower limit of that range and any other stated or intervening value in that stated range is encompassed within the claimed subject matter. The upper and lower limits of these smaller ranges may independently be included in the smaller ranges, and are also encompassed within the claimed subject matter, subject to any specifically excluded limit in the stated range. Where the stated range includes one or both of the limits, ranges excluding either or both of those included limits are also included in the claimed subject matter. This applies regardless of the breadth of the range.

As used herein, a composition refers to any mixture of two or more products, substances, or compounds, including cells. It may be a solution, a suspension, liquid, powder, a paste, aqueous, non-aqueous or any combination thereof.

As used herein, "enriching" when referring to one or more particular cell type or cell population, refers to increasing the number or percentage of the cell type or population, e.g., compared to the total number of cells in or volume of the composition, or relative to other cell types, such as by positive selection based on markers expressed by the population or cell, or by negative selection based on a marker not present on the cell population or cell to be depleted. The term does not require complete removal of other cells, cell type, or populations from the composition and does not require that the cells so enriched be present at or even near 100 % in the enriched composition.

As used herein, a statement that a cell or population of cells is "positive" for a particular marker refers to the detectable presence on or in the cell of a particular marker, typically a surface marker. When referring to a surface marker, the term refers to the presence of surface expression as detected by flow cytometry, for example, by staining with an antibody that specifically binds to the marker and detecting said antibody, wherein the staining is detectable by flow cytometry at a level substantially above the staining detected carrying out the same procedure with an isotype-matched control or fluorescence minus one (FMO) gating control under otherwise identical conditions and/or at a level substantially similar to that for cell known to be positive for the marker, and/or at a level substantially higher than that for a cell known to be negative for the marker.

As used herein, a statement that a cell or population of cells is "negative" for a particular marker refers to the absence of substantial detectable presence on or in the cell of a particular marker, typically a surface marker. When referring to a surface marker, the term refers to the absence of surface expression as detected by flow cytometry, for example, by staining with an antibody that specifically binds to the marker and detecting said antibody, wherein the staining is not detected by flow cytometry at a level substantially above the staining detected carrying out the same procedure with an isotype-matched control or fluorescence minus one (FMO) gating control under otherwise identical conditions, and/or at a level substantially lower than that for cell known to be positive for the marker, and/or at a level substantially similar as compared to that for a cell known to be negative for the marker.

The term "vector," as used herein, refers to a nucleic acid molecule capable of propagating another nucleic acid to which it is linked. The term includes the vector as a self-replicating nucleic acid structure as well as the vector incorporated into the genome of a host cell into which it has been introduced. Certain vectors are capable of directing the expression of nucleic acids to which they are operatively linked. Such vectors are referred to herein as "expression vectors."

### IX. EXAMPLES

The following examples are included for illustrative purposes only and are not intended to limit the scope of the invention.

### Example 1: Dose Determination of mTOR Kinase Inhibitor for Cultures of Primary Human T Cells

The dose effect of exemplary mTOR kinase inhibitors in primary human T cells was assessed by monitoring inhibition of ribosomal protein S6.

CD4+ and CD8+ T cells were isolated by immunoaffinity-based enrichment from leukapheresis samples from human donor subjects. Isolated CD4+ and CD8+ T cells were mixed 1: 1 and stimulated with anti-CD3/anti-CD28 magnetic beads in the presence of increasing concentrations of an mTOR kinase inhibitor, PI-103, Compound 155, Compound 63, or Compound 246. The T cell cultures were incubated overnight (approximately 16 hr) at 37 °C. Following incubation in the presence of PI-103, Compound 155, Compound 63, or Compound 246, T cells were assessed for intracellular S6 phosphorylation and co-stained for surface expression of CD4 or CD8, by flow cytometry.

Results are shown in **FIGS. 1A-D.** All assessed mTOR kinase inhibitors inhibited S6 phosphorylation in both CD4+ and CD8+ T cells. The IC50s for the inhibition of S6 phosphorylation in the T cells by PI-103, Compound 155, Compound 63, and Compound 246 were approximately 100 nM, 100 nM, 500 nM, and 500 nM, respectively. The IC50 in CD4+ and CD8+ T cells is shown in Table E1.

| **Table E1: IC50 for inhibition of Ribo-S6 phosphorylation** | | |
|---|---|---|
| Compound | **CD4** | **CD8** |
| PI-103 | 121 nM | 88.4 nM |
| Compound 155 | 121 nM | 127 nM |
| Compound 63 | 468 nM | 503 nM |
| Compound 246 | 475 nM | 502 nM |

### Example 2: Assessment of T Cell Expansion Following Incubation in the Presence of a mTOR Kinase Inhibitor

Separate compositions of CD4+ and CD8+ cells were isolated from human leukapheresis samples by immunoaffinity-based enrichment and cryofrozen. The CD4+ and CD8+ cells of the compositions were subsequently thawed and activated by separately culturing the cells under stimulating conditions in the presence of anti-CD3/anti-CD28 magnetic beads and recombinant IL-2, IL-7 and/or IL-15 for approximately 20 hours. Cells were then transduced with a viral vector encoding an anti-CD19 chimeric antigen receptor (CAR). After transduction, the CD4+ and CD8+ cells were separately incubated in the presence of an mTOR kinase inhibitor, either Compound 155, Compound 63, or Compound 246, at various concentrations. For controls, cells were incubated with media only, DMSO vehicle, or with 200 nM PI-103. Incubation was carried out at 37 °C for approximately 8 days post-thaw with one media change, at which point the compounds were re-added to the culture media at the same concentration.

The percent of CD8+ and CD4+ T cells at the end of the process as compared to the amount of cells seeded for culture following the transduction is shown in **FIG. 2****.** The cutoff for selecting a tolerated dose was set at 70% of the mean values of the media and DMSO controls. The highest tolerated dose of Compound 155, Compound 63, and Compound 246 that resulted in similar levels of CD8 and CD4 T cell expansion as observed in the vehicle control group, was 100 nM, 1 µM, and 100 nM, respectively.

### Example 3: Functional Assessment of Chimeric Antigen Receptor (CAR)-Transduced T Cells (CAR T Cells) Expanded in the Presence of a mTOR Kinase inhibitor

Separate compositions of CD4+ and CD8+ cells were isolated from three human donors, activated and transduced with a viral vector encoding an anti-CD19 CAR substantially as described in Example 2. After transduction, the CD4+ and CD8+ T cells derived from each donor were separately incubated for 8 days in the presence of a mTOR kinase inhibitor to expand T cells substantially as described in Example 2, except in the presence of 200 nM PI-103, 1 µM Compound 63, or with DMSO vehicle or media only controls. CD4+ and CD8+ T cells from each donor were then separately harvested, formulated, and cryofrozen. The cryofrozen engineered CD4+ and CD8+ T cells were thawed, washed to remove compound, and cells from the same donor were then combined at a ratio of 1:1 viable CD4+/CAR+ to viable CD8+/CAR+ T cells to produce an expanded anti-CD19 CAR-T cell composition containing CD4+ and CD8+ T cells. Functional activities of the generated anti-CD19 CAR-T cell compositions were assessed.

### Glycolytic Metabolism

Changes in cellular glycolytic metabolism in response to T cell receptor (TCR) stimulation were measured in CD8+ CAR-T cells from the generated anti-CD19 CAR-T cell composition prior to combining with CD4+ T cells. Glycolytic metabolism was assessed by measuring the extracellular acidification rate (ECAR) in CD8+ CAR-T cells in real time with an extracellular flux bioanalyzer (Seahorse Bioanalyzer, Agilent Technologies). Baseline ECAR measurements were taken from cultured the CD8+ CAR-T cells from the generated anti-CD19 CAR-T cell composition. After the third ECAR measurement (approximately 20 minutes into the assay), anti-CD3/anti-CD28 magnetic beads were added to the culture. Area under the curve (AUC) for the ECAR rates (mpH/min) from 0 to 76 minutes of the assay and maximal ECAR glycolytic burst ratio relative to media controls (n=3 donors) were determined.

As shown in **FIG. 3A****,** stimulation with anti-CD3/anti-CD28 beads increased the ECAR in CD8+ CAR-T cells among all generated anti-CD19 CAR-T cell compositions. A trend towards enhanced glycolytic burst upon TCR stimulation of CD8+ CAR-T among anti-CD19 CAR-T cells generated by expansion in the presence of Compound 63 was observed by ECAR AUC (**FIG. 3B**) or ECAR ratio (**FIG. 3C**) relative to media control in each of the 3 donors.

### CAR Signaling in Expanded CAR-T Cells

Antigen-dependent signaling of CAR-T cells among generated anti-CD 19 CAR-T cell compositions was assessed by monitoring activation of phospho-S6. Cells from generated anti-CD19 CAR-T cell compositions, expanded in the presence of media only, DMSO vehicle, PI-103 or Compound 63, were co-cultured with irradiated K562 cells transduced to express CD19 (K562-CD19 target cells) at a ratio of 1:1. After 20 hours, cells were assessed, for intracellular S6 phosphorylation and co-stained for surface expression of CD4 or CD8, by flow cytometry.

As shown in **FIG. 4A****,** following stimulation with antigen-expressing cells, higher levels of phospho-S6 was observed in CD4+ and CD8+ T cells among each of the generated anti-CD19 CAR-T cell compositions as compared to cells that were not stimulated with antigen. The phospho-S6 staining was similar in CD4+ and CD8+ T cells among compositions that were expanded in the presence of PI-103 or Compound 63 compared to among control compositions that were expanded with DMSO vehicle or media only. These results are consistent with a finding that cells expanded in the presence of mTOR kinase inhibitors retain normal mTOR kinase signaling activity after washout of the inhibitor.

### Cytolytic Activity

To assess cytolytic activity, the generated anti-CD19 CAR-T cell compositions were co-cultured with K562-CD19 target cells at a ratio of either 3:1 or 1: 1 effector cells to target cells. The target cells were labeled with NucLight Red (NLR) to permit tracking by fluorescent microscopy. As a negative control, K562-CD19 target cells were cultured alone or were co-cultured with CD4+ and CD8+ T cells that did not express an anti-CD19 CAR. Killing activity was assessed by measuring the loss of viable target cells after 80 hours, as determined by red fluorescent signal (using the INCUCYTE^{®} Live Cell Analysis System, Essen Bioscience). Cell killing was quantified as the inverse of the area under the curve as a function of amount of viable target cells over time.

As shown in **FIG. 4B****,** cells that were expanded in the presence of PI-103 or Compound 63 displayed similar cytolytic activity to control cells that were expanded with DMSO vehicle or media only. These results indicate that target cell killing function was retained in T cells that had been expanded in the presence of mTOR kinase inhibitors.

### Cytokine Measurement

To measure cytokines following antigen stimulation, cells from the generated anti-CD19 CAR-T cell compositions were co-cultured with irradiated K562-CD19 target cells or K562 parental target cells at an effector:target cell ratio of 1:1. After overnight (~16 hours) culture, cell culture supernatants were harvested and TNF-alpha, IFN-gamma, and IL-2 cytokine production were measured using a Luminex Multiplex Assay. The fold-change of cytokine production observed in co-culture supernatants was determined from generated anti-CD19 CAR-T cell compositions expanded in the presence of PI-103, Compound 63, or DMSO vehicle compared to cells expanded in media only.

As shown in **FIG. 5****,** production of TNF-alpha, IFN-gamma, and IL-2 was detected from T cells co-cultured with antigen-expressing cells. Cells from generated anti-CD19 CAR-T cell compositions that were expanded in the presence of PI-103 or Compound 63 exhibited improvements in cytokine production compared to control cells, particularly with respect to production of IFN-gamma, which was improved in cells expanded with either PI-103 or Compound 63.

Intracellular cytokines, CD107a, IFN-gamma (IFNγ), IL-2, IL-17a, and TNF-alpha (TNFα), were assessed in co-cultured T cells that were split into two groups and further incubated for 5 hours with PMA/Ionomycin and a Golgi Inhibitor or a Golgi Inhibitor alone. The intracellular cytokine accumulation was expressed as a fold change in frequency of cytokine positive cells from media-only controls. CD8+ T cells (**FIG. 6A**) and CD4+ T cells (**FIG. 6B**) that were expanded with PI-103 or Compound 63 exhibited an increased frequency of certain polyfunctional cytokine profiles as compared to control cells (boxed profiles in **FIGS. 6A** **and** **6B**). In particular, CD8+ T cells exhibited an increased polyfunctional profile of CD107a+IFNγ+TNFα+ positive cells following re-stimulation with PMA/Ionomycin and a Golgi Inhibitor, and an increased polyfunctional profile of CD107a+IFNγ+IL-2+ cells following incubation with a Golgi Inhibitor alone (**FIG. 6A**). In CD4+ cells, an increased polyfunctional profile of CD107a+IFNγ+TNFα+ cells was observed following treatment with PMA/Ionomycin and a Golgi Inhibitor, or a Golgi Inhibitor alone, while polyfunctional profiles of CD107a+IFNγ+IL-2+TNFα+ cells increased following restimulation with PMA/Ionomycin and a Golgi Inhibitor, and polyfunctional profiles of CD107a+IFNy+ cells increased following incubation with a Golgi Inhibitor alone (**FIG. 6B**).

### Serial Stimulation

The ability of cells to expand *ex vivo* following repeated stimulations in some aspects can indicate capacity of CAR-T cells to persist *(e.g*., following initial activation) and/or is indicative of function *in vivo* (Zhao et al. (2015) Cancer Cell, 28:415-28). To assess function of cells in a serial stimulation assay, the generated anti-CD19 CAR-T cell compositions were incubated with irradiated K562-CD19 target cells. Every 3-4 days, T cells were harvested, counted, and restimulated with new target cells using the same culture conditions after resetting cell number to initial seeding density for each round. After four rounds of restimulation, T cells were re-cultured for an additional 4 days with no further restimulation with target cells. The population doublings (**FIG. 7A**) and area under the curves (AUC) as a function of population doublings over time relative to AUC of cells expanded with media only (**FIG. 7B**) were determined.

As shown in **FIG. 7A****,** the number of anti-CD19 CAR-expressing T cells increased in this assay, consistent with the ability of these cells to proliferate in the presence of CD 19-expressing cells. As shown from the fold-change of AUC of population doublings, T cells from generated anti-CD19 CAR T cell compositions that had been expanded with Compound 63 had a larger mean AUC compared to T cells expanded with PI-103 or DMSO vehicle (**FIG. 7B**). This observation indicates that the presence of Compound 63 during the expansion of CAR-T cells supports sustained expansion and survival of the T cells even after repeated antigen stimulation.

To assess activity upon further secondary stimulation, CAR-T cells were harvested at day 11 following serial re-stimulation, and were stimulated with irradiated K562-CD19 target cells at an effector:target ratio of 1:1 for approximately 16 hours. Supernatant was collected and TNF-alpha, IFN-gamma, and IL-2 cytokine production were measured using a Luminex Multiplex Assay substantially as described above. The fold-change of cytokine production observed in co-culture supernatants from generated anti-CD19 CAR-T cell compositions expanded in the presence of PI-103, Compound 63, or DMSO vehicle compared to cells expanded in media only was determined and are shown in **FIG. 7C****.** Assessment of polyfunctional cytokine profiles of cells at day 11, following incubation with a Golgi Inhibitor for 4 hours substantially as described above, showed an increased CD8+ polyfunctional cytokine profile of CD107a+IFNy+ cells (**FIG. 7D**).

The ability of the generated anti-CD19 CAR-T cell compositions that were expanded in the presence of mTOR inhibitors to retain improved cytokine capacity and survival through sustained antigen exposure is consistent with a resistance to functional exhaustion.

### CAR-Specific Expansion

The ability of the cells to expand following stimulation of the CAR was assessed by incubating cells of the generated anti-CD19 CAR-T cell compositions with beads surface conjugated with an anti-idiotype antibody specific to the anti-CD19 CAR. The anti-idiotype antibody conjugated beads were incubated with cells at a 1:1 bead:cell ratio in wells of 24-well G-rex expansion vessels (Argos Technologies) for 15 days. The total live T cells per well was determined by counting cells in the cultures every 5 days (**FIG. 8A**). The mean area under the curve (AUC) as a function of T cell number over time was calculated relative to the AUC of cells expanded with media only (**FIG. 8B**).

As shown in **FIG. 8A****,** stimulation of cells with anti-idiotypic antibody conjugated beads resulted in an initial expansion that was followed by a decline in cell number. T cells from generated anti-CD19 CAR T cell compositions that had been previously expanded with Compound 63 or PI-103 had a larger mean AUC as compared to T cells previously expanded with DMSO vehicle (**FIG. 8B**). The results indicate that the presence of an mTOR kinase inhibitor during expansion supports enhanced expansion and survival following a single CAR-specific stimulation.

Secondary cytokine response after stimulation with antigen-expressing cells was assessed on CAR-T cells harvested at day 11 following expansion with anti-idiotype antibody conjugated beads. The anti-idiotype antibody stimulated cells were incubated with irradiated K562-CD19 target cells at an effector:target ratio of 1:1 for approximately 16 hours. Supernatant was collected and TNF-alpha, IFN-gamma, and IL-2 cytokine production were measured using a Luminex Multiplex Assay substantially as described above. The fold-change of cytokine production observed in co-culture supernatants was determined from generated anti-CD19 CAR-T cell compositions expanded in the presence of PI-103, Compound 63, or DMSO vehicle compared to cells expanded in media only. As shown in **FIG. 8C****,** T cells from generated anti-CD19 CAR T cell compositions that had been previously expanded with Compound 63 or PI-103 exhibited improved secondary cytokine production following subsequent stimulation with antigen. In addition, some donor-derived cells that were engineered and expanded with Compound 63 exhibited an increased frequency of CD8+ T cells that were CD107a+IFNγ+ cells at day 11, as determined by intracellular cytokine staining following incubation with a Golgi Inhibitor for 4 hours substantially as described above (**FIG. 8D**).

### Example 4: Gene expression Analysis of Engineered CD4+ and CD8+ T Cells Expanded in the Presence of a mTOR Kinase Inhibitor

Gene expression among cells of the generated anti-CD19 CAR-T cell compositions described in Example 2, generated by expanding the cells in the presence of PI-103, Compound 63, DMSO vehicle, or media only, was assessed by RNA sequencing (RNA-Seq). RNA was extracted from compositions generated from three donors under each expansion condition and an assessment of the whole-transcriptome was performed by RNA-Seq. FPKM and FPKQ values were determined; FPKQ values were log-transformed (log2). Gene expression was determined by comparing expression profiles of CD4+, CD8+ or combined CD4+/CD8+ cells among cells of the generated anti-CD 19 CAR-T cell compositions that were expanded in the presence of media only, PI-103, or Compound 63, as compared to cells expanded with DMSO vehicle. Gene products were identified that were different among each groups based on analysis of a volcano plot by imposing cutoff of FDR ≤ 10% between the two conditions.

As shown in the volcano plots in **FIG. 9A**, significantly changed gene expression was identified among CD4+ and/or CD8+ T cells expanded in the presence of PI-103 or Compound 63, with downregulated genes depicted to the left of the middle point ("0") of each plot and upregulated genes depicted to the right of the middle point.

The expression of each differentially expressed gene in cells expanded with PI-103 or Compound 63 was plotted as the Log² fold-change as compared to the expression in cells expanded with DMSO. As shown in **FIG. 9B**, a linear relationship between the expression of the differentially expressed genes in cells expanded with PI-103 or Compound 63 was identified, indicating a strong positive correlation between expression of the differentially expressed genes in cells expanded with PI-103 and cells expanded with Compound 63 (R² = 0.9).

Ontological enrichment analysis on the differentially expressed genes was carried out to identify gene ontology (GO) categories, based on transcriptional regulators of differentially expressed genes that were activated or inhibited as compared to expression in cells expanded with DMSO vehicle. TZ-scores of the transcriptional regulator were calculated based on the concordance of the expected transcriptional effect directions in each regulatory network relative to the observed transcriptional effects in cells expanded with PI-103 or Compound 63. **FIG. 9C** lists exemplary identified GO categories, defined by the regulatory member of each cluster, relative to their corresponding Z-scores.

### Example 5: Assessment of Tumor Burden and Survival in a Tumor Xenograft Model Following Administration of CAR-T Cells Expanded in the Presence of a mTOR Kinase Inhibitor

Anti-tumor effects of the generated anti-CD 19 CAR-T cell compositions, generated by expansion in the presence of PI-103, Compound 63 or media only as described in Example 2, was assessed. The tumor xenograft mouse model was generated by implanting nod scid gamma (NSG) immunodeficient mice with 0.5 ×10⁶ Raji cells (an immortalized human B lymphocyte tumor cell line that expresses CD19), which were allowed to engraft. The Raji cells were transfected with firefly luciferase to facilitate detection by bioluminescence imaging. After seven days, mice either received no treatment, DMSO vehicle, or a low dose (0.25×10⁶) or a high dose (1.0×10⁶) dose of CAR+ T cells of the generated anti-CD19 CAR-T cell compositions. Tumor burden was assessed by bioluminescence weekly or every 10 days.

Treatment of tumor-bearing mice with either the low or high dose of CAR-T cells improved tumor burden and survival as compared to no treatment or DMSO vehicle. Reduced tumor burden was observed following administration of a low or a high dose of CAR-T cells from an anti-CD 19 CAR-T cell composition that had been expanded in the presence of PI-103 (**FIG. 10A**, top panels) or Compound 63 (**FIG. 11A**, top panels) as compared with DMSO vehicle. Tumor-bearing mice administered a low or high dose of CAR-T cells that had been previously expanded in the presence of PI-103 or Compound 63 also displayed substantially improved survival as compared to tumor-bearing mice administered CAR-T cells expanded with the DMSO vehicle (**FIGS. 10A** and **11A****,** bottom panels). Tumor-bearing mice administered the high dose of CAR-T cells expanded in the presence of Compound 63 displayed 100% survival for at least 80 days following tumor cell implant. **FIGS. 10B** **and** **11B** show results for survival of tumor-bearing mice at later time points up to 100 days following tumor cell implant after treatment with CAR-T cells expanded in the presence of PI-103 or Compound 63, respectively; the results were consistent with the effect of cells produced in the presence of Compound 63 resulting in improved performance of administered CAR-T cells. The results indicate that a CAR-T cell composition produced in the presence of an mTOR kinase inhibitor, such as Compound 63, exhibits improved performance in in vivo assays. The improved tumor clearance and survival in the in vivo model are consistent with a qualitative improvement of the state and/or function of the CAR-T cells that was achieved through the inhibition of mTOR signaling during the CAR-T cell generation.

### Example 6: In Vitro Assay for Chronic Stimulation of CAR+ T Cells Utilizing Anti-Idiotype Conjugated Beads

Separate compositions of CD4+ and CD8+ cells were isolated from human donors, stimulated by activation with anti-CD3/anti-CD28 magnetic beads and transduced with a viral vector encoding an anti-CD 19 CAR having an scFv derived from FMC63. Following cultivation under conditions to expand the cells, T cell compositions containing engineered CD4+ and CD8+ T cells from each donor were then separately harvested, formulated, and cryofrozen. The cryofrozen engineered CD4+ and CD8+ T cells were thawed and formulated at a 1:1 ratio of CD4+ and CD8+ T cells from the same donor to generate a T cell composition containing CAR+ T cells. Anti-idiotype (ID) antibody conjugated beads against the anti-CD19 CAR were incubated with cells at a 1:1 bead:cell ratio for 14 days.

Secondary response of CAR-T cells harvested at day 14 following CAR-specific stimulation with anti-ID conjugated beads (Day 14; secondary) was assessed after stimulation with K562-CD19 antigen-expressing target cells at an effector to target ratio of 1:1 (to assess cytokine levels) or 3:1 (to assess cytolytic activity). The primary response of T cells from the T cell composition that had not been incubated with the anti-ID conjugated beads also was determined by similar stimulation with antigen-expressing cells (Day 0; "primary"). To assess cytolytic activity, the target cells were labeled with NucLight Red (NLR) to permit tracking by fluorescent microscopy. Killing activity was assessed by measuring the loss of viable target cells over 72 hours, as determined by loss of fluorescent signal over time by kinetic fluorescence microscopy (using the INCUCYTE^{®} Live Cell Analysis System, Essen Bioscience). Killing index was determined as the inverse of the area under the curve (AUC) for target fluorescence over time. Intracellular cytokine levels of IL-2 and TNF-alpha were assessed by flow cytometry in co-cultured T cells after incubation in the presence of a Golgi Inhibitor.

As shown in **FIG. 12A****,** target cell killing by a T cell composition containing CAR+ T cells collected following CAR-specific stimulation for 14 days with anti-ID conjugated beads was reduced compared to cytolytic activity of CAR+ T cells that did not undergo prior CAR-specific stimulation. Intracellular cytokine levels of IL-2 and TNF-alpha were also reduced in CAR+ T cells that had received long-term CAR-specific stimulation with the anti-ID conjugated beads (**FIG. 12B**). These results are consistent with an observation that long-term CAR-specific stimulation, such as by incubation with anti-ID conjugated beads for 14 days, leads to chronic stimulation of the CAR and loss of sustained function.

The chronic stimulation assay described above was used to assess the effects of PI-103 or Compound 63 on improving CAR+ T cell function after long-term stimulation. Anti-CD19 CAR+ T cell compositions were generated as described above, except in the presence of PI-103, Compound 63 or a vehicle control starting from the initiation of the stimulation. Cells from each generated CAR-T cell composition were incubated with anti-ID conjugated paramagnetic beads at 1:1 bead to cell ratio for 14 days.

Primary response of CAR-T cell compositions at thaw (no stimulation with anti-ID conjugated beads) or secondary response of CAR-stimulated CAR-T compositions (following 14 day CAR-specific stimulation with anti-ID conjugated beads) was assessed after stimulation with antigen-expressing cells. CAR-T cell compositions were cultured 1:1 with K562-CD19 antigen-expressing cells in the presence of a Golgi Inhibitor, and polyfunctional cytokine production was assessed by flow cytometry following intracellular cytokine staining for IL-2, IFN-gamma and TNF-alpha. A polyfunctional score was determined from cumulative levels of cytokines as determined in CD8+ cells after the data were normalized by scaling within donor cohorts **(****FIG. 13A****)**. Total secreted IL-2, TNF and IFN-gamma cytokines from cell culture supernatant of co-cultures after 20 hours of incubation with targets cells was determined, and the average of the scaled scores for all three cytokines was calculated as shown in **FIG. 13A**. As shown in **FIG. 13A****,** PI-103 or Compound 63 resulted in improved primary or secondary responses based on the ability of CAR-T cell compositions to produce cytokines. Improvements in primary or secondary cytolytic response, following co-culture with target cells at a 3:1 effector:target cell ratio as described above, also was observed among T cell compositions produced in the presence of PI-103 or Compound 63 (**FIGS. 13B** **and** **13C**).

These results demonstrate the utility of the chronic stimulation assays to evaluate CAR-T cell compositions, including different CAR-T cell compositions produced under different conditions or in the presence of PI-103 or Compound 63 or other agents, for their ability to exhibit long-term survival and/or sustain function after chronic CAR-T cell stimulation, such as may occur following prolonged exposure to antigen in vivo.

### Example 7: Functional Assessment of Chimeric Antigen Receptor (CAR)-Transduced T Cells (CAR T Cells) Prepared in the Presence of a mTOR Kinase inhibitor

The impact of the presence of an mTOR Kinase inhibitor during cell production was further assessed in a process for engineering T cells in which CD4+ and CD8+ T cell populations were separately enriched and then mixed and processed together in a single composition. The processing steps including those for stimulation, transduction with a vector encoding a chimeric antigen receptor, and expansion.

Separate compositions of CD4+ and CD8+ cells were selected from isolated PBMCs from a leukapheresis sample from the same human donor by immunoaffinity based selection, and the selected cell compositions were cryofrozen. The separate compositions of CD4+ and CD8+ T cells were subsequently thawed and mixed at a ratio of 1:1 of viable CD4+ T cells to viable CD8+ T cells. In this study, the mixed CD4+ and CD8+ cell populations were incubated in the presence of Compound 63, PI103 or no inhibitor (DMSO vehicle control) beginning at the initiation of stimulation. In more detail, the mixed CD4+ and CD8+ T cell composition was stimulated (in the presence or absence of an inhibitor, as indicated) in the presence of paramagnetic polystyrene-coated beads with attached anti-CD3 and anti-CD28 antibodies for between 18 to 30 hours, and were then transduced with a lentiviral vector encoding an anti-CD 19 CAR. The CAR contained an scFv antigen-binding domain specific for CD 19 (derived from FMC63), a CD28 transmembrane region, a 4-1BB costimulatory signaling region, and a CD3-zeta derived intracellular signaling domain. The cells were then expanded in the presence of cytokines typically for approximately 6-7 days. Beginning with stimulation and for the duration of the process, the media contained DMSO (vehicle control), 2 µM PI103 or 1 µM Compound 63. The vehicle control contained DMSO at a volume to match that in the mTOR inhibitor-treated samples. Expanded cells were cryopreserved. For assessment, the cryopreserved CAR-T cells were thawed and washed prior to assessment in assay media without supportive cytokines and without inhibitor or vehicle.

The cells were assessed by flow cytometry for levels of the cell surface markers and levels of pro-apoptotic marker, intracellular caspase 3. Representative flow cytometry plots from three donors are shown in **FIG. 14****.** As shown, CAR-T cells prepared in the presence of Compound 63 were observed to have decreased levels of the pro-apoptotic marker, intracellular caspase 3.

Functional attributes of the generated CAR-T cells were assessed in a serial stimulation assay by incubation of the generated CAR-T cells antibody conjugated to beads. The anti-CAR antibody was an anti-idiotypic antibody recognizing the FMC63-derived scFv of the CAR. Thawed CAR-T cells were mixed with CAR-specific beads, plated, and incubated for 14 days. Every 3-4 days, T cells were, counted. As shown in **FIG. 15****,** generated CAR-T cells prepared in the presence of PI103 or Compound 63 exhibited improved expansion after restimulation.

Cytolytic activity of the generated CAR-T cells was assessed by culturing the generated CAR-T cells, either freshly thawed or at day 14 of restimulation from above, with CD19-expressing target cells at a 3:1 effector to target ratio. Target cell death was quantitated over time. The tumor cell growth area under the curve (AUC) of signal over time for each concentration was determined. A killing index was calculated as the inverse of the area under the tumor cell growth curve (1/AUC). CAR-T cells that had been generated in a process in the presence of PI103 or Compound 63 exhibited improved target cell killing (**FIG. 16**) as compared to CAR-T cells prepared in the presence of DMSO.

Intracellular cytokine levels were monitored in cells of co-cultures incubated with freshly thawed generated CAR-T cells or CAR-T cells after secondary restimulation with CD19-expressing target cells. CAR-T cells were incubated with target-expressing cells in the presence of golgi inhibitor for 5 hours. Intracellular expression of IL-2, TNF-alpha and IFN-gamma was determined and a polyfunctional score was calculated by gating for cumulative CAR-T cells positive for IL-2 and any combination of IFN-gamma and TNF-alpha. As shown in **FIG. 17A****,** CAR-T cells prepared in the presence of PI103 or Compound 63 exhibited improved polyfunctional effector cytokine profiles in both CD8+ and CD4+ subsets after both primary or secondary stimulation (**FIG. 17A****)** as compared to CAR-T cells prepared in the presence of DMSO. Generated CAR-T cells were also cultured with CD19-antigen expressing cells for 20 hours and levels of IL-2, TNF-alpha and IFN-gamma were assessed in cell culture supernatants by ELISA. CAR-T cells generated with PI103 or Compound 63 exhibited increased levels of secreted cytokines in supernatants (**FIG. 17B**).

Together, these results are consistent with the observation that the presence of PI103 and Compound 63 during a process for producing CAR-T cells from a mixed population of CD4+ and CD8+ T cells improves CAR-T cell function and activity.

### Example 8: Gene expression Analysis of CAR-T Cells Prepared in the Presence of a mTOR Kinase Inhibitor

Gene expression among cells of the compositions containing anti-CD19 CAR-T cells (prepared in the presence DMSO, PI-103 or Compound 63 as described in Example 7) was assessed by differential expression (DESeq2) analysis of RNA-Seq. RNA-seq was performed on the complementary DNA (cDNA) samples prepared from the RNA isolated from the CAR-T cells. Principal component analysis (PCA) was performed for the RNA-seq data sets, generated from DESeq2-normalized counts. Gene level differential expression analyses were performed in R (version 3.4) using the DESeq2 package (version 1.16.1) by comparing compound treated samples to the DMSO control, controlling for donors. Prior to differential expression analysis, the gene set was filtered to exclude genes with zero counts across all samples. Differentially expressed (DE) genes were identified by imposing a log2 fold change cutoff of 0.5 and a Benjamini-Hochberg adjusted false discovery rate (FDR) cutoff of 0.1 Both overlapping and non-overlapping gene expression profiles were observed in compositions containing CAR-T cells prepared in the presence of PI103 or Compound 63 (**FIG. 18**).

### Example 9: Assessment of Tumor Burden and Survival in a Tumor Xenograft Model Following Administration of CAR-T Cells Prepared in the Presence of a mTOR Kinase Inhibitor

Anti-tumor effects of anti-CD 19 CAR-T cell compositions prepared in the presence DMSO, PI-103 or Compound 63 as described in Example 7 were assessed in vivo. The tumor xenograft mouse model was generated by implanting nod scid gamma (NSG) immunodeficient mice with 0.5 ×10⁶ Raji cells (an immortalized human B lymphocyte tumor cell line that expresses CD19), which were allowed to engraft. The Raji cells were transfected with firefly luciferase to facilitate measurement of tumor burden by bioluminescence imaging. After seven days, mice either received no treatment, or treatment with a low dose (0.25×10⁶) or a high dose (1.0×10⁶) of anti-CD19 CAR+ T cells prepared in the presence of DMSO (vehicle) or DMSO and a mTOR kinase inhibitor. Tumor burden and mortality were assessed weekly.

Anti-CD19 CAR-T cell compositions that, separately, had been prepared from cells of three different human donors, in a process including the presence of DMSO, were observed to have demonstrable anti-tumor effects on animals in this study, as compared to non-treated animals, with treated animals exhibiting decreased mortality. Results from cells prepared from a matched donor are shown in **FIGS. 19A-B** and **20A-20B.** The inclusion of the mTOR kinase inhibitor Compound 63 (as compared to the absence of inhibitor, i.e., DMSO vehicle) in the process used to generate the CAR-T cells was observed to result in an improvement in vivo anti-tumor responses in animals in this study. Exemplary results are shown in **FIGS. 20A** **and** **20B****.** Substantially similar results were seen with CAR-T cells generated from another donor. Results comparing CAR-T cells generated in the presence or absence of the inhibitor PI103 are shown in (**FIGS. 19A** **and** **19B**). For CAR-T cells generated from another donor, comparable anti-tumor effects were observed for cells produced in the presence of PI103 and for cells produced in the absence of inhibitor (DMSO vehicle).

### Example 10: Assessment of CAR-T Cell Persistence In Vivo

The presence and numbers of the anti-CD19 CAR-T cells were assessed in the blood of animals following administration of compositions containing the cells having been prepared in the presence of PI-103, Compound 63, or no inhibitor, as described in Example 7) to the animals. The Raji Burkitt's CD19+ lymphoma tumor xenograft model described in Example 9 was utilized. Mice either received no treatment, or treatment with a low dose (0.25×10⁶) or a high dose (1.0×10⁶) of the respective anti-CD19 CAR+ T cell compositions. Tumor burden and mortality were assessed every 7-10 days.

Mice were bled at days 18, 25 and 36 post-infusion, and the presence of circulating CAR+ CD4+ T cells or CAR+ CD8+ T cells in peripheral blood was assessed by flow cytometry. Inclusion of either Compound 63 or PI103 during preparation of the CAR-T cells from the same matched donor as described in Example 10 was observed to result in increased numbers of CAR+ T cells over time, consistent with an interpretation that the use of the inhibitors during production of the cell compositions resulted in increased exposure, such as due to increased expansion or persistence, of cells in vivo, following administration in this animal tumor model (**FIGS. 21A** **and** **21B**). Substantially similar results were seen with CAR-T cells generated from a second donor.

### SEQUENCES

| **SEQ ID NO.** | **SEQUENCE** | **DESCRIPTION** |
|---|---|---|
| **1** | ESKYGPPCPPCP | spacer (IgG4hinge) (aa) |
| | | Homo sapiens |
| **2** | GAATCTAAGTACGGACCGCCCTGCCCCCCTTGCCCT | spacer (IgG4hinge) (nt) |
| | | homo sapiens |
| **3** | ESKYGPPCPPCPGQPREPQVYTLPPSQEEMTKNQVSLTCLVKGFYPSDI AVEWESNGQPENNYKTTPPVLDSDGSFFLYSRLTVDKSRWQEGNVFSCS VMHEALHNHYTQKSLSLSLGK | Hinge-CH3 spacer Homo sapiens |
| **4** | ESKYGPPCPPCPAPEFLGGPSVFLFPPKPKDTLMISRTPEVTCVVVDVS QEDPEVQFNWYVDGVEVHNAKTKPREEQFNSTYRVVSVLTVLHQDWLNG KEYKCKVSNKGLPSSIEKTISKAKGQPREPQVYTLPPSQEEMTKNQVSL TCLVKGFYPSDIAVEWESNGQPENNYKTTPPVLDSDGSFFLYSRLTVDK SRWQEGNVFSCSVMHEALHNHYTQKSLSLSLGK | Hinge-CH2-CH3 spacer |
| | | Homo sapiens |
| **5** | RWPESPKAQASSVPTAQPQAEGSLAKATTAPATTRNTGRGGEEKKKEKE KEEQEERETKTPECPSHTQPLGVYLLTPAVQDLWLRDKATFTCFVVGSD LKDAHLTWEVAGKVPTGGVEEGLLERHSNGSQSQHSRLTLPRSLWNAGT SVTCTLNHPSLPPQRLMALREPAAQAPVKLSLNLLASSDPPEAASWLLC EVSGFSPPNILLMWLEDQREVNTSGFAPARPPPQPGSTTFWAWSVLRVP APPSPQPATYTCVVSHEDSRTLLNASRSLEVSYVTDH | IgD-hinge-Fc |
| | | Homo sapiens |
| **6** | LEGGGEGRGSLLTCGDVEENPGPR | T2A |
| | | artificial |
| **7** | MLLLVTSLLLCELPHPAFLLIPRKVCNGIGIGEFKDSLSINATNIKHFK NCTSISGDLHILPVAFRGDSFTHTPPLDPQELDILKTVKEITGFLLIQA WPENRTDLHAFENLEIIRGRTKQHGQFSLAVVSLNITSLGLRSLKEISD GDVIISGNKNLCYANTINWKKLFGTSGQKTKIISNRGENSCKATGQVCH ALCSPEGCWGPEPRDCVSCRNVSRGRECVDKCNLLEGEPREFVENSECI QCHPECLPQAMNITCTGRGPDNCIQCAHYIDGPHCVKTCPAGVMGENNT LVWKYADAGHVCHLCHPNCTYGCTGPGLEGCPTNGPKIPSIATGMVGAL LLLLVVALGIGLFM | tEGFR |
| | | artificial |
| **8** | FWVLVWGGVLACYSLLVTVAFIIFWV | CD28 (amino acids 153-179 of Accession No. P10747) |
| | | Homo sapiens |
| **9** | IEVMYPPPYLDNEKSNGTIIHVKGKHLCPSPLFPGPSKPFWVLVVVGGV LACYSLLVTVAFIIFWV | CD28 (amino acids 114-179 of Accession No. P10747) |
| | | Homo sapiens |
| **10** | RSKRSRLLHSDYMNMTPRRPGPTRKHYQPYAPPRDFAAYRS | CD28 (amino acids 180-220 of P10747) |
| | | Homo sapiens |
| **11** | RSKRSRGGHSDYMNMTPRRPGPTRKHYQPYAPPRDFAAYRS | CD28 (LL to GG) |
| | | Homo sapiens |
| **12** | KRGRKKLLYIFKQPFMRPVQTTQEEDGCSCRFPEEEEGGCEL | 4-1BB (amino acids 214-255 of Q07011.1) |
| | | Homo sapiens |
| **13** | RVKFSRSADAPAYQQGQNQLYNELNLGRREEYDVLDKRRGRDPEMGGKP RRKNPQEGLYNELQKDKMAEAYSEIGMKGERRRGKGHDGLYQGLSTATK DTYDALHMQALPPR | CD3 zeta |
| | | Homo sapiens |
| **14** | RVKFSRSAEPPAYQQGQNQLYNELNLGRREEYDVLDKRRGRDPEMGGKP RRKNPQEGLYNELQKDKMAEAYSEIGMKGERRRGKGHDGLYQGLSTATK DTYDALHMQALPPR | CD3 zeta |
| | | Homo sapiens |
| **15** | RVKFSRSADAPAYKQGQNQLYNELNLGRREEYDVLDKRRGRDPEMGGKP RRKNPQEGLYNELQKDKMAEAYSEIGMKGERRRGKGHDGLYQGLSTATK DTYDALHMQALPPR | CD3 zeta |
| | | Homo sapiens |
| **16** | RKVCNGIGIGEFKDSLSINATNIKHFKNCTSISGDLHILPVAFRGDSFT HTPPLDPQELDILKTVKEITGFLLIQAWPENRTDLHAFENLEIIRGRTK QHGQFSLAVVSLNITSLGLRSLKEISDGDVIISGNKNLCYANTINWKKL FGTSGQKTKIISNRGENSCKATGQVCHALCSPEGCWGPEPRDCVSCRNV SRGRECVDKCNLLEGEPREFVENSECIQCHPECLPQAMNITCTGRGPDN CIQCAHYIDGPHCVKTCPAGVMGENNTLVWKYADAGHVCHLCHPNCTYG CTGPGLEGCPTNGPKIPSIATGMVGALLLLLVVALGIGLFM | tEGFR |
| | | artificial |
| **17** | EGRGSLLTCGDVEENPGP | T2A artificial |
| **18** | GSGATNFSLLKQAGDVEENPGP | P2A |
| **19** | ATNFSLLKQAGDVEENP GP | P2A |
| **20** | QCTNYALLKLAGDVESNPGP | E2A |
| **21** | VKQTLNFDLLKLAGDVESNPGP | F2A |
| **22** | PGGG- (SGGGG) 5-P- wherein P is proline, G is glycine and S is serine | Exemplary linker |
| **23** | GSADDAKKDAAKKDGKS | Exemplary Linker |
| **24** | GSTSGSGKPGSGEGSTKG | Exemplary Linker |
| **25** | Glu Val Val Val Lys Tyr Gly Pro Pro Cys Pro Pro Cys Pro | Exemplary IgG Hinge |
| **26** | X1PPX2P X1 is glycine, cysteine or arginine X2 is cysteine or threonine | Exemplary IgG Hinge |
| **27** | Glu Pro Lys Ser Cys Asp Lys Thr His Thr Cys Pro Pro Cys Pro | Exemplary IgG Hinge |
| **28** | Glu Arg Lys Cys Cys Val Glu Cys Pro Pro Cys Pro | Exemplary IgG Hinge |
| **29** | ELKTPLGDTHTCPRCPEPKSCDTPPPCPRCPEPKSCDTPPPCPRCPEPK SCDTPPPCPRCP | Exemplary IgG Hinge |
| **30** | Glu Ser Lys Tyr Gly Pro Pro Cys Pro Ser Cys Pro | Exemplary IgG Hinge |
| **31** | Glu Ser Lys Tyr Gly Pro Pro Cys Pro Pro Cys Pro | Exemplary IgG Hinge |
| **32** | Tyr Gly Pro Pro Cys Pro Pro Cys Pro | Exemplary IgG Hinge |
| **33** | Lys Tyr Gly Pro Pro Cys Pro Pro Cys Pro | Exemplary IgG Hinge |
| **34** | MLGTGPAAATTAATTSSNVSVLQQFASGLKSRNEETRAKAAKELQHYVT MELREMSQEESTRFYDQLNHHIFELVSSSDANERKGGILAIASLIGVEG GNATRIGRFANYLRNLLPSNDPVVMEMASKAIGRLAMAGDTFTAEYVEF EVKRALEWLGADRNEGRRHAAVLVLRELAISVPTFFFQQVQPFFDNIFV AVWDPKQAIREGAVAALRACLILTTQREPKEMQKPQWYRHTFEEAEKGF DETLAKEKGMNRDDRIHGALLILNELVRISSMEGERLREEMEEITQQQL VHDKYCKDLMGFGTKPRHITPFTSFQAVQPQQSNALVGLLGYSSHQGLM GFGTSPSPAKSTLVESRCCRDLMEEKFDQVCQWVLKCRNSKNSLIQMTI LNLLPRLAAFRPSAFTDTQYLQDTMNHVLSCVKKEKERTAAFQALGLLS VAVRSEFKVYLPRVLDIIRAALPPKDFAHKRQKAMQVDATVFTCISMLA RAMGPGIQQDIKELLEPMLAVGLSPALTAVLYDLSRQIPQLKKDIQDGL LKMLSLVLMHKPLRHPGMPKGLAHQLASPGLTTLPEASDVGSITLALRT LGSFEFEGHSLTQFVRHCADHFLNSEHKEIRMEAARTCSRLLTPSIHLI SGHAHVVSQTAVQVVADVLSKLLVVGITDPDPDIRYCVLASLDERFDAH LAQAENLQALFVALNDQVFEIRELAICTVGRLSSMNPAFVMPFLRKMLI QILTELEHSGIGRIKEQSARMLGHLVSNAPRLIRPYMEPILKALILKLK DPDPDPNPGVINNVLATIGELAQVSGLEMRKWVDELFIIIMDMLQDSSL LAKRQVALWTLGQLVASTGYVVEPYRKYPTLLEVLLNFLKTEQNQGTRR EAIRVLGLLGALDPYKHKVNIGMIDQSRDASAVSLSESKSSQDSSDYST SEMLVNMGNLPLDEFYPAVSMVALMRIFRDQSLSHHHTMVVQAITFIFK SLGLKCVQFLPQVMPTFLNVIRVCDGAIREFLFQQLGMLVSFVKSHIRP YMDEIVTLMREFWVMNTSIQSTIILLIEQIVVALGGEFKLYLPQLIPHM LRVFMHDNSPGRIVSIKLLAAIQLFGANLDDYLHLLLPPIVKLFDAPEA PLPSRKAALETVDRLTESLDFTDYASRIIHPIVRTLDQSPELRSTAMDT LSSLVFQLGKKYQIFIPMVNKVLVRHRINHQRYDVLICRIVKGYTLADE EEDPLIYQHRMLRSGQGDALASGPVETGPMKKLHVSTINLQKAWGAARR VSKDDWLEWLRRLSLELLKDSSSPSLRSCWALAQAYNPMARDLFNAAFV SCWSELNEDQQDELIRSIELALTSQDIAEVTQTLLNLAEFMEHSDKGPL PLRDDNGIVLLGERAAKCRAYAKALHYKELEFQKGPTPAILESLISINN KLQQPEAAAGVLEYAMKHFGELEIQATWYEKLHEWEDALVAYDKKMDTN KDDPELMLGRMRCLEALGEWGQLHQQCCEKWTLVNDETQAKMARMAAAA AWGLGQWDSMEEYTCMIPRDTHDGAFYRAVLALHQDLFSLAQQCIDKAR DLLDAELTAMAGESYSRAYGAMVSCHMLSELEEVIQYKLVPERREIIRQ IWWERLQGCQRIVEDWQKILMVRSLVVSPHEDMRTWLKYASLCGKSGRL ALAHKTLVLLLGVDPSRQLDHPLPTVHPQVTYAYMKNMWKSARKIDAFQ HMQHFVQTMQQQAQHAIATEDQQHKQELHKLMARCFLKLGEWQLNLQGI NESTIPKVLQYYSAATEHDRSWYKAWHAWAVMNFEAVLHYKHQNQARDE KKKLRHASGANITNATTAATTAATATTTASTEGSNSESEAESTENSPTP SPLQKKVTEDLSKTLLMYTVPAVQGFFRSISLSRGNNLQDTLRVLTLWF DYGHWPDVNEALVEGVKAIQIDTWLQVIPQLIARIDTPRPLVGRLIHQL LTDIGRYHPQALIYPLTVASKSTTTARHNAANKILKNMCEHSNTLVQQA MMVSEELIRVAILWHEMWHEGLEEASRLYFGERNVKGMFEVLEPLHAMM ERGPQTLKETSFNQAYGRDLMEAQEWCRKYMKSGNVKDLTQAWDLYYHV FRRISKQLPQLTSLELQYVSPKLLMCRDLELAVPGTYDPNQPIIRIQSI APSLQVITSKQRPRKLTLMGSNGHEFVFLLKGHEDLRQDERVMQLFGLV NTLLANDPTSLRKNLSIQRYAVIPLSTNSGLIGWVPHCDTLHALIRDYR EKKKILLNIEHRIMLRMAPDYDHLTLMQKVEVFEHAVNNTAGDDLAKLL WLKSPSSEVWFDRRTNYTRSLAVMSMVGYILGLGDRHPSNLMLDRLSGK ILHIDFGDCFEVAMTREKFPEKIPFRLTRMLTNAMEVTGLDGNYRITCH TVMEVLREHKDSVMAVLEAFVYDPLLNWRLMDTNTKGNKRSRTRTDSYS AGQSVEILDGVELGEPAHKKTGTTVPESIHSFIGDGLVKPEALNKKAIQ IINRVRDKLTGRDFSHDDTLDVPTQVELLIKQATSHENLCQCYIGWCPF W | Human mTOR protein |
| **35** | QQGNTLPYT | CDR L3 |
| **36** | RASQDISKYLN | CDR L1 |
| **37** | SRLHSGV | CDR L2 |
| **38** | GNTLPYTFG | CDR L3 |
| **39** | DYGVS | CDR H1 |
| **40** | VIWGSETTYYNSALKS | CDR H2 |
| **41** | YAMDYWG | CDR H3 |
| **42** | EVKLQESGPGLVAPSQSLSVTCTVSGVSLPDYGVSWIRQPPRKGLEWLG VIWGSETTYYNSALKSRLTIIKDNSKSQVFLKMNSLQTDDTAIYYCAKH YYYGGSYAMDYWGQGTSVTVSS | VH |
| **43** | DIQMTQTTSSLSASLGDRVTISCRASQDISKYLNWYQQKPDGTVKLLIY HTSRLHSGVPSRFSGSGSGTDYSLTISNLEQEDIATYFCQQGNTLPYTF GGGTKLEIT | VL |
| **44** | DIQMTQTTSSLSASLGDRVTISCRASQDISKYLNWYQQKPDGTVKLLIY HTSRLHSGVPSRFSGSGSGTDYSLTISNLEQEDIATYFCQQGNTLPYTF GGGTKLEITGSTSGSGKPGSGEGSTKGEVKLQESGPGLVAPSQSLSVTC TVSGVSLPDYGVSWIRQPPRKGLEWLGVIWGSETTYYNSALKSRLTIIK DNSKSQVFLKMNSLQTDDTAIYYCAKHYYYGGSYAMDYWGQGTSVTVSS | scFv |
| **45** | KASQNVGTNVA | CDR L1 |
| **46** | SATYRNS | CDR L2 |
| **47** | QQYNRYPYT | CDR L3 |
| **48** | SYWMN | CDR H1 |
| **49** | QIYPGDGDTNYNGKFKG | CDR H2 |
| **50** | KTISSWDFYFDY | CDR H3 |
| **51** | EVKLQQSGAELVRPGSSVKISCKASGYAFSSYWMNWVKQRPGQGLEWIG QIYPGDGDTNYNGKFKGQATLTADKSSSTAYMQLSGLTSEDSAVYFCAR KTISSVVDFYFDYWGQGTTVTVSS | VH |
| **52** | DIELTQSPKFMSTSVGDRVSVTCKASQNVGTNVAWYQQKPGQSPKPLIY SATYRNSGVPDRFTGSGSGTDFTLTITNVQSKDLADYFCQQYNRYPYTS GGGTKLEIKR | VL |
| **53** | GGGGSGGGGSGGGGS | Linker |
| **54** | EVKLQQSGAELVRPGSSVKISCKASGYAFSSYWMNWVKQRPGQGLEWIG QIYPGDGDTNYNGKFKGQATLTADKSSSTAYMQLSGLTSEDSAVYFCAR KTISSVVDFYFDYWGQGTTVTVSSGGGGSGGGGSGGGGSDIELTQSPKF MSTSVGDRVSVTCKASQNVGTNVAWYQQKPGQSPKPLIYSATYRNSGVP DRFTGSGSGTDFTLTITNVQSKDLADYFCQQYNRYPYTSGGGTKLEIKR | scFv |
| **55** | HYYYGGSYAMDY | CDR H3 |
| **56** | HTSRLHS | CDR L2 |
| **57** | GSTSGSGKPGSGEGSTKG | Linker |
| **58** | gacatccagatgacccagaccacctccagcctgagcgccagcctgggcg accgggtgaccatcagctgccgggccagccaggacatcagcaagtacct gaactggtatcagcagaagcccgacggcaccgtcaagctgctgatctac cacaccagccggctgcacagcggcgtgcccagccggtttagcggcagcg gctccggcaccgactacagcctgaccatctccaacctggaacaggaaga tatcgccacctacttttgccagcagggcaacacactgccctacaccttt ggcggcggaacaaagctggaaatcaccggcagcacctccggcagcggca agcctggcagcggcgagggcagcaccaagggcgaggtgaagctgcagga aagcggccctggcctggtggcccccagccagagcctgagcgtgacctgc accgtgagcggcgtgagcctgcccgactacggcgtgagctggatccggc agccccccaggaagggcctggaatggctgggcgtgatctggggcagcga gaccacctactacaacagcgccctgaagagccggctgaccatcatcaag gacaacagcaagagccaggtgttcctgaagatgaacagcctgcagaccg acgacaccgccatctactactgcgccaagcactactactacggcggcag | Sequence encoding scFv |
| | ctacgccatggactactggggccagggcaccagcgtgaccgtgagcagc | |
| **59** | MPLLLLLPLLWAGALA | CD33 signal peptide |
| **60** | MALPVTALLLPLALLLHA | CD8 alpha signal peptide |
| **61** | atgcttctcctggtgacaagccttctgctctgtgagttaccacacccag cattcctcctgatccca | GMCSFR alpha chain signal sequence |
| **62** | MLLLVTSLLLCELPHPAFLLIP | GMCSFR alpha chain signal sequence |

## Claims

1. A method for producing a composition of engineered cells, the method comprising:
(a) incubating, under stimulating conditions, an input composition comprising primary human T cells, said stimulating conditions comprising the presence of (i) a stimulatory reagent capable of activating one or more intracellular signaling domains of one or more components of a TCR complex and/or one or more intracellular signaling domains of one or more costimulatory molecules and (ii) an agent that inhibits mTOR activity, wherein the agent that inhibits mTOR activity is 2-(3-hydroxyphenyl)-9-(2-isopropylphenyl)-8-oxo-8,9-dihydro-7H-purine-6-carboxamide (Compound 63); and
(b) introducing a recombinant receptor into the stimulated composition, thereby generating an engineered composition comprising engineered T cells.

2. The method of claim 1, comprising further incubating the engineered cells, wherein the incubating comprises the presence of the agent that inhibits mTOR activity, thereby producing an output composition, optionally wherein:
(a) the further incubating is carried out in the presence of one or more recombinant cytokines; and/or
(b) the further incubating comprises cultivation under conditions to result in the proliferation or expansion of cells in the composition.

3. A method for producing a composition of engineered cells, the method comprising cultivating, in the presence of an agent that inhibits mTOR activity, an engineered cell composition comprising enriched primary human T cells comprising T cells engineered with a recombinant receptor;
wherein the agent that inhibits mTOR activity is 2-(3-hydroxyphenyl)-9-(2-isopropylphenyl)-8-oxo-8,9-dihydro-7H-purine-6-carboxamide (Compound 63); and
wherein the method results in the proliferation or expansion of cells in the composition to produce an output composition comprising engineered T cells.

4. The method of claim 3, wherein, prior to the cultivating, the method further comprises:
(a) incubating, under stimulating conditions, an input composition comprising primary T cells in the presence of the agent that inhibits mTOR activity; wherein said stimulating conditions comprise the presence of a stimulatory reagent capable of activating one or more intracellular signaling domains of one or more components of a TCR complex and/or one or more intracellular signaling domains of one or more costimulatory molecules, thereby generating a stimulated composition; and
(b) introducing a recombinant receptor into the stimulated composition, thereby generating an engineered composition comprising engineered T cells.

5. The method of any of claims 1-4, wherein the primary T cells are CD4+ and/or CD8+ T cells.

6. The method of any of claims 3-5, wherein:
(a) the engineered T cell composition comprises enriched CD4+ T cells; or
(b) the engineered T cell composition comprises enriched CD8+ T cells.

7. The method of any of claims 3-6, wherein the cultivating is carried out in the presence of one or more recombinant cytokines.

8. The method of claim 2 or 7, wherein the one or more recombinant cytokines comprises one or more of IL-2, IL-4, IL-7, IL-9, IL-12, IL-15, G-CSF, and GM-CSF, optionally wherein the one or more recombinant cytokines comprises one or more of IL-2, IL-7, and IL-15.

9. The method of any of claims 3-8, wherein, prior to the cultivating, the method further comprises:
(a) incubating, under stimulating conditions, an input composition comprising primary T cells, said stimulating conditions comprising the presence of a stimulatory reagent capable of activating one or more intracellular signaling domains of one or more components of a TCR complex and/or one or more intracellular signaling domains of one or more costimulatory molecules, thereby generating a stimulated composition; and
(b) introducing a recombinant receptor into the stimulated composition, thereby generating an engineered composition comprising engineered T cells.

10. The method of any of claims 1, 2, and 9, wherein:
(a) the input composition, the stimulated composition, and/or the engineered composition comprises primary CD4+ and/or CD8+ T cells; and/or
(b) wherein the input composition, the stimulated composition, and/or the engineered composition comprises enriched CD4+ T cells.

11. The method of any of claims 1, 2, 9, and 10, wherein the input composition, the stimulated composition, and/or the engineered composition comprises enriched CD8+ T cells.

12. The method of any of claims 1, 2, 9, and 10, wherein:
(a)
(i) the input composition, the stimulated composition, and/or the engineered composition comprises greater than or greater than about 70%, greater than or greater than about 75%, greater than or greater than about 80%, greater than or greater than about 85%, greater than or greater than about 90%, greater than or greater than about 95% or greater than or greater than about 98% CD4+ primary human T cells; and/or
(ii) the input composition consists essentially of CD4+ primary human T cells; and/or
(b)
(i) the input composition, the stimulated composition, and/or the engineered composition comprises greater than or greater than about 70%, greater than or greater than about 75%, greater than or greater than about 80%, greater than or greater than about 85%, greater than or greater than about 90%, greater than or greater than about 95% or greater than or greater than about 98% CD8+ primary human T cells; and/or
(ii) the input composition consists essentially of CD8+ primary human T cells.

13. The method of claim 3 or claim 4, wherein the agent that inhibits mTOR activity is Compound 63 and the engineered cell composition is cultivated in the presence of between 500 nM and 2 µM, between 1 nM and 100 nM, between 50 nM and 250 nM, or between 100 nM and 500 nM of Compound 63.

14. The method of claim 1 or claim 2, wherein the agent that inhibits mTOR activity is Compound 63 and the engineered cell composition is incubated in the presence of between 500 nM and 2 µM, between 1 nM and 100 nM, between 50 nM and 250 nM, or between 100 nM and 500 nM of Compound 63.

15. The method of any of claims 1-2 and 9-14, wherein the stimulatory reagent comprises a primary agent that specifically binds to a member of a TCR complex, optionally wherein:
(a) the primary agent specifically binds to CD3;
and/or
(b) the stimulatory reagent further comprises a secondary agent that specifically binds to a T cell costimulatory molecule, optionally wherein the costimulatory molecule is selected from CD28, CD137 (4-1-BB), OX40, and ICOS;
and/or
(c) the primary and/or secondary agents comprise an antibody, optionally wherein the stimulatory reagent comprises incubation with an anti-CD3 antibody and an anti-CD28 antibody, or an antigen-binding fragment thereof; optionally wherein the primary agent and/or secondary agent are present on the surface of a solid support, optionally wherein the solid support is or comprises a bead.

16. The method of any of claims 1-2 and 9-15, wherein the introducing comprises transducing cells of the stimulated composition with a viral vector comprising a polynucleotide encoding the recombinant receptor; optionally wherein the viral vector is a retroviral vector; optionally wherein the viral vector is a lentiviral vector or a gammaretroviral vector.

17. The method of any of claims 2-16, wherein the method further comprises collecting cells of the output composition; optionally further comprising formulating cells of the output composition for cryopreservation and/or administration to a subject, optionally in the presence of a pharmaceutically acceptable excipient.

18. The method of any of 1-2 and 9-17, further comprising isolating the CD4+ and/or the CD8+ T cells from a biological sample prior to the incubating; optionally wherein the biological sample is or comprises a whole blood sample, a buffy coat sample, a peripheral blood mononuclear cells (PBMC) sample, an unfractionated T cell sample, a lymphocyte sample, a white blood cell sample, an apheresis product, or a leukapheresis product.

19. The method of any of claims 1-18, wherein:
(a) the recombinant receptor is capable of binding to a target antigen that is associated with, specific to, and/or expressed on a cell or tissue of a disease, disorder, or condition; optionally wherein the disease, disorder, or condition is an infectious disease or disorder, an autoimmune disease, an inflammatory disease, a tumor, or a cancer; optionally wherein the target antigen is a tumor antigen; and/or
(b) wherein the recombinant receptor is or comprises a functional non-TCR antigen receptor or a TCR or antigen-binding fragment thereof; and/or
(c) the recombinant receptor is a chimeric antigen receptor (CAR).

## Patentansprüche

1. Verfahren zum Erzeugen einer Zusammensetzung aus veränderten Zellen, das Verfahren umfassend:
(a) Inkubieren, unter stimulierenden Bedingungen, einer Eingabezusammensetzung, umfassend primäre humane T-Zellen, die stimulierenden Bedingungen umfassend das Vorliegen von (i) einem Stimulierungsreagens, das dazu in der Lage ist, eine oder mehrere intrazelluläre Signalisierungsdomänen einer oder mehrerer Komponenten eines TCR-Komplexes und/oder eine oder mehrere intrazelluläre Signalisierungsdomänen eines oder mehrerer kostimulierender Moleküle zu aktivieren, und (ii) einem Mittel, das mTOR-Aktivität hemmt, wobei das Mittel, das mTOR-Aktivität hemmt, 2-(3-Hydroxyphenyl)-9-(2-isopropylphenyl)-8-oxo-8,9-dihydro-7H-purin-6-carboxamid (Verbindung 63) ist; und
(b) Einführen eines rekombinanten Rezeptors in die stimulierte Zusammensetzung und dadurch Erzeugen einer veränderten Zusammensetzung, umfassend veränderte T-Zellen.

2. Verfahren nach Anspruch 1, umfassend weiteres Inkubieren der veränderten Zellen, wobei das Inkubieren das Vorliegen des Mittels umfasst, das mTOR-Aktivität hemmt, und dadurch das Erzeugen einer Ausgabezusammensetzung, optional wobei:
(a) das weitere Inkubieren in Gegenwart eines oder mehrerer rekombinanter Zytokine ausgeführt wird; und/oder
(b) das weitere Inkubieren das Kultivieren unter Bedingungen umfasst, um zu der Proliferation oder Expansion von Zellen in der Zusammensetzung zu führen.

3. Verfahren zum Erzeugen einer Zusammensetzung aus veränderten Zellen, das Verfahren umfassend das Kultivieren, in Gegenwart eines Mittels, das mTOR-Aktivität hemmt, einer Zusammensetung aus veränderten Zellen, umfassend angereicherte primäre humane T-Zellen, umfassend T-Zellen, die mit einem rekombinanten Rezeptor verändert sind;
wobei das Mittel, das mTOR-Aktivität hemmt, 2-(3-Hydroxyphenyl)-9-(2-isopropylphenyl)-8-oxo-8,9-dihydro-7H-purin-6-carboxamid (Verbindung 63) ist; und
wobei das Verfahren zur Proliferation oder zur Expansion von Zellen in der Zusammensetzung führt, um eine Ausgabezusammensetzung zu erzeugen, umfassend veränderte T-Zellen.

4. Verfahren nach Anspruch 3, wobei vor dem Kultivieren das Verfahren ferner umfasst:
(a) Inkubieren, unter stimulierenden Bedingungen, einer Eingabezusammensetzung, umfassend primäre T-Zellen, in der Gegenwart des Mittels, das mTOR-Aktivität hemmt; wobei die stimulierenden Bedingungen das Vorliegen eines Stimulierungsreagens umfassen, das dazu in der Lage ist, eine oder mehrere intrazelluläre Signalisierungsdomänen einer oder mehrerer Komponenten eines TCR-Komplexes und/oder eine oder mehrere intrazelluläre Signalisierungsdomänen eines oder mehrerer kostimulierender Moleküle zu aktivieren und dadurch eine stimulierte Zusammensetzung zu erzeugen; und
(b) Einführen eines rekombinanten Rezeptors in die stimulierte Zusammensetzung und dadurch Erzeugen einer veränderten Zusammensetzung, umfassend veränderte T-Zellen.

5. Verfahren nach einem der Ansprüche 1-4, wobei die primären T-Zellen CD4+ und/oder CD8+ T-Zellen sind.

6. Verfahren nach einem der Ansprüche 3-5, wobei:
(a) die veränderte T-Zell-Zusammensetzung angereicherte CD4+ T-Zellen umfasst; oder
(b) die veränderte T-Zell-Zusammensetzung angereicherte CD8+ T-Zellen umfasst.

7. Verfahren nach einem der Ansprüche 3-6, wobei das Kultivieren in Gegenwart eines oder mehrerer rekombinanter Zytokine ausgeführt wird.

8. Verfahren nach einem der Ansprüche 2 oder 7, wobei das eine oder die mehreren Zytokine eines oder mehrere von IL-2, IL-4, IL-7, IL-9, IL-12, IL-15, G-CSF und GM-CSF umfassen, optional wobei das eine oder die mehreren Zytokine eines oder mehrere von IL-2, IL-7 und IL-15 umfassen.

9. Verfahren nach einem der Ansprüche 3-8, wobei vor dem Kultivieren das Verfahren ferner umfasst:
(a) Inkubieren, unter stimulierenden Bedingungen, einer Eingabezusammensetzung, umfassend primäre T-Zellen, die stimulierenden Bedingungen umfassend das Vorliegen eines Stimulierungsreagens, das dazu in der Lage ist, eine oder mehrere intrazelluläre Signalisierungsdomänen einer oder mehrerer Komponenten eines TCR-Komplexes und/oder eine oder mehrere intrazelluläre Signalisierungsdomänen eines oder mehrerer kostimulierender Moleküle zu aktivieren und dadurch eine stimulierte Zusammensetzung zu erzeugen; und
(b) Einführen eines rekombinanten Rezeptors in die stimulierte Zusammensetzung und dadurch Erzeugen einer veränderten Zusammensetzung, umfassend veränderte T-Zellen.

10. Verfahren nach einem der Ansprüche 1, 2 und 9, wobei:
(a) die Eingabezusammensetzung, die stimulierte Zusammensetzung und/oder die veränderte Zusammensetzung primäre CD4+ und/oder CD8+ Zellen umfasst; und/oder
(b) wobei die Eingabezusammensetzung, die stimulierte Zusammensetzung und/oder die veränderte Zusammensetzung angereicherte CD4+ Zellen umfasst.

11. Verfahren nach einem der Ansprüche 1, 2, 9 und 10, wobei die Eingabezusammensetzung, die stimulierte Zusammensetzung und/oder die veränderte Zusammensetzung angereicherte CD8+ Zellen umfasst.

12. Verfahren nach einem der Ansprüche 1, 2, 9 und 10, wobei:
(a)
(i) die Eingabezusammensetzung, die stimulierte Zusammensetzung und/oder die veränderte Zusammensetzung mehr als oder mehr als etwa 70 %, mehr als oder mehr als etwa 75 %, mehr als oder mehr als etwa 80 %, mehr als oder mehr als etwa 85 %, mehr als oder mehr als etwa 90 %, mehr als oder mehr als etwa 95 % oder mehr als oder mehr als etwa 98 % CD4+ primäre humane T-Zellen umfasst; und/oder
(ii) die Eingabezusammensetzung im Wesentlichen aus CD4+ primären humanen T-Zellen besteht;
und/oder
(b)
(i) die Eingabezusammensetzung, die stimulierte Zusammensetzung und/oder die veränderte Zusammensetzung mehr als oder mehr als etwa 70 %, mehr als oder mehr als etwa 75 %, mehr als oder mehr als etwa 80 %, mehr als oder mehr als etwa 85 %, mehr als oder mehr als etwa 90 %, mehr als oder mehr als etwa 95 % oder mehr als oder mehr als etwa 98 % CD8+ primäre humane T-Zellen umfasst; und/oder
(ii) die Eingabezusammensetzung im Wesentlichen aus CD8+ primären humanen T-Zellen besteht.

13. Verfahren nach Anspruch 3 oder 4, wobei das Mittel, das mTOR-Aktivität hemmt, Verbindung 63 ist und die veränderte Zellzusammensetzung in Gegenwart von zwischen 500 nM und 2 µM, zwischen 1 nM und 100 nM, zwischen 50 nM und 250 nM oder zwischen 100 nM und 500 nM Verbindung 63 kultiviert wird.

14. Verfahren nach Anspruch 1 oder 2, wobei das Mittel, das mTOR-Aktivität hemmt, Verbindung 63 ist und die veränderte Zellzusammensetzung in Gegenwart von zwischen 500 nM und 2 µM, zwischen 1 nM und 100 nM, zwischen 50 nM und 250 nM oder zwischen 100 nM und 500 nM Verbindung 63 inkubiert wird.

15. Verfahren nach einem der Ansprüche 1-2 und 9-14, wobei das Stimulierungsreagens ein primäres Mittel umfasst, das spezifisch an ein Element eines TCR-Komplexes bindet, optional wobei:
(a) das primäre Mittel spezifisch an CD3 bindet;
und/oder
(b) das Stimulierungsreagens ferner ein sekundäres Mittel umfasst, das spezifisch an ein T-Zellkostimulatorisches Molekül bindet, optional wobei das kostimulatorische Molekül ausgewählt ist aus CD28, CD137 (4-1-BB), OX40 und ICOS;
und/oder
(c) das primäre und/oder das sekundäre Mittel einen Antikörper umfasst, optional wobei das Stimulierungsreagens Inkubierung mit einem Anti-CD3-Antikörper und einem Anti-CD28-Antikörper oder einem antigenbindenden Fragment davon umfasst; optional wobei das primäre Mittel und/oder das sekundäre Mittel auf der Oberfläche eines festen Trägers vorliegen, optional wobei der feste Träger ein Bead ist oder umfasst.

16. Verfahren nach einem der Ansprüche 1-2 und 9-15, wobei das Einführen das Transduzieren von Zellen der stimulierten Zusammensetzung mit einem viralen Vektor umfasst, umfassend ein Polynukleotid, das für den rekombinanten Rezeptor kodiert; optional wobei der virale Vektor ein retroviraler Vektor ist; optional wobei der virale Vektor ein lentiviraler Vektor oder ein gammaretroviraler Vektor ist.

17. Verfahren nach einem der Ansprüche 2-16, wobei das Verfahren ferner das Sammeln von Zellen der Ausgabezusammensetzung umfasst; optional ferner umfassend das Formulieren von Zellen der Ausgabezusammensetzung für Kryokonservierung und/oder Verabreichung an ein Subjekt, optional in Gegenwart eines pharmazeutisch annehmbaren Hilfsstoffs.

18. Verfahren nach einem von 1-2 und 9-17, ferner umfassend das isolieren der CD4+ und/oder der CD8+ T-Zellen aus einer biologischen Probe vor dem Inkubieren; optional wobei die biologische Probe eine Vollblutprobe, eine Buffy-Coat-Probe, eine Probe peripherer mononukleärer Blutzellen (PBMC), eine Probe unfraktionierter T-Zellen, eine Lymphozytenprobe, eine Probe weißer Blutzellen, ein Aphereseprodukt oder ein Leukaphereseprodukt ist oder umfasst.

19. Verfahren nach einem der Ansprüche 1-18, wobei:
(a) der rekombinante Rezeptor in der Lage ist, an ein Zielantigen zu binden, das verknüpft ist mit, spezifisch ist für und/oder exprimiert wird auf einer Zelle oder einem Gewebe einer Erkrankung, einer Störung oder eines Zustands; optional wobei die Erkrankung, die Störung oder der Zustand eine Infektionskrankheit oder -störung, eine Autoimmunerkrankung, eine entzündliche Erkrankung, ein Tumor oder Krebs ist; optional wobei das Zielantigen ein Tumorantigen ist; und/oder
(b) wobei der rekombinante Rezeptor ein funktioneller nicht-TCR-Antigenrezeptor oder ein TCR- oder antigenbindendes Fragment davon ist oder umfasst; und/oder
(c) der rekombinante Rezeptor ein chimärer Antigenrezeptor (CAR) ist.

## Revendications

1. Procédé pour la production d'une composition de cellules modifiées, le procédé comprenant :
(a) l'incubation, dans des conditions stimulantes, d'une composition d'entrée comprenant des cellule T humaines primaires, lesdites conditions stimulantes comprenant la présence de (i) un réactif stimulant capable d'activer un ou plusieurs domaines de signalisation intracellulaire d'un ou de plusieurs composants d'un complexe TCR et/ou un ou plusieurs domaines de signalisation intracellulaire d'une ou de plusieurs molécules costimulatrices et (ii) un agent qui inhibe l'activité de mTOR, l'agent qui inhibe l'activité de mTOR étant le 2-(3-hydroxyphényl)-9-(2-isopropylphényl)-8-oxo-8,9-dihydro-7H-purine-6-carboxamide (composé 63) ; et
(b) l'introduction d'un récepteur recombinant dans la composition stimulée, générant ainsi une composition modifiée comprenant des cellules T modifiées.

2. Procédé selon la revendication 1 , comprenant une incubation supplémentaire des cellules modifiées, l'incubation comprenant la présence de l'agent qui inhibe l'activité de mTOR, produisant ainsi une composition de sortie, éventuellement :
(a) l'incubation supplémentaire étant réalisée en la présence d'une ou de plusieurs cytokines recombinantes ; et/ou
(b) l'incubation supplémentaire comprenant une culture dans des conditions qui entraînent la prolifération ou l'expansion de cellules dans la composition.

3. Procédé pour la production d'une composition de cellules modifiées, le procédé comprenant une culture, en la présence d'un agent qui inhibe l'activité de mTOR, d'une composition de cellules modifiées comprenant des cellules T humaines primaires enrichies comprenant des cellules T modifiées par un récepteur recombinant ;
l'agent qui inhibe l'activité de mTOR étant le 2-(3-hydroxyphényl)-9-(2-isopropylphényl)-8-oxo-8,9-dihydro-7H-purine-6-carboxamide (composé 63) ; et
le procédé entraînant la prolifération ou l'expansion de cellules dans la composition pour produire une composition de sortie comprenant des cellules T modifiées.

4. Procédé selon la revendication 3, avant la culture, le procédé comprenant en outre :
(a) l'incubation, dans des conditions stimulantes, d'une composition d'entrée comprenant des cellules T primaires en la présence de l'agent qui inhibe l'activité de mTOR ; lesdites conditions stimulantes comprenant la présence d'un réactif stimulant capable d'activer un ou plusieurs domaines de signalisation intracellulaire d'un ou de plusieurs composants d'un complexe TCR et/ou un ou plusieurs domaines de signalisation intracellulaire d'une ou de plusieurs molécules costimulatrices, générant ainsi une composition stimulée ; et
(b) l'introduction d'un récepteur recombinant dans la composition stimulée, générant ainsi une composition modifiée comprenant des cellules T modifiées.

5. Procédé selon l'une quelconque des revendications 1 à 4, les cellules T primaires étant des cellules T CD4+ et/ou CD8+.

6. Procédé selon l'une quelconque des revendications 3 à 5,
(a) la composition de cellules T modifiées comprenant des cellules T CD4+ enrichies ; ou
(b) la composition de cellules T modifiées comprenant des cellules T CD8+ enrichies.

7. Procédé selon l'une quelconque des revendications 3 à 6, la culture étant réalisée en la présence d'une ou de plusieurs cytokines recombinantes.

8. Procédé selon la revendication 2 ou 7, la ou les cytokines recombinantes comprenant l'une ou plusieurs parmi IL-2, IL-4, IL-7, IL-9, IL-12, IL-15, G-CSF et GM-CSF, éventuellement, la ou les cytokines recombinantes comprenant l'une ou plusieurs parmi IL-2, IL-7 et IL-15.

9. Procédé selon l'une quelconque des revendications 3 à 8, avant la culture, le procédé comprenant en outre :
(a) l'incubation, dans des conditions stimulantes, d'une composition d'entrée comprenant des cellules T primaires, lesdites conditions stimulantes comprenant la présence d'un réactif stimulant capable d'activer un ou plusieurs domaines de signalisation intracellulaire d'un ou de plusieurs composants d'un complexe TCR et/ou un ou plusieurs domaines de signalisation intracellulaire d'une ou de plusieurs molécules costimulatrices, générant ainsi une composition stimulée ; et
(b) l'introduction d'un récepteur recombinant dans la composition stimulée, générant ainsi une composition modifiée comprenant des cellules T modifiées.

10. Procédé selon l'une quelconque des revendications 1, 2 et 9,
(a) la composition d'entrée, la composition stimulée et/ou la composition modifiée comprenant des cellules T CD4+ et/ou CD8+ primaires ; et/ou
(b) la composition d'entrée, la composition stimulée et/ou la composition modifiée comprenant des cellules T CD4+ enrichies.

11. Procédé selon l'une quelconque des revendications 1, 2, 9 et 10, la composition d'entrée, la composition stimulée et/ou la composition modifiée comprenant des cellules T CD8+ enrichies.

12. Procédé selon l'une quelconque des revendications 1, 2, 9 et 10,
(a)
(i) la composition d'entrée, la composition stimulée et/ou la composition modifiée comprenant plus de ou plus d'environ 70 %, plus de ou plus d'environ 75 %, plus de ou plus d'environ 80 %, plus de ou plus d'environ 85 %, plus de ou plus d'environ 90 %, plus de ou plus d'environ 95 % ou plus de ou plus d'environ 98 % de cellules T humaines primaires CD4+ ; et/ou
(ii) la composition d'entrée étant essentiellement constituée de cellules T humaines primaires CD4+ ;
et/ou
(b)
(i) la composition d'entrée, la composition stimulée et/ou la composition modifiée comprenant plus de ou plus d'environ 70 %, plus de ou plus d'environ 75 %, plus de ou plus d'environ 80 %, plus de ou plus d'environ 85 %, plus de ou plus d'environ 90 %, plus de ou plus d'environ 95 % ou plus de ou plus d'environ 98 % de cellules T humaines primaires CD8+ ; et/ou
(ii) la composition d'entrée étant essentiellement constituée de cellules T humaines primaires CD8+ ;

13. Procédé selon la revendication 3 ou la revendication 4, l'agent qui inhibe l'activité de mTOR étant le composé 63 et la composition de cellules modifiées étant cultivée en la présence d'entre 500 nM et 2 µM, entre 1 nM et 100 nM, entre 50 nM et 250 nM ou entre 100 nM et 500 nM du composé 63.

14. Procédé selon la revendication 1 ou la revendication 2, l'agent qui inhibe l'activité de mTOR étant le composé 63 et la composition de cellules modifiées étant incubée en la présence d'entre 500 nM et 2 µM, entre 1 nM et 100 nM, entre 50 nM et 250 nM ou entre 100 nM et 500 nM du composé 63.

15. Procédé selon l'une quelconque des revendications 1 et 2 et 9 à 14, le réactif stimulant comprenant un agent primaire qui se lie spécifiquement à un élément d'un complexe TCR, éventuellement :
(a) l'agent primaire se liant spécifiquement à CD3 ; et/ou
(b) le réactif stimulant comprenant en outre un agent secondaire qui se lie spécifiquement à une molécule costimulatrice de cellules T, éventuellement, la molécule costimulatrice étant choisie parmi CD28, CD137 (4-1-BB), OX40, et ICOS ;
et/ou
(c) les agents primaires et/ou secondaires comprenant un anticorps, éventuellement le réactif stimulant comprenant une incubation avec un anticorps anti-CD3 et un anticorps anti-CD28, ou un fragment de liaison à un antigène de celui-ci ; éventuellement, l'agent primaire et/ou l'agent secondaire étant présents sur la surface d'un support solide, éventuellement, le support solide étant ou comprenant une bille.

16. Procédé selon l'une quelconque des revendications 1 et 2 et 9 à 15, l'introduction comprenant une transduction de cellules de la composition stimulée avec un vecteur viral comprenant un polynucléotide codant pour le récepteur recombinant ; éventuellement, le vecteur viral étant un vecteur rétroviral ; éventuellement, le vecteur viral étant un vecteur lentiviral ou un vecteur gammarétroviral.

17. Procédé selon l'une quelconque des revendications 2 à 16, le procédé comprenant en outre la collecte de cellules de la composition de sortie ; éventuellement comprenant en outre la formulation de cellules de la composition de sortie pour une cryoconservation et/ou une administration à un sujet, éventuellement en la présence d'un excipient pharmaceutiquement acceptable.

18. Procédé selon l'une quelconque des revendications 1 et 2 et 9 à 17, comprenant en outre l'isolement des cellules T CD4+ et/ou CD8+ à partir d'un échantillon biologique avant l'incubation ; éventuellement, l'échantillon biologique étant ou comprenant un échantillon de sang entier, un échantillon de couche leucocyto-plaquettaire, un échantillon de cellules mononucléaire de sang périphérique (PBMC), un échantillon de cellules T non fractionné, un échantillon de lymphocytes, un échantillon de globules blancs, un produit d'aphérèse ou un produit de leucaphérèse.

19. Procédé selon l'une quelconque des revendications 1 à 18,
(a) le récepteur recombinant étant capable de se lier à un antigène cible qui est associé à, spécifique à, et/ou exprimé sur une cellule ou un tissu d'une maladie, d'un trouble ou d'une affection ; éventuellement, la maladie, le trouble ou l'affection étant une maladie infectieuse ou un trouble infectieux, une maladie auto-immune, une maladie inflammatoire, une tumeur ou un cancer ; éventuellement, l'antigène cible étant un antigène tumoral ; et/ou
(b) le récepteur recombinant étant ou comprenant un récepteur d'antigène non TCR fonctionnel ou un TCR ou fragment de liaison à un antigène de celui-ci ; et/ou
(c) le récepteur recombinant étant un récepteur antigénique chimérique (CAR).
